(19) Europäisches Patentamt
European Patent Office
Office européen des brevets

(11) **EP 3 633 046 A1**

(12) **EUROPEAN PATENT APPLICATION**

(43) Date of publication:
08.04.2020 Bulletin 2020/15

(51) Int Cl.:
*C12Q 1/68* (2018.01)    *C12N 15/10* (2006.01)
*C12Q 1/6844* (2018.01)

(21) Application number: 19170382.6

(22) Date of filing: 03.02.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 01.02.2013 GB 201301857

(62) Document number(s) of the earlier application(s) in accordance with Art. 76 EPC:
14703318.7 / 2 951 316

(71) Applicants:
• **Selvi, Ozan**
  **06800 Çayyoly, Ankara (TR)**
• **Orcan, Serkan**
  **Çankaya, Ankara (TR)**

(72) Inventors:
• **SELVI, Ozan**
  **06800 Ankara (TR)**

• **ORCAN, Serkan**
  **Ankara (TR)**
• **TOKSOZ, Sila**
  **Owings Mills, MD 21117 (US)**

(74) Representative: **Ruscoe, Peter James et al**
  **Sagittarius IP**
  **Marlow International**
  **Parkway**
  **Marlow SL7 1YL (GB)**

Remarks:
•Claims filed after the date of receipt of the divisional application (Rule 68(4) EPC).
•This application was filed on 19.04.2019 as a divisional application to the application mentioned under INID code 62.

(54) **PRIMER TECHNOLOGY**

(57) The present invenion provides a method of nucleic acid manipulation comprising:
(i) hybridizing a double stranded primer to the 3' end of a single stranded nucleic acid template, wherein said primer comprises a double stranded region and a single stranded region, wherein the single stranded region is a 3' overhang region and wherein the 3' overhang region enables the double stranded primer to target and hybridize to the 3' end of the nucleic acid template, wherein said single stranded 3' overhang region comprises a degenerate sequence or a sequence comprising universal bases, and wherein the double stranded primer is made up of two separate strands;
(ii) at least one round of polymerization using a polypeptide with 5' to 3' DNA polymerization activity, wherein at least the first round of polymerization comprises using a polypeptide with 5' to 3' DNA polymerization activity to carry out a primer extension reaction to synthesise nucleotides in a template dependent manner from the 3' end of the single stranded region of said hybridized double stranded primer;
wherein the hybridizing step (i) and at least the first primer extension reaction from the 3' end of the single stranded region of said hybridized double stranded primer in step (ii) takes place without the formation of a phosphodiester bond between the 3' end of the nucleic acid template and the double stranded primer of step (i). Products, kits and compositions suitable for use in such methods are also provided.

**Description**

This invention relates to improved methods of nucleic acid manipulation. A preferred use of such methods is in methods of nucleic acid amplification. However, the methods of the invention can also be used for other types of nucleic acid manipulation, for example to label nucleic acids or to join nucleic acid fragments together or to incorporate specific nucleic acid sequences into a template. The invention further relates to improved methods for Systematic Evolution of Ligands by Exponential Enrichment (SELEX).

Many of the prior art methods of nucleic acid manipulation, amplification or SELEX involve the use of standard or modified amplification methods, for example standard or modified PCR methods which involve the use of conventional (or other) single stranded primers (see for example US 8,071,311, US 8,143,001, US 8,034,568, US 7,939,258 of NuGEN Technologies, Inc) or adaptors (see for example US 8,192,941 of Life Technologies Corporation). See also "Principles and Technical Aspects of PCR Amplification", 2008, van Pelt-Verkuil et al., published by Springer.

As will be described in more detail below, the methods of the present invention use a double-stranded primer with a particular structure which can target the 3' ends of single stranded nucleic acid molecules and which results in many advantages over various prior art methods. Such advantages include for example eliminating the need to have any sequence information of the nucleic acid template before amplification (although the methods are equally applicable for pre-known sequences if desired); amplifying the template by totally preserving the original sequence; eliminating the need for incubation periods for any 5'- and 3'-end modification and ligation reactions (e.g. the addition of adaptor molecules by ligation, although again 5'- and 3'-end modification and ligation reactions, including the insertion of adaptor sequences can be carried out if desired); convenient and less time-consuming protocols; eliminating the need for extensive separation/purification steps; using less enzyme quantities, and strong cost-effectiveness.

As set out above, many prior art nucleic acid manipulation and amplification methods use ligation steps in order to ligate nucleic acid molecules (often termed as adaptor sequences) to the 5' end, and in particular the 3' end, of nucleic acid templates to enable manipulation and amplification (in particular polymerization), see for example US 8,192,941 of Life Technologies Corporation. Thus, an important advantage of the methods of the present invention is that no ligation reaction is required, thereby also avoiding the need for 5' and/or 3' end modifications, resulting in advantages in terms of time and also in terms of costs as fewer reagents are necessary.

In addition, ligation can result in the linkage of undesired nucleic acid fragments to each other leading to undesired by-products. Thus, avoiding the use of ligation in the present methods can reduce the need for further separation/purification steps. Moreover, a relatively high yield and specificity are maintained because of the absence of non-specific random priming reactions.

As discussed in more detail below, the double-stranded primers and/or the amplification methods of the invention can also advantageously be used in Systematic Evolution of Ligands by Exponential Enrichment (SELEX) protocols, which allow the selection of single stranded nucleic acid molecules (e.g. DNA or RNA) which specifically bind to a particular target ligand or ligands (e.g. a protein or small organic compound). Such single stranded nucleic acid molecules are also referred to as aptamers.

Standard SELEX protocols begin with the synthesis of a large oligonucleotide library which generally comprises randomly generated sequences of fixed length (e.g. a fixed length of the order of 20-60 nucleotides) flanked by constant (fixed sequence) 5' and 3' ends which serve as primers. The sequences in the library are then exposed to the target ligand and those that do not bind the target are removed, e.g. by affinity chromatography. The bound sequences are then eluted from the ligand and amplified by PCR using the constant primer regions, after which they are then subjected to subsequent rounds of selection.

Use of the double-stranded primers and/or the amplification methods of the invention provides an improved SELEX discovery platform for identification of single-stranded (or double-stranded) DNA/RNA oligonucleotides that can specifically bind to any specific target ligand (or ligands). The methods of the present invention can provide significant advantages over existing techniques (see for example US 6,855,496 of Gilead Science, Inc) such as generating length variations besides sequence diversity, always being able to select the shortest possible oligonucleotides if desired, eliminating the need for post-selection modifications after selection and elucidating conserved structural motifs with a single SELEX setup. Other advantageous features of the SELEX methods described herein are the preservation of the native selected sequence, suitability for automation, adaptability to every selection method, providing maximum selection library diversity, adaptability for multiplex strategies and suitability for both DNA and RNA oligonucleotide selection. Further advantageous features of the SELEX methods described herein are that they can directly select aptamer molecules lacking constant primer regions or only having 5'- primer regions. Selection of aptamer molecules from single-base truncated SELEX pools generated by the chain termination of a desired SELEX library (or pre-discovered aptamer molecules) is also possible, enabling the elucidation of conserved structural motifs of selected DNA/RNA aptamer molecules via parallel NGS sequencing after any round of the selection process. Adaptor sequences of any desired NGS device (such as Ion Torrent™ (Life Technologies, Inc) P1 and B adapters) can also be incorporated in selected aptamer molecules during the enrichment process, after any desired selection round, via dsPrimer reagents and amplification methods - no additional sequencing/NGS library preparation steps are required. Aptamer molecules can be selected from within SELEX pools that contain single-base decreasing length variations of the starting SELEX library, enabling the generation of aptamers with different sequence lengths via parallel NGS sequencing. Most significantly, the SELEX methods described herein include a novel method enabling the creation of a reusable backup of every SELEX cycle that can be used, for example, at a library enrichment step, via the use of nanoparticle-conjugated dsPrimers (as illustrated in Design NP2 or NP3).

An important and central feature of the present invention is the structure and function of the double stranded primer (dsPrimer) which is used. It should be noted that due to the diversity of the uses and sequences included in said dsPrimers (as will be described elsewhere herein) they can function as more than just a primer.

The double stranded primers of the invention have the ability to target the 3' ends of the nucleic acid templates. As will be evident from the numerous manipulation, amplification, SELEX and other methods described herein, this targeting can be exploited in a novel and previously undescribed manner to give rise to a wide range of convenient, time and cost saving protocols and their products. This targeting is important as the primers are then in the optimum position to allow primer extension and amplification (or other manipulation) of the full length of the template molecule. If a primer hybridises in the middle of the template molecule or away from the ends of the template molecule, as often happens in nucleic acid amplification (or other manipulation) then this means that shorter and less desirable primer extension (or polymerisation) products are produced. The ability of the double stranded primers of the invention to target the 3' ends of the template molecules or to precisely bind at the 3' terminus of the template molecules is thus an important advantage.

Thus, the present invention provides a method of nucleic acid manipulation, e.g. DNA manipulation, comprising:

(i) hybridizing a double stranded primer to a single stranded nucleic acid template, wherein said primer comprises a double stranded region and a single stranded region, wherein the single stranded region is a 3' overhang region and wherein the 3' overhang region enables the hybridization of said primer to the 3' end of the nucleic acid template;
(ii) at least one round of polymerization using a DNA polymerase, wherein at least the first round of polymerization comprises using a DNA polymerase with 5' to 3' polymerization activity to carry out a primer extension reaction to synthesise nucleotides in a template dependent manner from the 3' end of the single stranded region of said hybridized double stranded primer;

wherein the hybridizing step (i) and at least the first primer extension reaction from the 3' end of the single stranded region of said hybridized double stranded primer in step (ii) takes place without the formation of a phosphodiester bond between the 3' end of the nucleic acid template and the double stranded primer of step (i).

Preferred applications of the invention are in nucleic acid amplification, including amplification via the polymerase chain reaction (PCR). Thus, a preferred embodiment of the invention provides a method of nucleic acid amplification comprising:

(i) hybridizing a double stranded primer to a single stranded nucleic acid template, wherein said primer comprises a double stranded region and a single stranded region, wherein the single stranded region is a 3' overhang region and wherein the 3' overhang region enables the hybridization of said primer to the 3' end of the nucleic acid template;
(ii) at least one round of polymerization using a DNA polymerase in order to form an amplification template, wherein at least the first round of polymerization comprises using a DNA polymerase with 5' to 3' polymerization activity to carry out a primer extension reaction to synthesise nucleotides in a template dependent manner from the 3' end of the single stranded region of said hybridized double stranded primer; and
(iii) at least one amplification step to form an amplified nucleic acid product;

wherein the hybridizing step (i) and at least the first primer extension reaction from the 3' end of the single stranded region of said hybridized double stranded primer in step (ii) takes place without the formation of a phosphodiester bond between the 3' end of the nucleic acid template and the double stranded primer of step (i).

The present invention further provides a method of nucleic acid manipulation comprising:

(i) hybridizing a double stranded primer to the 3' end of a single stranded nucleic acid template, wherein said primer comprises a double stranded region and a single stranded region, wherein the single stranded region is a 3' overhang region and wherein the 3' overhang region enables the double stranded primer to target and hybridize to the 3' end of the nucleic acid template, wherein said single stranded 3' overhang region comprises a degenerate sequence or a sequence comprising universal bases, and wherein the double stranded primer is made up of two separate strands;
(ii) at least one round of polymerization using a polypeptide with 5' to 3' DNA polymerization activity, wherein at least the first round of polymerization comprises using a polypeptide with 5' to 3' DNA polymerization activity to carry out a primer extension reaction to synthesise nucleotides in a template dependent manner from the 3' end of the single stranded region of said hybridized double stranded primer;

wherein the hybridizing step (i) and at least the first primer extension reaction from the 3' end of the single stranded region of said hybridized double stranded primer in step (ii) takes place without the formation of a phosphodiester bond between the 3' end of the nucleic acid template and the double stranded primer of step (i).

The present invention further provides a method of nucleic acid amplification comprising:

(i) hybridizing a double stranded primer to the 3' end of a single stranded nucleic acid template, wherein said primer comprises a double stranded region and a single stranded region, wherein the single stranded region is a 3' overhang region and wherein the 3' overhang region enables the double stranded primer to target and hybridize to the 3' end of the nucleic acid template, wherein said single stranded 3' overhang region comprises a degenerate sequence or a sequence comprising universal bases, and wherein the double stranded primer is made up of two separate strands;
(ii) at least one round of polymerization using a polypeptide with 5' to 3' DNA polymerization activity in order to form an amplification template, wherein at least the first round of polymerization comprises using a polypeptide with 5' to 3' DNA polymerization activity to carry out a primer extension reaction to synthesise nucleotides in a template dependent manner from the 3' end of the single stranded region of said hybridized double stranded primer; and
(iii) at least one amplification step to form an amplified nucleic acid product;

wherein the hybridizing step (i) and at least the first primer extension reaction from the 3' end of the single stranded region of said hybridized double stranded primer in step (ii) takes place without the formation of a phosphodiester bond between the 3' end of the nucleic acid template and the double stranded primer of step (i).

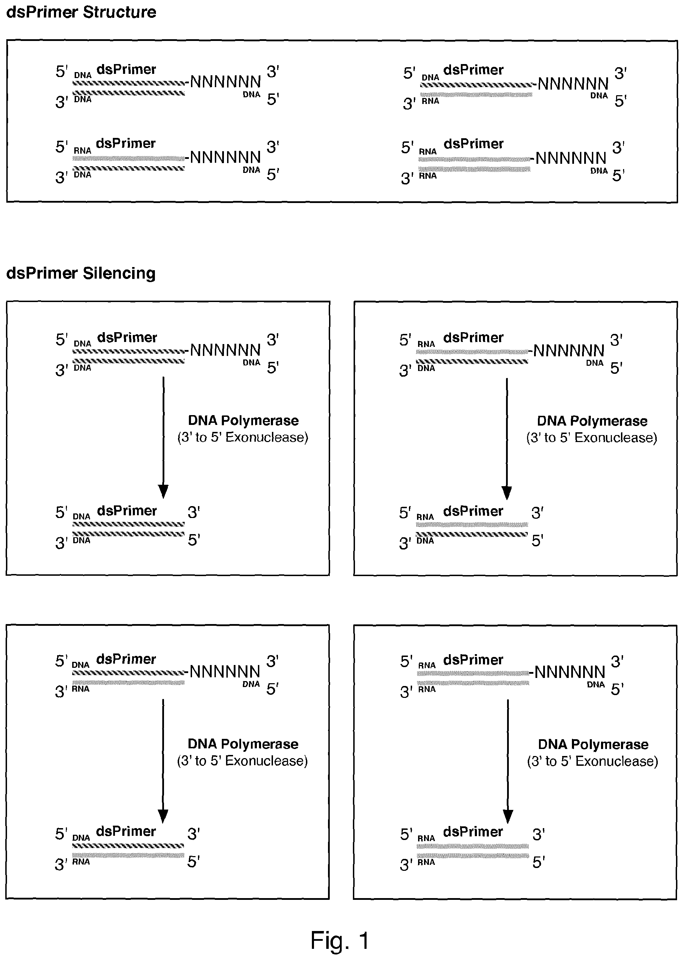

The term "double stranded primer" (dsPrimer) as used herein refers to a nucleic acid molecule which comprises a double stranded region (or part) and a single stranded region (or part), in other words the primer is partially double stranded. The single stranded region is a 3' overhang region which enables the hybridization of the primer to the 3' end of a single stranded nucleic acid template and, once hybridized, provides a 3' end which can be used as a starting point for nucleic acid synthesis (i.e. can be used as a primer). In the methods of the invention, a DNA polymerase or any polypeptide or enzyme with 5' to 3' polymerization activity acts at said 3' end to initiate a primer extension reaction to synthesise nucleotides in a template dependent manner. The general structure of the dsPrimer of the invention is shown in Figure 1 (such dsPrimers, with one 3' overhang region, are also referred to herein as basic or simple dsPrimers). Thus, in preferred embodiments of the invention, there is no 5' overhang region present in the dsPrimers. In such dsPrimers, the nucleic acid strand which comprises the 3' single stranded overhang region is also referred to herein as the "upper strand", "upper part", "upper portion", "sense strand", "sense part", "sense portion", or "overhang strand". The other nucleic acid strand of the dsPrimer (i.e. the strand without the single stranded 3' overhang region) is also referred to herein as the "lower strand" or "lower part" or "lower portion" or "complementary strand" or "complementary part" or "complementary portion" or "non-overhang strand or portion or part".

Thus, the double stranded primer of the invention is made up of two separate strands (which are complementary strands that are hybridized together), in other words, the two strands are not linked by the same sugar-phosphate backbone and any linkage between them in double stranded regions is between the bases of the primer strands. Thus, any secondary structure motifs of nucleic acids for example hair-pin type structures (or stem-loop type structures) where a single strand of nucleotides loops back on itself to form a double stranded region are not contemplated. In addition, it is important to the methods of the invention that the double stranded nature of the primer is retained during the hybridization step (i) and, if necessary, reaction conditions need to take this into account.

In the dsPrimers of the invention, the two strands of the dsPrimer can be made of any type of nucleic acid. For example, both strands can be DNA, both of the strands can be RNA, or one strand can be RNA and one strand can be DNA. Alternatively, one or both strands can contain a mixture of nucleic acid types (e.g. a mixture of RNA and DNA regions in the form of a chimeric nucleic acid molecule). In addition, the nucleotides making up the dsPrimer can be any type of natural nucleic acids or non-natural nucleic acids such as locked nucleic acids (LNAs), functionalized LNAs, nucleobase functionalized LNAs or other types of modified nucleic acid molecules, etc, in both strands or either strand and as mixtures of RNA, DNA and/or non-natural nucleotides or nucleic acids.

The structure of the dsPrimer is such that the dsPrimer can specifically or strictly target and hybridize to the 3' end of a single stranded nucleic acid template molecule or bind or hybridize exactly to the 3' terminus of a single stranded nucleic acid template molecule. In other words, the dsPrimers of the invention target only the 3' ends of the nucleic acid template and show no or no significant binding within (i.e. not at the end of) said template molecule. To this end, the 3' single stranded overhang region of the dsPrimer enables the hybridization to the 3' end/terminus and the double stranded region of the dsPrimer structure blocks the hybridization of the 3' overhang region elsewhere in the nucleic acid template molecule.

In this regard, in the dsPrimers of the invention, the 3' single stranded overhang sequence is covalently bound to the upper, sense strand of the double stranded region with a phosphodiester bond. An overhang nucleic acid sequence needs to have appropriate alignment and binding conditions in order to hybridize to its complementary sequence. Without wishing to be bound by theory, in the case of a dsPrimer of the invention, it is believed that the double-stranded region prevents the 3' overhang region from orientating in an appropriate alignment with a sequence within (i.e. not at the end of) a nucleic acid template molecule because of the bending limitations of the above mentioned phosphodiester bond between the single stranded region and the double stranded region of the dsPrimer. In addition, as the hybridization of the single stranded 3' overhang region is aided if the overhang portion has the appropriate alignment to the complementary base pairs (if this does not occur then it is difficult for proper hydrogen bonds to form), e.g. is linear and not self-hybridized, then, in addition, the size of the double stranded region of the dsPrimer sterically prevents such an appropriate alignment with a sequence within (i.e. not at the end of, not at the terminus of) a nucleic acid template molecule unless the phosphodiester bond is broken. It is believed that these reasons contribute to the ability of the dsPrimers of the invention to strictly and advantageously target only or solely the 3' ends (3' terminus) of the single stranded nucleic acid template molecules.

The dsPrimers of the invention can be used in an unmodified form (e.g. without any modifications being made to the 5' or 3' ends of the two strands). Indeed, such dsPrimers are preferred. However, in some embodiments the use of a modified dsPrimer is appropriate. For example, in embodiments where ligation of the dsPrimer is desired, then the 5' end of the lower strand/non-overhang strand (or a 5' end of the double stranded region which is at a complementary position to a 3' single stranded overhang region) of the dsPrimer would be modified to incorporate a 5' phosphate group. Of course in embodiments where ligation is not required then such phosphate groups need to not be present.

Thus, a yet further aspect of the invention provides a double stranded primer comprising a double stranded region and a single stranded region, wherein the single stranded region is a 3' overhang region comprising a degenerate sequence or a sequence comprising universal bases, wherein when only one of the strands has a 3' overhang region then the strand which does not contain the 3' overhang region does not have a 5'- $PO_3$ group, and wherein when both of the strands have a 3' overhang region then one or both of the strands does not have a 5'- $PO_3$ group. Such dsPrimers can be used in all aspects of the invention and are sometimes preferred.

The present invention also provides the dsPrimers of the invention for use in nucleic acid manipulation or amplification, for example in sequencing applications, nucleic acid labelling, joining nucleic acid fragments together, or for incorporation of primer sites, terminator sites and/or restriction sites. In the embodiments described herein where a 5'- $PO_3$ group is absent from the dsPrimer then the present invention provides such dsPrimers for use in nucleic acid manipulation or amplification, for example in sequencing applications, nucleic acid labelling, joining nucleic acid fragments together, or for incorporation of primer sites, terminator sites and/or restriction sites, wherein the strand which lacks the 5'- $PO_3$ group is not incorporated into the nucleic acid template with a covalent bond, or is not incorporated into a product containing the other strand.

Other modifications include those that increase the stability of dsPrimers or allow them to resist nuclease degradation (e.g. the inclusion of phosphorothioate bonds) or the inclusion of labelling moieties as also described elsewhere herein, e.g. flurophores or dyes, at appropriate ends (e.g. appropriate 5' ends such as the 5' end of the upper strand) of the dsPrimer. Other modifications include those necessary for allowing the dsPrimer to be immobilized to a solid phase. Such embodiments are described in more detail elsewhere herein but generally involve attachment via a modification of the 5' end of the upper strand or 3' end of the lower strand of the dsPrimer.

The double stranded region of the dsPrimer can be of any length and any sequence providing that the ability to hybridize to the 3' end of a single stranded nucleic acid template molecule is retained. A typical length could, for example, be between 10 to 100 base pairs or up to 200 base pairs, e.g. 20 to 70 or 80, or 25 to 40 or 50 or 60 base pairs. However, in some embodiments of the invention, for example where the methods are used to join larger nucleic acid fragments together or embodiments where chPrimers are used, then the length of the double stranded region can be increased, for example it can be up to several 100 or several 1000 base pairs in length. Thus, a typical length could range from a few base pairs, e.g. 10, to hundreds of base pairs, e.g. 200 or 500, or to thousands of base pairs, e.g. 3000 or 5000.

The sequence of the double stranded region of the dsPrimer can be engineered to contain any sequence of interest or use, or combinations thereof, or multiple copies (two or more) thereof, which may be the same or different. For example, the double stranded region can be designed to contain a sequence which acts as a genetic control sequence, for example primer binding site, i.e. which forms a site to which a primer can bind and can be used to initiate a primer extension reaction (i.e. can be used as a starting point for nucleic acid synthesis). In other embodiments of the invention, the double stranded region can be engineered to contain any other site of interest, for example a double stranded promoter region (a dsPrimer with a double stranded promoter region is also referred to herein as a "dsPromoter"), or a double stranded terminator region (a dsPrimer with a double stranded terminator region is also referred to herein as a "dsTerminator"). Thus, the dsPromoters or dsTerminators of the invention are more specialised forms of the dsPrimers of the invention and are useful in certain embodiments.

Equally, the double stranded region of the double stranded primer can be engineered to contain any other useful sequence, or combinations thereof, etc., for example a control region (for example a promoter, operator, terminator, repressor, enhancer, silencer, insulator or other response element) or any appropriate sequencing adaptor region or other element permitting or facilitating sequencing for example, such that sequencing adaptor regions/sequences are added to the ends of the nucleic acid molecule templates which are then compatable with appropriate sequencing applications, e.g. NGS sequencing. Equally such regions may contain a restriction site or some kind of label (e.g. an endogenous or exogenous label) or identifier sequence such as for example, a DNA barcode or a forensic DNA signature. Such a label can take any appropriate form for inclusion within (endogenous) or attaching to (exogenous) a nucleic acid sequence, for example endogenous labels could take the form of a nucleic acid code which is present within the dsPrimer sequence or could for example be 2-aminopurine (which is fluorescent) or phenylazide- or psoralen- based functional groups, etc., or it could for example be an exogenous label such as for example a fluorescent label or a radio-active label, etc. In other embodiments the double stranded region may contain a nucleic acid sequence or fragment of interest which is desired to be joined to the nucleic acid template molecule.

The double stranded region of the dsPrimer can be synthesised by any appropriate technology which would be well known to a person skilled in the art. A factor when selecting an appropriate technique to be used would for example be the length of the double stranded region required once the upper and lower strands are hybridized. For example, the current limit of most oligo synthesizers is almost 200 base pairs. Thus, the use of such synthesizers would be a convenient way to prepare any double stranded region of less than 200 bps. Longer double stranded regions could be prepared by any appropriate methods, for example by restriction digestion using restriction enzymes in order to prepare the double stranded region of the dsPrimer for example from a longer double stranded molecule. Longer double stranded regions are likely to be used in embodiments where the methods of the invention are used to join longer nucleic acid fragments together, where one of the fragments to be joined is contained in the double stranded region of the dsPrimer and the other fragment is within the nucleic acid template molecule. Fragments of over 1000 bps contained in the dsPrimer can be added in such embodiments.

A convenient way of preparing the dsPrimers of the invention (i.e. including both the double stranded region and the single stranded overhang region) is to synthesise the upper strand and lower strand separately (i.e. to synthesise both strands separately) and form dsPrimers by hybridizing these two strands. The ligation of a single stranded overhang region to a double stranded region could also be used.

The single stranded 3' overhang region of the dsPrimer of the invention can also be of any length providing that the length is sufficient and appropriate to enable the hybridization of said primer to the 3' end of a single stranded nucleic acid template, as in the methods of the present invention only the overhang region is hybridized to the nucleic acid template; the double stranded region, or part thereof, for example does not hybridize to the template. The length should also be appropriate and sufficient, for example, sufficiently short, so as to prevent its self-annealing and any resulting interference with the hybridization with the template. The length of the single stranded overhang region will generally be in the range of 3 to 25 or 3 to 20 base pairs, for example 3 to 12, or 3 to 15 base pairs or 4 to 12, or 4 to 15, or 5 to 10, or 10 to 15 base pairs, although longer lengths of e.g. 45 to 50 base pairs, for example lengths of up to 15 or 20 base pairs can also be made and used (even for a degenerate or universal overhang region). Preferred lengths are 3, 4, 5, 6, 7, 8, 9 or 10 base pairs, more preferred are 3, 4, 5, 6, 8, 10 or 12 base pairs, especially 6 base pairs. Longer lengths can be used, especially in the embodiments described below where the 3' overhang region is a known defined sequence which is designed on the basis of a known sequence at the 3' end of the nucleic acid template molecule. Again such longer lengths need to be sufficient and appropriate to enable the hybridization of said primer to the 3' end of a single stranded nucleic acid template. However, for example, lengths up to 30, 40, 50 or 60 base pairs could be used, for example 1 to 55 or 60, 1 to 40, 1 to 30, 3, 4, 5, 6, or 8 to 55 or 60, 3, 4, 5, 6 or 8 to 40 or 3, 4, 5, 6 or 8 to 30 base pairs. Although the lengths of the 3' overhang region can vary, generally, within a single method or experiment or embodiment, they generally have the same length.

The 3' overhang region is conveniently depicted as N residues in Figure 1. This sequence can be a known/specific defined sequence (for example if the sequence of the 3' end of the nucleic acid template is known). Alternatively and preferably, the single stranded 3' overhang region can be formed of or can comprise or consist of a degenerate (random) sequence of bases or nucleotides, conveniently A, T, U, C or G, or of inosine bases (or indeed can comprise or consist of universal bases other than or in addition to inosine such as 2-amino purine, diaminopurine, 5-nitroindole, 5-methyl isodeoxycytosine, iso deoxyguanine, xanthine, oxanine, etc., or mixtures thereof, including mixtures with A, T, U, C and/or G). Thus, where a single stranded 3' overhang region comprising inosine bases is referred to herein, a sequence comprising universal bases or a mixture thereof is also provided. A combination of degenerate sequences and universal bases might also be used.

Optionally, modified nucleotides (or unnatural nucleotides), such as locked nucleic acid (LNA) nucleotides, functionalized LNAs, nucleobase-functionalized LNAs, functionalized nucleic acids or nucleobase-functionalized nucleic acids etc. can be incorporated within the structure of single-stranded overhang or double-stranded regions of the dsPrimers of the invention. Modified nucleotides (or unnatural nucleotides) can be used in all, a portion, just several or individual bases of the primer sequence (single or double stranded portion or both) for example purines, pyrimidines and/or only some of the selected bases of a desired sequence for incorporation in the primer can be replaced with such modified nucleotides (or unnatural nucleotides). Modified nucleotides (or unnatural nucleotides) can also be included in a random or degenerate portion of the dsPrimers of the invention.

Such degenerate sequences, universal sequences, modified nucleotide, unnatural nucleotide or inosine- or inosine-like containing sequences, or combinations thereof, etc., can be designed and prepared by methods which are well known and standard in the art. Indeed, commercial oligonucleotide suppliers can synthesise and mass-produce both degenerate (random) and inosine sequences of a chosen length. In addition, methods of making random hexamers (and random oligos of other lengths) are described in the art and could be used. The advantage with using degenerate sequences or inosine or other universal or modified or unnatural nucleotide bases as the 3' overhang region is that no knowledge of the template sequence is required for the methods of the invention to be conducted and these primers can be used to hybridize to the 3' end of any nucleic acid template molecule. This is a significant advantage.

The use of degenerate sequences or inosine or other universal or modified or unnatural nucleotide base (or combinations thereof) sequences in the 3' overhang region can place a practical construction limitation on the length of the 3' overhang region. For each possible overhang sequence to be represented in the dsPrimer molecules (which is generally important to ensure that the dsPrimers can hybridize with any possible sequence which may be present at the 3' end of any nucleic acid template molecule), then $4^X$ different overhang sequences need to be generated (where x is the length of the 3' overhang). This means that if the overhang region were 25 base pairs, $4^{25}$ different dsPrimers would need to be constructed. In addition, for efficient hybridization, it is desirable to have more than one copy (ideally more like >6 copies) of each unique overhang sequence. Thus, it can be seen that the longer the 3' overhang sequence is, then the more dsPrimers have to be produced, which will have a knock on effect on costs. Thus, in practice, around 10 or 12 base pairs or less (e.g. 6 or 8 base pairs) is preferred for a degenerate or inosine, universal base or modified or unnatural nucleotide overhang region. The use of shorter overhangs will lead to cost advantages.

A population (or a library) of dsPrimers of the invention with the features as described herein (e.g. a population of dsPrimer molecules with different 3' overhang regions as described above, e.g. with $4^X$ different overhang sequences, present in one or more copies, e.g. up to 5 or 6 copies or > 5 or 6 copies, e.g. up to 10 copies of each dsPrimer, e.g. degenerate, inosine, universal or unnatural/modified nucleotide overhang regions) or kits comprising such molecules form a yet further aspect of the invention. Preferred methods of the invention which use such degenerate or universal or modified or unnatural nucleotide sequences (or combinations thereof) in the 3' overhang region will thus use such populations in which all possible combinations of overhang sequences are present in one or more copies so that all possible sequences at the 3' end of the nucleic acid templates can be targeted. At its broadest, a population or library comprises more than one member, but the size of the library or population for use in the methods or products of the invention can readily be determined by the skilled person depending on the desired application (e.g. an appropriate size of population or library is chosen in order for the method or application to function successfully).

In all aspects of the invention, multiple copies (or a plurality) of the dsPrimers will generally be present. Again, the appropriate number of copies required for the particular aspect concerned can readily be determined by the skilled person.

The dsPrimers and associated methods and kits of the invention described herein have applications in a wide range of sequencing and NGS methods for example, massively parallel signature sequencing (MPSS), polony sequencing, 454 pyrosequencing, Illumina (Solexa) sequencing, SOLiD sequencing, Ion Torrent™ (Life Technologies, Inc) semiconductor sequencing, DNA nanoball sequencing, heliscope single molecule sequencing, single molecule real time (SMRT) sequencing, nanopore DNA sequencing (including GridION™ and MinION™ systems), tunnelling currents DNA sequencing, sequencing by hybridization, sequencing with mass spectrometry, microfluidic Sanger sequencing, microscopy-based techniques, RNAP sequencing, in vitro virus high-throughput sequencing and other methods as known in the art.).

Also provided are compositions comprising the dsPrimers of the invention. Preferred compositions comprise a polypeptide or enzyme with DNA polymerase activity, deoxynucleotides, and at least one double stranded primer (for example a population of said double stranded primers) which is capable of hybridizing to a single stranded nucleic acid template, wherein said primer comprises a double stranded region and a single stranded region, wherein the single stranded region is a 3' overhang region and wherein the 3' overhang region enables the hybridization of said primer to the 3' end of the nucleic acid template. Said compositions may further comprise a target nucleic acid or a population thereof, and optionally in said compositions the 3' end of the single stranded region of said double stranded primer has been extended by the addition of nucleotides.

Preferred kits comprise a DNA polymerase, or a polypeptide or enzyme having DNA polymerase activity, and at least one dsPrimer of the invention (for example a population of dsPrimers). Optionally said kits further comprise deoxynucleotides. Kits containing dsPrimers of the invention can be produced for and have use in many applications including: PCR amplification, Diagnostic PCR, Real-time PCR (or quantitative PCR), Diagnostic Real-time PCR (or quantitative PCR), Nanoparticle-based nucleic acid separation and purification, Massively Parallel Sequencing (MPS), a.k.a. Next Generation Sequencing (NGS) library preparation and construction, Third Generation Sequencing library preparation and construction, in-vitro translation and cloning.

dsPrimers of the invention and kits containing them can be also be produced for adding adaptors to single stranded or double stranded nucleic acids solely via the hybridization of dsPrimers without any ligation or polymerisation reaction, for example by performing transcription without polymerization in methods known in the art in a manner similar to standard transcription but at reduced temperatures, for example 25°C, 20°C, 16°C or 4°C using an RNA polymerase, or polypeptide or enzyme with RNA polymerase activity, and its appropriate promoter in the dsPrimer. Suitable enzymes and kits for such transcription include, but are not limited to, T7 RNA polymerase, SP6 RNA Polymerase, T3 RNA polymerase etc., TranscriptAid T7 High Yield Transcription Kit, (Thermo Scientific) T7 High Yield RNA Synthesis Kit (NEB), etc., MEGAscript® T7 Transcription Kit, MAXIscript® SP6 Transcription Kit, MAXIscript® T3 Transcription Kit (all Invitrogen). One possible application of such methods is in the preparation of sequencing libraries for nanopore sequencing, or other sequencing methods where it would be possible or preferable to omit the ligation or polymerization steps.

The dsPrimers of the invention provide various advantages in their application to next generation sequencing (NGS) and high-throughput genomics workflow improvement, in particular an increase the efficiency of library construction and target enrichment processes after fragmentation of DNA samples. Further advantages in such applications include, but are not limited to: (i) no incorrectly positioned adapters are generated - the dsPrimers and methods of the present invention operate in such a way that each and every one of the 5' and 3' adapters will be attached to the correct ends of the fragmented dsDNA; (ii) no DNA fragments will have double copies of the same adapter at both their 5' and 3' ends; (iii) no self-conjugated adapters are created/present, (iv) a reusable backup template of the whole adapter-containing NGS library can be created without any dilution, via the use of nanoparticle-based dsPrimers (as illustrated in Design NP2); (v) such back up templates of the adapted NGS library can be amplified and/or sequenced again whenever necessary, including after long-term storage.

For the SELEX methods preferred compositions comprise one or more single stranded nucleic acid molecules selected for binding to a ligand of interest, a dsPrimer of the invention (for example a population of dsPrimers) and a DNA polymerase or a polypeptide or enzyme having DNA polymerase activity.

Any dsPrimer as described herein can be included in such compositions or kits, and preferred such dsPrimers are also described herein, e.g. a kit or composition with a combinaton of dsPromoter molecules and dsTerminator molecules as described herein is preferred, as they will in particular find utility as RNA (for example, mRNA, sRNA, siRNA, snRNA, miRNA, snoRNA, exRNA, piRNA and long ncRNA etc.) amplification and diagnostic kits; in-vitro translation kits; or cloning kits.

Thus, a preferred embodiment of the invention provides a kit or composition comprising:

(i) a double stranded primer which comprises a double stranded region and a single stranded region, wherein the single stranded region is a 3' overhang region and wherein the 3' overhang region enables the double stranded primer to target and hybridize to the 3' end of a single stranded nucleic acid template, wherein said single stranded 3' overhang region preferably comprises a degenerate sequence or a sequence comprising universal bases, wherein the double stranded primer is made up of two separate strands, and wherein said double stranded region of said double stranded primer comprises a promoter region; and

(ii) a double stranded primer which comprises a double stranded region and a single stranded region, wherein the single stranded region is a 3' overhang region and wherein the 3' overhang region enables the double stranded primer to target and hybridize to the 3' end of a single stranded nucleic acid template, wherein said single stranded 3' overhang region preferably comprises a degenerate sequence or a sequence comprising universal bases, wherein the double stranded primer is made up of two separate strands, and wherein said double stranded region of said double stranded primer comprises a terminator region or a primer binding site.

A yet further preferred embodiment of the invention provides a kit or composition comprising:

(i) a single type of double stranded primer wherein said double stranded primer comprises a double stranded region and a single stranded region, wherein the single stranded region is a 3' overhang region and wherein the 3' overhang region enables the double stranded primer to target and hybridize to the 3' end of a single stranded nucleic acid template, wherein said single stranded 3' overhang region preferably comprises a degenerate sequence or a sequence comprising universal bases, wherein the double stranded primer is made up of two separate strands and wherein said double stranded region of said double stranded primer comprises a primer binding site; and, optionally

(ii) a single type of single stranded primer which can hybridise to said primer binding site.

In such compositions or kits, preferably a population of dsPrimers are used. Preferably multiple copies of said dsPrimers are present. Preferably the compositions or kits are for use in the methods of the invention. Thus, in addition, such kits preferably also include instructions for performing the methods of the invention.

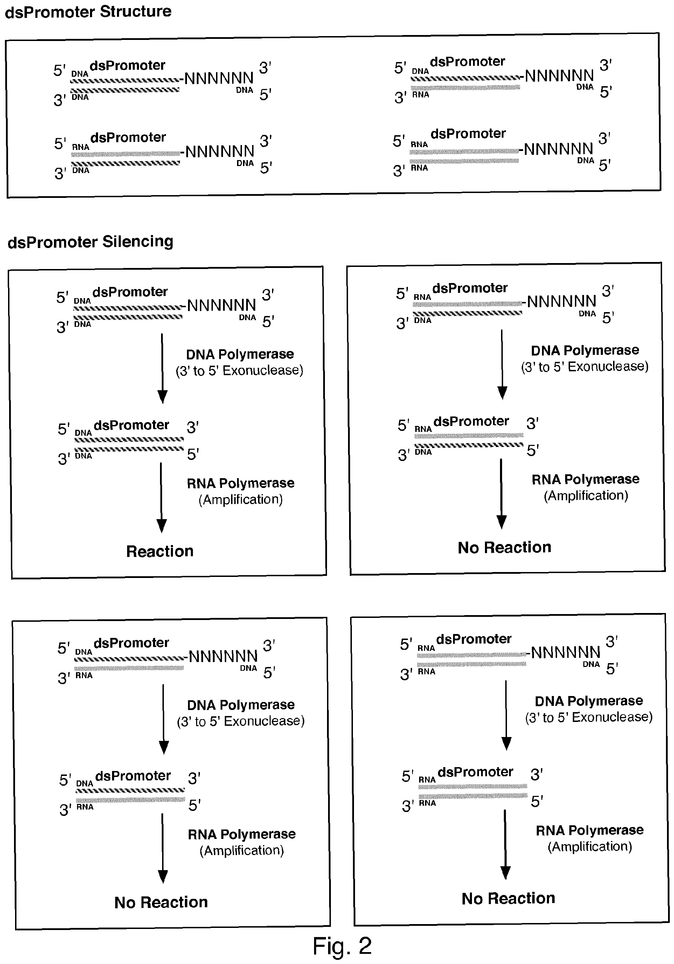

As mentioned above, the double stranded region of the dsPrimer can contain useful motifs such as a promoter region. The general structure of such a dsPromoter is shown in Figure 2. Again, as for the dsPrimer, it can be seen that there is no limitation on the length of the double stranded regions containing these promoter sequences or on the nucleic acid types which form them, for example the sequences can be made of DNA or RNA or can be hybrid DNA/RNA molecules for example where one strand of the double stranded region is DNA and the other strand is RNA. Such dsPromoters are thus alternative and sometimes preferred examples of the dsPrimers of the invention. Other exemplary features of the structure of these dsPromoters are as described above for the dsPrimers.

Advantageously, whether or not these dsPromoter molecules are functional will depend on the type of promoter and can thus be manipulated. For example, some promoters are only functional when they are in the form of double stranded DNA. Examples of such promoters are DNA dependent RNA polymerase promoters, such as the T7 promoter, which result in the synthesis of RNA from a double stranded DNA promoter. Other examples are the SP6 or T3 promoter.

Such properties can be harnessed in the methods of the invention, for example as shown in Design 1. Here a hybrid (DNA/RNA) dsT7Promoter is used in which the upper strand is DNA and the lower strand is RNA, meaning that the promoter is inactive or activity of promoter extremely decreased (McGinness and Joyce., J. Biol. Chem., 277:2987-2991, 2002; Arnaud-Barbe et al., Nucleic Acids Research, 26:3550-3554, 1998). This dsPromoter hybridizes with a single stranded nucleic acid template molecule in accordance with the invention and, after the first round of polymerization, i.e. in the case of Design 1, after the first primer extension reaction and the production of the complementary strand to replace the lower part of the dsPromoter (as will be described in more detail elsewhere herein), both the strands of the double stranded region of the original dsPromoter are now DNA molecules (the upper strand was always DNA and the lower DNA strand has been synthesised by a DNA polymerase using the upper strand of the dsPrimer/Promoter as a template (see Design 1). This has the effect that the T7 promoter region is now functional meaning that the double stranded molecule obtained as a result of these steps can act as an amplification template in which an appropriate RNA polymerase (T7 polymerase) can now bind to the functional T7 promoter site and produce multiple copies of an RNA molecule in a template dependant amplification reaction. Multiple copies of the RNA molecule can be synthesised. In this particular example, because of the nature of the T7 promoter which is used this will have the effect that the residues GGG will be added to the 5' end of the amplified nucleic acid product.

This step of starting with an inactive dsPromoter and designing the methods so that the promoter later becomes active is a useful tool in many of the design protocols described herein. Advantageously, this allows a degree of control to be exerted on the method in that amplification can only take place once the promoter has become functionally active.

The T7 promoter is only given as one particular example of a promoter which might be used in the methods of the invention and any other DNA dependent RNA polymerase promoter could be used in a similar way, e.g. the SP6 promoter. Other promoters with different functional properties could also be utilised. For example, the use of the phi6 promoter (also referred to as a phi6 initiation sequence) which is an RNA dependent RNA polymerase initiation sequence, is elegantly demonstrated in Designs 12-13. In contrast to the T7 promoter, the phi6 initiation sequence is active when in the form of a double stranded or a single stranded RNA molecule, and is particularly active when in a single stranded RNA form. In the case of a phi6Promoter sequence (initiation sequence), a phi6 RNA Replicase (which is an example of an RNA dependent RNA polymerase) can bind to the phi6 promoter and transcribe an RNA strand in a template dependent manner. Multiple copies of such RNA strands can be produced by the replicase. Any other RNA dependent RNA polymerase initiation sequence or RNA dependent RNA polymerase could equally be used.

In Designs 12-13, the active phi6 promoter is provided in the form of a single stranded RNA molecule (see step 07) and the phi6 replicase then transcribes the other strand of the RNA in a template dependent manner, initiating from the 3' end of the single stranded RNA molecule, to form double stranded RNA. Thus, again, protocols can be designed where the original dsPrimer/Promoter molecule incorporates a phi6 promoter sequence which is inactive as it is present in a double stranded DNA format or double stranded DNA/RNA format, but through the course of the method this phi6 promoter is incorporated into the template and eventually provided as an active single stranded (or double stranded) RNA phi6 promoter, which can then bind phi6 replicase and cause multiple copies of RNA (e.g. multiple copies of double stranded RNA molecules) to be produced in a template dependent manner from multiple copies of RNA molecules which contain the phi6 promoter sequence at the 3' end.

In other embodiments of the invention, the dsPrimer can be designed to contain a terminator region. Thus, a dsTerminator is an alternative and sometimes preferred form of a dsPrimer of the invention. Thus, other exemplary features of the structure of the dsTerminators are as described above for the dsPrimers. The dsTerminators of the invention can be particularly useful in combination with a dsPromoter of the invention. As will become clear from discussion elsewhere herein, methods can be designed in which a dsPromoter can be incorporated (inserted) at one end of the nucleic acid template and a dsTerminator can be incorporated at the other end of the template. Then, when the amplification step is carried out, appropriate polymerases can start at the promoter region and transcription will terminate at the incorporated terminator region at the other end of the template molecule. This design option is preferred in embodiments in which it is desired to obtain amplified copies of an RNA molecule in a native form (i.e. in a form which mimics that which would be generated in a natural reaction, e.g. in which an RNA polymerase would start transcription at a promoter site and stop at a terminator site). Design 5 gives an illustrative example of how such dsPromoters and dsTerminators of the invention can be used.

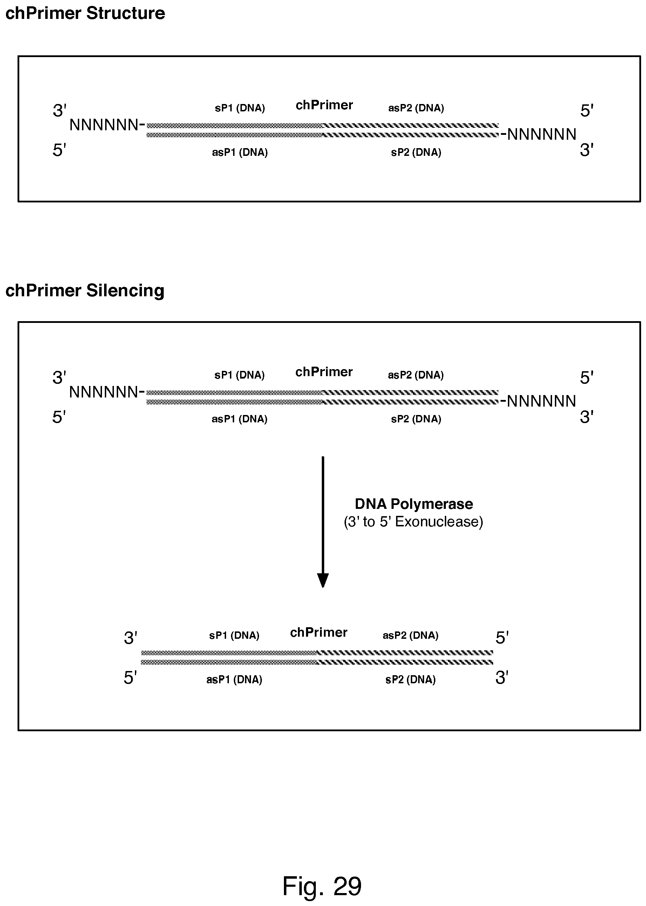

A particularly preferred dsPrimer of the invention is referred to herein as a chimeric primer (chPrimer). The general structure and features of the chPrimers of the invention are shown schematically in the Design Ch1 and Figure 29. As can be seen from Design Ch1, the chPrimers of the invention have two single stranded regions, i.e. two single stranded 3' overhang regions, which are present on the 3' end of each strand of the dsPrimer (see Design Ch1). In such chPrimers the single stranded 3' overhang regions function in the same way as in the simple dsPrimers described above, in that they still enable the hybridization of the chPrimer to the 3' end of a single stranded nucleic acid template. Thus, preferred features of the 3' single stranded overhang region of the chPrimers are as discussed above (e.g. they can comprise known defined sequences, degenerate (random) sequences or inosine (universal) sequences, and optionally the two single stranded regions could be of different types, e.g. one could be a known defined sequence and one could be a degenerate sequence or an inosine sequence, etc).

Thus, it can be seen that in the preferred primers of the invention, no 5' overhang regions are present and in such preferred embodiments there is only one (e.g. in a simple dsPrimer) or two (e.g. in a chPrimer) 3' overhang regions present. Thus, chPrimers form a further preferred embodiment of the invention and the present invention thus provides a double stranded primer which comprises a double stranded region and two single stranded regions, wherein the single stranded regions are 3' overhang regions which are present on the 3' end of each strand of the double stranded primer, and wherein the double stranded primer is made up of two separate strands. Preferably said single stranded 3' overhang regions comprise a degenerate sequence or a sequence comprising universal bases as described elsewhere herein.

Kits and compositions comprising such chPrimers of the invention form preferred aspects.

Advantageously however, the same chPrimer can hybridize to two nucleic acid template molecules at the same time. For example, in situations described in more detail elsewhere herein where the starting nucleic acid molecule is double stranded and is then denatured to form single stranded nucleic acid template molecules for use in the present methods, then the same chPrimer is able to hybridize to both the sense and the anti-sense strands at the same time (see Design Ch1).

Although the schematic designs show the binding of a sense template molecule to one of the 3' overhang regions of the chPrimer and the anti-sense molecule to the other 3' overhang region, it should be noted that the degenerate or inosine nature of the 3' overhang regions (when these are used) will mean that there is no restriction or preference as to whether the sense or anti-sense molecules bind (the chPrimer may bind to sense/sense, antisense/antisense or sense/antisense pairs of molecules). However, once the first round of polymerization has been completed, sense/anti-sense pairs will hybridize to each other after each step of denaturation and annealing. Thus, at the end of the amplification, it is the sense/antisense paired molecules which are amplified.

Another important distinctive feature of the chPrimers of the invention (see Figure 29) is that the double stranded region of the chPrimer generally comprises two different primer sequences/sites (also referred to herein as P1 and P2) which are adjacent to each other and give rise to the name "chimeric primer". The two primer sites (P1 and P2) can either be directly adjacent to each other or separated by one or more base pairs. In other embodiments the chPrimers can comprise one primer site or two or more primer binding sites, which can be the same or different. Equally, as described above for the types of regions which may be included in the simple dsPrimers, the double stranded region of the chPrimers can for example comprise one or more primer binding sites, promoter regions, terminator regions, nucleic acid fragments to be joined to said nucleic acid templates, restriction sites or labels, and other useful sequences described herein etc., which may be the same or different, or combinations thereof.

In essence, the chPrimers of the invention can be viewed conceptually as two of the simple dsPrimers as described above and depicted schematically in Figure 1 joined together (the chPrimer structure is shown in Figure 29). Thus, other exemplary features of the structure of the chPrimers are as described above for the simple dsPrimers. However, the existence of two (or more) same or different primer sites (or other types of region as described above) and the ability of an individual chPrimer molecule to hybridize to two template strands, allows for further variation and complexity in the subsequent steps of the methods. For this reason the chPrimers are sometimes referred to herein as complex dsPrimers.

For example, as shown in the schematic Design Ch1, such chPrimers can usefully be used to generate amplification products which have a different primer site at each end. In the schematic shown in Design Ch2, it is possible to incorporate both of the different primer sites at each end of the amplified product, which allows even more flexibility, for example with downstream steps (e.g. this allows the selection of the length and the primer site content of each end of the double stranded DNA (dsDNA) by using different sets of PCR primers).

Thus, the use of the chPrimers of the invention readily allows the formation of different primer sites at each end of the amplified nucleic acid product (amplification product). This feature can be particularly useful when the starting population is dsDNA, the desired amplified nucleic acid product is double stranded (ds) DNA and both the sense and antisense strands are used in the methods of the invention (after strand separation to form single strand nucleic acid template molecules). In contrast, if a single simple dsPrimer is used in such a method then identical primer sites will be formed at each end of the dsDNA amplification product (see for example Design 2 and 3). Thus chPrimers are particularly useful for any applications which particularly need different primer sequences (e.g. different adaptor sequences) at each end of the amplified dsDNA. An exemplary application would be Next-Generation Sequencing but others will be readily apparent to a skilled person in the field.

In addition, through the ability of the same chPrimer to bind to two nucleic acid strands at the same time, the use of the chPrimers of the invention allows the insertion of different orientations of the same primer sequences into the template molecules/amplification products. (The use of a single simple dsPrimer will insert the same orientations of the same sequence into the template molecules/ amplification products).

As for the simple dsPrimers described elsewhere herein, the chPrimers can comprise DNA, RNA or a mixture of types of nucleic acid. For example, in preferred embodiments of the invention (for example as illustrated in the schematic designs Ch5 and Ch6), a portion of the chPrimer is an RNA sequence and the rest is DNA, which can be used to advantageous effect, e.g. to form RNA/DNA hybrid double-stranded structures in which the RNA part can be degraded by any polypeptide with RNase H like-activity. For example, in the schematic designs Ch5 and Ch6, one of the anti-sense primer portions is RNA, and this is preferred. However, methods where one of the sense primer portions is RNA are also envisaged.

The nucleic acid templates of the invention, e.g. as referred to in step (i) of the methods, are single stranded nucleic acid molecules. The single stranded nature of the template is important in order to enable the single stranded 3' overhang region of the dsPrimer to hybridize to the 3' end (3' terminus) of the nucleic acid template. This does not mean however that the present methods are not suitable for manipulation or amplification of double stranded nucleic acid molecules (e.g. genomic DNA), as appropriate single stranded nucleic acid template molecules for manipulation or amplification using the methods of the invention can readily be derived or obtained from double stranded nucleic acid molecules by any appropriate means, for example by denaturation, e.g. heat denaturation or any other appropriate treatment such as the use of organic solvents (e.g. DMSO or formamide) or high pH (e.g. an alkaline pH, e.g. a pH greater than 11.3) to break the hydrogen bonding between the DNA strands, the use of low salt concentration, urea (e.g. 8M urea), formaldehyde (e.g. 70% formaldehyde). A DNA helicase enzyme could also be used or a DNA polymerase with helicase-like unwinding activity, e.g. phi29 polymerase. As the use of a DNA helicase would involve unwinding the dsDNA enzymatically, the heat denaturation and any subsequent PCR thermocycling steps could be omitted. Thus, a DNA helicase or another molecule with DNA helicase-like activity would be particularly useful for embodiments where the methods are desired to be carried out under isothermal conditions.

In addition, as will be clear from the design schematics shown and described herein, the nucleic acid template can be formed from any type of nucleic acid, for example any type of natural nucleic acid or non-natural nucleic acids (such as locked nucleic acids (LNAs), functionalized LNAs, nucleobase functionalized LNAs, or other types of artificial or modified nucleic acid molecules, etc) or mixtures thereof. For example, the nucleic acid template molecules may be DNA or RNA molecules, a mixture of DNA and RNA molecules, chimeric molecules or even modified or non-natural nucleic acids (such as locked nucleic acids (LNAs), functionalized LNAs, nucleobase-functionalized LNAs or other types of modified nucleic acid molecules, etc).

For example, the SELEX methods described herein can be applied to templates containing a mixture of natural, unnatural or modified bases, including templates that are partially or wholly constructed of locked nucleic acid. Some particular applications of targeting LNA containing templates include (i) the creation of LNA containing aptamers developed by the SELEX methods of the invention via chain terminators; (ii) the creation of advanced LNA aptamers containing functionalized LNAs or nucleobase-functionalized LNAs developed by the SELEX methods of the invention via chain terminators. In applications (i) and (ii) the use of the primers and methods of the invention ensures that there is no need for post-selection modifications to remove fixed (or constant) primer regions or to increase the stability against enzymatic degradation of discovered aptamer molecules and can also increase the affinity and stability of secondary structure originated ligand binding properties of LNA aptamers for the development of novel diagnostic and therapeutic applications.

There is also no particular restriction on the format of the nucleic acid templates which can be used in the present invention, apart from the requirement that the molecules are at least partially single stranded where targeting by the dsPrimers of the invention takes place. Thus, the nucleic acid templates may have modified ends or they may have been derived from modified nucleotides or nucleobases. Equally they may be derived from (denatured from) double stranded molecules with sheared ends or blunt ends (for example they can take the form of 5'end phosphorylated DNA e.g. 5' phosphorylated genomic DNA, see for example Designs 9 and 10, for example as typically prepared for use in a sequencing device, which can then be denatured into single stranded molecules). They can also take the form of nucleic acid molecules with a poly-A tail, for example can be mRNA molecules, or DNA molecules with an engineered poly-A tail at the 3' end (see for example Designs 14 - 17). Indeed, the use of the methods of the invention with various lengths of genomic DNA, specifically sheared genomic DNA, is illustrated in the attached experimental Examples. The methods of the invention can also be conducted on hybrid template molecules (i.e. template molecules with both single stranded and double stranded regions) where single stranded regions of such hybrid templates can be targeted. Such embodiments can be particularly useful for applications such as cloning or targeting of RNA interface pathways.

It should be noted that if the single stranded nucleic acid template molecules are derived from double stranded nucleic acid molecules, then both of the strands of the original double stranded molecules (i.e. both the sense and the anti-sense strands) can be included in the methods of the invention. In such embodiments and where a simple dsPrimer rather than a chPrimer is used, in the hybridizing step (i) then separate dsPrimer molecules can hybridize to the 3' ends of both of the single strands and the subsequent steps of polymerization and amplification can be carried out on both strands simultaneously. An example of such an embodiment is shown in Design 2. As described elsewhere herein, chPrimers can also be used with a starting population of double stranded nucleic acid molecules. Indeed a preferred use of chPrimers is with a starting population of dsDNA molecules.

Thus, it can be seen that the methods of the invention can be used to manipulate or amplify any type of nucleic acid molecule and can be used with a starting population of either double stranded or single stranded nucleic acid molecules.

In all the embodiments of the invention, it is important to note that if the starting population of nucleic acid molecules are double stranded molecules and such molecules are denatured to form the single stranded molecules for use in the methods of the invention, advantageously, both of the strands can remain in the reaction mixture and the dsPrimers of the invention can hybridize to the 3' ends of each of the strands.

The term "hybridizing", "hybridization" etc., as used herein in relation to step (i) of the methods describes the step in which the dsPrimers of the invention are brought into contact with the appropriate nucleic acid template molecules and then attach to or bind with the 3' ends of the nucleic acid templates by virtue of the (noncovalent, H bond mediated) interaction of base pairs in the 3' single stranded overhang region(s) of the dsPrimer with appropriate complementary base pairs at the 3' end of the single stranded nucleic acid template molecules. Such steps are also referred to herein as "annealing" and these terms should be regarded as interchangeable.

As with any method of nucleic acid manipulation or amplification, it is well within the normal capability of a person skilled in the art to provide the appropriate reagents and conditions (e.g. temperature) to carry out the hybridization step. For example a skilled person could readily determine the number of dsPrimer molecules of the invention which should be brought into contact with the nucleic acid template molecules to obtain sufficient hybridization to carry out the methods of the invention.

As will be well known to a person skilled in the art, exact complementarity is not always required for stable hybridization/annealing. However, exact complementarity will generally occur in embodiments where the sequence of the 3' end of the nucleic template is known and the sequence of the 3' single stranded overhang is designed to match it, or in embodiments where degenerate 3' overhang sequences are used (where in most cases, as discussed above, the population of dsPrimers which are used in the methods, will have been generated to have all possible sequence variations of a defined 3' overhang length, which means that an exact complementary sequence should be present to enable hybridization/annealing). In contrast, in embodiments where a length of inosine or other universal bases are used as the 3' overhang region, then as some of these universal bases cannot always pair with each of adenine, thymine, cytosine or guanine bases, then exact complementarity will not always occur. However, in general sufficient complementarity will be present in order for stable hybridization to take place with the 3' end of the template.

The nucleic acid manipulation methods of the invention thus comprise the steps of:

(i) hybridizing a double stranded primer to a single stranded nucleic acid template, wherein said primer comprises a double stranded region and a single stranded region, wherein the single stranded region is a 3' overhang region and wherein the 3' overhang region enables the hybridization of said primer to the 3' end of the nucleic acid template; and

(ii) at least one round of polymerization using a DNA polymerase, wherein at least the first round of polymerization comprises using a DNA polymerase with 5' to 3' polymerization activity to carry out a primer extension reaction to synthesise nucleotides in a template dependent manner from the 3' end of the single stranded region of said hybridized double stranded primer.

The nucleic acid amplification methods of the invention comprise the steps of:

(i) hybridizing a double stranded primer to a single stranded nucleic acid template, wherein said primer comprises a double stranded region and a single stranded region, wherein the single stranded region is a 3' overhang region and wherein the 3' overhang region enables the hybridization of said primer to the 3' end of the nucleic acid template;

(ii) at least one round of polymerization using a DNA polymerase in order to form an amplification template, wherein at least the first round of polymerization comprises using a DNA polymerase with 5' to 3' polymerization activity to carry out a primer extension reaction to synthesise nucleotides in a template dependent manner from the 3' end of the single stranded region of said hybridized double stranded primer; and

(iii) at least one amplification step to form an amplified nucleic acid product;

However, an important advantage of the method of the present invention is that the hybridizing step (i) does not involve and does not require the formation of a phosphodiester bond (or indeed any other type of covalent bond) between the 3' end of the nucleic acid template and the double stranded region of the dsPrimer used in step (i), more specifically such a bond is not required to be formed (or is not formed) between the 3' end of the nucleic acid template and the 5' end of the lower strand/non-overhang strand of the dsPrimer (or equivalent strand of a chPrimer) used in step (i). Such a step in which a phosphodiester (covalent) bond is formed between the 3' end of a nucleic acid molecule and another nucleic acid molecule is often termed a ligation step or reaction. Such ligation reactions, for example in the form of the ligation of an -OH group at the 3' end of a nucleic acid template to the 5' phosphate ($-PO_3$) group of an adaptor molecule, are used in many prior art techniques of nucleic acid manipulation and it is highly advantageous that the methods of the present invention can take place without the formation of such phosphodiester (covalent) bonds. For example, the absence of the need for a ligation reaction as part of or immediately subsequent to the hybridization of step (i) is advantageous in terms of time and also in terms of costs as fewer reagents are necessary. In addition, ligation can lead to undesired by-products. The formation of such covalent bonds also depends on the 5' end of the nucleic acid molecule that is being ligated to the 3' end of the nucleic acid template being modified, usually to include a 5' phosphate group. The fact that the methods of the invention can be carried out without requiring additional modification of the molecules to be used in the reaction provides a yet further advantage in terms of time and cost.

Thus, in the methods of the present invention in which certain steps take place without the formation of such phosphodiester (covalent) bonds, such bonds are not formed either because there is no appropriate phosphate group at the 5' end of the dsPrimer which has been hybridized to the nucleic acid template and/or beacuse no ligase enzyme is provided to ligate the -OH group at the 3' end of the nucleic acid template to a 5' phosphate ($-PO_3$) group at the 5' end of the dsPrimer (if the dsPrimer has been modified to include such a group).

Thus, in such methods the dsPrimers used in step (i) of the methods, and optionally in subsequent steps, do not contain a 5' phosphate group on the lower strand/non-overhang strand of the dsPrimer (or equivalent strand when the dsPrimer is a chPrimer).

The lack of a requirement for a ligation step in connection with the hybridization of the dsPrimers of the invention to the 3' end of the nucleic acid template in order for the methods of the present invention to work has the effect that both the hybridizing step (i) and at least the first primer extension reaction to synthesise nucleotides in a template dependent manner from the 3' end of the single stranded region of said hybridized dsPrimer in step (ii) can take place without the formation of a phosphodiester (covalent) bond between the 3' end of the nucleic acid template and the double stranded region of the double stranded primer of step (i), more specifically such a bond is not formed between the 3' end of the nucleic acid template and the 5' end of the lower strand/non-overhang strand of the dsPrimer (or equivalent strand when the dsPrimer is a chPrimer) used in step (i). Indeed, such embodiments are preferred.

Viewed alternatively, in the methods of the present invention, the steps up to and including the first primer extension reaction from the 3' end of the single stranded region of said hybridized dsPrimer in step (ii) can take place without the formation of a phosphodiester (covalent) bond between the 3' end of the nucleic acid template and the double stranded primer of step (i). Indeed, such embodiments are preferred.

Although not necessarily preferred, ligation reactions or the formation of phosphodiester (or other covalent) bonds, can be used in steps subsequent to the first primer extension reaction. Equally however all of the steps of the methods of the invention can take place without the formation of such covalent bonds (e.g. without any ligation reactions or steps). Indeed, such embodiments are preferred. Thus in a preferred embodiment of the present invention, the methods of the invention, for example said amplification or manipulation of nucleic acid or said formation of an amplified nucleic acid product, does not involve the use of a ligase activity or does not involve a ligation reaction or the use of a ligase enzyme or a ligation step.

In addition, unlike many prior art methods of nucleic acid manipulation, the methods of the present invention do not require the ligation or hybridization of a molecule (e.g. an adaptor molecule) to the 5' end of the initial nucleic acid template (e.g. the nucleic acid template as present in step (i) of the methods described herein), or do not require the use of a double stranded molecule with a 5' overhang region. Thus, in preferred embodiments of the present invention, the use of said steps or molecules are excluded.

In other aspects of the invention, methods without polymerization or ligation steps are provided. Such methods in which a dsPrimer is targeted and hybridized to the 3' (or 5') end of a single stranded nucleic acid template can allow the dsPrimer to be used to attach any desirable sequence or molecule contained in the dsPrimer to the end of the nucleic acid template, e.g. the dsPrimer can be used to attach a label (tag) molecule, or an adaptor, e.g. for end modification purposes.

Thus, a yet further aspect of the invention provides a method of nucleic acid manipulation comprising a hybridizing step wherein a double stranded primer is hybridized to the 3' end of a single stranded nucleic acid template, wherein said primer comprises a double stranded region and a single stranded region, wherein the single stranded region is a 3' overhang region and wherein the 3' overhang region enables the double stranded primer to target and hybridize to the 3' end of the nucleic acid template, wherein said single stranded 3' overhang region preferably comprises a degenerate sequence or a sequence comprising universal bases, and wherein the double stranded primer is made up of two separate strands;

wherein said hybridizing step takes place without the formation of a phosphodiester bond between the 3' end of the nucleic acid template and the double stranded primer of the hybridizing step.

A similar process can also be carried out at the 5' end of a single stranded nucleic acid template. Thus, a yet further aspect of the invention provides a method of nucleic acid manipulation comprising a hybridizing step wherein a double stranded primer is hybridised to the 5' end of a single stranded nucleic acid template, wherein said primer comprises a double stranded region and a single stranded region, wherein the single stranded region is a 5' overhang region and wherein the 5' overhang region enables the double stranded primer to target and hybridize to the 5' end of the nucleic acid template, wherein preferably said single stranded 5' overhang region comprises a degenerate sequence or a sequence comprising universal bases, and wherein the double stranded primer is made up of two separate strands; wherein said hybridizing step takes place without the formation of a phosphodiester bond between the 5' end of the nucleic acid template and the double stranded primer of the hybridising step.

Adding a dsPrimer structure to the 5' end of a single stranded nucleic acid template, can also be used e.g. for ligation, or tagging, or end modification purposes. These structures will not initiate conventional primer extension or amplification as they will not provide a substrate for DNA polymerase, though currently contemplated artificial polypeptides or as yet undiscovered natural enzymes with a reverse 5'-3' DNA polymerase activity (see for example, A design proposal of the reverse DNA polymerase (3'-5' direction), Penghui Shi, http://arxiv.org/abs/1310.4073) may permit primer extension or amplification using such primers in the future.

Step (ii) of the methods of the invention involves at least one round of polymerization using a DNA polymerase in order to form an amplification template, wherein at least the first round of polymerization comprises using a DNA polymerase with 5' to 3' polymerization activity to carry out a primer extension reaction to synthesise nucleotides in a template dependent manner from the 3' end of the single stranded region of the dsPrimer after it has hybridized to the 3' end of the nucleic acid template. Thus, for the avoidance of doubt, the reference to the single stranded region of the double stranded primer (dsPrimer) in step (ii) of the described methods refers to the part of the dsPrimer which was single stranded before hybridization, i.e. the single stranded region or 3' overhang region, but has become double stranded by virtue of the dsPrimer having hybridised to the 3' end of the nucleic acid template molecule.

Put another way, step (ii) of the method involves at least one round of polymerization using a DNA polymerase in order to form an amplification template, wherein at least the first round of polymerization comprises using a DNA polymerase with 5' to 3' polymerization activity to synthesise nucleotides in a template dependent manner from suitable 3' ends of nucleic acid strands which are present in the reaction mixture.

As used herein, the term "DNA polymerase" refers to a polypeptide or enzyme comprising DNA polymerase/DNA polymerization activity, e.g. DNA-dependent DNA polymerase and/or RNA-dependent DNA polymerase activity (5' to 3' polymerization activity). As described elsewhere herein, depending on the steps of the methods in which they are used, the DNA polymerase polypeptides as used herein may have various properties, for example as well as having 5' to 3' polymerization activity in order to be able to for example carry out primer extension reactions, the DNA polymerase polypeptides may have strand displacement activity or nick translation activity or reverse transcriptase activity or other useful activities. In addition, where appropriate for the steps of the methods in which they are used, the DNA polymerase polypeptides can optionally have multiple properties such as for example more than one of the properties discussed above.

The appropriate DNA polymerase to select will be readily apparent to the person skilled in the art. For example, if a DNA polymerase with strand displacement activity is required then a convenient choice of DNA polymerase would be DNA polymerase I, Klenow Fragment (such a DNA polymerase also has 5' to 3' polymerization activity) or Phi29 or variants known in the art for example as described in de Vega, Miguel, et al. "Improvement of φ29 DNA polymerase amplification performance by fusion of DNA binding motifs." PNAS 107.38 (2010): 16506-16511, (which has a strong strand displacement ability and is active at a moderate temperature, around 20-37 °C). If a DNA polymerase with nick translation activity is required then a convenient choice of DNA polymerase would be Bst DNA polymerase, Full length (such a DNA polymerase also has 5' to 3' polymerization activity and a good strand displacement activity, and is active at elevated temperatures, around 65 °C), or E. coli DNA polymerase I. T4 and T7 DNA polymerases lack strand displacement activity and 5' to 3' exonuclease activity but have 5' to 3' polymerization activity and a 3' to 5' exonuclease activity which is significantly better than the Klenow fragment. Such polymerases are also active at moderate temperatures (around 20-37 °C). The polymerases which are active at moderate temperatures are appropriate for the isothermal embodiments of the invention. Thermostable DNA polymerases, e.g. Taq, in particular thermostable DNA polymerase enzymes with one or more of strand displacement activity, nick translation activity or reverse transcriptase activity can also be highly useful in the methods of the invention. For example there are modifed versions of Taq polymerase described which have strand displacement activity. In addition VentR, Deep VentR and their exo-variants are thermostable and have strand displacement activity. Additional DNA polymerases that may be selected for use with the dsPrimers, methods and kits of the invention include uracil tolerant DNA polymerases such as the Phusion U Hot Start DNA Polymerase from Thermo Fisher Scientific Inc, the EpiMark® Hot Start Taq DNA Polymerase from New England Biolabs, Inc, the moderately thermostable (active upto 65C) reverse transcriptase "Maxima Reverse Transcriptase" from Thermo Fisher Scientific Inc and the KOD DNA polymerase of Toyobo Co. Ltd and its variants, particularly useful for their ability to function with modified nucleotides.

Some methods of the invention can be carried out using only one round of polymerization using a DNA polymerase. However, as will be described elsewhere herein, other methods of the invention will involve more than one round of polymerization using a DNA polymerase, for example may involve two or three rounds of polymerization and possibly more. In the methods of the present invention, one, two or three rounds of polymerization are generally preferred.

Although the number of rounds of polymerization will vary between the different embodiments of the methods of the invention, a common step to all the methods of the invention is that at least the first round of polymerization comprises using a DNA polymerase with 5' to 3' polymerization activity to carry out a primer extension reaction to synthesise nucleotides in a template dependent manner from the 3' end of the single stranded region of the dsPrimer which has become hybridized to the nucleic acid template.

As with any step of polymerization, it is well within the normal capability of a person skilled in the art to provide the appropriate reagents (deoxynucleotides, etc) and conditions (e.g. thermal cycling conditions) to carry out the first round (and any subsequent rounds) of polymerization. Indeed, some exemplary reagents and conditions are described in the experimental Examples section.

After hybridization of the dsPrimer in step (i) of the method, the 3' end of the single stranded overhang region is available for primer extension using an appropriate DNA polymerase. As described herein, this step of primer extension (also referred to as step (ii)(a)) involves the use of a DNA polymerase with 5' to 3' polymerization activity to synthesise nucleotides in a template dependent manner from the 3' end of the single stranded region of the dsPrimer of the invention. In other words, this first primer extension reaction involves the filling in of nucleotides next to the 3' single stranded overhang region of the dsPrimer (e.g. next to the degenerate or inosine or defined sequences) which has hybridized to the 3' end of the nucleic acid template.

Depending on the particular design of the method, advantageously the DNA polymerase used to carry out this primer extension reaction can be selected to have additional activities to the required 5' to 3' polymerization activity. For example, depending on the method, the DNA polymerase can be selected to also have strand displacement activity (see for example Design 1 and Design 3), and/or reverse transcriptase activity (see for example Design 8 and Designs Ch5 and Ch6) and/or nick-translation activity.

In addition, step (ii) of the amplification and manipulation methods of the invention will generally involve a further step which involves the production or synthesis of the lower strand (complementary strand, non-overhang strand) of the dsPrimer in order to fill in or complete the 3' end of the second strand of the template, e.g. to polymerise the 3' end of the template. Thus, such a step is referred to herein as the production of (or replacing) the complementary strand (complementary part) or non-overhang strand (non-overhang part) of the dsPrimer or the step of production of or replacing the lower part (lower strand) of the dsPrimer. Such a step is also referred to herein as step (ii)(b). In this step, the strand of the dsPrimer which does not contain the 3' overhang region is produced or synthesised or replaced. Such a step generally takes place after the first primer extension step (i.e. after step (ii) (a)) and, in the amplification methods, takes place before the amplification step (iii).

Put simply, such a step involves or has the effect of incorporating the lower strand of the dsPrimer, i.e. the strand which is not directly attached to the single stranded 3' overhang region/the strand which does not contain the 3' overhang region (the non-overhang strand), or the equivalent strand in a chPrimer of the invention, into the nucleic acid template, for example, by joining the lower strand of the double stranded primer onto the 3' end of the nucleic acid template or by in effect incorporating said lower strand of the double stranded primer onto the 3' end of the nucleic acid template by synthesis using the upper strand of the dsPrimer as a template (in other words "replacing the lower strand/part of the dsPrimer"). Any appropriate technique can be used to carry out this lower strand replacement, many of which are illustrated schematically in the various designs of the invention shown herein.

One convenient method/technique is referred to herein as "strand displacement". This technique involves the use of a DNA polymerase to synthesise the lower strand of the dsPrimer using the upper strand of the dsPrimer as a template. During this step, the original lower strand of the dsPrimer is displaced away from the nucleic acid template (and more specifically from the original upper strand of the dsPrimer), hence the terminology "strand displacement" which describes the ability to displace downstream DNA encountered during synthesis. The way in which strand displacement is carried out can be varied appropriately depending on the molecular entities involved and various examples are depicted herein. For example, if the upper strand of the dsPrimer being used as a template is DNA, then a DNA polymerase with strand displacement activity can be used (see for example Design 1). Equally however the upper strand of the dsPrimer can be in the form of an RNA molecule and in this case, a DNA polymerase with strand displacement activity and reverse transcriptase activity is required (see for example Design 8). In carrying out this step the DNA polymerase acts on the 3' end of the original nucleic acid template molecule (see also Designs 3 and 5).

Strand displacement is a preferred and convenient technique to carry out the step of replacement of the lower strand of the dsPrimer. However, there are other techniques that can be used, which would be within the normal skill of a person in this field.

An example of an alternative technique to that of strand displacement is that of a nick translation step. After hybridization of the dsPrimers of the invention to the 3' end of the nucleic acid template in step (i) of the methods a single stranded ("nick") site is formed (see the design schematics included herewith). A DNA polymerase (which has the appropriate nick translation ability) can then be used to replace the single stranded "nick" sites by elongating the 3'OH terminus of the original nucleic acid template molecule while removing nucleotides by 5' to 3' exonuclease activity (which involves degrading the encountered downstream strand via the 5' to 3' exonuclease activity) and replacing them with new nucleotides in order to replace the lower strand of the dsPrimer.

Alternatively to strand displacement or nick translation a step of denaturation, conveniently heat denaturation, could be used to replace the lower strand of the dsPrimer. In such a step, denaturation is used to remove the lower part of the dsPrimer (which is not covalently attached to the nucleic acid template molecule as shown by the presence of a single stranded "nick"), after which a DNA polymerase with 5' to 3' polymerase activity can be used to produce the complementary strand/lower strand by extension from the 3' end of the nucleic acid template using the upper strand of the dsPrimer as a template. An example of such a heat denaturation step to allow replacement of the lower strand of the dsPrimer is shown for example in steps 04 and 05 of Design 2. Such a step can conveniently be carried out by heat denaturation in which a temperature is selected so as to enable the denaturation of the short lower part of the dsPrimer while the long template strand and its complementary strand can still maintain their double stranded structure. Such a temperature will vary depending on the length of the double stranded region of the dsPrimer and the length of the template molecule but can readily be determined by a person skilled in the art. Both generally and particularly in the use of heat denaturation, it is envisaged that a thermostable DNA polymerase with strand displacement activity (for example, those previously mentioned herein or such as described in WO/2011/055737 or EP2377929) can be used in the methods or kits of the invention.

A final specific example of a method/technique of replacing the lower strand of the dsPrimer would be to form a covalent bond between the 3' end of the nucleic acid template and the dsPrimer, more specifically between the 3' end of the nucleic acid template and the 5' end of the lower strand of the dsPrimer (or an equivalent strand when the dsPrimer is a chPrimer), i.e. in effect repairing the single stranded "nick". This would be an example of a technique in which the lower strand of the dsPrimer was joined to the end of the nucleic acid template rather than being synthesised from the upper strand. A convenient way to carry out such a step would be to use a ligase enzyme and a ligase reaction in which case the 5' end of the lower strand of the dsPrimer would need to be modified to incorporate a 5' phosphate group in order that the ligase enzyme could act. This is therefore a less preferred embodiment as an additional processing step will need to be carried out on the dsPrimer and an additional ligation step will need to be carried out which will lengthen the procedure. In addition, the process will be more expensive in terms of reagents. However, there are situations that can be envisaged where the use of a ligase enzyme to carry out this step would be appropriate and convenient.

Steps (ii)(a) and (ii)(b), i.e. the step of primer extension and the step of replacing the lower part of the dsPrimer can, if desired, be carried out at the same time in a single step or can be carried out in separate steps. If these two steps are carried out at the same time, then a single type of DNA polymerase can be used, which is selected to have any necessary additional activity (e.g. strand displacement activity, nick displacement activity, etc) to the 5' to 3' polymerase activity. Equally, different types of DNA polymerases to supply all the necessary activities can be added to the reaction mixture at the same time. It can be seen that carrying out steps (ii)(a) and (ii)(b) at the same time and with a single polymerase can result in a time and cost advantage. Thus, in some aspects of the invention the use of such methods is preferred.

Exemplary methods of the invention which involve only one round of polymerization in order to form an amplification template are shown in Design 1 and Design 8. The Design Ch1 is a further example, although here it is optional as to whether or not one or two rounds of polymerization are carried out.

Other embodiments of the invention involve more than one round of polymerization.

As described above, the first round of polymerization involves the steps of primer extension and optionally the production of the complementary strand/replacement of the lower strand of the dsPrimer. Depending on the design of the method, the techniques of hybridization/annealing, primer extension, strand displacement, nick translation, denaturation, ligation and production of the complementary strand/lower strand of the dsPrimer (or equivalent strand of a chPrimer), as described above and elsewhere herein, can equally be used in the further rounds of polymerization (and indeed in the amplification step (iii)).

Second and further rounds of polymerization, if they are carried out, will generally each involve a cycle of the steps of the preparation of single stranded molecules, hybridization and polymerization. In the methods of the present invention, these steps can take various forms depending on the design of the protocol and examples are described below.

The first step in the cycle is thus the treatment of the molecules present in order to obtain single stranded molecules. Such a step could take the form of a denaturation step, for example a heat denaturation step or an alternative denaturation method as described elsewhere herein such as the use of dimethyl sulfoxide, formamide, high pH or DNA helicase, etc. Alternatively, if the molecules resulting from the first round of polymerization comprised strands which were RNA molecules (for example had one strand which was DNA and one strand which was RNA, for example by using a DNA polymerase with reverse transcriptase activity as described elsewhere herein), then the formation of single strands could be carried out by adding an RNase H enzyme (or indeed any other enzyme which has the effect of degrading RNA). Such an RNAse degradation step is exemplified and illustrated in Designs 4 and 7 for reference.

The second step in the cycle is a further hybridization/annealing step, i.e. a hybridization/annealing step which is distinct from step (i) of the methods described herein. Similarly to step (i), such a further hybridization step still takes the form of a conventional hybridization step involving the interaction of nucleic acid molecules by virtue of the formation of hydrogen bonds between complementary (or sufficiently complementary) nucleic acid sequences as described above. Depending on the embodiment of the invention, such a further hybridization step may involve the use of additional dsPrimers of the invention, the addition of conventional single stranded primers, or may, in the most simple methods, comprise further hybridization of the molecules already present in the reaction mixture without requiring the addition of further primers. Again a skilled person could readily devise appropriate reagents and conditions (e.g. temperature) to carry out such a further annealing step and ensure that the desired molecules (or parts thereof) hybridize with each other depending on the molecular entities present in the mixture. Examples of each of these options are described below using the design protocols to illustrate techniques which could be used.

Designs 4 and 5 give exemplary protocols in which the second round of polymerization and the further hybridization step involves the use of an additional dsPrimer of the invention. Designs 4 and 5 happen to use a second dsPrimer which is different to the dsPrimer used in the hybridization step (i). However this is not compulsory and it would equally be possible to add an additional dsPrimer in this second round of polymerization which was the same as that used in the first round of polymerization. In some embodiments the dsPrimers used can be dsPromoters and/or dsTerminators, which represents a highly useful embodiment of the invention (see for example Design 5).

Design 3 gives an exemplary protocol in which the second round of polymerization and the further hybridization step involves the use of conventional primers (here conventional sense and anti-sense primers) which are added in the second round of polymerization. These single stranded primers are designed to bind to appropriate ends of the nucleic acid molecules which are present in the reaction mixture and in which one of the strands of the dsPrimer has been incorporated. Appropriate single stranded primers are readily designed given that the sequences of the strands of the dsPrimer are known. In some embodiments (e.g. the embodiments using a simple dsPrimer of the invention), these single stranded primers are designed to bind to the 3' ends of the nucleic acid molecules which are present in the reaction mixture and in which the lower strand of the dsPrimer has been incorporated. Appropriate single stranded primers are readily designed given that the sequence of the lower strand of the dsPrimer is known. A further advantage with protocols such as those shown in Design 3 (e.g. protocols where a single type of simple dsPrimer is used) is that a single single stranded primer can be used as the sequences are the same. Such embodiments, where a single type of dsPrimer is used are thus preferred as they, for example, allow 3' end targeting of both strands of a dsDNA molecule at the same time (after denaturation) and only require a single single stranded primer, e.g. a single conventional single stranded primer, for amplification. Kits and compositions with only a single type of dsPrimer and optionally a single type of single stranded primer are preferred. A "single type" of dsPrimer as used herein is generally a reference to the same double stranded region being present. Thus, "single type" of dsPrimer as used herein with embodiments comprising degenerate or universal 3' overhang regions, generally means that the double stranded region of the primer is the same in all the dsPrimers but the single stranded 3' overhang region may vary in sequence, e.g. to provide a full set of combinations of the degenerate bases as described elsewhere herein.

In embodiments where chPrimers are used (which contain two different primer sites) then two different types of single stranded primer will need to be used if a step using conventional single stranded primers forms part of the second or subsequent rounds of polymerization (see for example Designs Ch1 and Ch2).

Design 2 (and Design Ch3, Ch4, Ch5 and Ch6) give exemplary protocols where no additional primers are required in the second round of polymerization and the further hybridization step. In such protocols the further hybridization step involves the hybridization of molecules which are already present in the reaction mixture as a result of the first round of polymerization, and, for example, appropriate conditions, e.g. annealing temperatures, can be selected to ensure that the correct molecules (or parts thereof) hybridize to each other.

Thus, at its most general, this second step in the cycle may or may not involve the addition of further primers. If it does involve the addition of further primers, then these may be conventional single stranded primers designed to bind to a strand of the dsPrimers of the invention which have been incorporated into the ends of the nucleic acid molecules of the reaction mixture by virtue of the first round of polymerization, or may involve the addition of further dsPrimers of the invention.

The third step in the cycle which forms the second (or subsequent) round of polymerization involves the use of a DNA polymerase enzyme with 5' to 3' polymerase activity which then acts on any appropriate 3' ends of nucleic acid molecules present in the reaction mixture and as a result fills in any gaps which are present.

As is clear from the exemplary design protocols described herein, this use of a DNA polymerase enzyme to fill in gaps which are present can take several forms depending on the design of the method. Depending on the molecules which are present in the reaction mixture, then more that one of these forms may be involved in any particular method.

Thus, this DNA polymerase step can take the form of the completion of 3' ends of various strands, for example by production of the sequence corresponding to the lower strand of the dsPrimer using the upper strand sequence as a template (see for example Design 2, step 05, and some of the reactions of Design 3, see for example step 05 shown on the left hand side of the schematic). See also designs Ch3 to Ch6 using chPrimers of the invention in which in the second round of polymerization a DNA polymerase is used to complete the second strand from the 3' end of the nucleic acid molecules present using the other strand as a template (see step 06).

A similar step is carried out in Design Ch1 and Design Ch2 using chPrimers of the invention. However here, in order to fully complete the 3' ends of the molecules the use of a DNA polymerase which also has strand displacement or nick translation activity is preferred.

Alternatively, in some protocols, the DNA polymerase is used to carry out a primer extension reaction from newly added conventional single stranded primers (for example Design 3, step 05) or from a newly added dsPrimer of the invention (see for example Designs 4, 5 and 7).

In methods where another dsPrimer of the invention is used, then such methods generally involve the additional step of producing the complementary strand of the dsPrimer, i.e. replacing the lower strand of the dsPrimer, as described above. As also described above, a preferred technique of carrying out this step will be to use a DNA polymerase with strand displacement activity (as depicted in Designs 4, 5 and 7) or nick translation activity, although equally the other techniques as described above could be used. Again as described above, if a DNA polymerase with strand displacement or nick translation activity is the chosen method, then this activity may be provided by way of an additional DNA polymerase or by way of a single type of DNA polymerase which has both 5' to 3' polymerase activity and strand displacement or nick translation activity, as appropriate.

Design 2 and Designs 4 to 7 are examples where two rounds of polymerization are used in order to form an amplification template. Design 3 is an example where three rounds of polymerization are used to arrive at the amplification template.

Thus, it can be seen that in methods of the invention the dsPrimers can act before the amplification step and add primer regions for amplification primers to target. In other words, the method steps (i) and (ii) can be used to form double stranded template molecules for amplification via another (amplification) primer by the addition of appropriate primer binding sites to the ends of the template molecules.

Once the amplification template has been produced by carrying out the hybridizing step (i) and one or more rounds of polymerization in accordance with step (ii) of the method, at least one amplification step (iii) is then carried out on the amplification template in order to generate an amplified nucleic acid product (also referred to herein as an amplification product). Thus, it can be seen that the steps of the method are preferably carried out in the order presented. In some embodiments steps (ii) and (iii) can be performed in a single process, for example in embodiments where the same primers are used for both steps (see e.g. Design Ch1). The amplification step may also be referred to herein as an amplification reaction.

The number of amplification steps to be carried out can be readily determined by a person skilled in the art and will depend on the amount of nucleic acid product it is desired to generate. It is also well within the normal skill of such a person skilled in the art to provide the necessary reagents and conditions in order to carry out the amplification steps. Indeed, some exemplary reagents and conditions are described in the experimental Examples section.

The amplification step(s) performed on the amplification template to form an amplified nucleic acid product can take any appropriate form depending on the nature of the amplification template which has been produced. Thus, the amplification step can take the form of conventional or standard PCR using forward and reverse single stranded primers which are designed to bind to the sequences (primer sites), originally present in the strands of the dsPrimers used in the methods and now incorporated at the ends of the nucleic acid strands present (see for example and illustration Designs 2 to 4 and Designs 9 and 10 where sequences originally present in the lower strand of the dsPrimers have been incorporated at the 3' ends of both the nucleic acid strands present).

As has been mentioned elsewhere herein the sequence of the double stranded region of the dsPrimer(s) used is known, so the sequences of the forward and reverse primers can be designed accordingly. The PCR amplification is carried out in accordance with standard protocols as will be well known to a person skilled in the art, any of which should be appropriate for use. Such protocols generally involve the steps of denaturation, hybridization/annealing and polymerization, said steps being repeated as many times as is necessary to achieve the desired level of amplification. As well as standard PCR, any variant PCR method can be used, for example Hot start PCR, Touchdown PCR or Step-down PCR. Indeed, methods involving both these types of PCR are described in the experimental Examples section.

In addition, the methods are compatible with the use of extension primers, for example, it would be possible to use two extension primers instead of using a single primer in the standard PCR step of Design 2 and Design 3. The use of two different extension primers (e.g. termed E1 and E2) would give rise to 3 different amplification products (i.e. the template with E1 at both ends, the template with E2 at both ends and the template with E1 at one end and E2 at the other end), which could then, if desired, be separated by size separation based on the difference in length between E1 and E2.

In some embodiments of the invention, advantageously the same single stranded primer can be used for both forward and reverse reactions (i.e. the method only requires a single primer, see for example Designs 2, 3, 9 and 10).

Equally however the primer sites can be designed to be different, in which case two different primers are used (see for example Design 4).

In embodiments of invention where chPrimers are used, which as described elsewhere herein have two adjacent but different primer sites (P1 and P2), then appropriate combinations of single stranded primers can be chosen for the PCR amplification step in order to generate an amplification product which for example has a different primer site at each end (see for example Design Ch1 in which the amplification product shown at the bottom of the figure has P1 at one end and P2 at the other) or both different primer sequences at each end (see for example Design Ch2 in which the amplification product shown at the bottom of the figure has P1 and P2 at each end).

In embodiments of the invention where the amplified nucleic acid product is to take the form of amplified RNA molecules, then the amplification step is generally carried out using an RNA polymerase. In such methods the RNA polymerase needs to bind to a specific (conserved) sequence (called a "promoter" sequence) in the amplification template and be able to synthesise an RNA molecule. In such embodiments, amplified nucleic acid products can be generated continuously as new RNA polymerase molecules can bind to the appropriate sequences in the amplification template and synthesise nucleic acid products one after the other providing that sufficient reagents and the appropriate conditions are provided.

Any appropriate RNA polymerase enzyme can be used in the methods of the present invention providing the appropriate binding site (promoter site) has been incorporated in the dsPrimers of the invention which are used. Thus, in exemplary embodiments the dsPrimer is a dsPromoter which an appropriate RNA polymerase (DNA dependent RNA polymerase) can bind or recognise and thereby initiate RNA synthesis. As described elsewhere herein the T7 promoter is particularly preferred and in such embodiments a double stranded DNA dependent RNA polymerase (T7 RNA polymerase) binds to the T7 promoter region and RNA is transcribed.

T7 RNA polymerase starts transcription at the final G in the promoter sequence (5'-taatacgactcactataG-3'). The polymerase then transcribes using the opposite strand as a template for 5' to 3' transcription. The first base in the transcript will therefore be a G. T7 RNA Polymerase is one of a group of very active phage polymerases, that use a short double-stranded promoter sequence to initiate transcription at a specific base in the template, and then transcribe single or double-stranded DNA with good fidelity until the end of the DNA bottom strand is reached. T7 polymerase strongly prefers to start with a G (GG is better still), but otherwise will transcribe virtually any sequence. In preferred embodiments of the invention, to increase the yield of the T7 RNA polymerase, 3 G residues are added as GGG to the end of the minimal promoter sequence (taatacgactcactataGGG) in which case a GGG sequence will be added to the 5' end of the amplified product (see for example Design 1 and Design 5).

Any appropriate DNA dependent RNA polymerases can be used providing the appropriate promoter sequence is present in the dsPrimers of the invention. Another specific example is the SP6 RNA polymerase.

In certain embodiments, a dsTerminator sequence can be incorporated at the opposite end of the amplification template from the dsPrimer sequence or the dsPromoter sequence. If both a dsTerminator and a dsPromoter are used then in preferred embodiment the terminator sequence is selected to match the promoter sequence, i.e. they are selected to both be recognised by the same enzyme (e.g. a polymerase enzyme). Thus, in preferred embodiments a T7 terminator sequence can be incorporated at the opposite end of the amplification template from a T7 promoter sequence, in which case the appropriate RNA polymerase will recognise and initiate transcription at the T7 promoter sequence and terminate the transcription at the T7 terminator sequence thereby meaning that molecules will be produced which resemble native transcripts (see for example Design 5 and Design 7). Other appropriate promoter/terminator pairs can equally be used, e.g. the SP6 promoter and terminator sequences which are recognized by the SP6 RNA polymerase.

Alternatively, a dsPrimer sequence can be incorporated at the opposite end from the dsPromoter (e.g. the T7 promoter) in which case more elongated amplification products are produced as the transcription is not terminated before the end of the amplification template (see for example Design 6). The 3' end elongated amplification products, such as the type of molecules produced in Design 6, can be particularly useful for any application that requires modified (or elongated) 3' ends. For example such elongated 3' ends can be designed to incorporate or insert adaptor sequences and/or nucleic acid labels or tags, for example in the form of a barcode or other identifier sequence. Such elongated 3' ends can then be used for example in next generation sequencing reactions, third generation sequencing, etc. 3' end elongated amplification products can also conveniently be immobilized to any surface (such as nanoparticles, microarrays, etc), for example via hybridization of the inserted sequence with a pre-immobilized complementary sequence.

Other amplification reactions of the invention can involve the use of a single stranded RNA primer which can hybridize to a sequence corresponding to one of the strands of the primer site present in the original dsPrimer of the invention which has become incorporated into the 3' end of one of the strands of the amplification template. In embodiments of the invention where the simple dsPrimers of the invention are used then such amplification reactions involve the use of a single stranded RNA primer which can hybridise to the sequence corresponding to the lower strand of the original dsPrimer, which has become incorporated into the 3' end of one of the strands of the amplification template.

Such methods involving the single stranded RNA primer for amplification can be used for continuous amplification. Such methods require the dsPrimer of the invention to be a hybrid molecule with the upper strand composed of RNA and the lower strand composed of DNA (or in the case of a chPrimer of the invention then one of the primer sites, P1 or P2, is a hybrid primer site with one strand of said site composed of RNA and the other strand of DNA).

These hybrid double stranded primers are then incorporated into the amplification templates by way of steps (i) and (ii) of the methods of the invention. A step in which DNA hybridized RNA molecules are degraded, for example using a polypeptide comprising RNase H activity, is then carried out in order to remove the RNA strand of the dsPrimer from the amplification template thereby allowing a single stranded RNA primer to be hybridized in its place. After hybridization, this RNA primer has a free 3' end on which a DNA polymerase comprising both 5' to 3' polymerization activity and strand displacement activity can act in order to extend the sequence of the RNA primer to form another copy (of the amplification template whilst at the same time displacing the strand which was previously joined to the RNA part of the dsPrimer away from the newly forming amplification template. This step thus has the effect of generating an amplified nucleic acid product (these are the displaced molecules) and regenerating the amplification template to be subjected to a further round of amplification using RNA degradation and the hybridization of a single stranded RNA primer, i.e. these steps can form a continuous cycle. Exemplary designs in which such an amplification step is used are shown in Design 8 (see steps 3) and also in Design Ch5 and Ch6 (see steps 7).

Such methods involving amplification using an RNA primer and strand displacement can readily be adapted to amplify sense or antisense DNAfrom sense or antisense DNA or RNA templates.

The methods of the invention can be varied to be used on different types of template and to produce different types of amplification product. For, example the methods can be used to amplify antisense DNA from sense DNA templates, or sense or antisense DNA from RNA templates. The amplification products may for example be DNA or RNA and may be single or double stranded.

Once generated, the amplification products may be used in any appropriate subsequent methods in which an amplified product is desirable or advantageous. Some uses are described elsewhere herein. An exemplary use where a double stranded PCR product is used as a template for *in vitro* transcription (and hence, if desired, subjected to a further round of amplification) is described in the experimental Examples section.

The next section describes some optional steps and preferred embodiments of the methods of the invention.

### dsPrimer Silencing

The term "dsPrimer silencing" as used herein refers to a process to stop the dsPrimer activity, i.e. to remove the ability of the dsPrimer to act as a primer, by removing the 3' single stranded overhang region. Any appropriate method of removing the 3' single stranded overhang region can be used, however, conveniently, this is done by using a DNA polymerase with both a DNA dependent 5' to 3' DNA polymerase activity and a 3' to 5' exonuclease activity. The principal of dsPrimer silencing is shown in Figure 1 and the step can be used to prevent undesired hybridization of dsPrimer molecules, as once the 3' single stranded overhang region is removed then no hybridization can occur. Thus, such optional steps of dsPrimer silencing can be used after step (i) of hybridization, e.g. after hybridization step (i) or after the first primer extension reaction of step (ii) or after the replacement of the lower strand of the dsPrimer in step (ii) or after the first round of polymerization in step (ii). Equally said silencing step can be used at similar points after any other subsequent step of hybridization of the dsPrimers of the invention. When used at these points in the methods, this step has the effect of inactivating any primers which have not hybridized to the relevant nucleic acid molecules. In other words the step of silencing can be used to stop the activity of unhybridized dsPrimers. As shown in Figure 1, the step of dsPrimer silencing can be used with all types of dsPrimer (including chPrimers, see Figure 29) regardless of whether they are comprised of DNA or RNA strands or both.

An equivalent silencing procedure can be used with the dsTerminators of the invention and is termed "dsTerminator silencing".

### dsPromoter Silencing

"dsPromoter silencing" as used herein works on the same principle as the dsPrimer silencing described above. However, this step is carried out on dsPromoters of the invention, i.e. in embodiments where the dsPrimer is a dsPromoter. The dsPromoter silencing is also carried out by removing the 3' single stranded overhang region and is again conveniently carried out with a DNA polymerase which additionally has 3' to 5' exonuclease activity. The principal of dsPrimer silencing is shown in Figure 2. Again this step can be used to remove the hybridization activity of the dsPromoter structures which can help prevent undesired hybridization of the dsPromoter molecules. This optional step can be carried out at the same times as the dsPrimer silencing steps described above. As shown in Figure 2, dsPromoter silencing can be used with all types of dsPromoter regardless of whether they are comprised of DNA or RNA strands or both in terms of stopping the hybridization activity. As discussed elsewhere herein, as such promoters are only active when in a double stranded DNA form, in preferred embodiments of the invention, such promoters are used in a form which has a disturbed structure, e.g. has an RNA strand or part, e.g. is a DNA/RNA hybrid or is in an RNA/RNA form, and is therefore inactive. The protocols are designed to convert this inactive form to a DNA/DNA form when RNA polymerase activity is desired.

### RNase H Degradation

In embodiments of the invention where a double stranded nucleic acid molecule is generated in which one of the strands (or part of the strands) is RNA and the other strand (or part of the strand) is DNA, e.g. a DNA/RNA hybrid molecule, then an enzyme which degrades RNA, e.g. an RNase H enzyme (a polypeptide comprising RNase H activity), can optionally be used to remove the RNA strands from the reaction mixture, for example to arrive at single stranded DNA molecules. Such steps are exemplified in Design 4 and Design 7, wherein the original nucleic acid template molecule is RNA and the dsPrimer used has a lower strand which is RNA. The step of RNase H degradation is used after the hybridizing step (i) and the first primer extension reaction in order to remove the RNA strand and give rise to a preparation of single stranded DNA molecules. In Design 7 the initial dsPrimer which is hybridized is a T7 terminator sequence whereas in Design 4 a basic dsPrimer is used, which shows how this step of RNA degradation is versatile and can be used with many designs of the invention.

### Use of a Lock Primer

In some embodiments of the invention, the methods comprise a step in which a lock primer is used. The term "lock primer" is used herein to describe a single stranded or double stranded nucleic acid molecule (e.g. a DNA or RNA or DNA/RNA chimeric single or double stranded primer) which is incapable of being subjected to primer extension because it cannot be used as a substrate by a DNA polymerase, e.g. because the 3' end of the DNA/RNA molecule/primer has been modified so that it does not have a 3' OH group and chain elongation is thereby inhibited. The incorporation of chain terminators such as ddNTP or 3'dNTP residues provides a convenient way of making such DNA or RNA or DNA/RNA molecules with 3' modified ends. When such lock primers bind to nucleic acid molecules in the reaction mixture (e.g. bind to the sense or the antisense strands in the reaction mixture), as they cannot be subjected to chain elongation, they block the reaction, i.e. they block the further amplification of the molecule to which they bind.

Lock primers when used in the methods of the present invention are generally designed to hybridize to the 3' ends of nucleic acid molecules or unbound dsPrimers or chPrimers the amplification of which is not desired. In other words lock primers are used to block unwanted polymerization reactions. Such lock primers are conveniently designed to bind to a sequence (e.g. to be complementary to a sequence or to be complementary to only a region) corresponding to the lower strands of the dsPrimer (or equivalent strand of a chPrimer) of the invention which have been incorporated at the 3' end of a proportion of the nucleic acid molecules which are present in a reaction mixture (see for example Design 5, 6 and Design 13). Thus, if in a particular method it is desired that the amplification of the sense strand is blocked and the amplification of the anti-sense strand proceeds, then a lock primer can be designed which binds to and "locks" the 3' end of the sense strands (see Design 5 and 13 where the sense strand is DNA and Design 6 where the sense strand is RNA). After the lock primer has bound then only the anti-sense molecule is available to be amplified. Equally such lock primers can be used to block amplification of the antisense strand. In such embodiments lock primers can be designed so that the lock primer binds to and locks the 3' end of the antisense strands instead, by following the same design parameters and used to block amplification of the antisense strand or unbound dsPrimer and chPrimers. Essentially, designing lock primers for blocking sense or antisense amplification is the same as designing forward or reverse primers for a a standard PCR amplification.

Such lock primers can either be in a single stranded (see for example Designs 5, 6 and 13) or in a double stranded form (see for example Designs 14, 16 and 17). When in a double stranded form, in order to bind to or target the 3' end of the nucleic acid strand or strands it is desired to block, then the lock primers can conveniently use the same structure as the dsPrimers of the invention in which the single stranded 3' overhang region is designed to hybridize to the sequence at the 3' end of the strand it is desired to block and, of course, the 3' end of the single stranded overhang region is modified so that it does not have a 3'OH group. Thus, although, for example, Design 5 illustrates the use of a single stranded lock primer, equally such a double stranded dsLockPrimer may also be used (providing the 3' single stranded overhang region of said primer is designed to contain a sequence which was complementary to the sequence of the terminal, e.g. Terminator sequence present at the 3' end of the sense DNA strand).

A particularly preferred form of the above described dsLockPrimer and its use is, for example, the Lock Primer T as shown in Designs 14, 16 and 17. Such lock primers are specifically designed to target and block the amplification of nucleic acid molecules which have a poly(A) tail at the 3' end and thus, in such lock primers, the single stranded 3' overhang region is composed of a series of (multiple) T residues (e.g. 6 T residues) and (as for the dsPrimers described herein) this single stranded region is attached to a double stranded nucleic acid (DNA and/or RNA) region. These primers function as lock primers since the last T base of the 3' overhang region does not have a 3'OH group and thus chain elongation cannot occur.

Another usage of the lock primer concept is to inhibit the hybridization of dsPrimers to the 3'-end of nucleic acid molecules. For example, hybridization of a Lock Primer (in either a single stranded or a double stranded form as described above) to the 3'-end of a nucleic acid strand in the second (or subsequent) round of polymerization, e.g. after the denaturation step (formation of single stranded molecules) which takes place at the beginning of the second (or subsequent) round of polymerization, does not just inhibit chain elongation, it also inhibits hybridization of a second (or subsequent) set of dsPrimers (or other primers) to this strand (see e.g. Design 5, 6 and Design 13). In such embodments Lock Primers are added first to the reaction mixture and then for example appropriate secondary, alternative, dsPrimers are added to the solution.

***Use of Two Different dsPrimers of the Invention***

In preferred embodiments of the invention, two or more different dsPrimers of the invention are used in separate rounds of polymerization, for example in order to incorporate two different primers at each end of the nucleic acid molecules. In this way two different regions (with different sequences) are added to the 3' and 5' ends of the initial nucleic acid template. Illustrative and exemplary protocols showing this technique are shown schematically in Designs 4, 5 and 7. "Different" dsPrimers is thus generally used to mean dsPrimers with different sequences or regions in the double stranded region of said primers.

The types of dsPrimer to use, in addition to the sequences and structures of said dsPrimers can readily be selected by a skilled person. For example, at its most simple, two different dsPrimers (for example with a different primer sequence contained within the double stranded region of each of the two different dsPrimers) can be used in such embodiments (see for example dsPrimer 01 and dsPrimer 02 as shown in Design 4). In alternative embodiments, a dsPrimer can be combined with a dsPromoter or dsTerminator as described elsewhere herein. Alternatively, a dsPromoter can be combined with a dsTerminator.

In such embodiments, the two different dsPrimers of the invention are added in the appropriate order depending on the type of amplified nucleic acid product being produced and the type of molecule which is being used as the nucleic acid template in step (i) of the methods. Thus, for example Design 4 involves the use of two different dsPrimers of the invention in separate rounds of polymerization in order to incorporate different primers at each end of the amplification template molecule which can then be used for standard PCR amplification.

However, in Design 5, one of the dsPrimers is a dsTerminator (illustrated in Design 5 as a T7 terminator, however any appropriate terminator site for an RNA polymerase could be used), and the second dsPrimer used in the second round of polymerization is a dsPromoter (in design 5 this is illustrated as a T7 promoter, however this could be any other DNA dependant RNA polymerase promoter). In Design 5, sense DNA is the original nucleic acid template molecule and sense RNA is the desired amplified product. The dsTerminator is added in the first round of polymerization and the dsPromoter is added in the second round. As can be seen from the schematic of Design 5, such protocols involving the use of dsPromoters and dsTerminators of the invention are a particularly good way to achieve amplification of native forms of the molecules as the relevant RNA polymerase will start at its natural promoter sequence and finish transcribing RNA at its natural terminator sequence, making its natural modifications at both ends of the molecule.

Such protocols can readily be modified however. For example, if rather than obtaining a natural form of the 3' end of the amplified product as shown in Design 5, it is preferred that a more extended 3' end is obtained, then a dsPrimer of the invention can be combined with a ds (e.g. T7) promoter of the invention, for example as illustrated in Design 6. It can be seen that in the amplified products of Design 6 (bottom molecule in the Figure), the 5' ends of the amplified products are in the native form produced from the T7 promoter (with a GGG sequence) but the 3' ends are extended as the dsPrimer used did not contain a terminator sequence. However, such amplified products now contain a primer sequence (or some kind of other useful sequence which was present in the double stranded region of the dsPrimer used) at the 3' end which can be useful for further manipulation, for example for use like an adaptor in for example next generation sequencing.

Thus, the 3' end elongated amplification products, such as the type of molecules produced in Design 6, can be particularly useful for any application that requires modified (or elongated) 3' ends. For example such elongated 3' ends can be designed to incorporate or insert adaptor sequences, RNA or DNA affinity sequences/tags (which for example allow the isolation of RNA or DNA molecules, respectively, by affinity purification), and/or nucleic acid labels or tags, for example in the form of a barcode sequence. Such elongated 3' ends can then be used for example in next generation sequencing reactions, third generation sequencing, affinity purification, etc. 3' end elongated amplification products can also conveniently be immobilized to any surface (such as nanoparticles, microarrays, etc), for example via hybridization of the inserted sequence with a pre-immobilized complementary sequence.

Design 5 and Design 6 also illustrate that such embodiments where two different dsPrimers of the invention are used, can, if desirable and appropriate, be combined with the use of a lock primer (e.g. a single or double stranded lock primer as described above).

Design 7 shows a yet further way in which two different dsPrimers of the invention (again a dsTerminator and a dsPromoter) can be used in separate rounds of polymerization in order to produce amplified RNA molecules with natural ends. Design 7 will be discussed in more detail below as it also involves the use of a hybrid RNA/DNA dsPrimer which is another preferred embodiment of the invention.

In other embodiments of the invention, more than two different dsPrimers of the invention are used, depending on the method concerned. For example, for standard amplification techniques three different dsPrimers could readily be used. On the other hand, for methods in which the dsPrimers are used to incorporate nucleic acid labels or tags (e.g. in the form of barcode sequences) then the number of different types of dsPrimers required would depend on the number of different types of tags to be used. For example, for DNA barcoding applications for next generation sequencing systems, up to 64 different dsPrimers would conveniently be used.

### Use of DNA/RNA hybrid dsPromoter - Upper strand DNA Lower strand RNA

As described above, yet further embodiments of the invention involve the use of hybrid RNA/DNA dsPrimers. In some embodiments the upper strand of the double stranded region of the dsPrimer is DNA and the lower strand is RNA. When such dsPrimers are hybridized to nucleic acid molecules in the reaction mixture, if these molecules are RNA strands then this will open up the possibility of using an RNA degradation step to remove the RNA strand and end up with a preparation of single stranded molecules. This is indeed shown in Design 7 (see the RNase H step 3). In Design 7, again a dsT7 terminator sequence is used in the first round of polymerization and a dsT7 promoter (which is also a hybrid molecule in which the lower strand is RNA and the upper strand is DNA) is used in the second round of polymerization. In the case of the dsT7 terminator used in the first round of polymerization, as the original nucleic acid template molecule is RNA (in this case sense RNA), once hybridization step (i) of the method and the first primer extension reaction has taken place, this has the effect that the whole of one of the strands of the double stranded molecule produced is RNA. This then allows the use of an RNA degradation enzyme such as RNase H in order to remove this whole strand and give rise to a single stranded DNA template to be used in the second round of polymerization.

A hybrid dsT7 Promoter is used in the second round of polymerization and, as described above, this makes use of the property of the T7 promoter that it is only active/functional when it is in double stranded DNA form. In other words, the T7 RNA polymerase cannot bind to the T7 promoter and produce any RNA molecules (e.g. random or undesired RNA molecules) while the double strand structure of the T7 promoter contains at least one RNA strand. Thus, as can be seen from Design 7, as the lower strand of the dsPromoter is RNA, an RNA polymerase can only bind to the ds T7Promoter once hybridization and replacement of the lower stand of the dsPromoter have been carried out. Here the lower RNA part of the dsPromoter is replaced with DNA in the polymerization step after the hybridization of the dsPromoter to the template has taken place in the second round of polymerization. Thereby, an active double stranded DNA dsPromoter is formed such that T7 RNA polymerase can bind and initiate RNA production. In this way, no amplification will occur until the lower part of the dsPrimer (the dsT7Promoter) is replaced by DNA. This illustrates a yet further preferred use of the hybrid dsPromoters of the invention, a use which is also illustrated in Design 5 where a hybrid dsT7Promoter is used in the second round of polymerization.

### Use of DNA/RNA hybrid dsPrimer - Upper Strand RNA and Lower Strand DNA

Design 8 illustrates another useful embodiment of the invention using a hybrid dsPrimer. In this case, the upper strand (of the double stranded region) is RNA and the lower strand is DNA. When such dsPrimers are used in the methods of the invention, because the upper strand is RNA, a DNA polymerase with reverse transcriptase activity and strand displacement activity will need to be used to replace the lower strand of the dsPrimer once it has hybridized in step (i). The advantage and usefulness of such dsPrimers is however that once the step of reverse transcription has been carried out, both the upper RNA strand of the dsPrimer and the lower DNA strand of the dsPrimer become covalently incorporated into the double stranded molecule produced at the end of the first round of polymerization. This upper RNA strand of the original dsPrimer can then be selectively digested away with an enzyme which degrades RNA (for example RNase H), thereby leaving a gap in the amplification template to which a single stranded RNA primer can bind and a DNA polymerase used to carry out primer extension from said RNA single stranded primer as described elsewhere herein, which will result in a further copy of the amplification template being produced (with the same RNA segment at the 5' end of the upper strand), whilst at the same time displacing the amplification product nucleic acid. This reaction or amplification step (iii) can be cycled for as long as required, i.e. in a continuous cycle to obtain the desired amount of the amplification product.

### Isothermal Reactions

A further highly advantageous feature of some of the methods of the invention is that they can be designed to be carried out in an isothermal manner (under isothermal conditions), e.g. are amplification reactions which can take place at a constant temperature rather than for example cycling through thermal denaturation and annealing/extension steps as carried out in conventional PCR amplification. The ability to carry out isothermal reactions is highly advantageous. Design 8 is a illustration of such a method in which no thermal denaturation is involved and all the steps of the method are enzymatic. Design Ch5 and Ch6 are further examples.

Isothermal amplification is highly advantageous in that it does not require a thermal cycler, which greatly simplifies and enhances techniques in which it is used, for example point-of-care diagnosis. In most cases, it is cost effective, easy-to-use and more tolerant to inhibitory components from a crude sample compared with standard PCR (involving thermocycling), showing equivalent or higher sensitivity and reliability, for example in clinical diagnosis.

A further advantage of isothermal reactions is that in addition to DNA, other biomolecules (for example RNA and proteins) can be used as input signals. For example, mRNAs in a few homogeneous cells were successfully amplified with an isothermal method after converting into complementary DNA. Proteins have also been successfully assayed with immuno-detection followed by isothermal signal amplification.

Furthermore, Whole Genome Amplification (WGA) is achievable with a linear, isothermal amplification, owing to the large size of amplifiable genomic segments (>10 Kb) by *Bacillus subtilis* bacteriophage phi29 DNA polymerase. This enzyme has extremely high processivity (up to 70 Kb within 20 min), strong proofreading, strand-displacement activity and long a half-life. Usually, micrograms of amplified genomic segments can be obtained from nanograms of initial genomic DNA within several hours.

### Use of Modified Ends

Although it is an advantage that the methods of the invention do not require either the nucleic acid template molecules or the dsPrimers of the invention to have any end modifications (i.e. can be used in an unmodified form), it should be noted that the use of such end modifications is not incompatible with the methods of the invention. Thus, if desirable, in certain embodiments of the invention such end modifications can be used. An example of this would be if it was desired to use a ligation reaction, for example in order to replace the lower stand of the dsPrimer (or the equivalent strand in a chPrimer) in the first (or subsequent) round of polymerization as described elsewhere herein. In such a case, the 5' end of the lower strand/non-overhang strand of the dsPrimer (or the equivalent parts of the chPrimers of the invention), in other words a 5' end of the double stranded region of the dsPrimer/chPrimer which is at a complementary position to a 3' single stranded overhang region, would be phosphorylated as described above and a ligase enzyme could be used to fill the gap/nick between the 3' end of the template and the 5' phosphate on the dsPrimer (or chPrimer). In other specific embodiments no 5' phosphate is present at those positions in the dsPrimer or chPrimer.

Another example is illustrated in Designs 9 and 10 in which the template nucleic acid molecules have been phosphorylated at the 5' ends. Optionally in such protocols the 5' end of the lower strand of the dsPrimer (or the equivalent parts of the chPrimers of the invention), in other words a 5' end of the double stranded region of the dsPrimer/chPrimer which is at a complementary position to a 3' single stranded overhang region, can also be phosphorylated. A population of template molecules such as those shown in Designs 9 and 10 can take the form of a template which has been sheared, blunt ended and 5' end phosphorylated double stranded genomic DNA, which is an appropriate template for use in DNA sequencing devices.

The advantage of using such templates with 5' phosphorylated ends is that they allow steps in which a lambda exonuclease is used which will degrade 5' end phosphorylated DNA until it reaches a gap or a nick site. (Lambda exonuclease is unable to initiate DNA digestion at nicks or gaps). Thus, in such embodiments, the lambda exonuclease activity will remove DNA from the 5' phosphorylated end until it reaches the nick site as exemplified in Designs 9 and 10. This thus provides an alternative way of removing one of the strands (and forming a population of single stranded molecules) after which further denaturing and hybridization (annealing) steps can occur in order to allow the second round of polymerization to be carried out. The denaturing step will also deactivate the lambda exonuclease so that this will not have an effect in the subsequent rounds of polymerization and amplification.

As will be described elsewhere herein labels can also be incorporated at the ends of the dsPrimers and are thus another example of a dsPrimer with a modified end.

### Production of ds RNA as an Amplified Product

Advantageously the methods of the invention can be used to produce double stranded RNA as an amplified product, see for example the steps shown in Design 11 in which a hybrid dsT7Promoter of the invention is used with a double stranded DNA template (once it has been subjected to denaturation to form single stranded template molecules) in order to produce a double stranded RNA as an amplification product. Designs 12 and 13 illustrate yet further ways in which the methods of the invention can be used to produce a double stranded RNA molecule as an amplified product. These methods involve the use of a phi6 promoter (or any other RNA-dependent RNA polymerase initiation sequence) in order to produce multiple copies of a dsRNA molecule from amplified single stranded RNA molecules.

*phi6 Promoters/ initiation sequences*

As described elsewhere herein there is no restriction on the type of promoter which can be used in the methods of the invention by incorporation into the dsPrimers. Another promoter which can be particularly useful in the methods of the invention is the phi6 promoter. This sequence acts as an initiation sequence and, indeed, any other RNA-dependent RNA polymerase initiation sequence can be used in place of phi6. As shown in Designs 12 and 13, an RNA replicase (the phi6 Replicase, which is for example commercially available from Thermo Scientific - Finnzyme) can bind to the phi6 promoter (when in single stranded (or in double stranded, not shown) RNA form) and synthesise an RNA strand from the 3' end of the template. Thus, if the phi6 promoter has been incorporated into the 3' end of a single stranded RNA molecule using the methods of the invention, then the phi 6 replicase can be used to create a double stranded RNA molecule.

The phi6 promoters/initiators of the invention are preferably used in protocols in which a second dsPrimer of the invention is used, for example protocols in which a dsPromoter is used as a second dsPrimer of the invention (see for example the use of the dsT7Promoter as a second dsPrimer in Design 12). As with the dsT7Promoters, the ds phi6Promoters of the invention can be hybrid molecules, for example in which the lower strand is RNA, thereby allowing a step of RNA degradation (preferably RNase H degradation) to take place in order to remove one of the strands. Such methods can also be used with lock primers as described elsewhere herein in order to ensure that the correct strand is available for interaction with the dsPrimer of the invention in the second round of polymerization (see Design 13). It can thus be seen that the use of the dsphi6Promoter is fully compatible with other embodiments of the invention as described herein.

*Template Nucleic Acid is mRNA*

Conveniently, the methods of the invention can also be carried out using mRNA molecules as template nucleic acid molecules, for example the original nucleic acid template molecules can be mRNA molecules with a poly-A tail, which can for example be a naturally occurring poly-A tail or can be enzymatically added to a nucleic acid molecule using for example a terminal transferase enzyme or a poly(A) polymerase (or equally can be DNA molecules with an engineered poly-A tail at the 3' end for example a poly A tail added using a terminal transferase enzyme). Examples of such embodiments are illustrated in Designs 14-17.

In such embodiments, an oligo-dT primer can conveniently be used with a DNA polymerase to produce a second (complementary) strand which contains the oligo-dT at the 5' end. After production of the second (complementary) strand with oligo-dT at the 5' end, then dsPrimers of the invention can be used to generate an amplified RNA molecule with a poly-A stretch at the 3' end of the RNA. Preferred oligo-dT primers are shown in Designs 14 to 17 and contain multiple thymine bases (e.g. 6 bases), together with at least one "not thymine" base (e.g. A, C or G) at the 3' end (e.g. VTTTTTT). Optionally said oligo-dT primer also contains a primer region at the 5' end, which, if present, will also be present in the amplified product (see Design 14 and Design 17).

As described above, these oligo-dT methods can be combined with other embodiments and preferred steps of the invention. Thus, again, a preferred use is with a dsPromoter of the invention (for example the dsT7Promoter), see Designs 14 to 17. In addition, such methods are also compatible and useful in combination with the use of lock primers, in particular the double stranded Lock Primer T as discussed above (see for example Designs 14, 16 and 17). Thus, if the method of creating the oligo-dT strand with the oligo-dT at the 5' end means that there are nucleic acid molecules with poly-A resides on the 3' ends which are not desired to be amplified, then it can be advantageous to use a lock primer which comprises T residues. To ensure that the lock primer which comprises T residues binds at the 3' end of the poly-A tail rather than binding anywhere in the poly-A tail and leaving the 3' end free, it is particularly preferred to use a ds Lock Primer T as described above for this step which will strictly target the 3' ends.

**Solid Phase Based dsPrimers and related Methods**

In preferred embodiments, the dsPrimers of the invention can be attached or immobilized or conjugated to a solid phase or solid support. Such dsPrimers immobilized to a solid phase can be used in any of the methods of the invention, e.g. in any methods employing PCR or other nucleic acid amplification and manipulation techniques (e.g. in-vitro translation, isothermal amplification, reverse transcription reactions, in-vitro diagnostics, sequencing applications, nucleic acid molecule or library construction for sequencing, SELEX etc). Methods of attaching or immobilizing nucleotide sequences to solid supports are known in the art and any of these may be used to immobilize the dsPrimers of the invention, for example amine (-NH2), thiol (-SH2), aldehyde (-COOH) coupling chemistries or avidin-biotin interactions. Preferably the immobilization is covalent.

The attachment or immobilization might take place at any convenient time point during the methods of the invention but conveniently takes place before the hybridization step (i).

Any appropriate solid phase may be used for such attachment, for example beads or other particles, plates, filters, columns, column matrices, membranes, or wires. Particularly preferred solid supports are particles, in particular nanoparticles. Such solid phases and in particular the particles or nanoparticles are preferably magnetic or paramagnetic, although any type of particle or nanoparticle may be used (e.g. semi conducting particles or nanoparticles, or beads or particles tagged with affinity molecules such as streptavidin or biotin).

Magnetic or paramagnetic nanoparticles are a class of nanoparticles which can be manipulated using a magnetic field. Such particles commonly consist of magnetic elements such as iron, nickel, cobalt and their chemical compounds. Nanoparticle-conjugated dsPrimers can conveniently be constructed by immobilization of appropriately modified 5' ends of upper strand or appropriately modified 3' ends of lower strand of dsPrimer structures to the reactive surface of magnetic nanoparticles by using any suitable immobilization techniques such as amine (-NH2), thiol (-SH2), aldehyde (-COOH) coupling chemistries or avidin-biotin interactions (see Design NP1).

Thus, the dsPrimers of the invention are generally attached to the solid support through either the 5' end of the upper strand or the 3' end of the lower strand. Appropriate modifications of the 5' or 3' ends may be required. As in the other embodiments of the invention, the double stranded region of said dsPrimer can include any useful sequence as detailed elsewhere herein. However, preferably a primer binding site, adaptor sequence restriction site, promoter or terminator region, transcription initiation site, restriction site or 5'-end or 3'-end labels for non-immobilized strands etc will be present.

The dsPrimers of the invention can conveniently be immobilized in double stranded form via the 5' end of the upper strand or the 3' end of the lower strand (as shown schematically in Design NP1A, where the dsPrimers are immobilized through the 3' end of the lower strand). Alternatively, the 5' end of the upper strand or the 3' end of the lower strand of a dsPrimer in single stranded form can be immobilized alone in a first step (see for example Design NP1B) after which the complementary strand can be hybridized to the immobilized strand in order to form the dsPrimer of the invention (in other words the dsPrimer can be formed in situ on the solid support by hybridization).

Thus, in preferred embodiments, dsPrimers can be immobilized to the reactive surface of the magnetic nanoparticle in double-structured form via the modified 5' end of the upper strand or the modified 3' end of the lower strand (see Design NP1A). Alternatively, the single-stranded 5' end of the upper strand or the single-stranded 3' end of the lower strand of a dsPrimer structure can be immobilized in a standalone manner to the reactive surface of the magnetic nanoparticle and the complementary strand hybridized after this immobilization step (see Design NP1B).

The advantages of immobilizing the dsPrimers of the invention to a solid phase will be clear to a person skilled in the art, e.g. ease of manipulation; ability to separate or remove dsPrimers and any attached products (e.g. polymerization products) from the remaining reaction mixture, e.g. from unbound and unreacted dsPrimers, buffer, enzymes, dNTPs etc. e.g. after the hybridization or polymerization steps; ability to reuse solid phases and ability to store nucleic acid molecules in a stable way when immobilized to such a solid support.

Multiple dsPrimer molecules of the invention are generally immobilized to an appropriate solid phase. Preferably, multiple copies of the dsPrimers are immobilized. In embodiments where the 3' overhang regions of said dsPrimers comprise degenerate sequences, then a population of dsPrimers comprising a full set of degenerate sequences should be immobilized to the solid phase in order to ensure that the dsPrimers can hybridize with any 3' end present in a nucleic acid template sequence. Multiple copies of each type of degenerate sequence will preferably be used as described elsewhere herein. However, it is not necessary for the full set to be immobilized on an individual solid phase but the full set should be immobilized in the total population of solid phase used. For example, in the case of nanoparticles, the full set of degenerate sequences should be represented on the total population of the nanoparticles not on the individual nanoparticle.

Appropriate densities of immobilization can readily be established in order to ensure that the dsPrimers remain functional, e.g. in terms of allowing hybridization of single stranded nucleic acid template molecules and polymerization to occur. As described for magnetic nanoparticles in more detail below, the attachment of multiple dsPrimers to a solid phase allows for the formation of libraries of nucleic acid molecules by way of single stranded nucleic acid templates becoming hybridized to the immobilized dsPrimers in accordance with step (i) of the methods of the invention, and optionally carrying out a round of polymerization as described above in the methods of the invention.

In this way libraries e.g. for sequencing (e.g. NGS, Nanopore or Sanger sequencing) can be generated. In addition, as described in more detail below, reusable backup libraries can also be generated. Such libraries form a yet further aspect of the invention, as do dsPrimers of the invention immobilized to solid phases.

Thus, a further aspect of the invention provides a solid phase comprising immobilized double stranded primers, wherein said double stranded primers comprise a double stranded region and a single stranded region, wherein the single stranded region is a 3' overhang region, wherein said single stranded 3' overhang region preferably comprises a degenerate sequence or a sequence comprising universal bases, and wherein the double stranded primer is made up of two separate strands. In such embodiments, the single stranded 3' overhang region enables the double stranded primer to target and hybridize to the 3' end of a single stranded nucleic acid template.

Thus, in a preferred aspect of the invention, libraries (and optionally backup libraries) can be prepared using dsPrimers of the invention attached to a solid phase via the 3' end of the lower (non-overhang) strand or the 5' end of the upper (overhang containing) strand. In such methods, appropriate single stranded nucleic acid templates are hybridized to said immobilized dsPrimers in accordance with step (i) of the methods of the invention, after which a polymerization step is carried out in accordance with step (ii) of the methods of the invention, i.e. using a DNA polymerase or other polypeptide with 5' to 3' polymerization activity to carry out a primer extension reaction to synthesize nucleotides in a template dependent manner from the 3' end of the single stranded region of the dsPrimer. The non-attached (non-immobilized) strands are then removed, e.g. by heat denaturation, to provide a library of single stranded molecules, which can then optionally be subjected to further manipulation such as amplification or sequencing (e.g. NGS). The immobilized single stranded molecules are preferably retained and stored as a backup library in order to be able to create the same library in the future or to increase the size of the library by amplification or replication.

In such embodiments of the invention, it is preferable to carry out a step in which the "nick" (gap) between the hybridized single stranded nucleic acid molecules and the 5' end of the lower (non-overhang) strand of the dsPrimer is repaired. This can for example be carried out using a ligase reaction or other reactions as described elsewhere herein. After the polymerization step, such a ligase reaction can also optionally be used to ligate for example a conventional double stranded adaptor, for example with a structure such as that outlined below in Design NP2, to the end of the double stranded polymerised product (which comprises the dsPrimer and the polymerization product) which is not in contact with the solid phase (the free end). Such adaptor sequences can for example be used in conjunction with the sequences contained in the double stranded region of the dsPrimer (e.g. primer binding sites or adaptor sequences) in order to aid any further manipulation steps (e.g. amplification or sequencing) of the products which are carried out after the non-attached/non-immobilized strands have been removed to form the library. Alternatively, as in the other embodiments of the invention, another set of dsPrimers (which are free and non-immobilized to any solid support) can be used instead of double stranded adaptors as detailed elsewhere herein.

A preferred and exemplary way of forming such libraries and reusable backup libraries is described and illustrated in Design NP2 which gives examples of appropriate and preferred steps and of storage conditions for the reusable backup libraries, any of which may be used in the above more general methods.

Any appropriate type of single stranded nucleic acid template molecules as described herein can be used to generate such libraries and immobilized backup libraries. For example, such methods can be used with any DNA or RNA template molecules as described elsewhere herein, including with SELEX aptamers or SELEX LNA aptamers. Design NP2 illustrates how such techniques might be used with genomic DNA.

In addition, in an alternative preferred aspect of the invention, libraries (and optionally backup libraries) can be prepared using dsPrimers of the invention attached or immobilized to a solid phase via the 5' end of the upper (overhang containing) strand. Such methods are particularly useful for application on SELEX to discover aptamers, for example in order to produce a library of selected candidate aptamers for further investigation or manipulation (such as amplification and sequencing), and optionally producing a backup library. Thus, such methods could readily be used after step (b) of any of the SELEX methods described herein in which the aptamers which bind to the target ligand are selected. The methods are particularly useful for truncated aptamers, e.g. aptamers selected from single base truncated libraries of improved SELEX methods constructed by using chain terminators which are truncated nucleic acid molecules in which the 3' ends of said nucleic acid molecules do not have a 3'OH group. The methods are also particularly useful for aptamers which have a known or fixed primer site, i.e. a specific known sequence, at the 5' end of each of the candidate aptamer molecules. Alternatively, a version of the NP3 methods where the 3' end of the lower (non-overhang) strand is immobilized may be used in classical SELEX applications in a manner similar to Design NP2 with a ligase enzyme.

In such methods, appropriate single stranded nucleic acid templates, for example selected or candidate SELEX Aptamers, with a known primer site at the 5' end, e.g. selected or candidate truncated aptamers, are hybridized to said immobilized dsPrimers in accordance with step (i) of the methods of the invention, after which a polymerization step is carried out in accordance with step (ii) of the methods of the invention, i.e. using an enzyme with 5' to 3' DNA polymerization activity to carry out a primer extension reaction to synthesize nucleotides in a template dependent manner from the 3' end of the single stranded region of the dsPrimer. The non-attached (non-immobilized) strands (in this case the original nucleic acid template molecules, e.g. the candidate SELEX aptamers) are then removed, e.g. by heat denaturation, so that the immobilized polymerized single strands remain attached to the solid support. These strands contain the upper (overhang) strand of the dsPrimer which is immobilized to the solid phase but also contain the polymerized product which corresponds to the complementary sequence of the original nucleic acid template molecule (e.g. the complementary sequence of the candidate aptamer molecule), together with a fixed region at the 3' end corresponding to the complementary sequence of the fixed primer region present in the original nucleic acid template molecules.

A single stranded primer is then added, which hybridizes to the fixed region at the 3' end of the polymerized strand, after which an enzyme with 5' to 3' DNA polymerization activity is used to carry out a primer extension reaction to synthesize nucleotides in a template dependent manner from the 3' end of said single stranded primer, in this way resynthesizing the nucleic acid template molecules, e.g. the candidate SELEX aptamers, now with fixed sequences at the 5' and 3' ends.

The non-attached (non-immobilized) strands (in this case the resynthesized nucleic acid template molecules, e.g. the candidate SELEX aptamers) are then removed, e.g. by heat denaturation, so that the immobilized polymerized single strands remain attached to the solid support. The removed library of single stranded molecules with fixed sequences at the 5' and 3' ends (e.g. the candidate SELEX aptamers), can then optionally be subjected to further manipulation such as amplification or sequencing (e.g. NGS), where the fixed sequences can aid any further such manipulation. Alternatively, the removed library of single stranded molecules with fixed sequences at the 5' and 3' ends (e.g. the candidate SELEX aptamers), can be subjected to a further round of screening using the above method steps, e.g. a further round of SELEX. The immobilized single stranded molecules are preferably retained and stored as a reusable backup library in order to be able to create the same SELEX cycle pool or library in the future or to increase the size of the SELEX cycle pool or library by amplification or replication.

A preferred and exemplary way of forming such libraries and reusable backup libraries is described and illustrated in Design NP3 which gives examples of appropriate and preferred steps any of which may be used in the above more general methods.

Appropriate storage conditions for the backup libraries are provided in Design NP2.

Design NP3 illustrates how such techniques might be used with truncated SELEX aptamers with fixed regions at the 5' end.

Some particular embodiments and advantages are outlined below for magnetic nanoparticles but these may equally be applied to other solid supports such as those described herein.

A highly advantageous feature of magnetic nanoparticle-conjugated dsPrimers is that they can be magnetically separated or extracted from the reaction mixture after hybridization, or polymerization steps to remove excess dsPrimers, which can decrease the overall amplification efficiency by hybridizing to PCR or other amplification/extension primers (see Design NP2 and Design NP3).

As shown in Design NP2, magnetic nanoparticle-conjugated dsPrimers can be used to construct Next Generation Sequencing (NGS) and other sequencing libraries. Double stranded genomic DNA from target organisms, tissues or cells is extracted via desired methods. Shorter DNA fragments can be prepared by enzymatic digestion or sonication, to be separated by size separation of fragments between 200-300 bp lengths. Fragmentation creates fragments with ends that are missing hydroxyl and phosphate groups. Enzymes, such as Klenow fragment or T4 polymerase, or commercial end-repair kits, can be used to repair the ends of the fragments to generate 3'-OH and 5'-$PO_4$ groups. Heat denaturation is used to obtain single stranded molecules from the fragmented double stranded molecules.

Magnetic nanoparticle-conjugated dsPrimers, can be employed in a manner to immobilize dsPrimers such that the lower (non-overhang) strand of the primer is attached to the bead through its 3' end, while the 5' end of the upper (overhang) strand is not attached to the bead.

After heat denaturation, the fragmented single stranded genomic DNA molecules can hybridize to the random nucleotide regions of the dsPrimers that are attached to the magnetic nanoparticles.

To form a covalent bond between the 3' end of the nucleic acid fragments and 5' end of the lower portion of the dsPrimers, a ligase enzyme and a ligase reaction can conveniently be used. At the same time, one round of polymerization using a DNA polymerase, for example a mesophilic DNA polymerase, such as Klenow Fragment, can be carried out to synthesize nucleotides in a template dependent manner from the 3' end of the single stranded region of the magnetic nanoparticle-conjugated dsPrimers.

Conventional double stranded adaptors, (herein referred to as dsAdaptors), comprise an upper strand that contains a hydroxyl group on its 3' end, and lower strand that contains a phosphate group on its 5' end. The 5' end of the upper strand and the 3' end of the lower strand lack phosphate and hydroxyl groups, respectively, which provides specificity.

The addition of DNA ligase in the solution can also be used to add dsAdaptor regions to the free ends of the polymerized double stranded structures on the nanoparticles which comprise the dsPrimer and polymerized genomic DNA fragments.

A further advantage of the magnetic nanoparticle-conjugated dsPrimers is that separated magnetic nanoparticle-conjugated dsPrimers can be used as a reusable and stable backup of the sequencing library, for example an NGS library. Magnetic separation of nanoparticles allows the selection of nucleic acid molecules which bind to the dsPrimers on nanoparticles and removal of nucleic acid molecules which do not bind to the dsPrimers on nanoparticles. After selection, heat denaturation can be used to obtain the complementary strand of the primer binding strand of the double stranded structure that is not attached to the nanoparticles, namely 5' end antisense asPrimer and 3' end sense Adaptor (sAdaptor) i.e. the primer binding strand of the double stranded adaptor containing single stranded fragments, ready for subsequent steps, such as emulsion PCR or other NGS steps. If desired, the nanoparticles comprising the still immobilized single stranded fragments, namely fragments capped with sPrimer and antisense Adaptor (asAdaptor), i.e. complementary strand of the primer binding strand of the double stranded adaptor can be stored as a backup library to create the same library, e.g. NGS library, in the future or increase the size of the library via amplification, or replication, with respective adaptor primers. Storage of the backup library can be done for example at 2-8°C, in solutions such as nuclease free water or in a buffer solution appropriate for the type of nucleic acid on the nanoparticle, such as 10mM Tris-HCl 1 mMEDTA pH 8.0 for DNA or 1 mM Sodium Citrate, pH 6.4. for RNA. Under such conditions, backup libraries are expected to be stable for at least 12 months.

Such conditions could be used for storing any of the backup libraries described herein.

As shown in Design NP3, magnetic nanoparticle-conjugated dsPrimers can also be used to amplify selected aptamers from single base truncated SELEX pools in which 3' ends of the nucleic acid molecules do not have a 3' -OH group and 5' ends have a fixed primer sequence and to construct NGS libraries of selected aptamer amplicons.

A single base truncated SELEX pool, in which the 3' ends of the nucleic acid molecules do not have a 3' OH group, can be produced by using an RNA or DNA polymerase in the presence of chain terminators (e.g. ddNTP or 3'-dNTPs). The single base truncated RNA or DNA SELEX pool may comprise oligonucleotides that have a proprietary, identification, target, control or functional sequence at their 5' ends, referred to herein as asPrimer 01.

Magnetic nanoparticle-conjugated dsPrimers may comprise dsPrimers immobilized such that the upper (overhang) strand of the primer is attached to the bead through its 5' end, while the 3' end of the lower (non-overhang) strand is not attached to the bead.

The magnetic nanoparticle-conjugated dsPrimers for example contain a specific sequence within their double stranded region that can be used as a primer binding site, adaptor sequence restriction site, promoter or terminator region or other useful sequence as described herein. For example, in the Design NP3, a single stranded primer site defined as "asPrimer02" is located in the upper strand of the dsPrimer (which also contains the 3' overhang region), and sequences complementary to the asPrimer 02 in the lower strand are hereupon referred as sPrimer 02. The selected single stranded truncated aptamer molecules can hybridize from their 3' ends to the random nucleotide regions of the upper strands of dsPrimers on the magnetic beads. One round of polymerization using a DNA polymerase, for example a mesophilic DNA polymerase, such as Klenow Fragment, or reverse transcriptase, as appropriate, can be carried out to synthesise nucleotides in a template dependent manner from the 3' end of the single stranded upper strand region of the magnetic nanoparticle-conjugated dsPrimers. Heat denaturation can be used to separate the lower strand sPrimer 02 and hybridized aptamers from the nanoparticles, and to obtain the extended upper strand attached to the nanoparticles. Magnetic separation of nanoparticles allows the selection of nanoparticle attached single stranded structures in the form of 5' end asPrimer 02 and 3' end sPrimer containing complementary sequences of selected aptamer candidates.

Following separation, an asPrimer 01 can be added into the solution such that it hybridizes to the 3' end of the sPrimer 01 region of the nanoparticle bound nucleic acid molecules. One round of polymerization using a DNA polymerase, for example mesophilic DNA polymerase, such as Klenow Fragment, can be carried out to synthesise nucleotides in a template dependent manner from the 3' end of the hybridized asPrimer 01.

Magnetic separation of nanoparticles allows the selection of double stranded nucleic acid molecules that are bound to the nanoparticles through the 5' end of the asPrimer 02 region. After selection, heat denaturation can be used to obtain the complementary strand of the double stranded structure that is not attached to the nanoparticles, namely the 5' asPrimer 01 and the 3' sPrimer 02 bound single stranded fragments, making them ready for subsequent steps such as PCR amplification, NGS, Nanopore or Sanger sequencing or another SELEX cycle round.

Another advantage of the magnetic nanoparticle-conjugated dsPrimers, is that magnetically separated nanoparticles comprising single stranded fragments capped with 5' asPrimer 02 and 3' sPrimer 01 can be stored as a reusable backup SELEX cycle pool or library to create the same SELEX cycle for future purposes or increase the size of the library via amplification, or replication, with respective primers.

**Other Applications**

As mentioned elsewhere herein, as well as utility in amplification methods, and any methods employing PCR or other amplification techniques (e.g. in-vitro diagnostics, sequencing applications, nucleic acid molecule or library construction for sequencing, etc), the dsPrimers of the invention find utility in many other methods of nucleic acid manipulation. For example, the dsPrimers and methods of the invention can find utility in the field of personalised medicine such as in companion diagnostics, e.g. for targeted therapies, and in applications relating to DNA based data storage, e.g. to store digital data in the base sequence of DNA via encoding/writing/reading/decoding of the DNA sequence..Additional in-vivo and in-vitro applications of the dsPrimers, methods and kits of the invention include, but are not limited to: linear or circular plasmid (or vector) construction; construction of synthetic DNA or RNA molecules; construction of synthetic DNA or RNA circuits; oligonucleotide synthesis; insertion of functional RNA or DNA domains, such as RNA localization elements or protein binding domains to nucleic acid templates via dsPrimers; amplification of nucleic acid templates containing modified (or unnatural) nucleotides by using dsPrimers as polymerization initiation regions; miRNA sequencing; miRNA-based diagnostics; sequencing-based (sanger, NGS, Third generation devices etc.) diagnostics; identification and sequencing of Circulating DNAs; identification and sequencing of noncoding RNAs; gene therapy methods; RNA silencing (or RNA interference) methods; constructing molecular recognition units of biosensors devices; the use of dsPrimers to add adaptors to single stranded or double stranded nucleic acids solely depending on the hybridization of dsPrimers witout any ligation or polymerisation reaction.

Such diverse utility derives mainly from the fact that the double stranded region of the double stranded primer (dsPrimer) can be engineered to contain any useful sequence.

Thus, for example this region can be engineered to contain some kind of label, thereby resulting in methods of nucleic acid labelling. Such a label can take any appropriate form for inclusion within (endogenous) or attaching to (exogenous) a nucleic acid sequence, for example it could take the form of a nucleic acid code which is present within the dsPrimer sequence or it could for example be an exogenous label such as for example a fluorescent label or a radioactive label, etc. If an exogenous label is used then this can conveniently be attached to the 5' end of the upper strand of the dsPrimer (or the equivalent site in a chPrimer). In other words such labels can be attached to the 5' end of the strand in the dsPrimer/chPrimer which also comprises the 3' single stranded overhang region. Techniques to attach exogenous labels or to incorporate endogenous labels into a nucleic acid sequence would be well known to a skilled person.

Such labelled dsPrimers can then be used in the hybridization step (i) of the methods of nucleic acid manipulation described herein. Then, after the polymerization step (ii) as described herein (which will generally involve primer extension and the replacement of the lower strand of the dsPrimer (or chPrimer equivalent strand)) the label will have become incorporated at the end of the nucleic acid molecules produced.

In other embodiments, the dsPrimers of the invention can be used in the construction of nucleic acid molecules, for example by being used to join nucleic acid fragments together. In such embodiments the double stranded region of the dsPrimer can be engineered to contain a first nucleic acid sequence or fragment which it is desired to join to a second nucleic acid sequence or fragment which is contained within the nucleic acid template molecule.

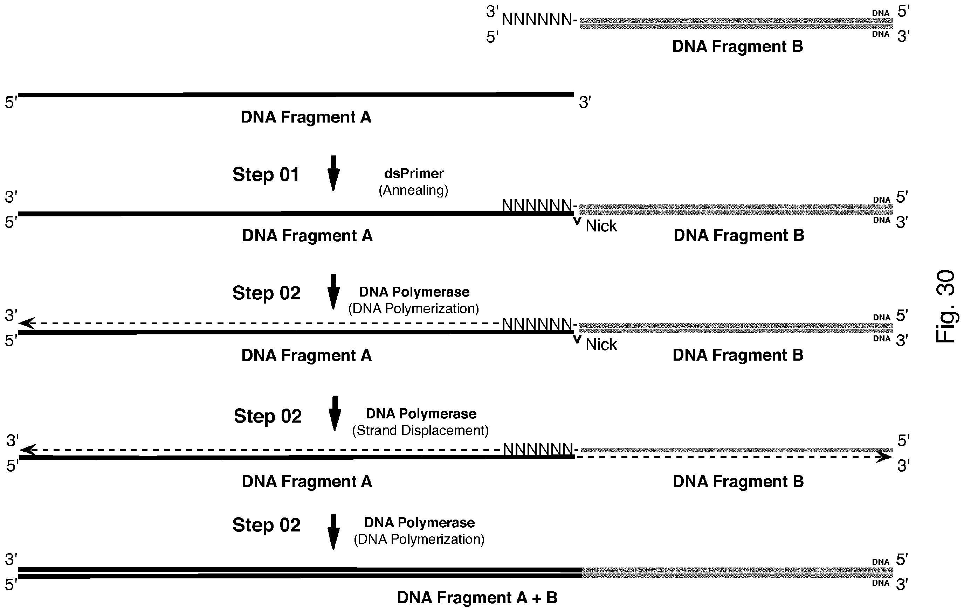

Such fragment containing dsPrimers can then be used in the hybridization step (i) of the methods of nucleic acid manipulation described herein. Then, after the polymerization step (ii) as described herein (which will generally involve primer extension and the replacement of the lower strand of the dsPrimer (or chPrimer equivalent strand)) the fragment contained in the dsPrimer will have become incorporated into the nucleic acid molecules produced and thereby joined to the fragment which was contained in the nucleic acid template molecule. Such methods can thus be used to add any nucleic acid sequence (contained in the double stranded region of the dsPrimer) to a nucleic acid template and conveniently such methods do not require the use of a ligation reaction. Furthermore, large fragments, e.g. fragments of over 1000 bps contained in the dsPrimer can be added in such embodiments. A schematic illustrating such an embodiment is shown in Figure 30.

Such methods can readily be adapted to incorporate any useful sequence by designing the dsPrimer to include such sequences. A preferred example would be to design the dsPrimer to include a restriction site, which, after hybridization and polymerization as discussed above would be incorporated at the end of the nucleic acid molecules produced.

**SELEX methods**

The dsPrimers and/or the amplification methods of the invention can also advantageously be used in Systematic Evolution of Ligands by Exponential Enrichment (SELEX) protocols. SELEX protocols allow the selection of single stranded nucleic acid molecules (e.g. DNA or RNA) which specifically bind to a particular target ligand or ligands (e.g. a protein or small organic compound). Such single stranded nucleic acid molecules are also referred to as aptamers.

Standard SELEX protocols begin with the synthesis of a large oligonucleotide library which generally comprises randomly generated sequences of fixed length (e.g. a fixed length of the order of 20-60 nucleotides, although sometimes libraries of molecules with lengths of over 100 base pairs can be used) flanked by constant (fixed sequence) 5' and 3' ends which serve as primers. The sequences in the library are then exposed to the target ligand and those that do not bind the target are removed, e.g. by affinity chromatography. The bound sequences are then eluted from the ligand and amplified by PCR using the constant primer regions, after which they are then subjected to subsequent rounds of selection in which for example the stringency of the elution conditions can be increased in order to identity the highest affinity binders.

Problems with the constant (fixed sequence) primer (oligonucleotide) regions have been encountered with standard SELEX methods in that such sequences have to be removed after the selection process and can be difficult to remove as they for example stabilize secondary structures that are unstable when formed by the random region alone. In addition, the presence of the constant sequences when the aptamers are brought into contact with the target ligand can contribute to binding to the target ligand which is also undesirable. The use of the dsPrimers and/or the amplification methods of the present invention in SELEX methods thus has the significant advantage and improvement that no constant primer regions need be present in the oligonucleotide library which is screened for binding to the target ligand and also amplification products can be produced which have no constant primer regions present. Thus, there is no need to remove these sequences after selection or amplification and the selection should identify true binders. As will be described in more detail below, a further advantage of the use of the methods of the present invention in SELEX methods is that aptamers of varying length can be screened in the same SELEX procedure, thereby allowing the smallest possible nucleic acid molecules (aptamers) which bind to the ligand to be selected.

Although some of the existing SELEX protocols, e.g. those described by Gilead Sciences, Inc., (see for example US 6,855,496), provide methods in which the participation of fixed (constant) regions is eliminated or minimised when the interaction of the aptamers with the target ligand occurs, such methods do not involve the use of dsPrimers as described herein. In addition, many of the Gilead Sciences, Inc., methods which eliminate the fixed regions when the interaction of the aptamers with the target ligand occurs, do require that fixed regions are added to and then physically removed from the amplified nucleic acid molecules (aptamers) in the amplification steps (e.g. to enable the amplification steps) after the interaction of the aptamers with the target ligand has taken place. In contrast, in the preferred amplification methods used herein with the SELEX protocols, amplification products (aptamers) can be produced which have no constant primer (oligonucleotide) regions present and thus no step in which fixed regions have to be physically removed from the amplified nucleic acid products, e.g. by cleavage or digestion, has to take place in order to obtain the selected aptamer.

Thus, a yet further aspect of the present invention provides a SELEX method in which a dsPrimer of the invention is used in order to amplify the aptamers which bind to the target ligand. Put another way, the present invention provides a SELEX method in which the dsPrimer and/or amplification methods of the invention are used to amplify the aptamers which bind to the target ligand. Such SELEX methods comprise the steps of:

(a) contacting a library of single stranded nucleic acid molecules with a ligand of interest;
(b) selecting for nucleic acid molecules which bind said ligand;
(c) use a dsPrimer of the invention to amplify the nucleic acid molecules which bind to said ligand; and optionally
(d) use said amplified nucleic acid molecules as a new library of nucleic acid molecules to repeat steps (a) to (c) one or more times.

These SELEX methods can thus be used to identify or select a single stranded nucleic acid molecule (aptamer) which can specifically bind to a target ligand, which can then, if desired, be modified, e.g. by restriction digestion or any other desirable technique. Such aptamers have many potential applications, including therapeutic applications, and thus, once identified by the methods of the invention, such aptamers can optionally be manufactured and/or formulated into pharmaceutical compositions by methods well known in the art.

Additional and preferred features of the dsPrimers for use in the SELEX methods are as described elsewhere herein. For example, in such methods it is preferred that the single stranded 3' overhang region comprises degenerate or inosine (universal) sequences. In addition any appropriate amplification method of the invention as described elsewhere herein can be selected for use in the SELEX methods, depending on the intention of the skilled person carrying out the SELEX protocols and on the format of the aptamers it is desired to obtain. For example, as will be described below, in some embodiments it is desirable to obtain an aptamer which has an extended 3' end beyond the aptamer sequence, which extended end can add an additional utility to the ability of the aptamer to specifically bind to the target ligand.

In other embodiments for example, it is desirable that aptamer sequences are produced with no additional structural or functional motifs present (e.g. no fixed structural or functional motifs such as primer sequences or other structural or functional oligonucleotides, i.e. the aptamer sequence alone is produced). In such embodiments therefore the methods do not comprise a step in which constant oligonucleotide regions have to be physically removed from the amplified nucleic acid products, e.g. the amplified products produced at the end of step (c). In such embodiments the amplified nucleic acid products (aptamers) do not contain additional constant oligonucleotide regions at the 5' or 3' end of the molecule.

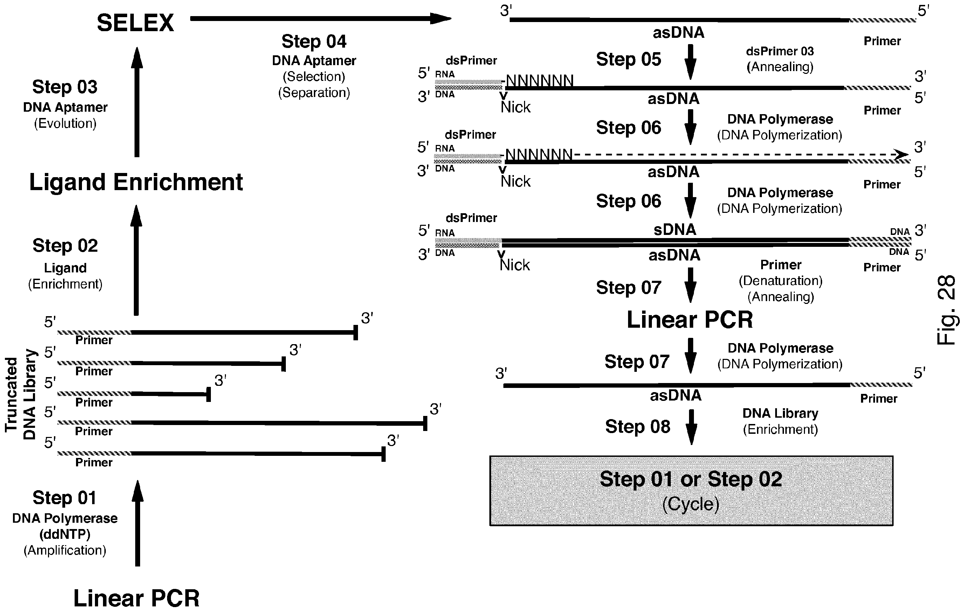

Equally however, if desired, the library of single stranded nucleic acid molecules (aptamers) and amplification products (aptamers) can be produced which have constant or fixed primer (oligonucleotide) regions present at the 5' and/or the 3' end (see for example Design S3, Figure 28), in which case a step in which such fixed regions are physically removed from the amplified nucleic acid products, e.g. by cleavage or digestion, can take place in order to obtain the selected aptamer.

In some embodiments, as described above, it is preferred that certain steps of the amplification methods take place without the formation of a phosphodiester bond between the 3' end of the nucleic acid molecule (aptamer) and the dsPrimer, more specifically in such embodiments such a bond is not required to be formed between the 3' end of the nucleic acid molecule (aptamer) and the 5' end of the lower strand/non-overhang strand of the dsPrimer (or equivalent strand when the dsPrimer is a chPrimer) as described above. Thus, in such steps, preferably the dsPrimers used do not contain a 5' phosphate group on the lower strand/non-overhang strand of the dsPrimer (or equivalent strand when the dsPrimer is a chPrimer).

Any appropriate library of single stranded nucleic acid molecules can be used in step (a). Thus, standard nucleic acid libraries obtained from any source can be used in such methods. In terms of the lengths of the single stranded aptamers to be used, a skilled person will be well aware of lengths which are appropriate for SELEX protocols. As mentioned above, standard SELEX protocols use oligonucleotide libraries with lengths of the order of 20-60 nucleotides, although sometimes libraries of molecules with lengths of over 100 base pairs can be used, and such libraries of oligonucleotides generally comprise randomly generated sequences. Such libraries and lengths of molecule are also appropriate for use in the present methods.

Importantly, in the SELEX methods of the present invention, the nucleic acid molecules which are screened (brought into contact with the ligand of interest) do not require the presence of constant or fixed primer regions. Indeed, it is preferred that the nucleic acid molecules of step (a) do not contain constant or fixed primer regions (although, as described above, and as shown in Design S3, Figure 28, where a library in which a constant primer region has been incorporated at the 5' end is shown, the use of such constant or fixed primer regions at the 5' and/or 3' end of the aptamers is not incompatible with the methods of the invention and screening of such aptamers is envisaged and indeed is preferred in some embodiments, particularly aptamers with constant or fixed regions at the 5' end). The methods where the nucleic acid molecules of step (a) do not contain constant primer regions advantageously allows the nucleic acid molecules which are screened to comprise only nucleotides which can potentially contribute to ligand binding, e.g. comprise short oligonucleotide sequences.

In a preferred embodiment of the invention the library used in step (a) is a truncated nucleic acid library in which the 3' ends of the nucleic acid molecules do not have a 3' OH group. Such libraries can conveniently be produced using an RNA or DNA polymerase in the presence of ddNTPs or 3'-dNTPs to act as chain terminators (or other chain terminators known in the art) to generate single base truncated DNA or RNA pools for SELEX applications. The use of such truncated libraries has the advantage that it is possible to generate truncated random nucleic acid libraries for SELEX screening that not only contain all possible randomly generated sequences of a predetermined fixed length, but also contain randomly generated sequences with decreasing length variations. Therefore, it is possible to create a true random nucleic acid library with maximum variation that increases the theoretical diversity of random sequences

$$\text{from } 4^N \text{ to } \sum_{M=0}^{N} 4^{N-M}.$$

where N means number of the degenerate sequences within the designed nucleic acid library. Such methods thus allow the smallest possible nucleic acid molecules (aptamers) which bind to the ligand to be selected, i.e. to select the aptamer molecule of minimum size. When reference is made herein to the selection of the smallest possible aptamers or aptamers of a minimum size, generally what is meant is that after obtaining varying lengths of aptamer, they are separated through size separation or they are sequenced, after which the shortest one can be selected, if desired.

The selection of aptamers of minimum size is advantageous both in terms of stability and production cost of the selected aptamers. In this regard, one of the fundamental purposes of aptamer research is to identify the shortest binding motifs (or domains) of aptamers to increase the stability of the molecules. Thus, the SELEX methods as described herein which use a truncated library enables the selection of minimal motifs within a single experimental set up because of the size and length variation of the nucleic acid library, which is highly advantageous. In addition, such methods can be used to engineer synthetic molecules (synthetic genes) by combining these shortest binding motifs (or domains). The cost efficiency is another important advantage as the production and modification costs of nucleic acid molecules increase with the length of the molecules. Therefore only the shortest forms of the selected nucleic acids are suitable for mass production. It can thus be seen that the suitability of this method to screen a library of aptamers which have different lengths is highly advantageous.

Importantly the use of such truncated molecules lacking an 3'OH group at the 3' terminus as a nucleic acid library has not been possible in SELEX methods described to date as it was previously not possible to amplify the truncated molecules (aptamers) which would have been selected for binding to the ligand, as previously no technique has been available to elongate a nucleic acid without any 3' end OH group. In other words it was not possible to amplify such truncated molecules as they were unsuitable for conventional PCR techniques. In addition, with such truncated molecules, no constant primer regions are present at the 3' ends to enable PCR. However, the methods of the present invention enable such truncated molecules (aptamers) to be used in SELEX methods as they allow truncated nucleic acid molecules, i.e. nucleic acid molecules with no 3' OH groups, to be amplified using the dsPrimers of the invention and then optionally be used for another round of selection.

The nucleic acid libraries for use in step (a) can be generated by any appropriate methods well known and described in the art, or pre-existing libraries can be obtained and screened. As is demonstrated in the attached SELEX design protocols (Design S1 and S2), dsPrimers of the invention can conveniently be used in the preparation of the nucleic acid libraries for use in step (a) and indeed this is advantageous in some embodiments, but is by no means compulsory (see for example Design S3).

The SELEX methods of the invention can be carried out on any single stranded nucleic acid library, e.g. RNA molecules, DNA molecules, PNA molecules, etc. The exemplary use of an RNA and a DNA library are shown in the Designs S1 and S2, S3, respectively. In such methods the preferred embodiment of a truncated (no 3'OH) library is shown. The libraries can have unmodified 5' ends as shown in Design S2 or 5' ends with native or non-native modifications such as for example shown in Design S1 (native modification), where the library has been prepared using an RNA polymerase acting on a T7 promoter region meaning that the characteristic and native motif (start point) generated by a T7 RNA polymerase is present at the 5' ends (as described elsewhere herein the particular promoter shown in the Designs results in the generation of the residues GGG at the 5' ends). Equally it is sometimes advantageous to use nucleic acid libraries with modified ends other than a truncated 3' end. For example, Design S3 shows the use of a library in which a constant primer region has been incorporated at the 5' end.

In certain embodiments it may be advantageous to use a library with a phosphorylated 5' end so that for example lambda exonuclease digestion can be used in the amplification step (c) (see Design S2). Methods of amplification using lambda exonuclease are described elsewhere herein.

The step of selecting for nucleic acids which bind to the selected ligand of interest (step (b)) can conveniently be carried out by removing those sequences which do not bind to the target ligand after the library has been exposed to the target ligand. The selection step (b) is carried out in conventional SELEX methods and thus appropriate methods are well known and described in the art. Thus, any conventional selection and/or separation methods can be used, for example membrane (nitrocellulose etc.) filtration, affinity beads, capillary electrophoresis, flow cytometry, gel mobility shift, affinity column matrices, centrifugation, surface plasmon resonance, high-throughput automated selections, nanoparticles (magnetic beads), or microarray systems.

Once the nucleic acid molecules which bind to the target ligand have been selected in step (b), and the nucleic acids molecules which do not bind to the target ligand have been removed from the reaction mixture (usually by physical separation of aptamer-target complexes from the reaction mixture), the target ligand is then eluted from the nucleic acid molecules (from the aptamer-target molecule complexes). Such elution methods are carried out in conventional SELEX methods and thus appropriate elution methodology is well known and described in the art (e.g. the aptamers are separated from the target ligands by denaturation methods such as increasing pH or temperature, etc). The nucleic acid molecules (aptamers) are then usually purified from the reaction mixture and subjected to amplification.

Thus, in the SELEX methods of the invention, dsPrimers of the invention are used to amplify the selected nucleic acid molecules. Any method of amplification as described herein (involving the described steps of hybridization, polymerization and amplification) may be used and can readily be selected depending on the type of nucleic acid template molecule (aptamer) to be amplified (e.g. DNA or RNA) and the particular format of template nucleic acid molecule (aptamer), e.g. 3' truncated, 5' phosphorylated, etc.

By way of example, some preferred amplification methods are selected so as to produce an amplification product without any 3' or 5' insertions or additions, i.e. the amplification product is the aptamer molecule itself without any additional regions, e.g. does not contain any fixed or constant oligonucleotide regions or primer regions, or at least does not contain unnatural (non-native, non-natural) 5' and 3' end modifications, although they may optionally contain natural/native 5' and/or 3' end modifications such as those introduced by the action of an RNA polymerase on a promoter and/or terminator region (see for example the methods such as those shown in Designs 1, 5, 7 and 8, S1, S2). Such methods have the advantage that they do not involve or require a step in which constant oligonucleotide sequences or regions are removed after amplification, for example by subjecting the amplified molecules (aptamers) to physical removal methods such cleavage or digestion.

Other preferred amplification methods will result in an additional 3' end insert (e.g. an additional 3' end oligonucleotide region) to the end of the aptamer (amplification product) which can be useful in further manipulation. For example methods such as those described herein in which 3' end elongated amplification products are produced are useful for this. For example they allow the aptamer to be immobilized for example for nanoparticle or microarray-based selection processes. In addition, in such embodiments, if the aptamer molecules are immobilized by hybridization using the 3' end elongated sequence before selection then this additional 3' end sequence should not interfere with the binding of the actual aptamer sequence. Furthermore, the 3' elongation ends can be designed so that they can be removed after selection, e.g. by digestion, which again should ensure that they do not interfere with the interaction between the aptamer and the target ligand. In other methods, if helpful, the additional 3' end inserts can be blocked from interacting with the target ligand by hybridization to a complementary oligonucleotide.

Other preferred amplification methods will result in an additional 5' end insert (e.g. an additional 5' end oligonucleotide region) to the end of the aptamer (amplification product) which can be useful in further manipulation (see for example, Design S3, in which a library with a constant 5' primer region is used).

Preferred amplification methods involve the use of DNA dependent RNA polymerase promoters and terminators which result in amplification products with native ends (such as the T7 promoter and terminator, see for example Design 5 and Design 7, Design S1) or involve other methods which result in the amplification of only the aptamer (nucleic acid template) molecule itself, e.g. methods involving amplification using an RNA primer such as those shown in Design 8, Design S2. Other preferred methods involve the use of DNA dependent RNA polymerase promoters (e.g. the T7 promoter) which result in amplification products with native 5' ends (see for example Design 1) and optionally with modified or extended/elongated 3' ends, for example elongated with an oligonucleotide sequence or phi6 or polyA tails, see for example Design 6, Design 12, Design 13, Designs 14 to 17.

For illustrative purposes, some preferred and exemplary SELEX protocols are shown in Design S1 and Design S2. For example preferred protocols of RNA amplification involve the use of a dsTerminator, for example dsT7Terminator (see Design S1) in one round of polymerization and a dsRNA polymerase promoter such as a dsT7Promoter in another round of polymerization (see Design S1), after which amplification of an aptamer molecule with native ends can occur using an RNA polymerase (see Design S1).

Design S2 shows an exemplary DNA amplification using two different dsPrimers of the invention for amplification (called dsPrimer 02 and dsPrimer 03). In the particular embodiment shown, the use of lock primers is illustrated demonstrating that such steps can be used where appropriate. In addition in these protocols a hybrid RNA/DNA dsPrimer is used for the amplification step, the RNA portion of which is degraded away by for example RNase H after which single stranded RNA primers and displacement of amplified product via strand displacement are used to produce the selected aptamer (with no additions to the 5' or 3' ends) in a continuous amplification reaction as described elsewhere herein.

In embodiments where truncated (no 3'OH) aptamers are used then during the polymerization rounds the step of producing the complementary strand (replacement of the lower strand) cannot be carried out by a strand displacement protocol as described elsewhere herein as there is no 3' end to act as a substrate for a DNA polymerase with strand displacement activity. Alternative methods therefore have to be used which do not require a 3' OH group in order to produce the lower strand. A convenient method would be the use of the enzyme RNase H to remove the undesired strand, for example as shown in Design S1, or to use a 5' phosphate label in order to allow degradation of the undesired strand by lambda exonuclease activity as described elsewhere herein.

A preferred second dsPrimer to be used in step (c) is a dsPromoter. Preferably the dsPromoter is selected to match up with the dsTerminator. Thus, in embodiments of the invention where a T7 dsTerminator was used then a preferred second dsPrimer of the invention to be used in the second round of polymerisation would be a ds T7 Promoter.

SELEX kits or compositions comprising appropriate dsPrimers of the invention, e.g. as outlined above, for carrying out the SELEX protocols as outlined herein are further aspects of the invention. A library of single stranded nucleic acid molecules (aptamers) is a further optional component. In a preferred embodiment, said aptamers take the form of a library of single stranded nucleic acid molecules (optionally of varying lengths) in which the 3' ends are truncated (i.e. do not have a 3'OH group).

Thus, a yet further preferred aspect of the invention provides a SELEX kit or composition comprising:

(i) a double stranded primer which comprises a double stranded region and a single stranded region, wherein the single stranded region is a 3' overhang region and wherein the 3' overhang region enables the double stranded primer to target and hybridize to the 3' end of a single stranded nucleic acid template, wherein said single stranded 3' overhang region preferably comprises a degenerate sequence or a sequence comprising universal bases, wherein the double stranded primer is made up of two separate strands; and
(ii) a library of single stranded nucleic acid aptamers, wherein said library is a library of truncated nucleic acid molecules in which the 3' ends of the nucleic acid molecules do not have a 3'OH group.

As described above, once an amplification template has been produced then amplification can be carried out in accordance with step (iii) of the methods of the invention as described elsewhere herein. The appropriate amplification methods will depend on the nature of the amplified product and the nature of the amplification template. In examples such as those shown in Design S1 where a promoter and terminator that are recognised by an RNA polymerase are used, then the amplification step is carried out using an RNA polymerase as described elsewhere herein.

The end result after step (c) of the method is an enriched library comprising amplified copies of nucleic acid molecules which bind to the target ligand. Thus, an optional and preferable step is to repeat the SELEX cycle one or more times by contacting said amplified (and enriched) molecules again with target ligand.

As will be clear from the discussion elsewhere herein, several Design protocols are included in order to illustrate the methods of the invention and some preferred steps and embodiments. The Designs provided are however illustrative of the invention and are included to show both preferred methods and to illustrate various exemplary steps of the methods of the invention. It is envisaged that a skilled person could readily derive alternative methods of the invention by combining various and appropriate steps shown in the schematic designs, and all such embodiments are encompassed within this disclosure.

In the above discussion and the Design protocols some abbreviations are used to describe the molecular entities used. Thus "ds" means double stranded, "ss" means single stranded, "sDNA" means sense DNA, "asDNA" means antisense DNA, "sRNA" means sense RNA, "asRNA" means antisense RNA, "asPrimer" means antisense primer, which is a primer which is designed to hybridize or anneal to the sense strand of the RNA or DNA which is present, "sPrimer" means sense primer, which is a primer which is designed to hybridize or anneal to the antisense strand of the RNA or DNA which is present.

A more detailed description of the specific Design protocols is included below with reference to drawings in which:

Figure 1 shows the structure of the dsPrimers of the invention, together with an illustration on the step of dsPrimer Silencing.

Figure 2 shows the structure of the dsPromoters of the invention, together with an illustration on the step of dsPromoter Silencing.

Figure 3 shows Design 1 - asRNA Amplification from sDNA.

Figure 4 shows Design 2 - dsDNA Amplification from dsDNA.

Figure 5 shows Design 3 - dsDNA Amplification from dsDNA (Strand Displacement).

Figure 6 shows Design 4 - dsDNA Amplification from sRNA (Initial RNase H).

Figure 7 shows Design 5 - sRNA Amplification from sDNA (Terminator).

Figure 8 shows Design 6 - sRNA Amplification from sRNA (Single Primer Region).

Figure 9 shows Design 7 - sRNA Amplification from sRNA (Initial RNase H + Terminator).

Figure 10 shows Design 8 - asDNA Amplification from sDNA (Isothermal + Single Primer).

Figure 11 shows Design 9 - dsDNA Amplification from dsDNA (Lambda).

Figure 12 shows Design 10 - dsDNA Amplification from dsDNA (Lambda + Strand Displacement).

Figure 13 shows Design 11 - dsRNA Amplification from dsDNA (Strand Displacement).

Figure 14 shows Design 12 - dsRNA Amplification from sDNA (Initial RNase H).

Figure 15 shows Design 13 - dsRNA Amplification from sDNA.

Figure 16 shows Design 14 - sRNA Amplification from sDNA (Polyadenylation + Oligo dT + Primer Region).

Figure 17 shows Design 15 - sRNA Amplification from sRNA (Initial RNase H + Oligo dT).

Figure 18 shows Design 16 - sRNA Amplification from sRNA (Oligo dT).

Figure 19 shows Design 17 - sRNA Amplification from sRNA (Polyadenylation + Oligo dT + Primer Region).

Figure 20 shows Design Ch1 - dsDNA Amplification from dsDNA (chPrimer + Strand Displacement + Initial Primer).

Figure 21 shows Design Ch2 - dsDNA Amplification from dsDNA (chPrimer + Strand Displacement + Initial Primer + 2 Primer Sites).

Figure 22 shows Design Ch3 - dsDNA Amplification from dsDNA (chPrimer).

Figure 23 shows Design Ch4 - dsDNA Amplification from dsDNA (chPrimer + 2 Primer Sites).

Figure 24 shows Design Ch5 - sDNA Amplification from dsDNA (chPrimer + RNase H).

Figure 25 shows Design Ch6 - asDNA Amplification from dsDNA (chPrimer + RNase H).

Figure 26 shows Design S1 - RNA SELEX (Terminator).

Figure 27 shows Design S2 - Truncated library formation (DNA SELEX) (Isothermal) in which a truncated library is formed which can then be subjected to DNA SELEX (Lambda and Isothermal).

Figure 28 shows Design S3 - DNA SELEX (Single Primer Region)

Figure 29 shows the structure of the chPrimers of the invention, together with an illustration on the step of chPrimer Silencing.

Figure 30 shows a schematic for the joining of two DNA fragments (DNA Fragment addition).

Figure 31 shows Design Ch7 - dsDNA Amplification from dsDNA (chPrimer + Denaturation).

Figure 32 shows Design Ch8 - dsDNA Amplification from dsDNA (chPrimer + Denaturation + 2 Primer Regions).

Figure 33 shows dsDNA to dsDNA with dsPrimer (Optimization) experimental data.

Figure 34 shows dsDNA to dsDNA with dsPrimer (Size comparison to standard PCR) experimental data.

Figure 35 shows dsDNA to ssRNA with dsPrimer (Transcription) experimental data.

Figure 36 shows gDNA to gDNA with chPrimer experimental data.

Figure 37 shows gDNA to gDNA with chPrimer (Size comparison of single and multiple primer sites) experimental data.

Figure 38 shows gDNA to gDNA with dsPrimer experimental data.

Figure 39 shows gDNA to ssRNA with chPrimer (Transcription) experimental data.

Figure 40 shows sDNA to asRNA with dsPrimer (Transcription) experimental data.

Figure 41 shows sequence data of dsDNA Fragment 02 amplicons amplified by dsPrimer.

Figure 42 shows Design NP1 Magnetic Nanoparticle-conjugated dsPrimer structure

Figure 43 shows Design NP2 Construction of a Backup NGS Library

Figure 44 shows Design NP3 Construction of a Backup SELEX Cycle Pool.

Figure 45 shows gDNA Fragmentation.

Figure 46 shows gDNA to gDNA with dsPrimers (Ion Torrent Adaptation).

### Figure Explanations

**Figure 1. dsPrimer Structures:**

**Figure 1A: dsPrimer Structure:**

- dsPrimer structure formed by; a double-stranded part and a single-stranded degenerate sequence or inosine bases or can be a specific sequence.
- The double-stranded part can be made of any type of nucleic acid. For example, both of the strands can be DNA, both of the strands can be RNA, or one strand can be RNA and one strand can be DNA. Alternatively, one or both strands can contain a mixture of nucleic acid types (e.g. a mixture of RNA and DNA regions in the form of a chimeric nucleic acid molecule).
- The single-stranded part can be formed by; degenerate sequences, any natural or unnatural nucleic acid molecules, inosine bases or can be a specific sequence located at the 3'-end of one of the strands as an overhang.

**Figure 1B: dsPrimer Silencing:**

- dsPrimer silencing is the name of the process to stop the activity of the unhybridized dsPrimer molecules after their first hybridization to the 3'- end of the single-stranded template molecule.
- Degradation of the 3' overhang part of the dsPrimer structure with a polymerase, which has 3'-5' exonuclease activity.
- Hybridization activity of all unhybridized dsPrimer structures is stopped after degradation in order to prevent undesired hybridization of dsPrimer molecules.

**Figure 2. dsPromoter Structure**

**Figure 2A: dsPromoter Structure**

- dsPromoter structure formed by; a double-stranded part and a single-stranded degenerate sequence or inosine bases or can be a specific sequence.
- The double-stranded part can be made of any type of nucleic acid. For example, both the strands can be DNA, both of the strands can be RNA, or one strand can be RNA and one strand can be DNA. Alternatively, one or both strands can contain a mixture of nucleic acid types (e.g. a mixture of RNA and DNA regions in the form of a chimeric nucleic acid molecule).
- Double-stranded part contains a promoter (T7, SP6 etc.) sequence.
- Single-stranded part can be formed by; degenerate sequences, any natural or unnatural nucleic acid molecules, inosine bases or can be a specific sequence located at the 3'-end of one of the strands as an overhang.

**Figure 2B: dsPromoter Silencing:**

- dsPromoter silencing is the name of the process to stop the activity of the unhybridized dsPromoter molecules after their first hybridization to the 3'- end of the single-stranded template molecule.
- Degradation of the 3' degenerate or inosine overhang of the dsPromoter structure with a polymerase, which has 3'-5' exonuclease activity.
- Hybridization activity of all unhybridized dsPromoter structures stopped after degradation in order to prevent undesired hybridization of dsPromoter molecules.
- Polymerization activity of DNA/RNA, RNA/DNA and RNA/RNA structures is stopped or extremely decreased, because T7 DNA-dependent RNA polymerase only works efficiently on DNA/DNA double-stranded structures.

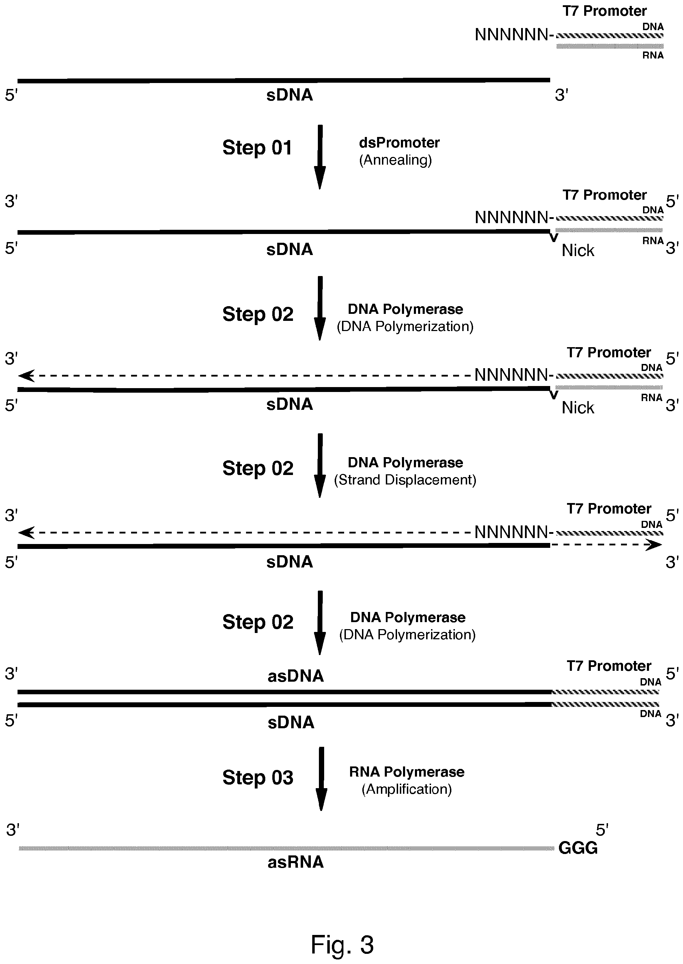

**Figure 3. Design 1 asRNA Amplification from sDNA:**

- The molecular structure of the dsPromoter contains a T7 promoter site (or any other DNA-depended RNA polymerase promoter) with a degenerate oligomer (e.g. hexamer) at the 3'-end of the DNA strand, and the complimentary strand is RNA.

  • Step 01: dsPromoter hybridizing to the 3' end of single-stranded sDNA template.
  • Step 02a: DNA polymerase with 5'-3' polymerization activity filling next to the degenerate sequences.
  • Step 02b: DNA polymerase with both 5'-3' polymerization and strand displacement (or nick translation) activity replacing lower part of the promoter.
  • *Note: A single DNA polymerase with both 5'-3' polymerization and strand displacement (or nick translation) capability can perform those two steps (02a and 02b) at the same time.*
  • *Note: A DNA polymerase with 3'-5' exonuclease activity can perform dsPromoter silencing at this step.*
  • Step 03: DNA-dependent RNA polymerase binding to promoter part and transcribing the sense strand into asRNA with three guanine bases at the 5'-end due to T7 promoter's feature.

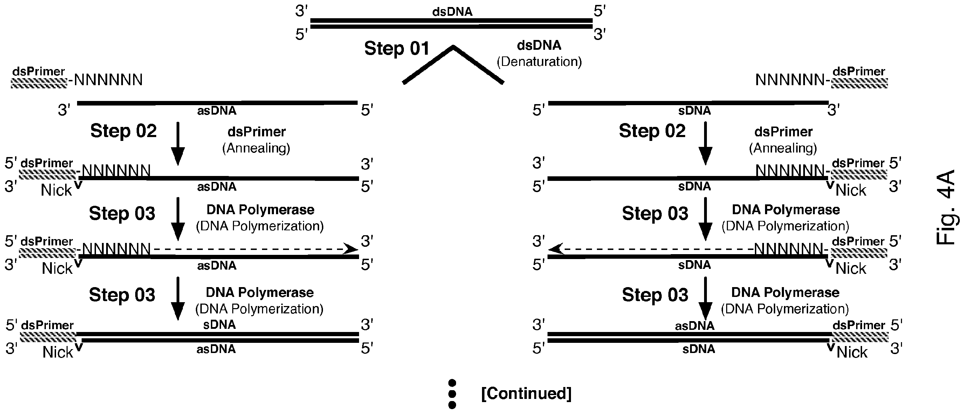

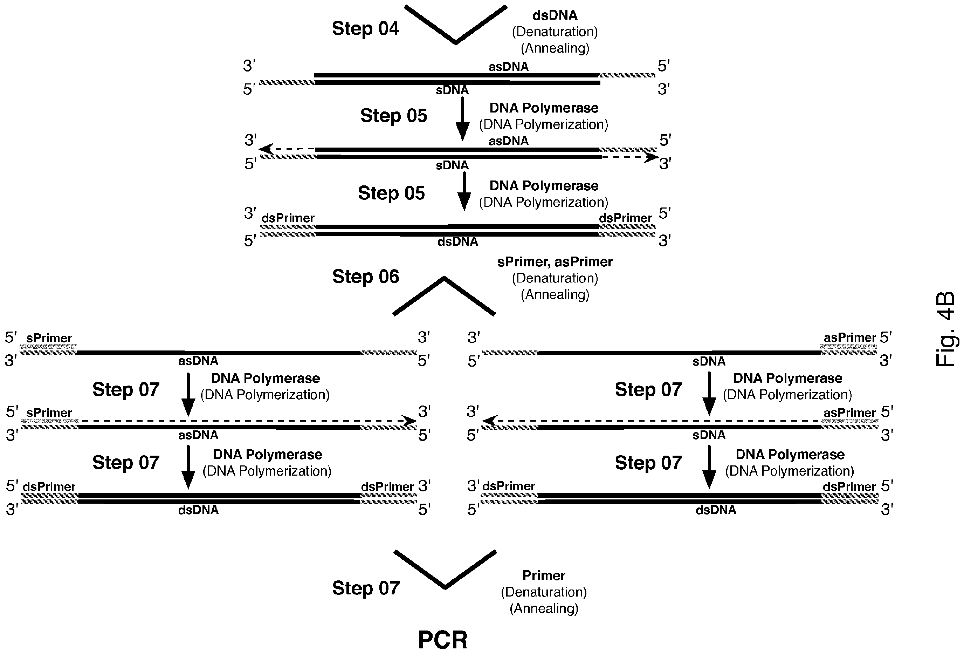

**Figure 4. Design 2 dsDNA Amplification from dsDNA:**

- The molecular structure of the dsPrimer contains a double-stranded DNA primer site and a degenerate oligomer (e.g. hexamer) at the 3'-end.

  • Step 01: dsDNA template denatured by heat or some other treatment.
  • Step 02: dsPrimer molecules hybridizing to the 3'-ends of the both strands.
  • Step 03: DNA polymerase with 5'-3' polymerization capability filling next to the degenerate sequences.
  • *Note: A DNA polymerase with 3'-5' exonuclease activity can perform dsPrimer silencing activity at this step.*
  • Step 04: Denaturing all fragments by heat or some other treatment.
  • Step 05a: asDNA with the dsPrimer upper site and sDNA with the dsPrimer upper site hybridizing to each other
  • *Note: Also sDNA and asDNA of the original template strands hybridized to each other - Since there is no primer site, there is no reaction.*
  • *Note: dsPrimer fragment with degraded degenerate sequences - Since no template, no reaction.*
  • Step 05b: DNA polymerase with 5'-3' polymerization capability filling the complementary sequences of primer sites.
  • Step 06: dsDNA denatured by heat or some other treatment.
  • Step 07: PCR reaction with a single primer. A single primer added to the solution to bind the primer sites at the 3'-end of the both sDNA and asDNA strands. DNA polymerase with 5'-3' polymerization capability filling the complementary strands.

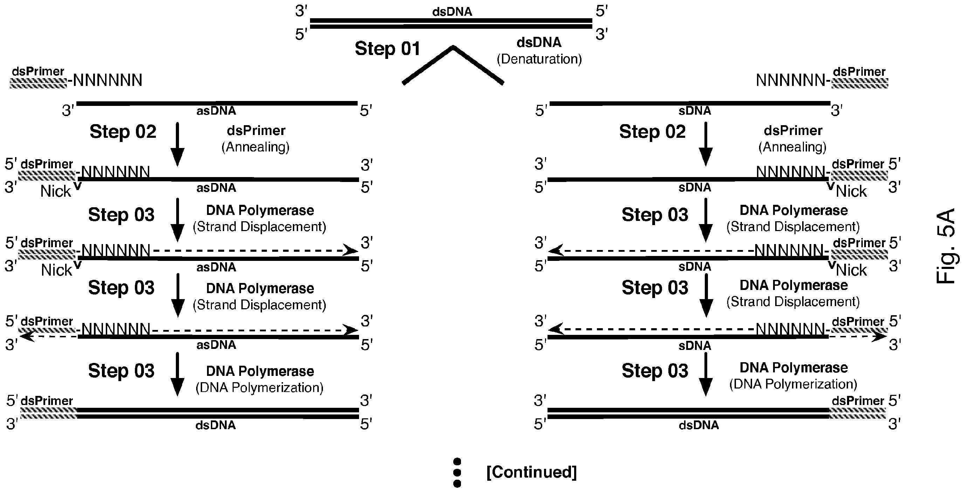

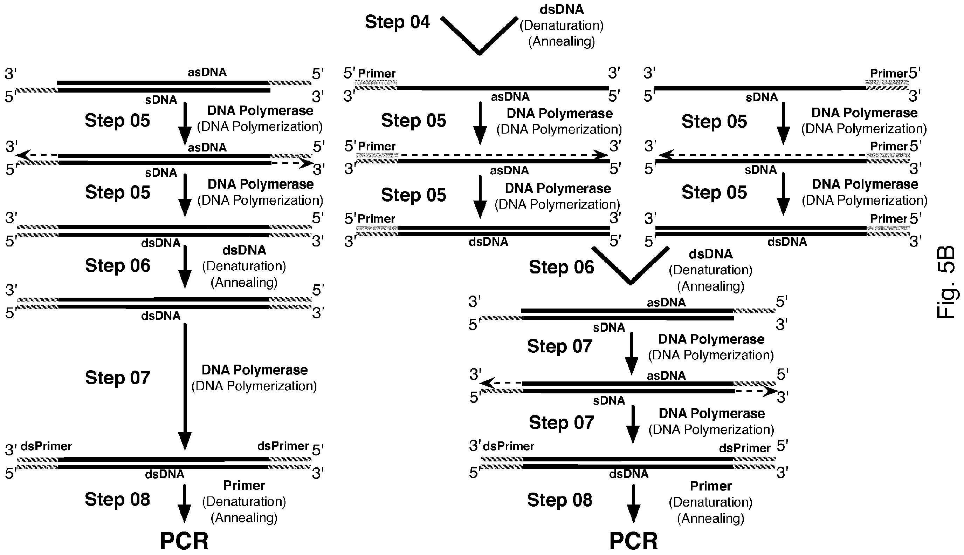

**Figure 5. Design 3 dsDNA Amplification from dsDNA (Strand Displacement):**

- The molecular structure of the dsPrimer contains a double-stranded DNA primer site and a degenerate oligomer (e.g. hexamer) at the 3'-end.

  • Step 01: dsDNA template denatured by heat or some other treatment.
  • Step 02: dsPrimer molecules hybridizing to the 3'-ends of the both strands.
  • Step 03a: DNA polymerase with 5'-3' polymerization capability filling next to the degenerate sequences.
  • Step 03b: DNA polymerase with both 5'-3' polymerization and strand displacement (or nick translation) activity replacing the lower part of the primer.
  • *Note: A single DNA polymerase with both 5'-3' polymerization and strand displacement (or nick translation) capability can perform those two steps (03a and 03b) at the same time.*
  • *Note: A DNA polymerase with 3'-5' exonuclease activity can perform dsPrimer silencing at this step.*
  • Step 04: Denaturing all fragments by heat or some other treatment.
  • Step 05a: asDNA with the dsPrimer upper site and sDNA with the dsPrimer upper site hybridizing to each other.

At the same time single primers hybridizing to the remaining DNA strands.

- Step 05b: DNA polymerase with 5'-3' polymerization capability filling the gaps.
- Step 06: dsDNA denatured by heat or some other treatment.
- Step 07a: asDNA with the dsPrimer upper site and sDNA with the dsPrimer upper site hybridizing to each other.
- Step 07b: DNA polymerase with 5'-3' polymerization capability filling the gaps.
- Step 08: PCR reaction with a single primer. Single primer added to the solution to bind the primer sites at the 3'-end of the both sense and antisense strands. DNA polymerase with 5'-3' polymerization capability filling the complementary strands.

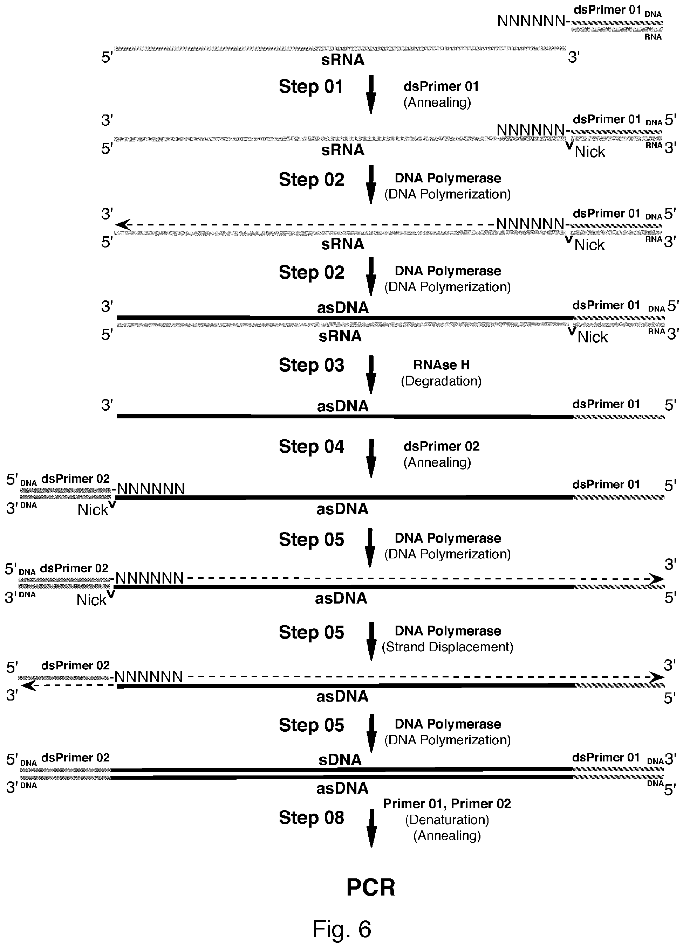

**Figure 6. Design 4 dsDNA Amplification from sRNA (Initial RNase H):**

- The molecular structure of the dsPrimer 01 contains a primer site with a degenerate oligomer (e.g. hexamer) at the 3'-end of the DNA strand and the complimentary strand is RNA.
- The molecular structure of the dsPrimer 02 contains a double-stranded DNA primer site (different from dsPrimer 01) and a degenerate oligomer (e.g. hexamer) at the 3'-end.

- Step 01: dsPrimer 01 hybridizing to the 3'-end of the single-stranded sRNA template.
- Step 02: DNA polymerase with 5'-3' polymerization and reverse transcription capability filling next to the degenerate sequences.
- *Note: A DNA polymerase with 3'-5' exonuclease activity can perform dsPrimer silencing at this step.*
- Step 03: RNase H degradation of the DNA hybridized RNA strands.
- Step 04: dsPrimer 02 hybridizing to the 3'-end of the single-stranded asDNA.
- Step 05a: DNA polymerase with 5'-3' polymerization capability filling next to the degenerate sequences.
- Step 05b: DNA polymerase with both 5'-3' polymerization and strand displacement (or nick translation) activity replacing the lower part of the primer.
- *Note: A single polymerase with both 5'-3' polymerization and strand displacement (or nick translation) capability can perform those two steps (05a and 05b) at the same time.*
- *Note: A DNA polymerase with 3'-5' exonuclease activity can perform dsPrimer silencing at this step.*
- Step 06: PCR reaction with two different primers.

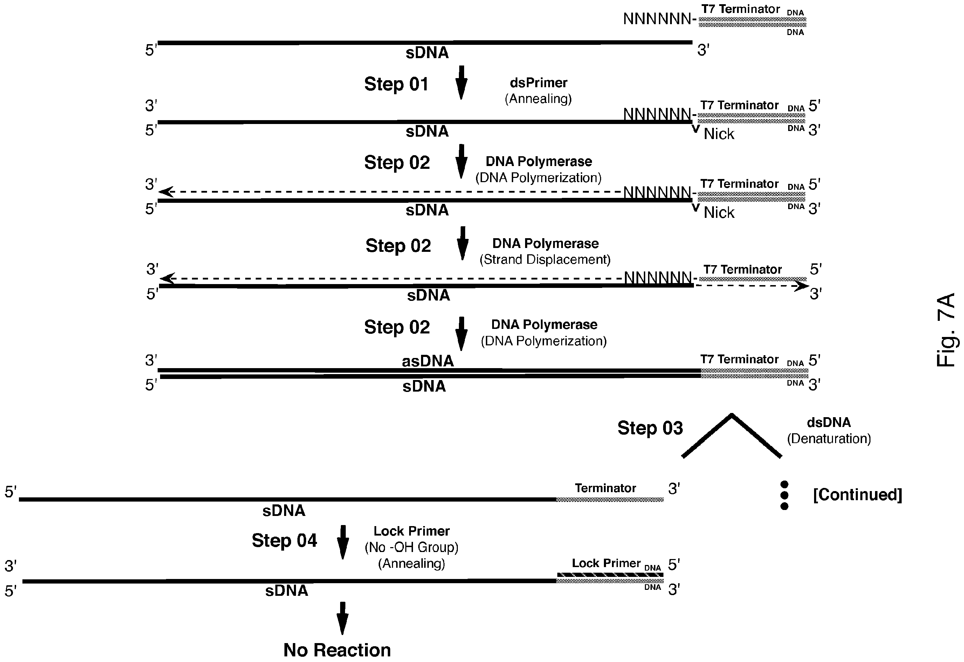

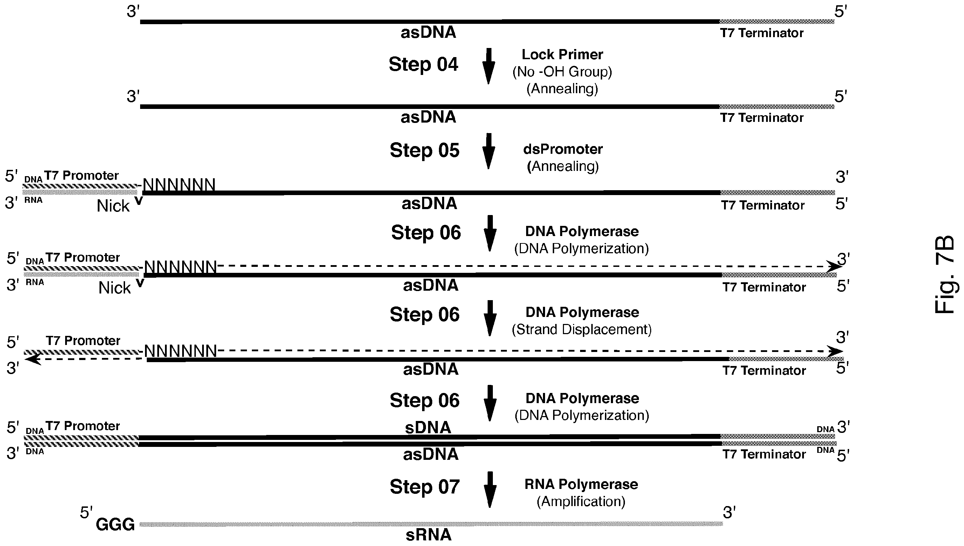

**Figure 7. Design 5 sRNA Amplification from sDNA (Terminator):**

- The molecular structure of the dsTerminator is a double-stranded DNA containing terminator site of T7 promoter (could be any other DNA-dependent RNA polymerase promoter) with a degenerate oligomer (e.g. hexamer) at the 3'-end.
- The molecular structure of the dsPromoter contains a T7 promoter site (could be any other DNA-dependent RNA polymerase promoter) with a degenerate oligomer (e.g. hexamer) at the 3'-end of the DNA strand and the complimentary strand is RNA.
- The molecular structure of the lock primer contains a single-stranded DNA primer region (complementary to the dsTerminator site at the 5'-end of the sDNA) lacking any hydroxyl (-OH) group at its 3'-end that inhibits chain elongation.

- Step 01: dsTerminator hybridizing to the 3'-end of the single-stranded sDNA template.
- Step 02a: DNA polymerase with 5'-3' polymerization capability filling next to the degenerate sequences.
- Step 02b: A polymerase with both 5'-3' polymerization and strand displacement (or nick translation) activity replacing the lower part of the terminator.
- *Note: A single DNA polymerase with both 5'-3' polymerization and strand displacement (or nick translation) capability can perform those two steps (02a and 02b) at the same time.*
- *Note: A DNA polymerase with 3'-5' exonuclease activity can perform dsTerminator silencing at this step.*
- Step 03: Denaturing all fragments by heat or some other treatment.
- Step 04: sDNA at its terminator site at the 3'-end hybridizing with a complementary lock primer.
- Step 05: dsPromoter hybridizing to the 3'-end of the single-stranded asDNA template.
- Step 06a: DNA polymerase with 5'-3' polymerization capability filling next to the degenerate sequences.
- Step 06b: DNA polymerase with both 5'-3' polymerization and strand displacement (or nick translation) activity replacing the lower part of the promoter.

- *Note: A single DNA polymerase with both 5'-3' polymerization and strand displacement (or nick translation) capability can perform those two steps (06a and 06b) at the same time.*
- *Note: A DNA polymerase with 3'-5' exonuclease activity can perform dsPromoter silencing at this step.*
- Step 07: RNA polymerase binding to promoter part and transcribing the antisense strand into sRNA with three guanine bases at the 5'-end due to T7 promoter's feature.
- *Note: RNA polymerase stops its activity at the T7 terminator.*

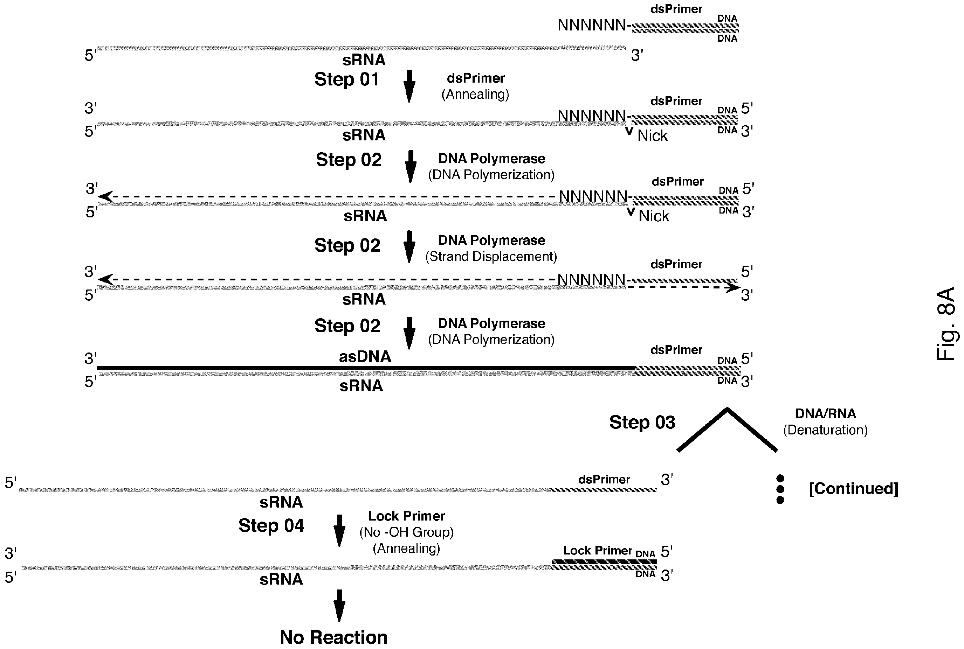

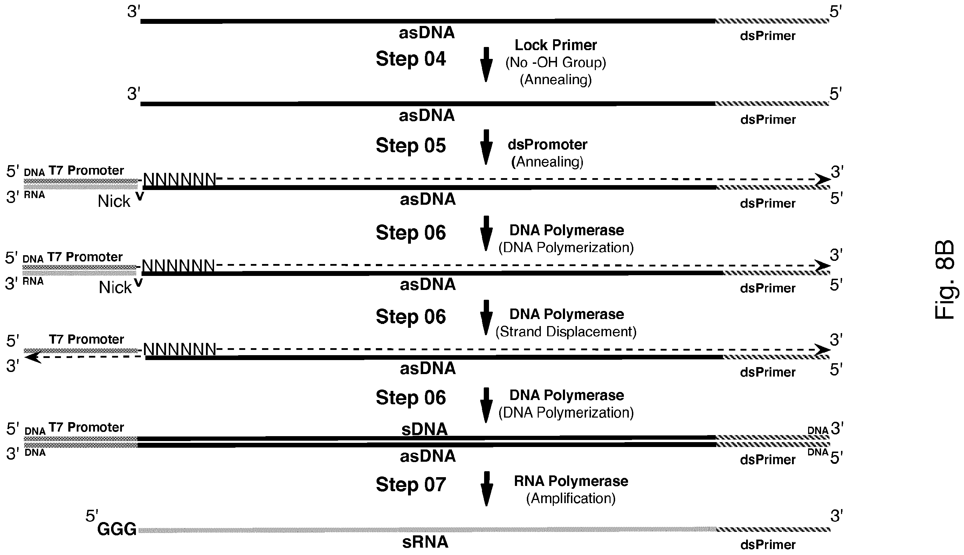

**Figure 8. Design 6 sRNA Amplification from sRNA (Single Primer Region):**

- The molecular structure of the dsPrimer is a double-stranded DNA containing a primer site with a degenerate oligomer (e.g. hexamer) at the 3'-end.
- The molecular structure of the dsPromoter contains a T7 promoter site (could be any other DNA-dependent RNA polymerase promoter) with a degenerate oligomer (e.g. hexamer) at the 3'-end of the DNA strand and the complimentary strand is RNA.
- The molecular structure of lock primer contains a single-stranded DNA primer region (complementary to the dsPrimer site at the 5'-end of the sDNA) lacking any hydroxyl (-OH) group at its 3'-end that inhibits chain elongation.

- Step 01: dsPrimer hybridizing to the 3'-end of the single-stranded sRNA template.
- Step 02a: DNA polymerase with 5'-3' polymerization and reverse transcription capability filling next to the degenerate sequences.
- Step 02b: DNA polymerase with both 5'-3' polymerization and strand displacement (or nick translation) activity replacing the lower part of the primer.
- *Note: A single DNA polymerase with both 5'-3' polymerization, reverse transcription and strand displacement (or nick translation) capability can perform those two steps (02a and 02b) at the same time.*
- *Note: A DNA polymerase with 3'-5' exonuclease activity can perform dsPrimer silencing at this step.*
- Step 03: Denaturing all fragments by heat or some other treatment.
- Step 04: sRNA at its terminator site at the 3'-end hybridizing with a complementary lock primer.
- Step 05: dsPromoter hybridizing to the 3'-end of the single-stranded asDNA template.
- Step 06a: DNA polymerase with 5'-3' polymerization capability filling next to the degenerate sequences.
- Step 06b: DNA polymerase with both 5'-3' polymerization and strand displacement (or nick translation) activity replacing the lower part of the promoter.
- *Note: A single DNA polymerase with both 5'-3' polymerization and strand displacement (or nick translation) capability can perform those two steps (06a and 06b) at the same time.*
- *Note: A DNA polymerase with 3'-5' exonuclease activity can perform dsPromoter silencing at this step.*
- Step 07: RNA polymerase binding to promoter part and transcribing the antisense strand into sRNA with three guanine bases at the 5'-end due to T7 promoter's feature.

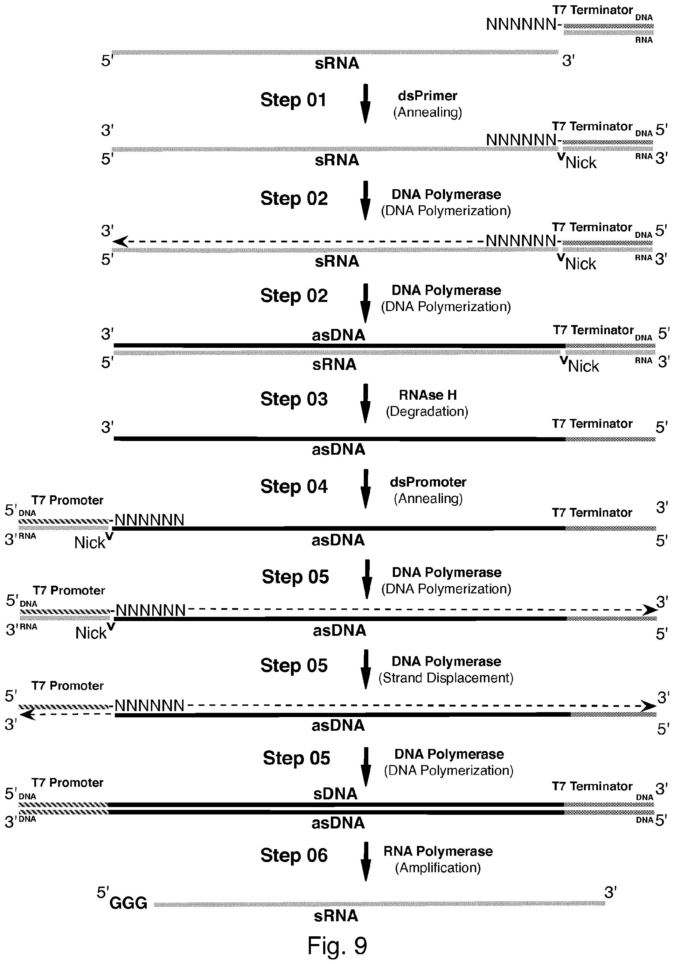

**Figure 9. Design 7 sRNA Amplification from sRNA (Initial RNase H + Terminator):**

- The molecular structure of the dsTerminator contains a terminator site of the T7 promoter (could be any other DNA-dependent RNA polymerase promoter) with a degenerate oligomer (e.g. hexamer) at the 3'-end of the DNA strand and the complimentary strand is RNA.
- The molecular structure of the dsPromoter contains a T7 promoter site (could be any other DNA-dependent RNA polymerase promoter) with a degenerate oligomer (e.g. hexamer) at the 3'-end of the DNA strand and the complimentary strand is RNA.

- Step 01: dsTerminator hybridizing to the 3'-end of the single-stranded sRNA template.
- Step 02: DNA polymerase with 5'-3' polymerization and reverse transcription capability filling next to the degenerate sequences.
- *Note: A DNA polymerase with 3'-5' exonuclease activity can perform dsTerminator silencing at this step.*
- Step 03: RNase H degradation of the DNA hybridized RNA strands.
- Step 04: dsPromoter hybridizing to the 3'-end of the single-stranded asDNA template.
- Step 05a: DNA polymerase with 5'-3' polymerization capability filling next to the degenerate sequences.

- Step 05b: A polymerase with both 5'-3' polymerization and strand displacement (or nick translation) activity replacing the lower part of the promoter.
- *Note: A single DNA polymerase with both 5'-3' polymerization and strand displacement (or nick translation) capability can perform those two steps (05a and 05b) at the same time.*
- *Note: A DNA polymerase with 3'-5' exonuclease activity can perform dsPromoter silencing at this step.*
- Step 06: RNA polymerase binding to promoter part and transcribing the antisense strand into sRNA with three guanine bases at the 5'-end due to T7 promoter's feature.
- *Note: RNA polymerase stops its activity at the T7 terminator.*

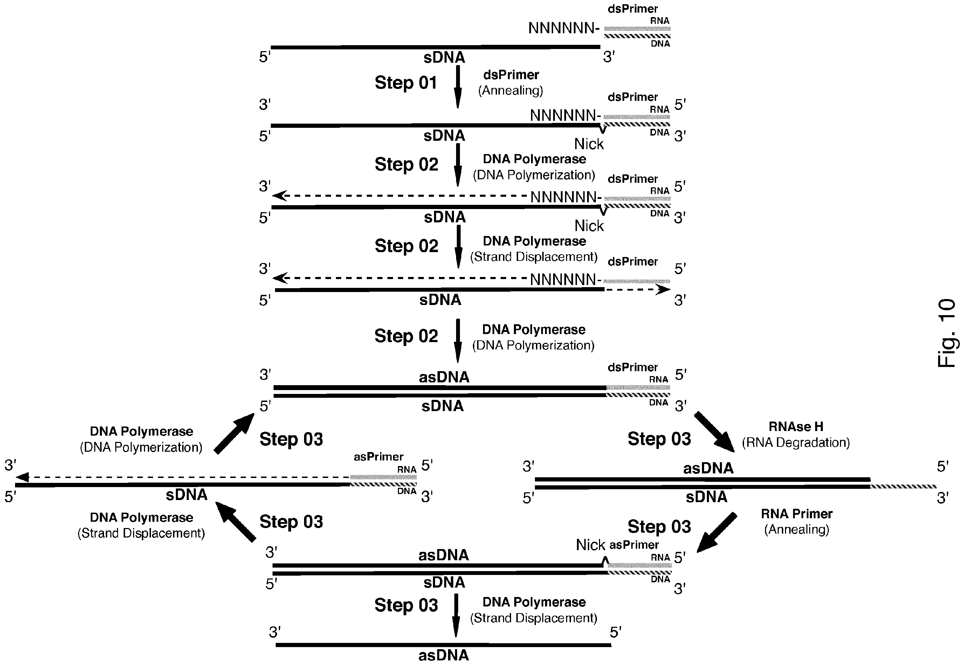

**Figure 10. Design 8 asDNA Amplification from sDNA (Isothermal + Single Primer):**

- The molecular structure of the dsPrimer contains a primer site with a degenerate oligomer (e.g. hexamer) at the 3'-end of the RNA strand and the complimentary strand is DNA.

- Step 01: dsPrimer hybridizing to the 3'-end of the single-stranded sDNA template.
- Step 02a: DNA polymerase with 5'-3' polymerization capability filling next to the degenerate sequences.
- Step 02b: A polymerase with 5'-3' polymerization, reverse transcription and strand displacement (or nick translation) activity replacing the lower part of the primer.
- *Note: Also another denaturation method such as heat or some other treatments can be use to replace the lower part of the primer.*
- *Note: A single DNA polymerase with 5'-3' polymerization, and reverse transcription and strand displacement (or nick translation) capability can perform those two steps (02a and 02b) at the same time.*
- *Note: A DNA polymerase with 3'-5' exonuclease activity can perform dsPrimer silencing at this step.*
- Step 03a: RNase H degradation of the DNA hybridized RNA part at the 3'-end of sDNA strand.
- Step 03b: Single-stranded RNA primer hybridizing to the 3'-end of sDNA.
- Step 03c: DNA polymerase with both 5'-3' polymerization and strand displacement activities replacing the asDNA by polymerization of single-stranded RNA primer.
- Step 03d: asDNA obtained (continuously).
- Step 03e: DNA polymerase with both 5'-3' polymerization and strand displacement activities continues the polymerization of single-stranded RNA primer after replacing the asDNA.
- Note: Step 03 is a continuous cycle. After Step 03e, the same cycle starts again with Step 03a.

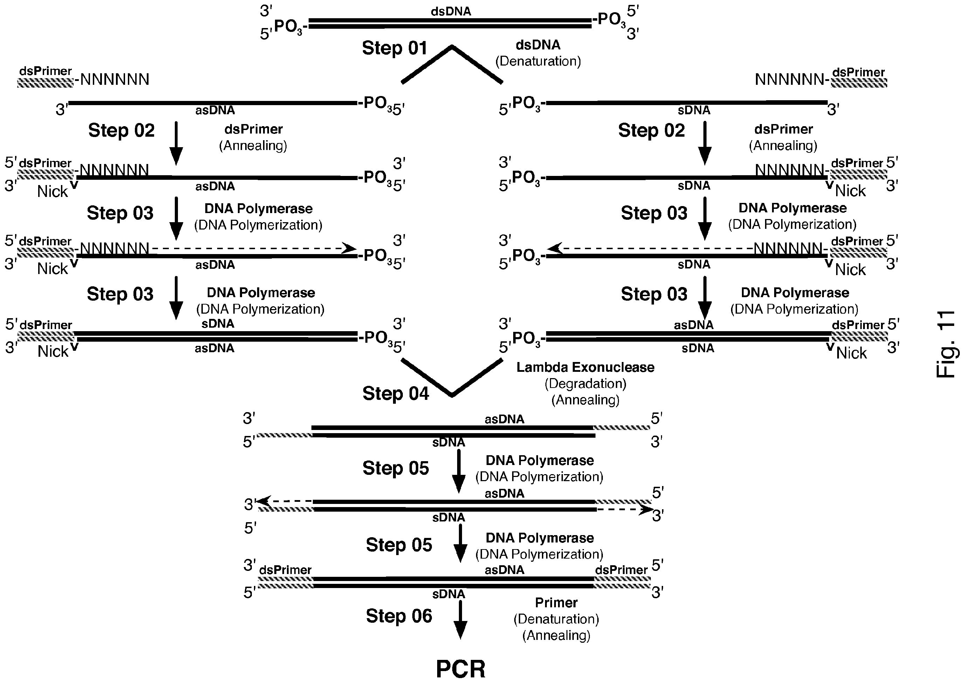

**Figure 11. Design 9 dsDNA Amplification from dsDNA (Lambda):**

- The molecular structure of the standard dsPrimer contains a double-stranded DNA primer site and a degenerate oligomer (e.g. hexamer) at the 3'-end with no 5'-end phosphorylated DNA strands.
- As an option, dsPrimer contains 5'-end phosphorylated DNA (complementary to the primer site adjacent to the 3'-end degenerate sequence) can be used as an alternative instead of the standard one described above.
- The initial template is sheared, blunted and 5'-ends phosphorylated to give double-stranded genomic DNA (gDNA) ready to use for DNA sequencing devices.

- Step 01: dsDNA template with 5'-ends phosphorylated denatured by heat or some other treatment.
- Step 02: dsPrimer molecules hybridizing to the 3'-ends of the both strands.
- Step 03: DNA polymerase with 5'-3' polymerization capability filling next to the degenerate sequences.
- *Note: A DNA polymerase with 3'-5' exonuclease activity can perform dsPrimer silencing activity at this step.*
- Step 04a: Lambda exonuclease degrades 5'-end phosphorylated sDNA and asDNA simultaneously until it reaches to the nick site.
- *Note: If a dsPrimer containing 5'-end phosphorylated DNA strand (complementary to the primer site adjacent to the 3'-end degenerate sequence) is used, the 5'-end phosphorylated DNA strand of the dsPrimer will also be degraded by Lambda exonuclease.*
- Step 04b: Denaturing all fragments by heat or some other treatment.
- Step 04c: asDNA with the dsPrimer upper site and sDNA with the dsPrimer upper site hybridizing to each other.
- Step 05: DNA polymerase with 5'-3' polymerization capability filling the complementary sequences of primer sites.

- Step 06: PCR reaction with a single primer. A single primer added to the solution binds the primer sites at the 3'-end of the both sense and antisense strands. DNA polymerase with 5'-3' polymerization capability filling the complementary strands.

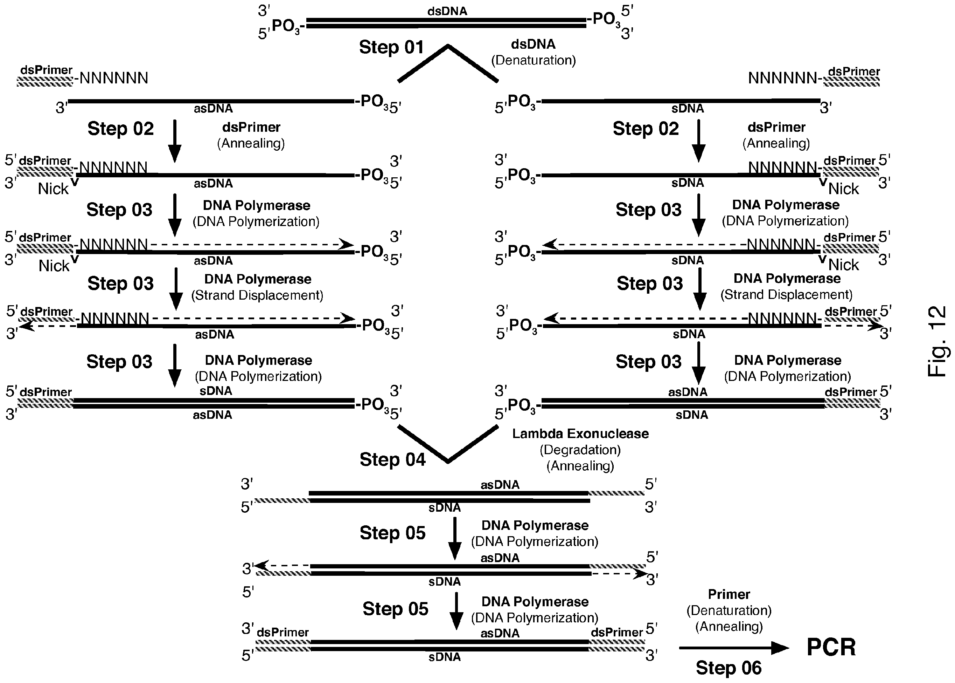

**Figure 12. Design 10 dsDNA Amplification from dsDNA (Lambda + Strand Displacement):**

- The molecular structure of the standard dsPrimer contains a double-stranded DNA primer site and a degenerate oligomer (e.g. hexamer) at the 3'-end with no 5'-end phosphorylated DNA strands.
- The initial template is sheared, blunted ended and 5'-ends phosphorylated to give double-stranded genomic DNA (gDNA) ready to use for DNA sequencing devices.

  - Step 01: dsDNA template with 5'-ends phosphorylated denatured by heat or some other treatment.
  - Step 02: dsPrimer molecules hybridizing to the 3'-ends of the both strands.
  - Step 03a: DNA polymerase with 5'-3' polymerization capability filling next to the degenerate sequences.
  - Step 03b: DNA polymerase with both 5'-3' polymerization and strand displacement (or nick translation) activity replacing the lower part of the primer.
  - *Note: A single DNA polymerase with both 5'-3' polymerization and strand displacement (or nick translation) capability can perform those two steps (03a and 03b) at the same time.*
  - *Note: A DNA polymerase with 3'-5' exonuclease activity can perform dsPrimer silencing at this step.*
  - Step 04a: Lambda exonuclease degrades 5'-end phosphorylated sDNA and asDNA simultaneously.
  - Step 04b: Denaturing all fragments by heat or some other treatment.
  - Step 04c: asDNA with the dsPrimer upper site and sDNA with the dsPrimer upper site hybridizing to each other.
  - Step 05: DNA polymerase with 5'-3' polymerization capability filling the complementary sequences of primer sites.
  - Step 06: PCR reaction with a single primer. A single primer added to the solution binds the primer sites at the 3'-end of the both sense and antisense strands. DNA polymerase with 5'-3' polymerization capability filling the complementary strands.

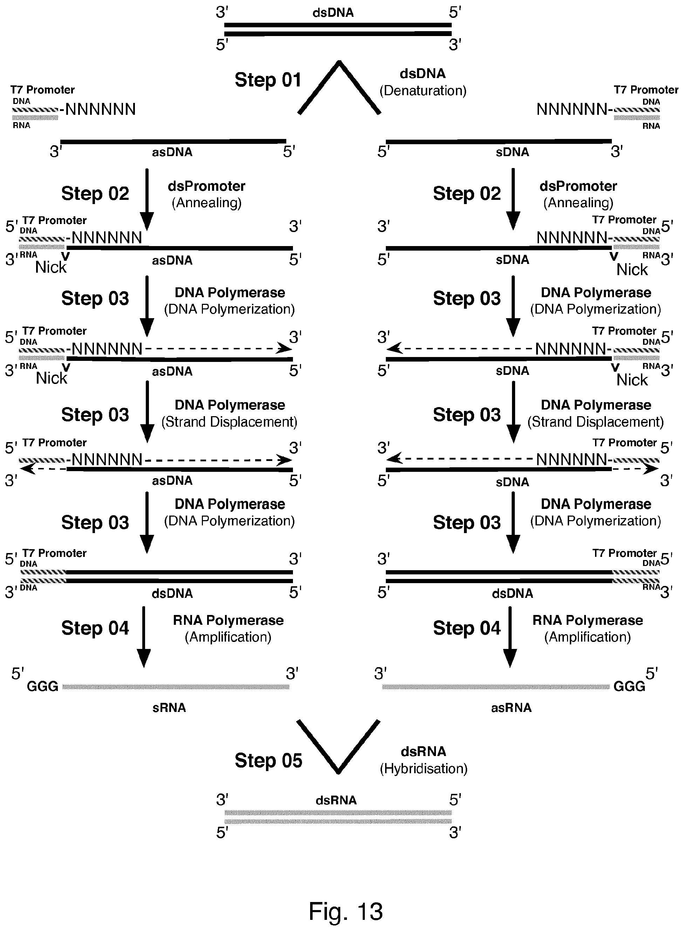

**Figure 13. Design 11 dsRNA Amplification from dsDNA (Strand Displacement):**

- The molecular structure of the dsPromoter contains a T7 promoter site (could be any other DNA-dependent RNA polymerase promoter) with a degenerate oligomer (e.g. hexamer) at the 3'-end of the DNA strand and the complimentary strand is RNA.

  - Step 01: dsDNA template denatured by heat or some other treatment.
  - Step 02: dsPromoter hybridizing to the 3'-ends of the both strands.
  - Step 03a: DNA polymerase with 5'-3' polymerization capability filling next to the degenerate sequences.
  - Step 03b: DNA polymerase with both 5'-3' polymerization and strand displacement (or nick translation) activity replacing the lower part of the promoter.
  - *Note: A single DNA polymerase with both 5'-3' polymerization and strand displacement (or nick translation) capability can perform those two steps (03a and 03b) at the same time.*
  - *Note: A DNA polymerase with 3'-5' exonuclease activity can perform dsPromoter silencing at this step.*
  - Step 04a: RNA polymerase binding to promoter part and transcribing the sense strand into asRNA with three guanine bases at the 5'-end due to T7 promoter's feature.
  - Step 04b: RNA polymerase binding to promoter part and transcribing the antisense strand into sRNA with three guanine bases at the 5'-end due to T7 promoter's feature.
  - Step 05: sRNA and asRNA hybridized to each other and form dsRNA.

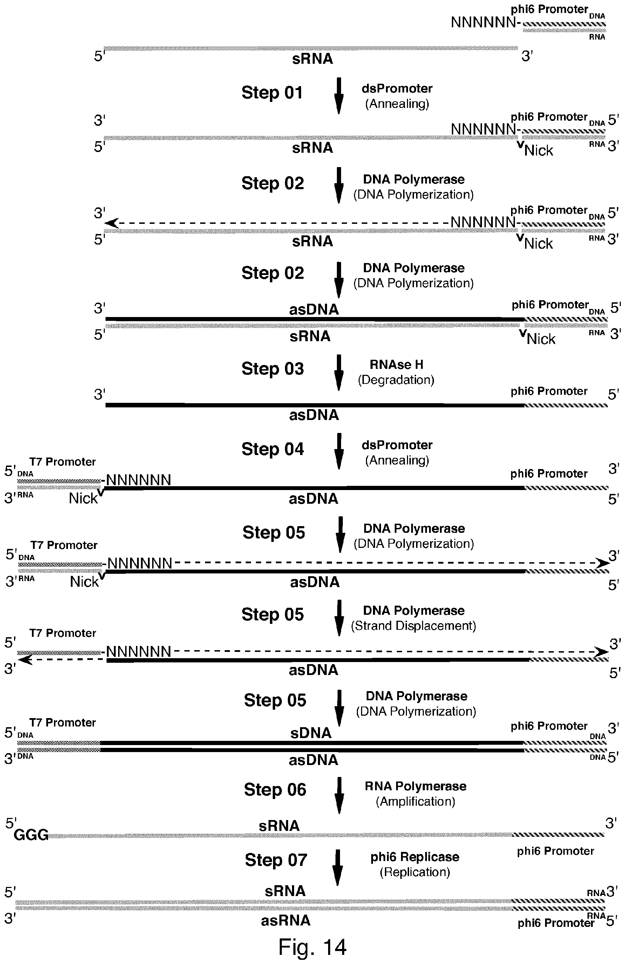

**Figure 14. Design 12 dsRNA Amplification from sRNA (Initial RNase H):**

- The molecular structure of the first dsPromoter contains a phi6 promoter site with a degenerate oligomer (e.g. hexamer) at the 3'-end of the DNA strand, and the complimentary strand is RNA.

- The molecular structure of the second dsPromoter contains a T7 promoter site (or any other DNA-dependent RNA polymerase promoter site) with a degenerate oligomer (e.g. hexamer) at the 3'-end of the DNA strand, and the complimentary strand is RNA.

• Step 01: First dsPromoter molecules hybridizing to the 3'-end of the sRNA template.
• Step 02: DNA polymerase with 5'-3' polymerization capability filling next to the degenerate sequences.
• *Note: A DNA polymerase with 3'-5' exonuclease activity can perform dsPrimer silencing activity at this step.*
• Step 03: RNase H degradation of the DNA hybridized RNA strands.
• Step 04: Second dsPromoter hybridizing to the 3'-end of the single-stranded asDNA.
• Step 05a: DNA polymerase with 5'-3' polymerization capability filling next to the degenerate sequences.
• Step 05b: DNA polymerase with both 5'-3' polymerization and strand displacement (or nick translation) activity replacing the lower part of the promoter.
• *Note: A single DNA polymerase with both 5'-3' polymerization and strand displacement (or nick translation) capability can perform those two steps (05a and 05b) at the same time.*
• *Note: A DNA polymerase with 3'-5' exonuclease activity can perform dsPromoter silencing at this step.*
• Step 06: RNA polymerase binding to T7 promoter part and transcribing the antisense strand into sRNA with three guanine bases at the 5'-end due to T7 promoter's feature.
• Step 07: Phi6 RNA replicase binding to phi6 promoter part and transcribing the antisense strand of the sRNA and form the dsRNA.

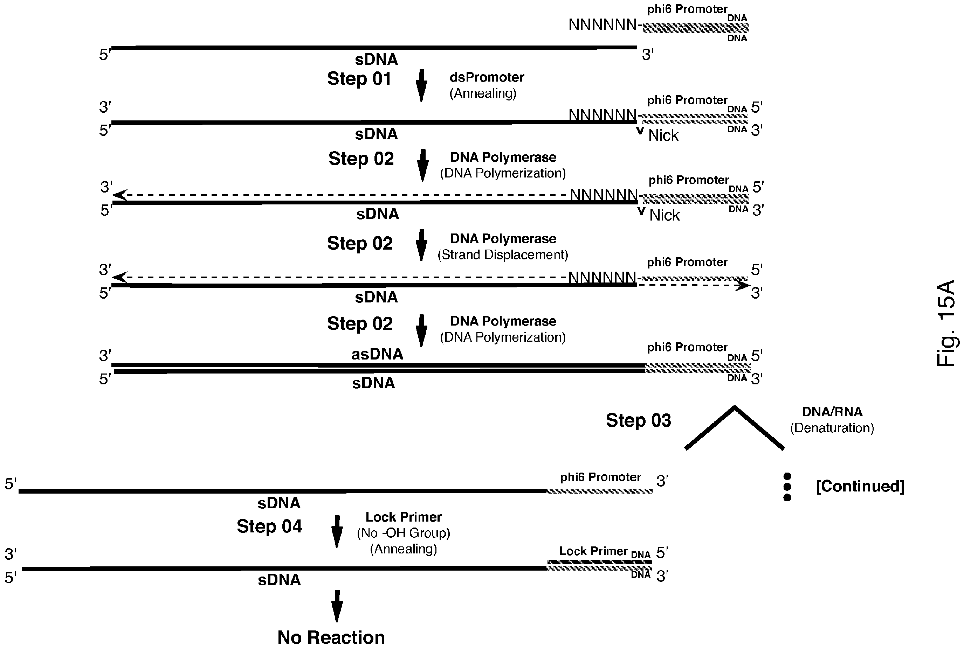

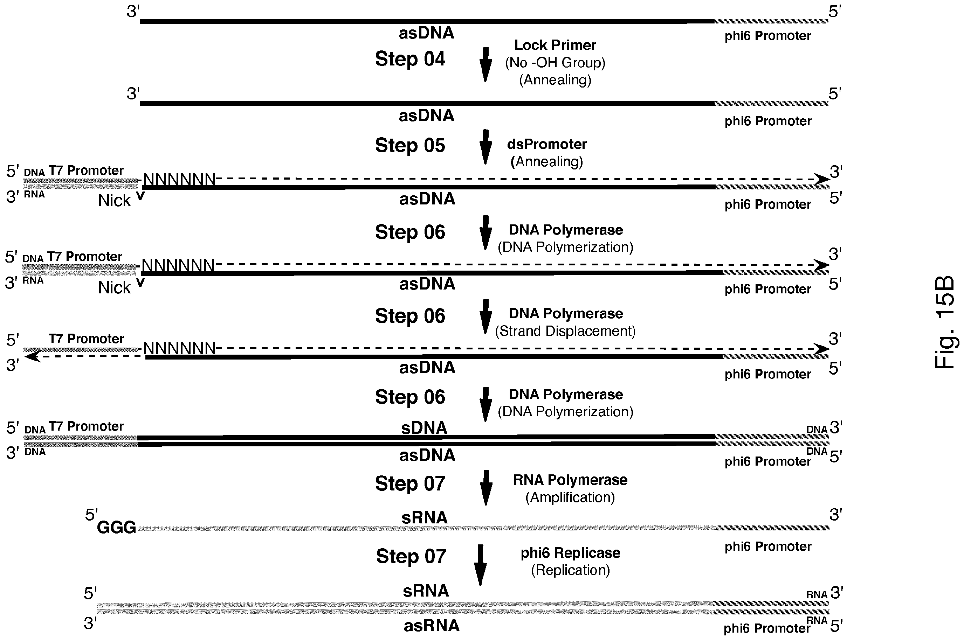

**Figure 15. Design 13 dsRNA Amplification from sDNA:**

- The molecular structure of the first dsPromoter contains a double-stranded DNA phi6 promoter site with a degenerate oligomer (e.g. hexamer) at the 3'-end.
- The molecular structure of the second dsPromoter contains a T7 promoter site (or any other DNA-dependent RNA polymerase promoter site) with a degenerate oligomer (e.g. hexamer) at the 3'-end of the DNA strand, and the complimentary strand is RNA.
- The molecular structure of lock primer contains a single-stranded DNA primer region (complementary to the phi6 promoter site at the 5'-end of the sDNA) lacking any hydroxyl (-OH) group at its 3'-end that inhibits chain elongation.

• Step 01: First dsPromoter molecules hybridizing to the 3'-ends of the sDNA template.
• Step 02a: DNA polymerase with 5'-3' polymerization capability filling next to the degenerate sequences.
• Step 02b: A polymerase with both 5'-3' polymerization and strand displacement (or nick translation) activity replacing the lower part of the terminator.
• *Note: A single DNA polymerase with both 5'-3' polymerization and strand displacement (or nick translation) capability can perform those two steps (02a and 02b) at the same time.*
• *Note: A DNA polymerase with 3'-5' exonuclease activity can perform dsPromoter silencing at this step.*
• Step 03: Denaturing all fragments by heat or some other treatment.
• Step 04: sDNA at its phi6 promoter site at the 3'-end hybridizing with a complementary lock primer.
• *Note: Hybridization of lock primer to the target strand inhibits any polymerization reaction at the binding site.*
• Step 04: Second dsPromoter hybridizing to the 3'-end of the single-stranded asDNA.
• Step 05a: DNA polymerase with 5'-3' polymerization capability filling next to the degenerate sequences.
• Step 05b: DNA polymerase with both 5'-3' polymerization and strand displacement (or nick translation) activity replacing the lower part of the promoter.
• *Note: A single DNA polymerase with both 5'-3' polymerization and strand displacement (or nick translation) capability can perform those two steps (05a and 05b) at the same time.*
• *Note: A DNA polymerase with 3'-5' exonuclease activity can perform dsPromoter silencing at this step.*
• Step 06: RNA polymerase binding to T7 promoter part and transcribing the antisense strand into sRNA with three guanine bases at the 5'-end due to T7 promoter's feature.
• Step 07: Phi6 RNA replicase binding to phi6 promoter part and transcribing the antisense strand of the sRNA and form the dsRNA.

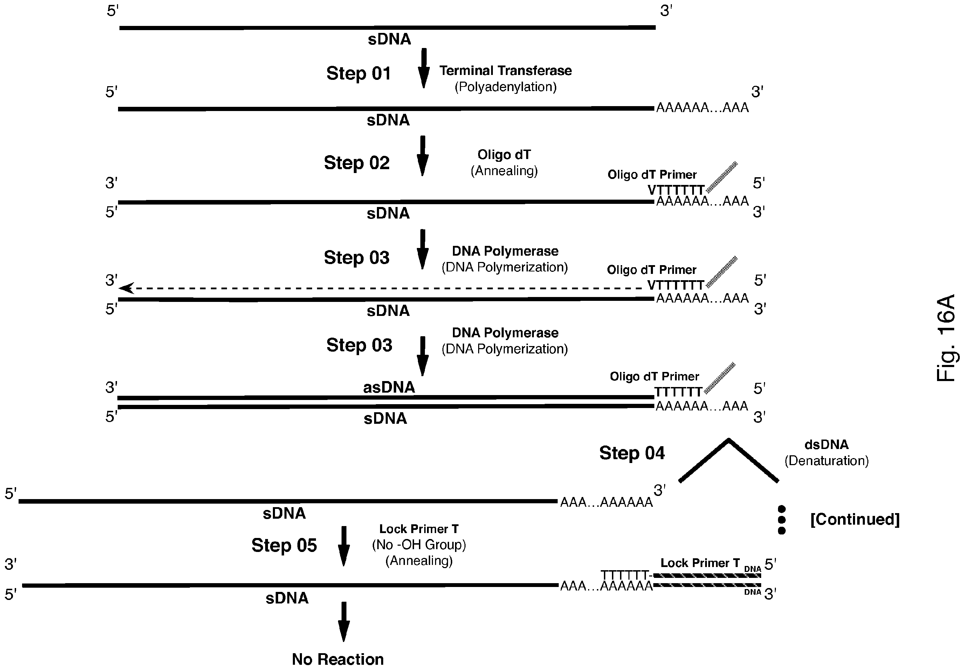

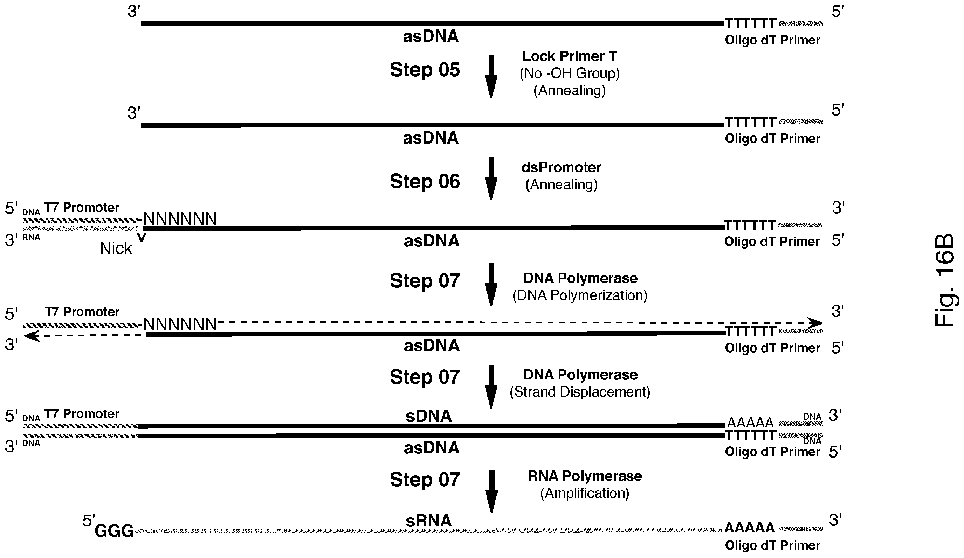

**Figure 16. Design 14 sRNA Amplification from sDNA (Polyadenylation + Oligo dT + Primer Region)**

- The molecular structure of the second dsPromoter contains a T7 promoter site (or any other DNA-dependent RNA polymerase promoter site) with a degenerate oligomer (e.g. hexamer) at the 3'-end of the DNA strand, and the complimentary strand is RNA.
- The molecular structure of oligo dT primer contains six thymine bases with at least one "not thymine" base (adenine, cytosine or guanine) at the 3'-end (VTTTTTT) and a primer region at the 5'-end.
- The molecular structure of lock primer T contains a double-stranded DNA primer site with six thymine bases at the 3'-end. The last T nucleotide at the 3'-end of the lock primer T lacks any hydroxyl group at its 3'-end inhibiting chain elongation.

- Step 01: Terminal transferase adds multiple adenine bases as a poly(A) tail to the 3'-end of the sDNA template.
- Step 02: Oligo dT primer hybridizes to the polyadenylated sDNA template from the at least one "not thymine" base of the original sDNA template and from six recently added adenine bases right next to the last original "not thymine" base at the 3'-end.
- Step 03: DNA polymerase with 5'-3' polymerization capability filling next to the oligo dT Primer sequences.
- Step 04: Denaturing all fragments by heat or some other treatment.
- Step 05: Lock primer T molecules hybridizing to the 3'-end of the sDNA.
- *Note: Hybridization of lock primer T to the target strand inhibits any polymerization reaction at the binding site.*
- Step 06: dsPromoter hybridizing to the 3'-end of the single-stranded asDNA.
- Step 07a: DNA polymerase with 5'-3' polymerization capability filling next to the degenerate sequences.
- Step 07b: DNA polymerase with both 5'-3' polymerization and strand displacement (or nick translation) activity replacing the lower part of the promoter.
- *Note: A single DNA polymerase with both 5'-3' polymerization and strand displacement (or nick translation) capability can perform those two steps (07a and 07b) at the same time.*
- *Note: A DNA polymerase with 3'-5' exonuclease activity can perform dsPromoter silencing at this step.*
- Step 08: RNA polymerase binding to T7 promoter part and transcribing the antisense strand into sRNA with three guanine bases at the 5'-end due to T7 promoter's feature and oligo dT primer site at the 3'-end.

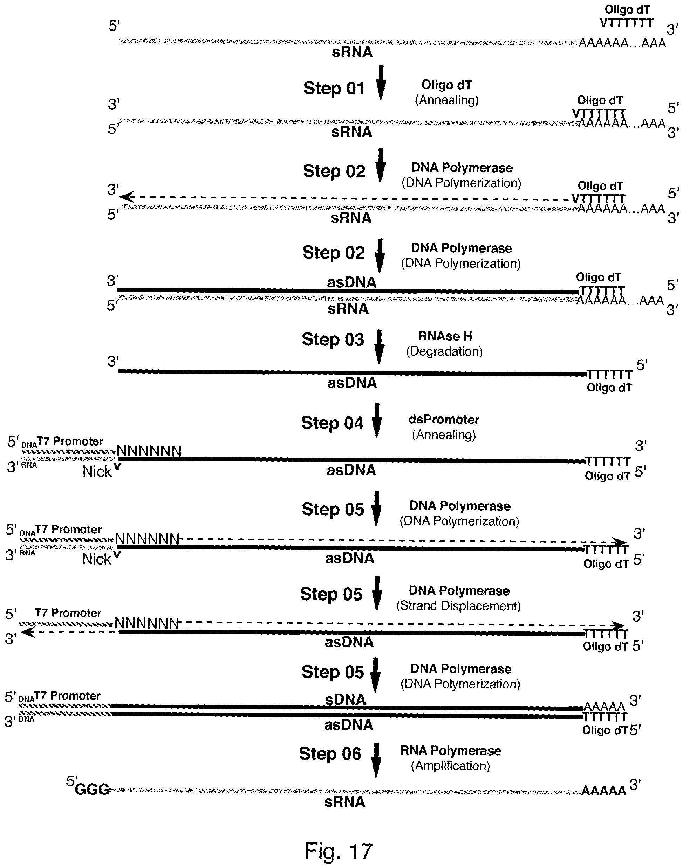

**Figure 17. Design 15 sRNA Amplification from sRNA (Initial RNase H + Oligo dT)**

- The molecular structure of the second dsPromoter contains a T7 promoter site (or any other DNA-dependent RNA polymerase promoter site) with a degenerate oligomer (e.g. hexamer) at the 3'-end of the DNA strand, and the complimentary strand is RNA.
- The molecular structure of oligo dT contains six thymine bases with at least one "not thymine" base (adenine, cytosine or guanine) at the 3'-end (VTTTTTT).
- Initial sRNA template is mRNA with poly(A) tail at the 3'-end.

- Step 01: Oligo dT hybridizes to the poly(A) tail of the sRNA template from the at least one "not thymine" base of the original sRNA template and from six recently added adenine bases right next to the last original "not thymine" base at the 3'-end.
- Step 02: DNA polymerase with 5'-3' polymerization capability filling next to the oligo dT sequences.
- Step 03: RNase H degradation of the DNA hybridized RNA strands.
- Step 04: dsPromoter hybridizing to the 3'-end of the single-stranded asDNA.
- Step 05a: DNA polymerase with 5'-3' polymerization capability filling next to the degenerate sequences.
- Step 05b: DNA polymerase with both 5'-3' polymerization and strand displacement (or nick translation) activity replacing the lower part of the promoter.
- *Note: A single DNA polymerase with both 5'-3' polymerization and strand displacement (or nick translation) capability can perform those two steps (05a and 05b) at the same time.*
- *Note: A DNA polymerase with 3'-5' exonuclease activity can perform dsPromoter silencing at this step.*
- Step 06: RNA polymerase binding to T7 promoter part and transcribing the antisense strand into sRNA with three guanine bases at the 5'-end due to T7 promoter's feature and 6 adenine bases at the 3'-end.

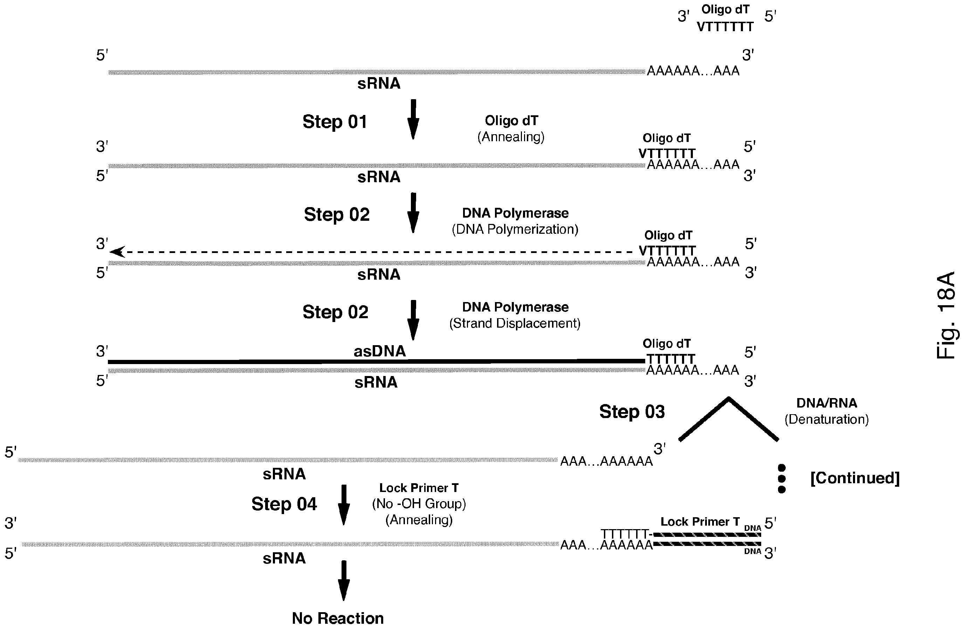

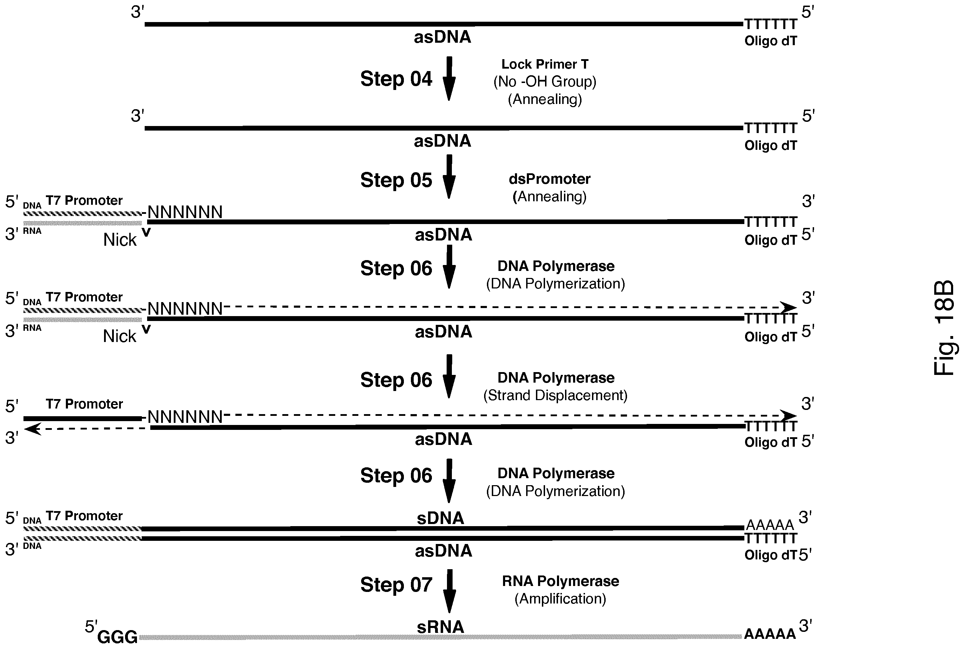

**Figure 18. Design 16 sRNA Amplification from sRNA (Oligo dT)**

- The molecular structure of the second dsPromoter contains a T7 promoter site (or any other DNA-dependent RNA polymerase promoter site) with a degenerate oligomer (e.g. hexamer) at the 3'-end of the DNA strand, and the complimentary strand is RNA.

- The molecular structure of oligo dT contains six thymine bases with at least one "not thymine" base (adenine, cytosine or guanine) at the 3'-end (VTTTTTT).
- The molecular structure of lock primer T contains a double-stranded DNA primer site with six thymine bases at the 3'-end. The last T nucleotide at the 3'-end of the lock primer T lacks any hydroxyl group at its 3'-end inhibiting chain elongation.
- Initial sRNA template is mRNA with poly(A) tail at the 3'-end.

- Step 01: Oligo dT hybridizes to the the poly(A) tail of the sRNA template from the at least one "not thymine" base of the original sRNA template and from six recently added adenine bases right next to the last original "not thymine" base at the 3'-end.
- Step 02: DNA polymerase with 5'-3' polymerization capability filling next to the oligo dT sequences.
- Step 03: Denaturing all fragments by heat or some other treatment.
- Step 04: Lock primer T hybridizing to the 3'-end of the sRNA.
- *Note: Hybridization of lock primer T to the target strand inhibits any polymerization reaction at the binding site.*
- Step 05: dsPromoter hybridizing to the 3'-end of the single-stranded asDNA.
- Step 06a: DNA polymerase with 5'-3' polymerization capability filling next to the degenerate sequences.
- Step 06b: DNA polymerase with both 5'-3' polymerization and strand displacement (or nick translation) activity replacing the lower part of the promoter.
- *Note: A single DNA polymerase with both 5'-3' polymerization and strand displacement (or nick translation) capability can perform those two steps (06a and 06b) at the same time.*
- *Note: A DNA polymerase with 3'-5' exonuclease activity can perform dsPromoter silencing at this step.*
- Step 07: RNA polymerase binding to T7 promoter part and transcribing the antisense strand into sRNA with three guanine bases at the 5'-end due to T7 promoter's feature and 6 adenine bases at the 3'-end.

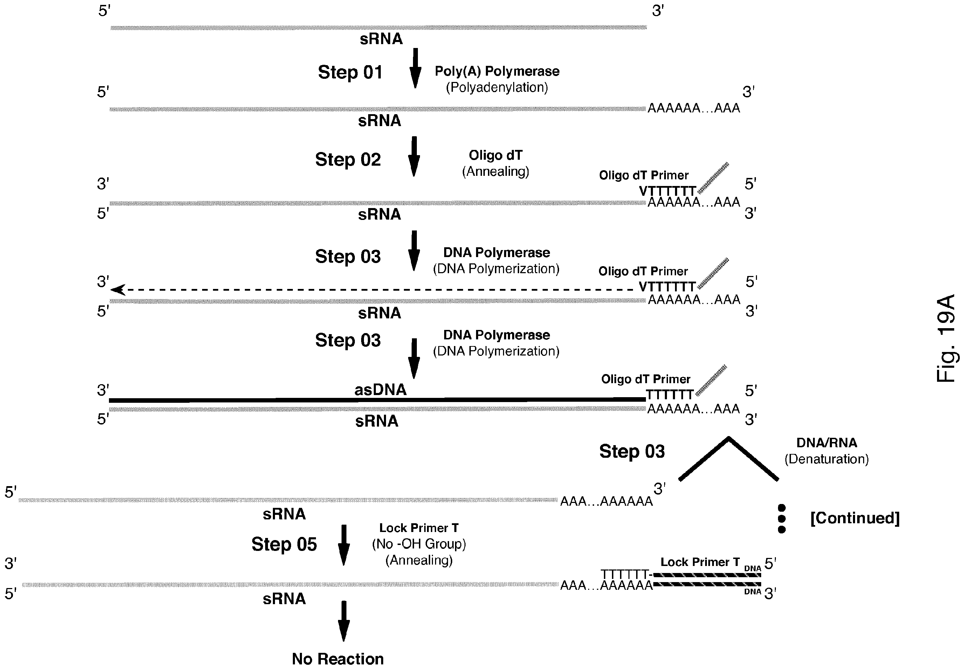

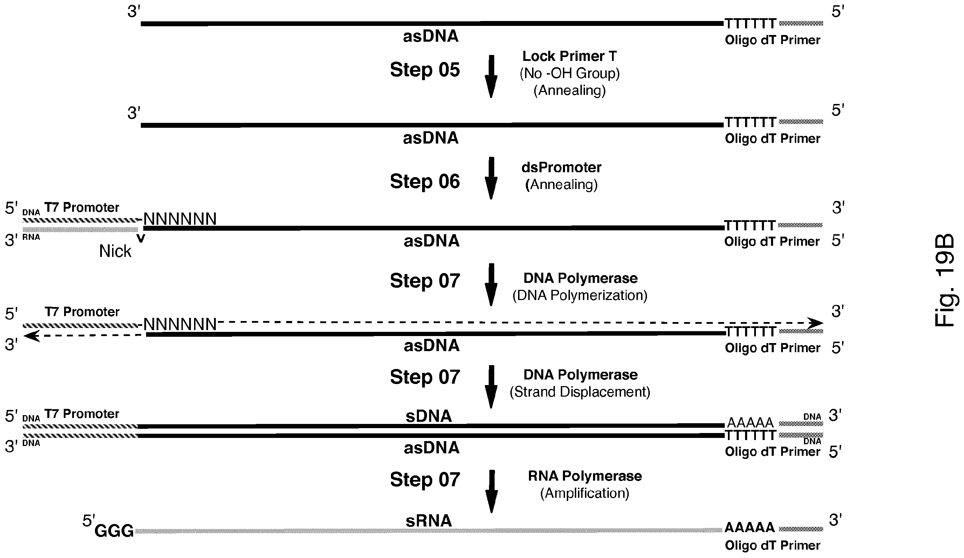

**Figure 19. Design 17 sRNA Amplification from sRNA (Polyadenylation + Oligo dT + Primer Region)**

- The molecular structure of the second dsPromoter contains a T7 promoter site (or any other DNA-dependent RNA polymerase promoter site) with a degenerate oligomer (e.g. hexamer) at the 3'-end of the DNA strand, and the complimentary strand is RNA.
- The molecular structure of oligo dT primer contains six thymine bases with at least one "not thymine" base (adenine, cytosine or guanine) at the 3'-end (VTTTTTT) and a primer site at the 5'-end.
- The molecular structure of lock primer T contains a double-stranded DNA primer site with six thymine bases at the 3'-end. The last T nucleotide at the 3'-end of the lock primer T lacks any hydroxyl group at its 3'-end inhibiting chain elongation.

- Step 01: Poly(A) polymerase adds multiple adenine bases as a poly(A) tail to the 3'-end of the sRNA template.
- Step 02: Oligo dT Primer hybridizes to the polyadenylated sRNA template from the at least one "not thymine" base of the original sRNA template and from six recently added adenine bases right next to the last original "not thymine" base at the 3'-end.
- Step 03: DNA polymerase with 5'-3' polymerization capability filling next to the oligo dT Primer sequences.
- Step 04: Denaturing all fragments by heat or some other treatment.
- Step 05: Lock primer T hybridizing to the 3'-end of the sRNA.
- *Note: Hybridization of lock primer T to the target strand inhibits any polymerization reaction at the binding site.*
- Step 06: dsPromoter hybridizing to the 3'-end of the single-stranded asDNA.
- Step 07a: DNA polymerase with 5'-3' polymerization capability filling next to the degenerate sequences.
- Step 07b: DNA polymerase with both 5'-3' polymerization and strand displacement (or nick translation) activity replacing the lower part of the promoter.
- *Note: A single DNA polymerase with both 5'-3' polymerization and strand displacement (or nick translation) capability can perform those two steps (07a and 07b) at the same time.*
- *Note: A DNA polymerase with 3'-5' exonuclease activity can perform dsPromoter silencing at this step.*
- Step 08: RNA polymerase binding to T7 promoter part and transcribing the antisense strand into sRNA with three guanine bases at the 5'-end due to T7 promoter's feature and oligo dT primer site at the 3'-end.

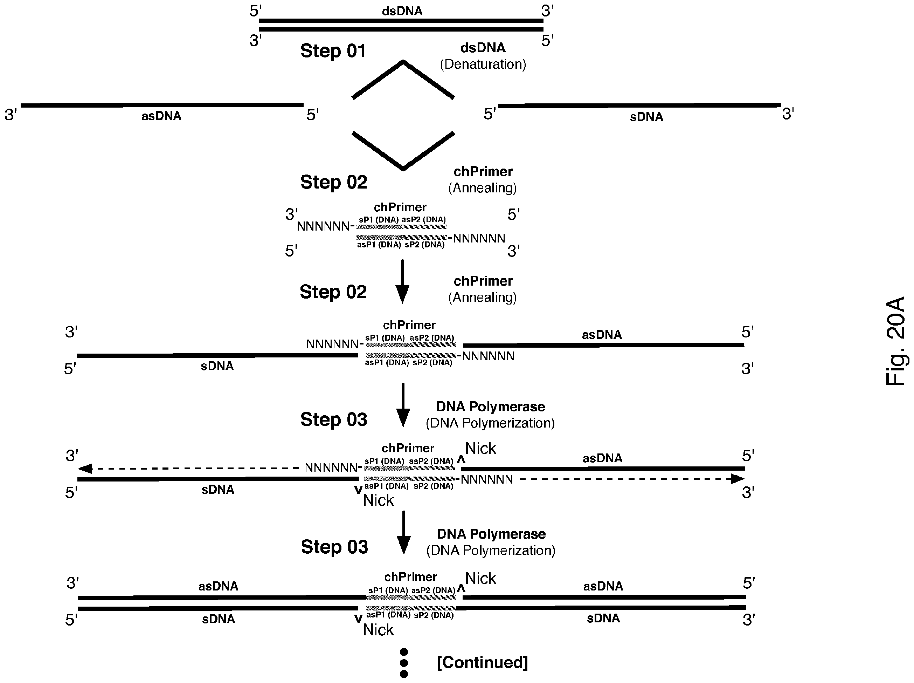

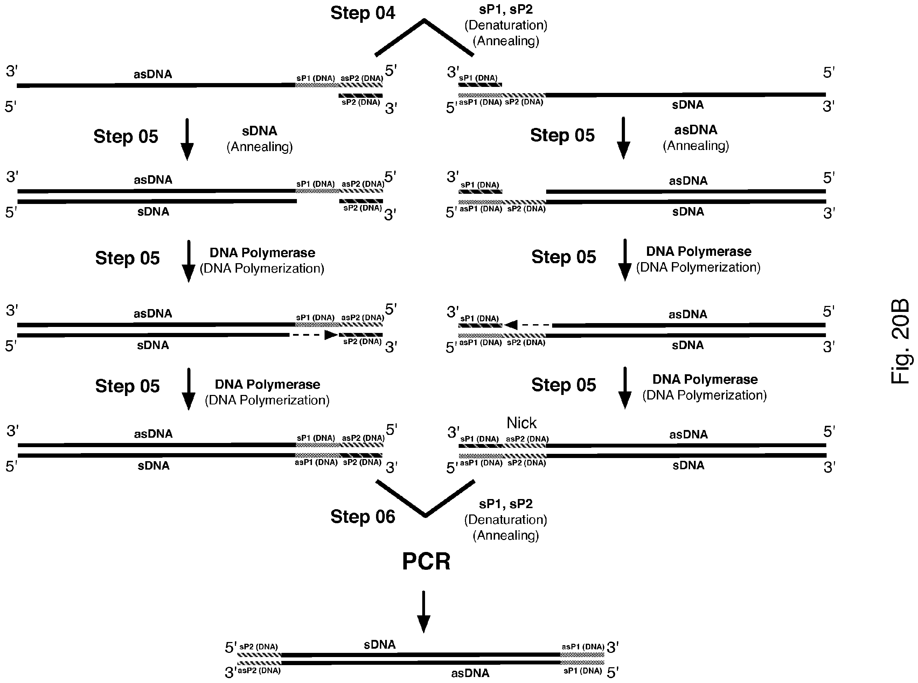

**Figure 20. Design Ch1 dsDNA Amplification from dsDNA (chPrimer + Strand Displacement + Initial Primer):**

- The molecular structure of the standard chPrimer contains a double-stranded DNA primer site with at least two different adjacent primer sites (P1 and P2) and two degenerate oligomers (e.g. hexamer) at the 3'-ends.

  • Step 01: dsDNA template denatured by heat or some other treatment.
  • Step 02: Separated sDNA and asDNA strands hybridizing to the 3' degenerate oligomer overhangs of the chPrimer from their 3'-ends simultaneously at this step.
  • *Note: Both sDNA and asDNA strands can hybridize to any of the two degenerate oligomer overhangs of the chPrimer with no predefined order or preferences.*
  • Step 03: DNA polymerase with 5'-3' polymerization capability filling next to the degenerate sequences.
  • *Note: A DNA polymerase with 3'-5' exonuclease activity can perform chPrimer silencing at this step.*
  • Step 04: Denaturing all fragments by heat or some other treatment.
  • Step 05a: sDNA with sP2 and asP1 primer sites at the 5'-end hybridizing with a complementary sP1 primer. asDNA with sP1 and asP2 primer sites at the 5'-end hybridizing with a complementary sP2 primer.
  • *Note: Simultaneous hybridization of sP1 and sP2 primer to sDNA and asDNA respectively blocks hybridization of sDNA and asDNA to each other from their complementary primer sites.*
  • Step 05b: sDNA and asDNA of the original template hybridizing to their complementary DNA strands.
  • Step 05c: DNA polymerase with 5'-3' polymerization capability filling next to the hybridized sDNA and asDNA of the original template. DNA polymerase with both 5'-3' polymerization and strand displacement (or nick translation) activity replacing the lower part of the primer.
  • *Note: A single DNA polymerase with both 5'-3' polymerization and strand displacement (or nick translation) capability can perform those three steps (05a, 05b and 05c) at the same time.*
  • Step 06: PCR reaction with two primers (sP1 and sP2). sP1 and sP2 primers added to the solution to bind the primer sites at the 3'-end of the both sense and antisense strands. DNA polymerase with 5'-3' polymerization capability filling the complementary strands. End PCR products (dsDNA) contain only one primer site at each of their 3'- and 5'-ends as P2 and P1 respectively.
  • *Note: Since same sP1 and sP2 primers can be used at both step 05 and step 06, PCR reaction can be started at step 05 and all steps after step 04 can be performed as a single process.*

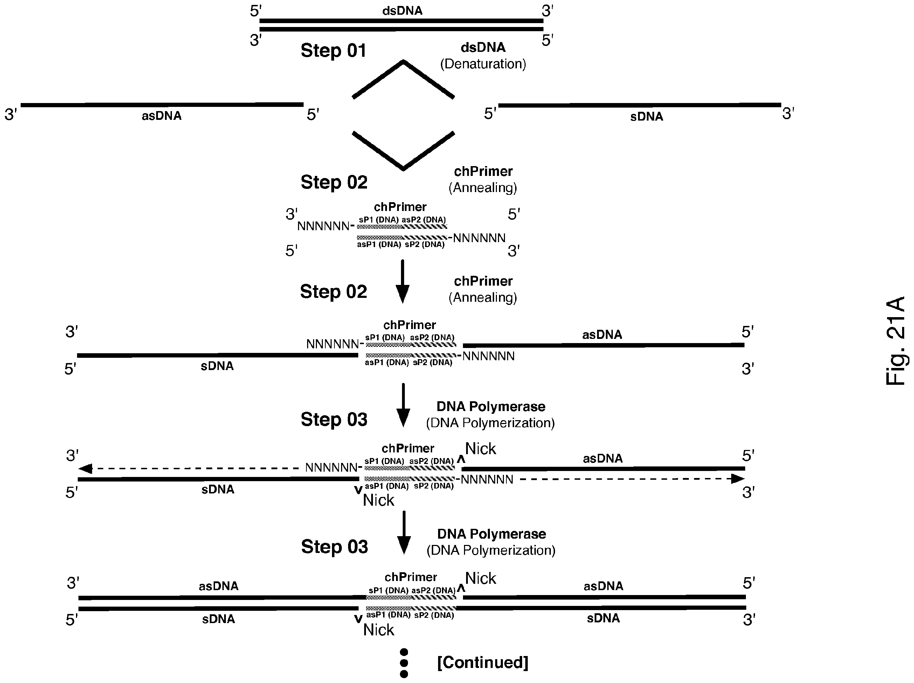

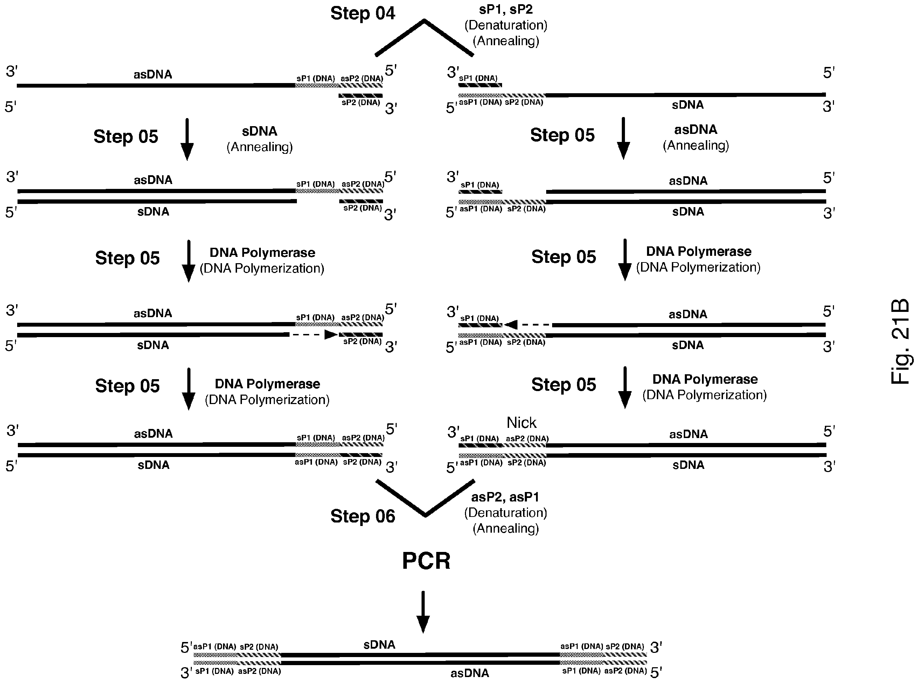

**Figure 21. Design Ch2 dsDNA Amplification from dsDNA (chPrimer + Strand Displacement + Initial Primer + 2 Primer Sites):**

- The molecular structures and steps 01 to 04 are as for Figure 20.
- Step 05a: sDNA with sP2 and asP1 primer sites at the 5'-end hybridizing with a complementary sP1 primer. asDNA with sP1 and asP2 primer sites at the 5'-end hybridizing with a complementary sP2 primer.
- *Note: Simultaneous hybridization of sP1 and sP2 primer to sDNA and asDNA respectively blocks hybridization of sDNA and asDNA to each other from their complementary primer sites.*
- Step 05b: sDNA and asDNA of the original template hybridizing to their complementary DNA strands.
- Step 05c: DNA polymerase with 5'-3' polymerization capability filling next to the hybridized sDNA and asDNA of the original template. DNA polymerase with both 5'-3' polymerization and strand displacement (or nick translation) activity replacing the lower part of the primer.
- *Note: A single DNA polymerase with both 5'-3' polymerization and strand displacement (or nick translation) capability can perform those three steps (05a, 05b and 05c) at the same time.*
- Step 06: PCR reaction with two primers (asP1 and asP2). asP1 and asP2 primers added to the solution to bind the primer sites at the 3'-end of the both sense and antisense strands. DNA polymerase with 5'-3' polymerization capability filling the complementary strands. End PCR products (dsDNA) contain two primer sites at each of their 3'- and 5'-ends as P1-P2 and P1-P2 respectively.

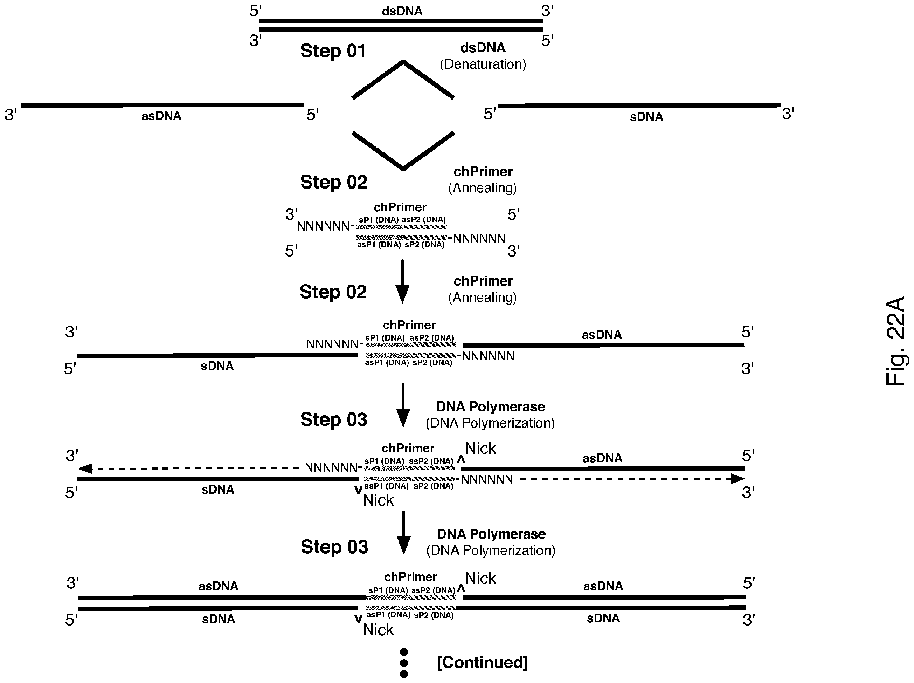

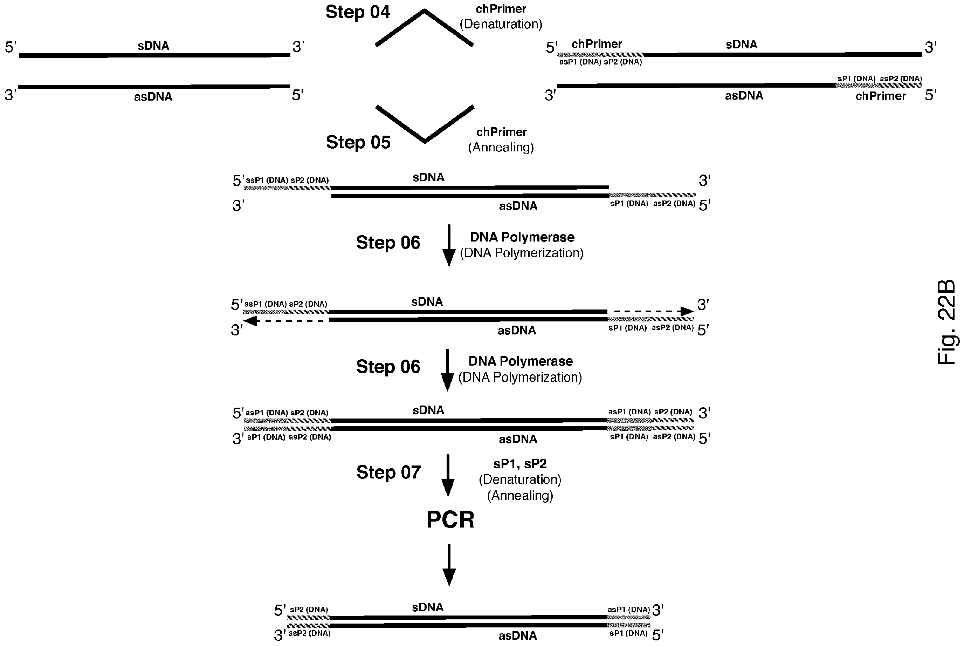

**Figure 22. Design Ch3 dsDNA Amplification from dsDNA (chPrimer):**

- The molecular structures and steps 01 to 04 are as for Figure 20.

- Step 05: sDNA with 5' primer sites (asP1 and sP2) and asDNA with primer sites (sP1 and asP2) hybridizing to each other.
- *Note: Also sDNA and asDNA of the original template strands hybridized to each other - Since there is no primer site, there is no reaction.*
- Step 06: DNA polymerase with 5'-3' polymerization capability filling the complementary sequences of primer sites.
- Step 07: PCR reaction with two primers (sP1 and sP2). sP1 and sP2 primers added to the solution to bind the primer sites at the 3'-end of the both sense and antisense strands. DNA polymerase with 5'-3' polymerization capability filling the complementary strands. End PCR products (dsDNA) contain only one primer site at each of their 3'- and 5'-ends as P2 and P1 respectively.

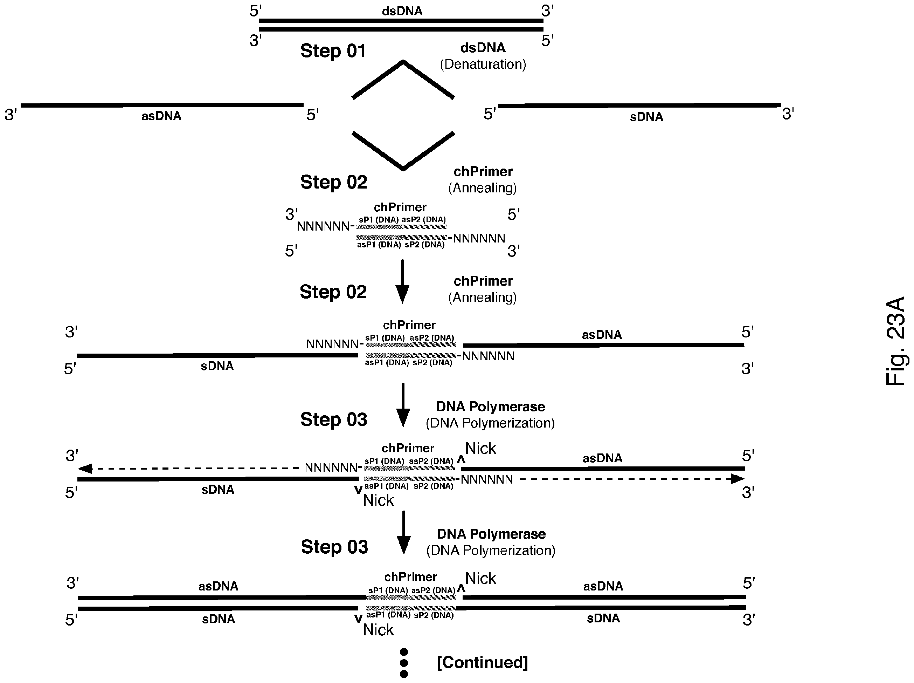

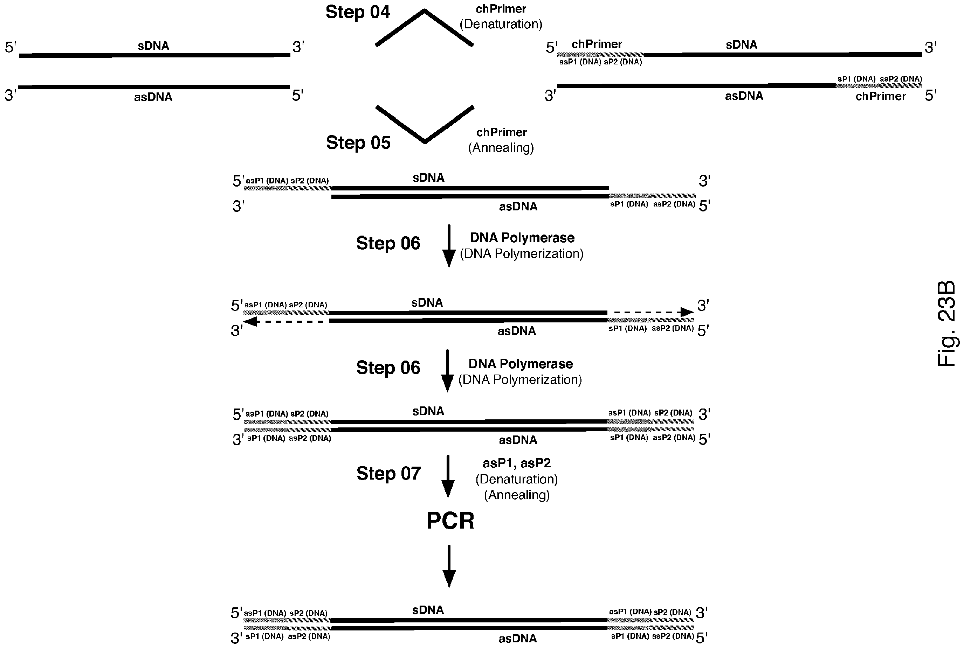

**Figure 23. Design Ch4 dsDNA Amplification from dsDNA (chPrimer + 2 Primer Sites):**

- The molecular structures and steps 01 to 04 are as for Figure 20.
- Step 05: sDNA with 5' primer sites (asP1 and sP2) and asDNA with primer sites (sP1 and asP2) hybridizing to each other.
- *Note: Also sDNA and asDNA of the original template strands hybridized to each other - Since there is no primer site, there is no reaction.*
- Step 06: DNA polymerase with 5'-3' polymerization capability filling the complementary sequences of primer sites.
- Step 07: PCR reaction with two primers (asP1 and asP2). asP1 and asP2 primers added to the solution to bind the primer sites at the 3'-end of the both sense and antisense strands. DNA polymerase with 5'-3' polymerization capability filling the complementary strands. End PCR products (dsDNA) contain two primer sites at each of their 3'- and 5'-ends as P1-P2 and P1-P2 respectively.

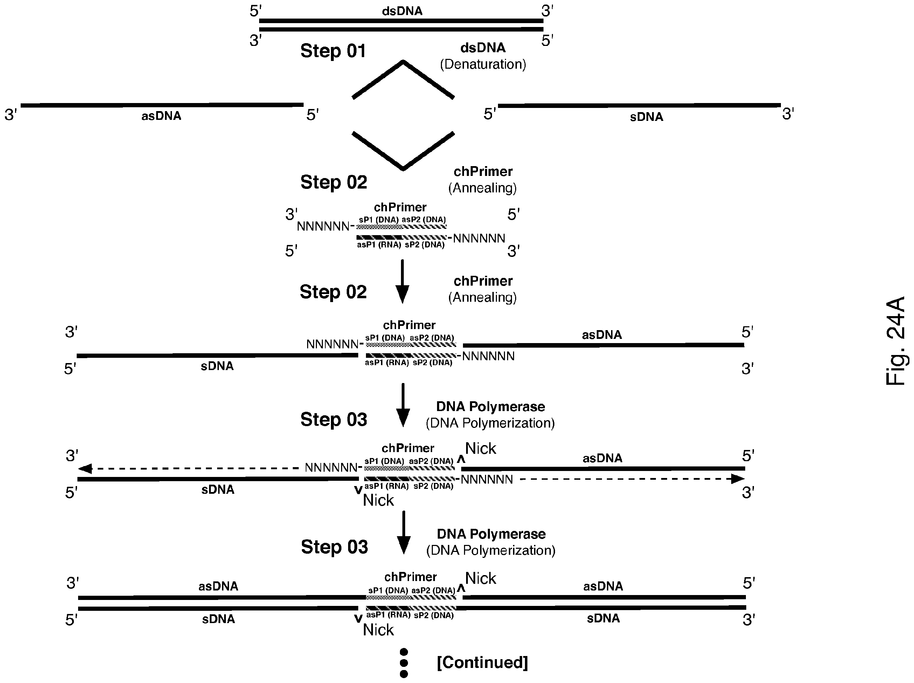

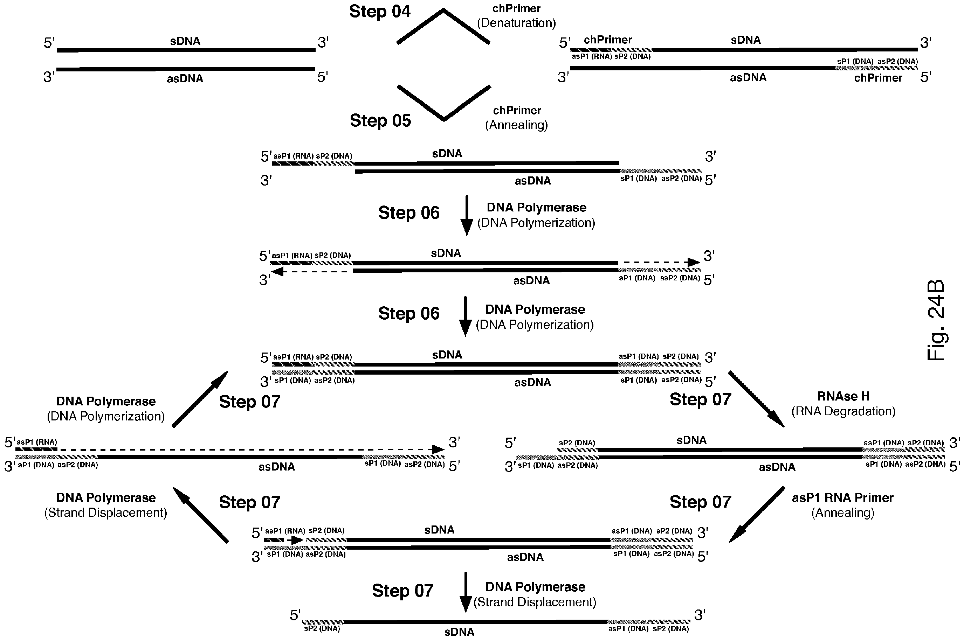

**Figure 24. Design Ch5 sDNA Amplification from dsDNA (chPrimer + RNase H):**

- The molecular structure of the standard chPrimer contains a double-stranded DNA primer site with at least two different adjacent primer sites (P1 and P2) where asP1 primer site is RNA and two degenerate oligomers (e.g. hexamer) at the 3'-ends.

  - Step 01: dsDNA template denatured by heat or some other treatment.
  - Step 02: Separated sDNA and asDNA strands hybridizing to the 3' degenerate oligomer overhangs of the chPrimer from their 3'-ends simultaneously at this step.
  - Step 03: DNA polymerase with 5'-3' polymerization capability filling next to the degenerate sequences.
  - *Note: A DNA polymerase with 3'-5' exonuclease activities can perform chPrimer silencing activity at the above step.*
  - Step 04: Denaturing all fragments by heat or some other treatment.
  - Step 05: sDNA with 5' primer sites (asP1 and sP2) and asDNA with primer sites (sP1 and asP2) hybridizing to each other.
  - *Note: Also sDNA and asDNA of the original template strands hybridized to each other - Since there is no primer site, there is no reaction.*
  - Step 06: DNA polymerase with 5'-3' polymerization and reverse transcription capability filling the complementary sequences of primer sites.
  - Step 07a: RNase H degradation of the DNA hybridized RNA part (asP1) at the 3'-end of asDNA strand.
  - Step 07b: Single-stranded asP1 RNA primer hybridizing to the 3'-end of asDNA.
  - Step 07c: DNA polymerase with both 5'-3' polymerization and strand displacement activities replacing the sDNA by polymerization of single-stranded asP1 RNA primer.
  - Step 07d: sDNA obtained (continuously).
  - Step 07e: DNA polymerase with both 5'-3' polymerization and strand displacement activities continues the polymerization of single-stranded asP1 RNA primer after replacing the sDNA.
  - *Note: Step 07 is a continuous cycle. After Step 07e, the same cycle starts again with Step 07a.*

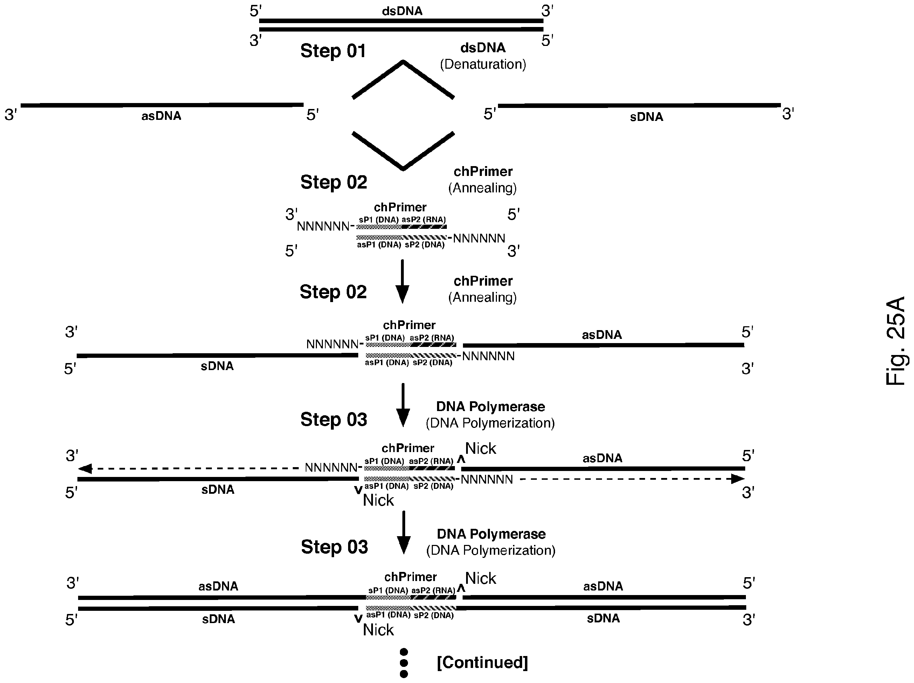

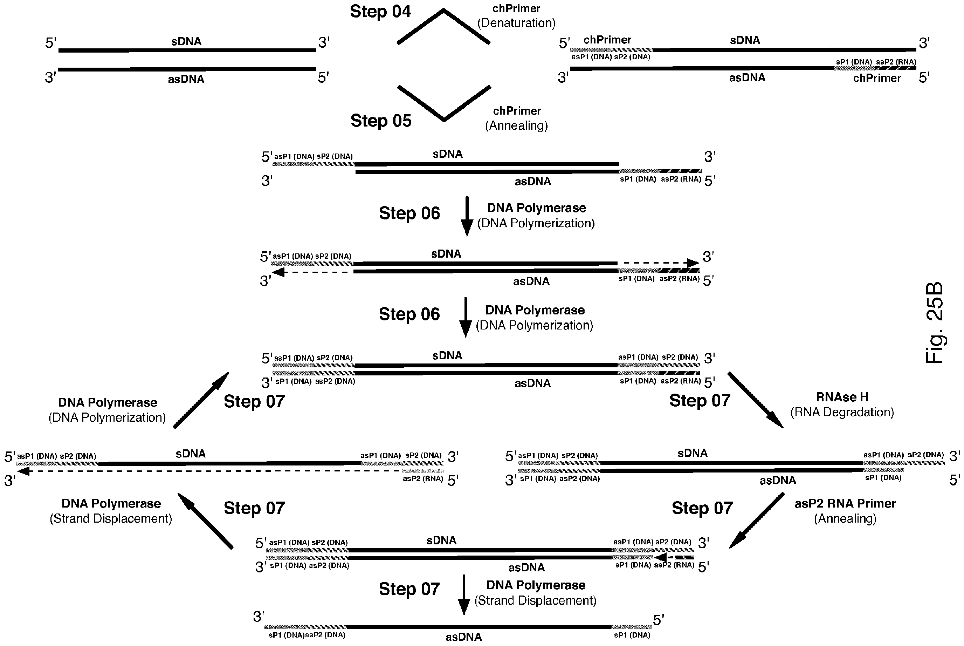

**Figure 25. Design Ch6 asDNA Amplification from dsDNA (chPrimer + RNase H):**

- The molecular structure of the standard chPrimer contains a double-stranded DNA primer site with at least two different adjacent primer sites (P1 and P2) where asP2 primer site is RNA and two degenerate oligomers (e.g. hexamer) at the 3'-ends.

- Step 01: dsDNA template denatured by heat or some other treatment.
- Step 02: Separated sDNA and asDNA strands hybridizing to the 3' degenerate oligomer overhangs of the chPrimer from their 3'-ends simultaneously at this step.
- Step 03: DNA polymerase with 5'-3' polymerization capability filling next to the degenerate sequences.
- *Note: A DNA polymerase with 3'-5' exonuclease activities can perform chPrimer silencing activity at the above step.*
- Step 04: Denaturing all fragments by heat or some other treatment.
- Step 05: sDNA with 5' primer sites (asP1 and sP2) and asDNA with primer sites (sP1 and asP2) hybridizing to each other.
- *Note: Also sDNA and asDNA of the original template strands hybridized to each other - Since there is no primer site, there is no reaction.*
- Step 06: DNA polymerase with 5'-3' polymerization and reverse transcription capability filling the complementary sequences of primer sites.
- Step 07a: RNase H degradation of the DNA hybridized RNA part (asP2) at the 3'-end of sDNA strand.
- Step 07b: Single-stranded asP2 RNA primer hybridizing to the 3'-end of sDNA.
- Step 07c: DNA polymerase with both 5'-3' polymerization and strand displacement activities replacing the asDNA by polymerization of single-stranded asP2 RNA primer.
- Step 07d: asDNA obtained (continuously).
- Step 07e: DNA polymerase with both 5'-3' polymerization and strand displacement activities continues the polymerization of single-stranded asP2 RNA primer after replacing the asDNA.
- *Note: Step 07 is a continuous cycle. After Step 07e, the same cycle starts again with Step 07a.*

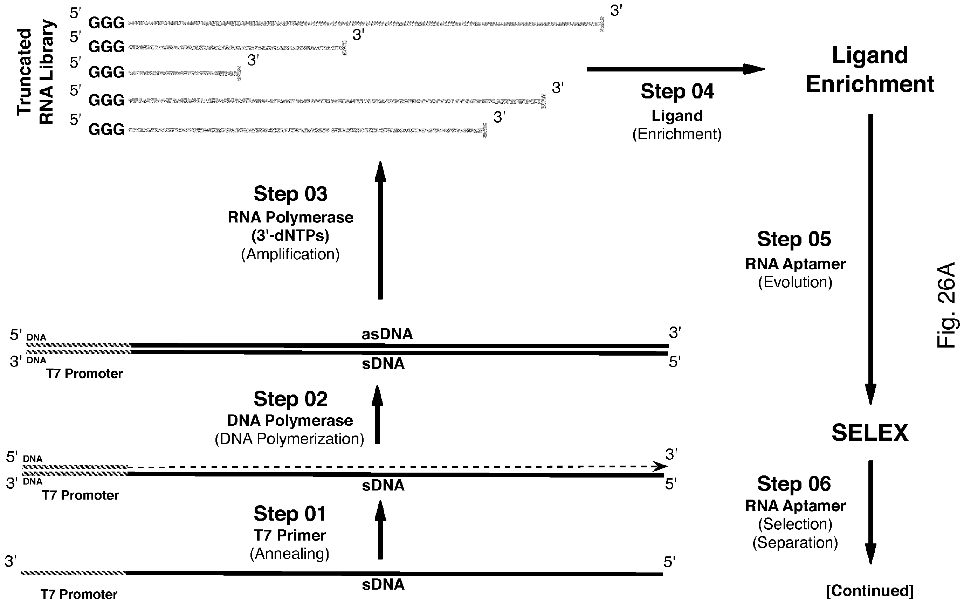

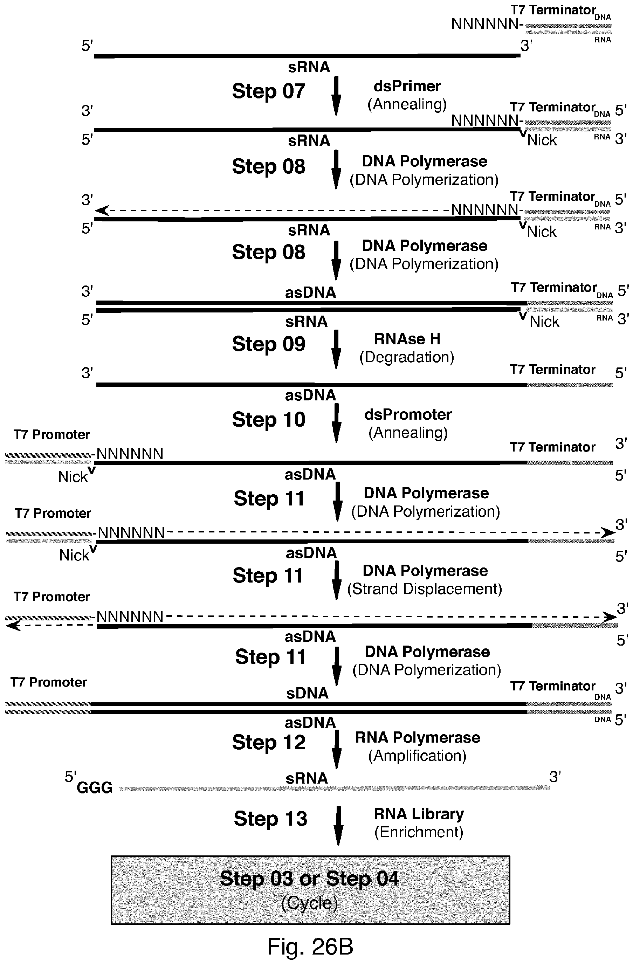

### Figure 26. Design S1 RNA Aptamer SELEX (Teminator):

- The molecular structure of the dsTerminator contains a terminator site of T7 promoter (could be any other DNA-dependent RNA polymerase promoter) with a degenerate oligomer (e.g. hexamer) at the 3'-end of the DNA strand and the complimentary strand is RNA.
- The molecular structure of the dsPromoter contains a T7 promoter site (could be any other DNA-dependent RNA polymerase promoter) with a degenerate oligomer (e.g. hexamer) at the 3'-end of the DNA strand and the complimentary strand is RNA.
- The molecular structure of the initial single-stranded DNA template contains a single-stranded T7 promoter site at the 3'-end.

- Step 01: T7 promoter site complementary primer hybridizes to the 3'-end of sDNA template.
- Step 02: DNA Polymerase with 5'-3' polymerization capability filling next to the primer.
- Step 03: RNA polymerase binds to the T7 promoter site and transcribes the antisense strand into single-stranded RNA molecules (with three guanine bases at the 5'-end due to T7 promoter's feature) in presence of ddNTPs to produce a truncated RNA library that contains different lengths of single-stranded RNA molecules.
- Step 04: Truncated RNA library exposed to the desired ligand.
- Step 05: Single-stranded RNA molecules inside the truncated RNA library bind to the ligand depending on their 3D conformation.
- Step 06: Ligand-bound single-stranded RNA molecules removed from truncated RNA library by any desired selection methods such as capillary electrophoresis, nanoparticles and chromatography techniques etc.
- Step 07: dsTerminator hybridizing to the 3'-end of the selected single-stranded sRNA molecules.
- Step 08: DNA polymerase with 5'-3' polymerization and reverse transcription capability filling next to the degenerate sequences.
- *Note: A DNA polymerase with 3'-5' exonuclease activity can perform dsTerminator silencing at this step.*
- Step 09: RNase H degradation of the DNA hybridized RNA strands.
- Step 10: dsPromoter hybridizing to the 3'-end of the single-stranded asDNA template.
- Step 11a: DNA polymerase with 5'-3' polymerization capability filling next to the degenerate sequences.
- Step 11b: A polymerase with both 5'-3' polymerization and strand displacement (or nick translation) activity replacing the lower part of the promoter.

- *Note: A single DNA polymerase with both 5'-3' polymerization and strand displacement (or nick translation) capability can perform those two steps (011a and 011b) at the same time.*
- *Note: A DNA polymerase with 3'-5' exonuclease activity can perform dsPromoter silencing at this step.*
- Step 12: RNA polymerase binding to promoter part and transcribing the antisense strand into sRNA with three guanine bases at the 5'-end due to T7 promoter's feature.
- *Note: RNA polymerase stops its activity at the T7 terminator.*
- Step 13: Amplified sRNA molecules form an enriched truncated RNA library and then enter the SELEX cycle again.

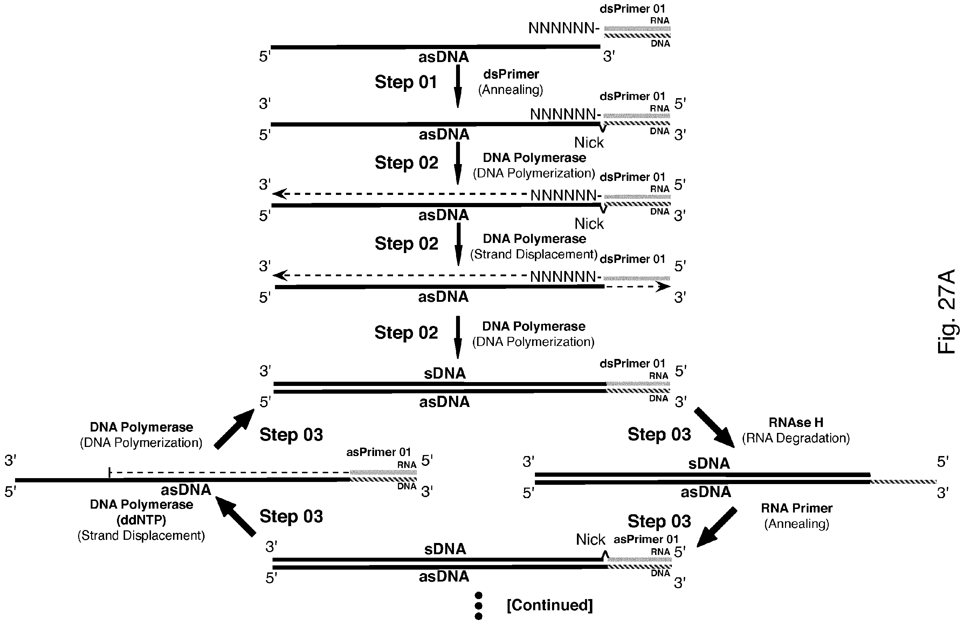

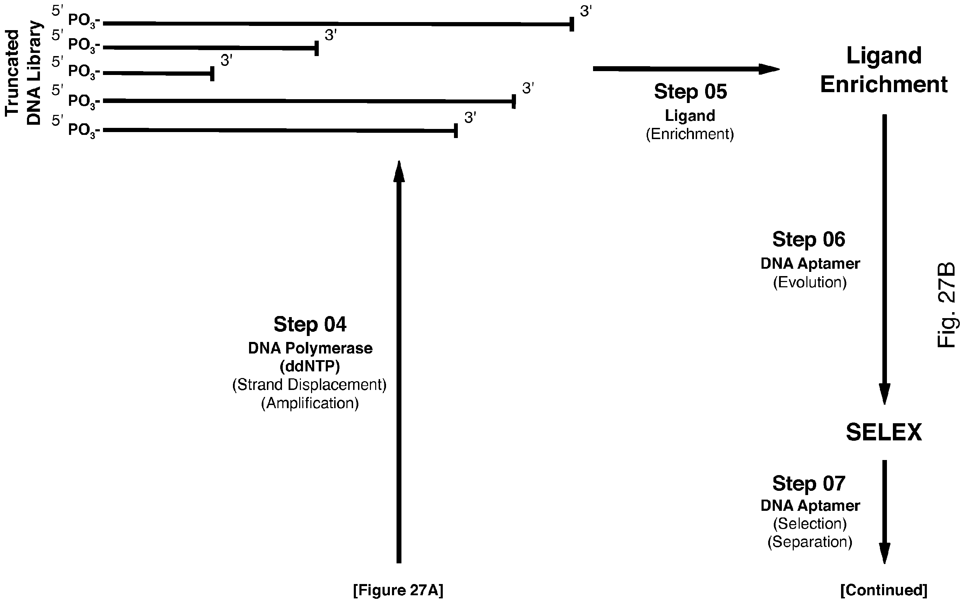

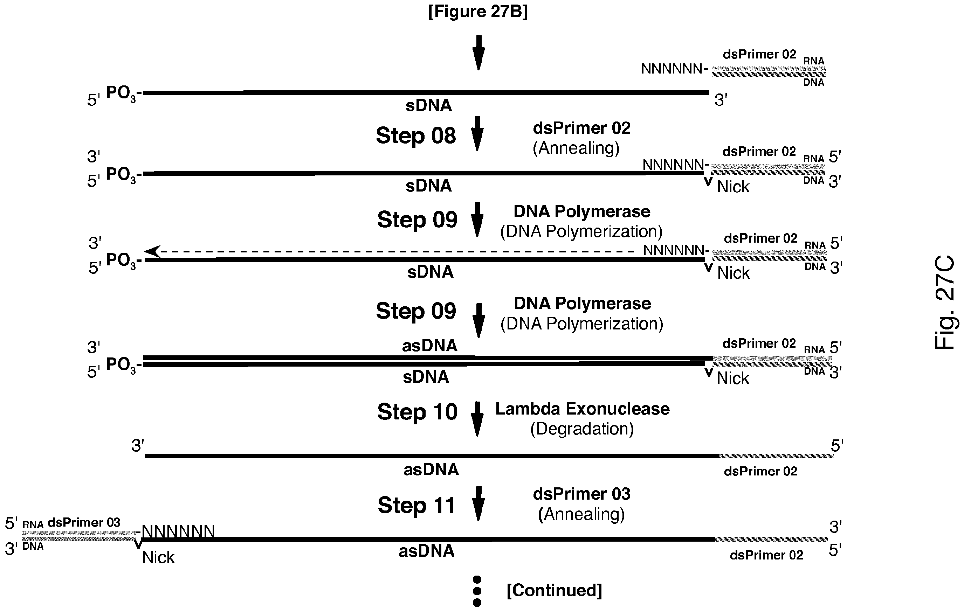

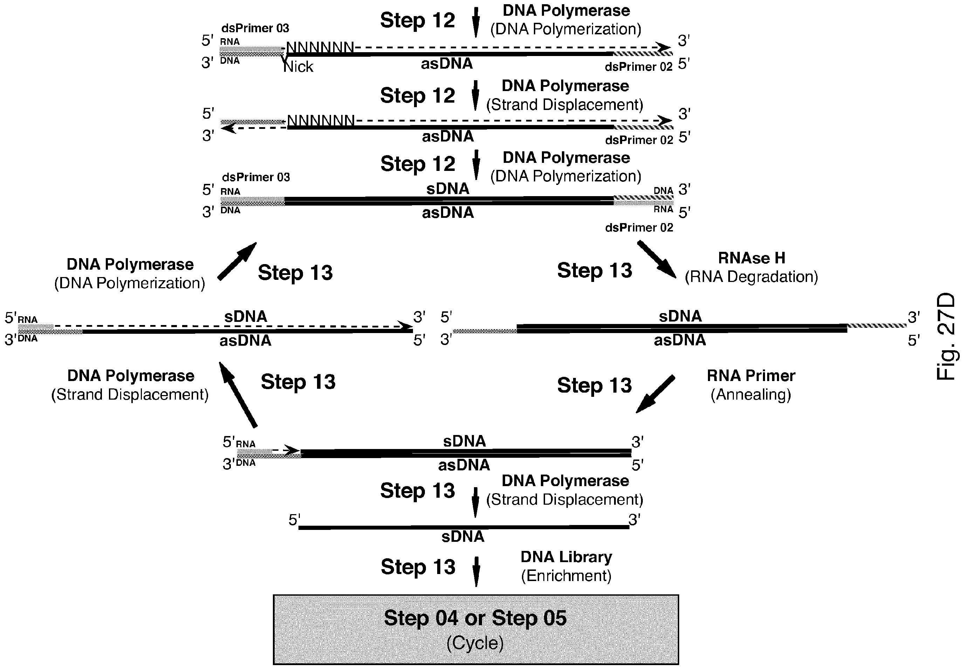

**Figure 27. Design S2 DNA Aptamer SELEX (Lambda + Isothermal):**

- The molecular structure of the dsPrimer 01 contains a primer site with a degenerate oligomer (e.g. hexamer) at the 3'-end of the RNA strand and the complimentary strand is DNA.
- The molecular structure of the dsPrimer 02 contains a primer site with a degenerate oligomer (e.g. hexamer) at the 3'-end of the RNA strand with no 5'-end phosphorylated complimentary DNA strand.
- As an option, dsPrimer 02 contains a primer site with a degenerate oligomer (e.g. hexamer) at the 3'-end of the RNA strand with 5'-end phosphorylated complimentary DNA strand can be used as an alternative instead of standard one described above.
- The molecular structure of the dsPrimer 03 contains a primer site (different from dsPrimer 01 and dsPrimer 02) with a degenerate oligomer (e.g. hexamer) at the 3'-end of the RNA strand and the complimentary strand is DNA.


- Step 01: dsPrimer 01 hybridizing to the 3'-end of the single-stranded asDNA template.
- Step 02a: DNA polymerase with 5'-3' polymerization capability filling next to the degenerate sequences.
- Step 02b: DNA polymerase with 5'-3' polymerization, reverse transcription and strand displacement (or nick translation) activity replacing the lower part of the primer.
- *Note: Also another denaturation methods such as heat or some other treatments can be use to replace the lower part of the primer.*
- *Note: A single DNA polymerase with 5'-3' polymerization, reverse transcription and strand displacement (or nick translation) capability can perform those two steps (02a and 02b) at the same time.*
- *Note: A DNA polymerase with 3'-5' exonuclease activity can perform dsPrimer silencing at this step.*
- Step 03a: RNase H degradation of the DNA hybridized RNA part at the 3'-end of asDNA strand.
- Step 03b: Single-stranded RNA primer hybridizing to the 3'-end of asDNA.
- Step 03c: DNA polymerase with both 5'-3' polymerization and strand displacement activities replacing the sDNA by polymerization of single-stranded RNA primer.
- Step 03d: 5'-end phosphorylated sDNA obtained (continuously).
- Step 03e: DNA polymerase with both 5'-3' polymerization and strand displacement activities continues the polymerization of single-stranded RNA primer after replacing the sDNA.
- *Note: Step 03 is a continuous cycle. After Step 03e, the same cycle starts again with Step 03a.*
- Step 04: DNA polymerase binds to the dsPrimer 01 primer site and replicates the antisense strand into single-stranded DNA molecules in presence of ddNTPs to produce a truncated DNA library that contains different lengths of single-stranded DNAs.
- Step 05: Truncated DNA library exposed to the desired ligand.
- Step 06: Single-stranded DNA molecules inside the truncated DNA library bind to the ligand depending on their 3D conformation.
- Step 07: Ligand-bound single-stranded DNA molecules removed from truncated DNA library by any desired selection method (capillary electrophoresis, nanoparticles, chromatography techniques etc.).
- Step 08: dsPrimer 02 hybridizing to the 3'-end of the selected single-stranded sDNA molecules.
- Step 09: DNA polymerase with 5'-3' polymerization capability filling next to the degenerate sequences.
- *Note: A DNA polymerase with 3'-5' exonuclease activity can perform dsPrimer silencing activity at this step.*
- Step 10: Lambda exonuclease degrades 5'-end phosphorylated sDNA and asDNA simultaneously until it reached to the nick site.
- *Note: If a dsPrimer containing 5'-end phosphorylated DNA strand (complementary to the primer site adjacent to the 3'-end degenerate sequence) is used, 5'-end phosphorylated DNA strand of the dsPrimer will also be degraded by Lambda exonuclease.*
- Step 11: dsPromoter 03 hybridizing to the 3'-end of the single-stranded asDNA template.
- Step 12a: DNA polymerase with 5'-3' polymerization capability filling next to the degenerate sequences.

- Step 12b: DNA polymerase with both 5'-3' polymerization, reverse transcription and strand displacement (or nick translation) activity replacing the lower part of the primer.
- *Note: Also another denaturation methods such as heat or some other treatment can be use to replace the lower part of the primer.*
- *Note: A single DNA polymerase with 5'-3' polymerization, reverse transcription and strand displacement (or nick translation) capability can perform those two steps (12a and 12b) at the same time.*
- *Note: A DNA polymerase with 3'-5' exonuclease activity can perform dsPrimer silencing at this step.*
- Step 13a: RNase H degrades DNA hybridized RNA part at the 3'-ends of both DNA strands at the first cycle. After the first cycle RNase H degradation occurs in the DNA hybridized RNA part at the 3'-end of asDNA.
- Step 13b: Single-stranded RNA primer hybridizing to the 3'-end of asDNA.
- *Note: Hybridization of single-stranded RNA primer to the 3'-end of asDNA at the first cycle leaves the primer site of sDNA as a 3' overhang.*
- Step 13c: DNA polymerase with both 5'-3' polymerization and strand displacement activity replaces sDNA by polymerization of single-stranded RNA primer.
- *Note: DNA polymerase with 5'-3' polymerization, 3'-5' exonuclease and strand displacement activities also degrades the 3' overhang primer site of sDNA at the first cycle while replacing the sDNA strand.*
- Step 13d: sDNA obtained (continuously).
- Step 13e: DNA polymerase with both 5'-3' polymerization and strand displacement activities continues the polymerization of single-stranded RNA primer after replacing the sDNA part.
- *Note: Step 13 is a continuous cycle. After Step 13e, the same cycle starts again with Step 13a.*
- Step 14: Amplified sDNA molecules form an enriched truncated DNA library and then enter the SELEX cycle again.

**Figure 28. Design S3 DNA Aptamer SELEX (Single Primer Region):**

- The molecular structure of the dsPrimer contains a double-stranded DNA primer site and a degenerate oligomer (e.g. hexamer) at the 3'-end.

- Step 01: Linear amplification of the template with a single primer by a DNA polymerase that binds to the primer site of the template. DNA polymerase replicates the antisense strand into single-stranded DNA molecules in presence of ddNTPs to produce a truncated DNA library that contains different lengths of single-stranded DNAs.
- Step 02: Truncated DNA library exposed to desired ligand.
- Step 03: Single-stranded DNA molecules inside the truncated DNA library bind to the ligand depending on their 3D conformation.
- Step 04: Ligand-bound single-stranded DNA molecules removed from truncated DNA library by any desired selection method (capillary electrophoresis, nanoparticles, chromatography techniques etc.).
- Step 05: dsPrimer hybridizing to the 3'-end of the selected single-stranded sDNA molecules.
- Step 06: DNA polymerase with 5'-3' polymerization capability filling next to the degenerate sequences.
- *Note: A DNA polymerase with 3'-5' exonuclease activity can perform dsPrimer silencing activity at this step.*
- Step 07: Linear amplification of the template with a single primer by a DNA polymerase that binds to the primer site of the template.
- Step 08: Amplified sDNA molecules form an enriched truncated DNA library and then enter the SELEX cycle again.

**Figure 29. chPrimer Structure:**

**Figure 29A: chPrimer:**

- chPrimer structure formed by; a double-stranded part and two single-stranded degenerate sequences or inosine bases or can be a specific sequence.
- The double-stranded part can be made of any type of nucleic acid. For example, both the strands can be DNA, both of the strands can be RNA, or one strand can be RNA and one strand can be DNA. Alternatively, one or both strands can contain a mixture of nucleic acid types (e.g. a mixture of RNA and DNA regions in the form of a chimeric nucleic acid molecule).

- Single-stranded parts can be formed by; degenerate sequences, any natural or unnatural nucleic acid molecules, inosine bases or can be a specific sequence located at the 3'-end of the each strand of the double-stranded part as an overhang.

**Figure 29B: chPrimer Silencing (to stop the activity of the primers):**

- chPrimer silencing is the name of the process to stop the activity of the unhybridized chPrimer molecules after their first hybridization to the 3'- ends of the single-stranded template molecules.
- Degradation of the 3' overhang parts of the chPrimer structure with a polymerase, which has 3'-5' exonuclease activity.
- Hybridization activity of all unhybridized chPrimer structures stopped after degradation in order to prevent undesired hybridization of chPrimer molecules.

**Figure 30. DNA Fragment Addition:**

- The molecular structure of the DNA Fragment A contains single-stranded DNA part.
- The molecular structure of the DNA Fragment B contains a double-stranded DNA part and a degenerate oligomer (e.g. hexamer) at the 3'-end.

    • Step 01: DNA Fragment A hybridizing to the 3' end of single-stranded DNA Fragment B.
    • Step 02a: DNA polymerase with 5'-3' polymerization activity filling next to the degenerate sequences of DNA Fragment B.
    • Step 02b: DNA polymerase with both 5'-3' polymerization and strand displacement (or nick translation) activity replacing lower part of the DNA Fragment B.
    • *Note: A single DNA polymerase with both 5'-3' polymerization and strand displacement (or nick translation) capability can perform those two steps (02a and 02b) at the same time.*
    • *Note: A DNA polymerase with 3'-5' exonuclease activity can perform dsPromoter silencing at this step.*
    • Step 02c: DNA Fragment A + B formed by the addition of DNA Fragment B to DNA Fragment A.

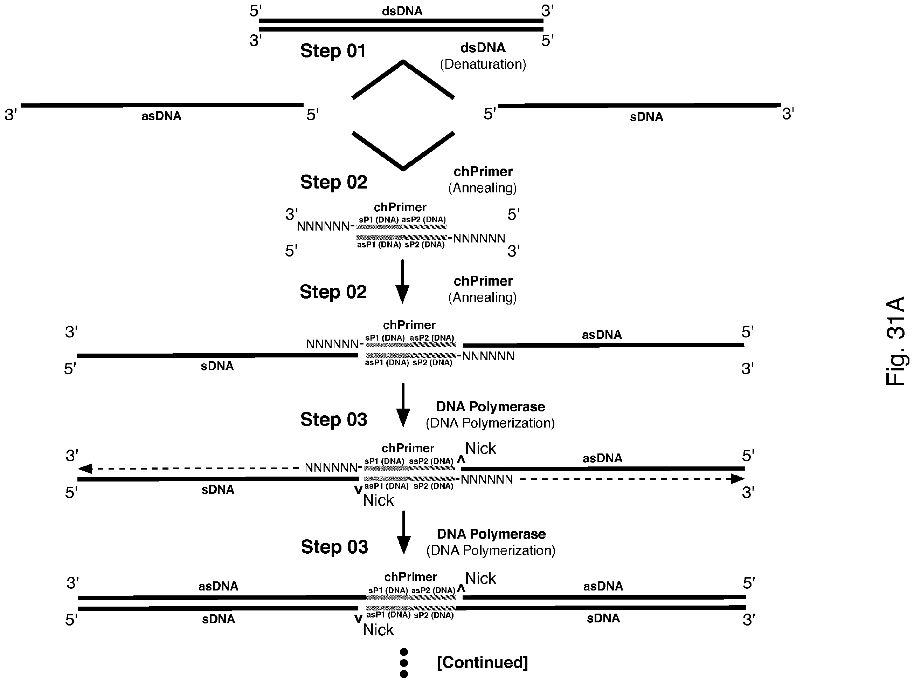

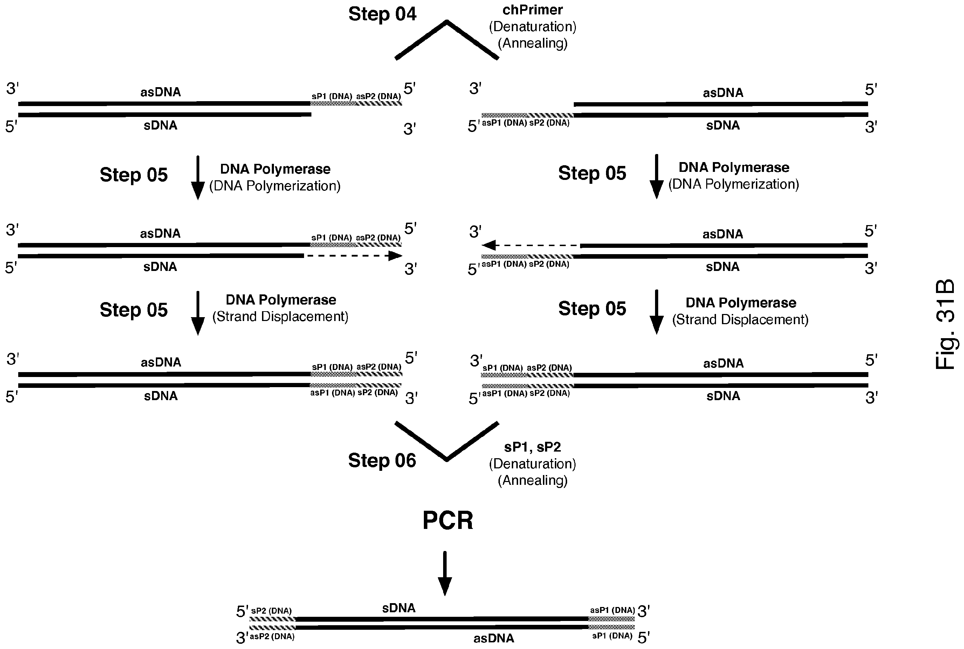

**Figure 31. Design Ch7 dsDNA Amplification from dsDNA (chPrimer + Denaturation):**

- The molecular structures and steps 01 to 04 are as for Figure 20.
- *Note: Denaturation from the "nick" regions by heat or some other treatment.*
- Step 05: DNA polymerase with 5'-3' polymerization capability filling the complementary sequences of primer sites.
- Step 06: PCR reaction with two primers (sP1 and sP2). sP1 and sP2 primers added to the solution to bind the primer sites at the 3'-end of the both sense and antisense strands. DNA polymerase with 5'-3' polymerization capability filling the complementary strands. End PCR products (dsDNA) contain only one primer site at each of their 3'- and 5'-ends as P2 and P1 respectively.

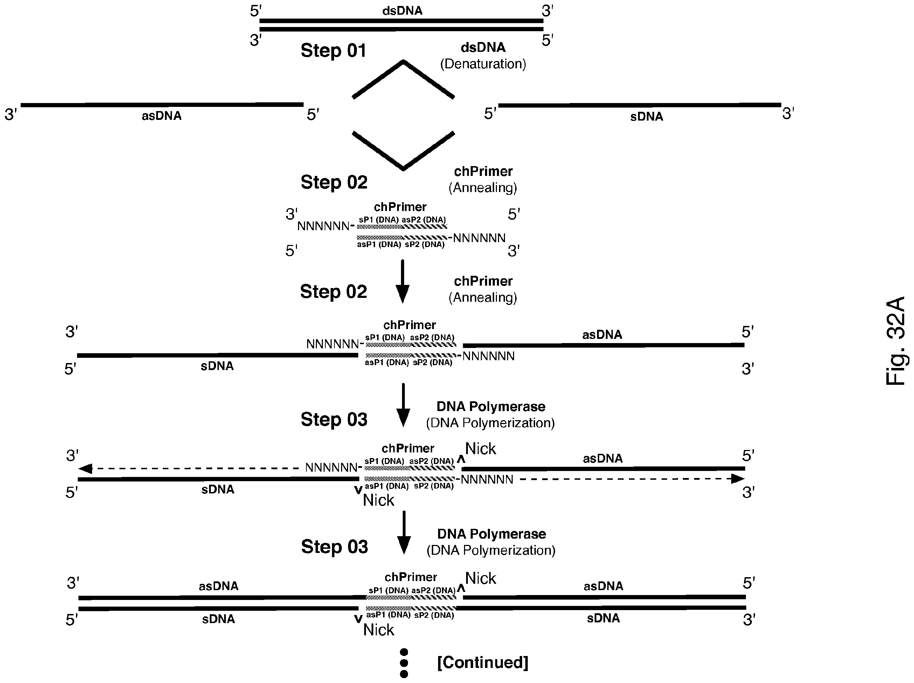

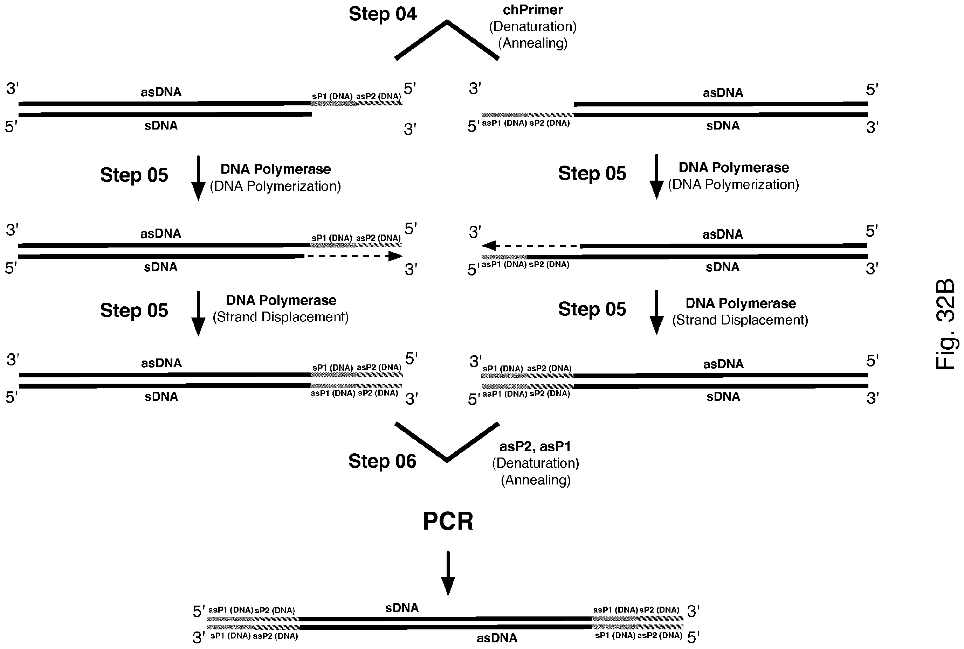

**Figure 32. Design Ch8 dsDNA Amplification from dsDNA (chPrimer + Denaturation + 2 Primer Regions):**

- The molecular structures and steps 01 to 04 are as for Figure 20.
- *Note: Denaturation from the "nick" regions by heat or some other treatment.*
- Step 05: DNA polymerase with 5'-3' polymerization capability filling the complementary sequences of primer sites.
- Step 06: PCR reaction with two primers (asP1 and asP2). asP1 and asP2 primers added to the solution to bind the primer sites at the 3'-end of the both sense and antisense strands. DNA polymerase with 5'-3' polymerization capability filling the complementary strands. End PCR products (dsDNA) contain two primer sites at each of their 3'- and 5'-ends as P1-P2 and P1-P2 respectively.

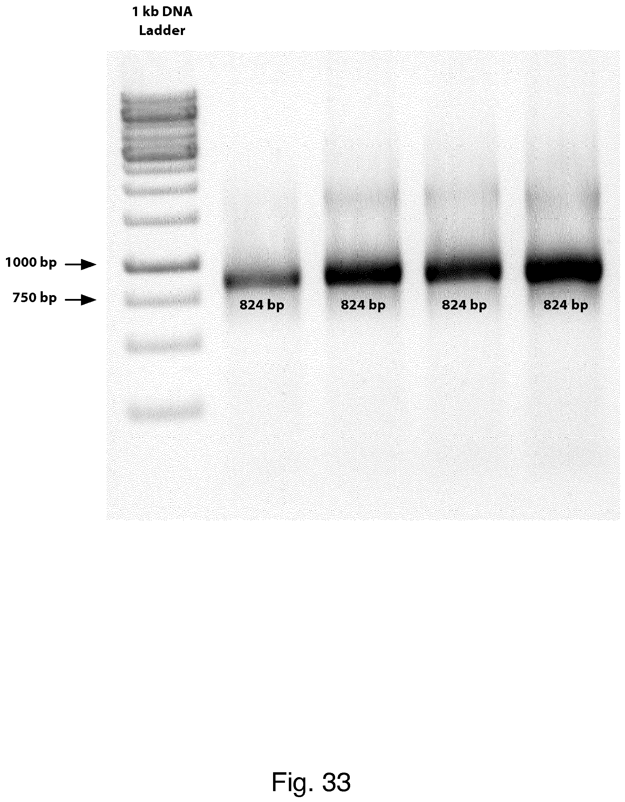

**Figure 33. Example 1** - **dsDNA to dsDNA with dsPrimer (Optimization):**

1- 1kb DNA ladder (750bp, 1000bp)

2- Amplification of dsDNA fragment 02 with 1.5mM $MgCl_2$ and 1.4$\mu$M primer

3- Amplification of dsDNA fragment 02 with 2mM $MgCl_2$ and 2$\mu$M primer

4- Amplification of dsDNA fragment 02 with 1.5mM $MgCl_2$ and 2$\mu$M primer

5- Amplification of dsDNA fragment 02 with 2mM $MgCl_2$ and 2.4$\mu$M primer

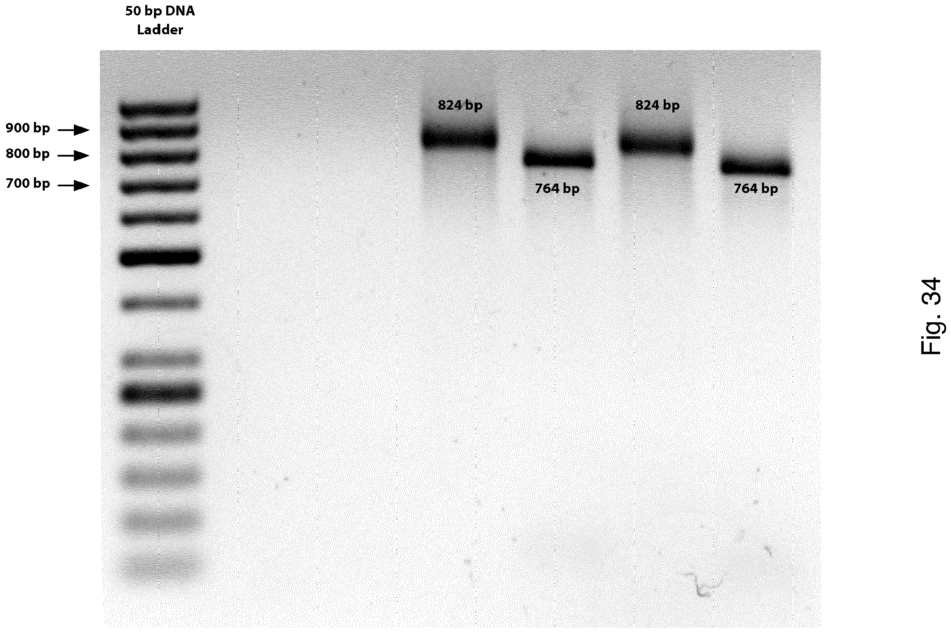

**Figure 34. Example 2** - **dsDNA to dsDNA with dsPrimer (Size comparison to standard PCR):**

1- 100bp DNA ladder
4- Amplification of dsDNA fragment 02 with dsPrimer
5- Amplification of dsDNA fragment 02 with dsDNA fragment 02 primers
6- Amplification of dsDNA fragment 02 with dsPrimer
7- Amplification of dsDNA fragment 02 with dsDNA fragment 02 primers

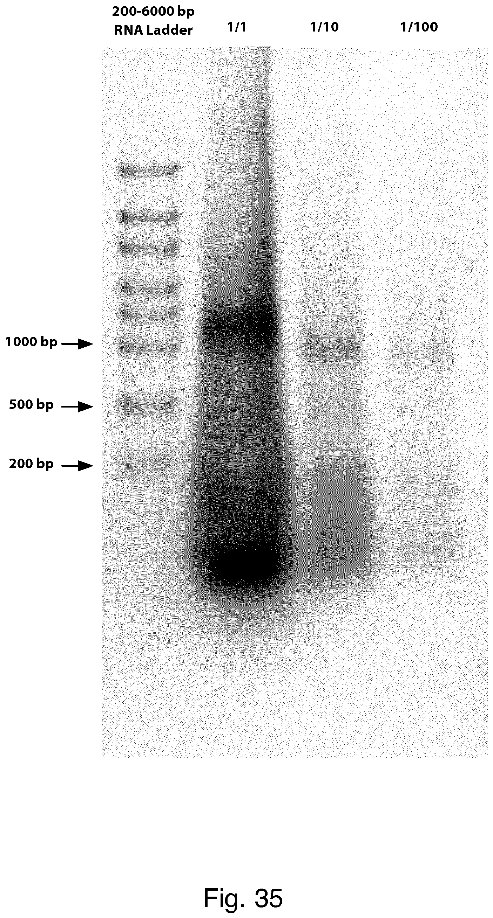

**Figure 35. Example 3** - **dsDNA to ssRNA with dsPrimer (Transcription):**

1- RNA ladder (200-6000bp)
2- Transcription product of the amplification product of dsPrimer amplification of dsDNA Fragment 02, undiluted
3- Transcription product of the amplification product of dsPrimer amplification of dsDNA Fragment 02, 1/10 diluted
4- Transcription product of the amplification product of dsPrimer amplification of dsDNA Fragment 02, 1/100 diluted

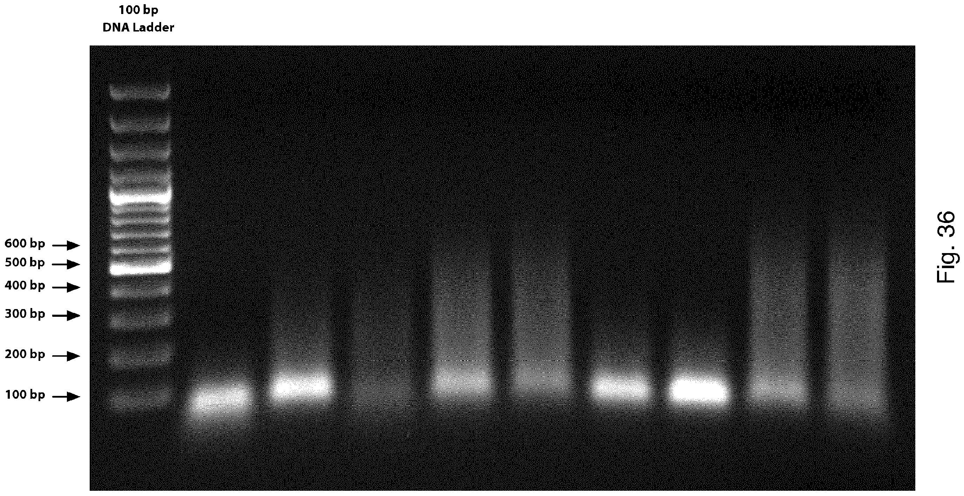

**Figure 36 Example 4** - **gDNA to gDNA with chPrimer:**

1- 100bp DNA ladder
5- 300-400bp sheared gDNA with chPrimer
6- 300-400bp sheared gDNA with chPrimer
9- 400-500bp sheared gDNA with chPrimer
10-400-500bp sheared gDNA with chPrimer

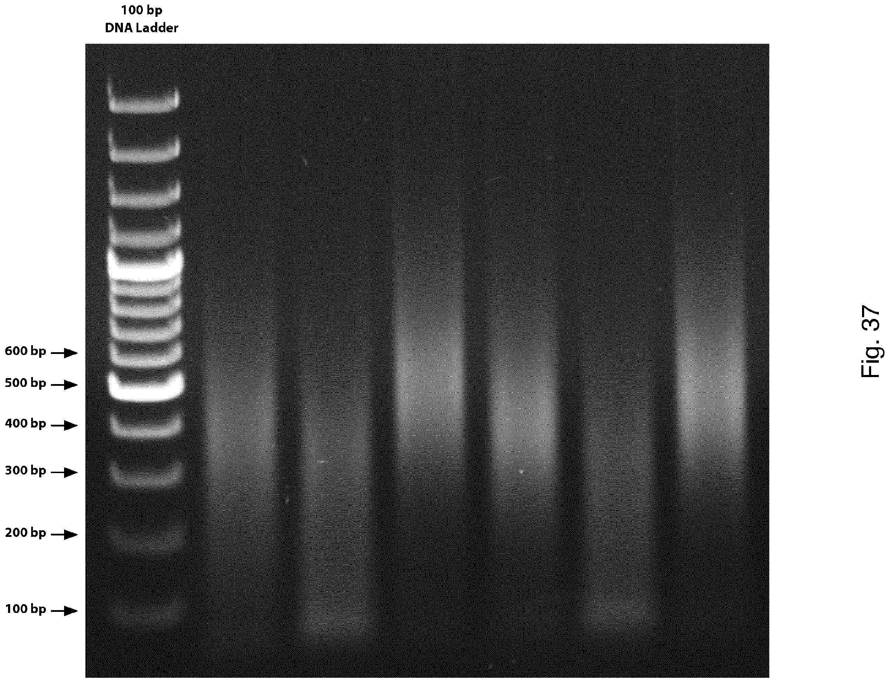

**Figure 37 Example 5** - **gDNA to gDNA with chPrimer (Size comparison of single and multiple primer sites):**

1- 100bp DNA Ladder
2- 300-400bp sheared gDNA with chPrimer, multiple primer sites
3- 300-400bp sheared gDNA with chPrimer, single primer site
4- 400-500bp sheared gDNA with chPrimer, multiple primer sites
5- 400-500bp sheared gDNA with chPrimer, single primer site
6- 400-500bp sheared gDNA with chPrimer, single primer site
7- 400-500bp sheared gDNA with chPrimer, multiple primer sites

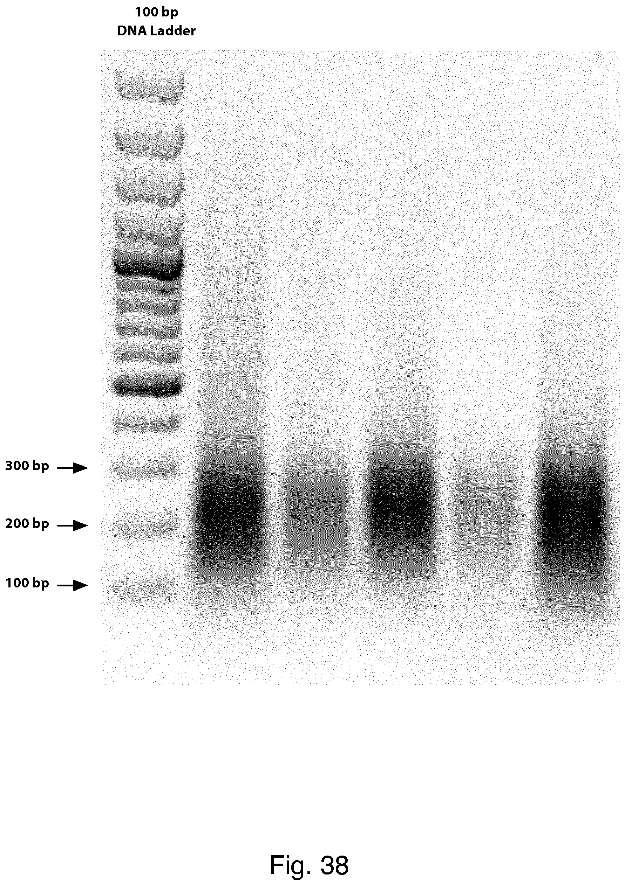

**Figure 38. Example 6** - **gDNA to gDNA with dsPrimer:**

1- 100bp DNA ladder

2- dsPrimer amplification of 100-200bp sheared gDNA using Hot-start Touchdown protocol 2, 1.5mM MgCl$_2$

3- dsPrimer amplification of 100-200bp sheared gDNA using Hot-start Touchdown protocol 1, 2mM MgCl$_2$

4- dsPrimer amplification of 100-200bp sheared gDNA using Hot-start Touchdown protocol 2, 1.75mM MgCl$_2$

5- dsPrimer amplification of 100-200bp sheared dNA using Hot-start Touchdown protocol 1, 1.5mM MgCl$_2$

6- dsPrimer amplification of 100-200bp sheared gDNA using Hot-start Touchdown protocol 2, 2mM MgCl$_2$

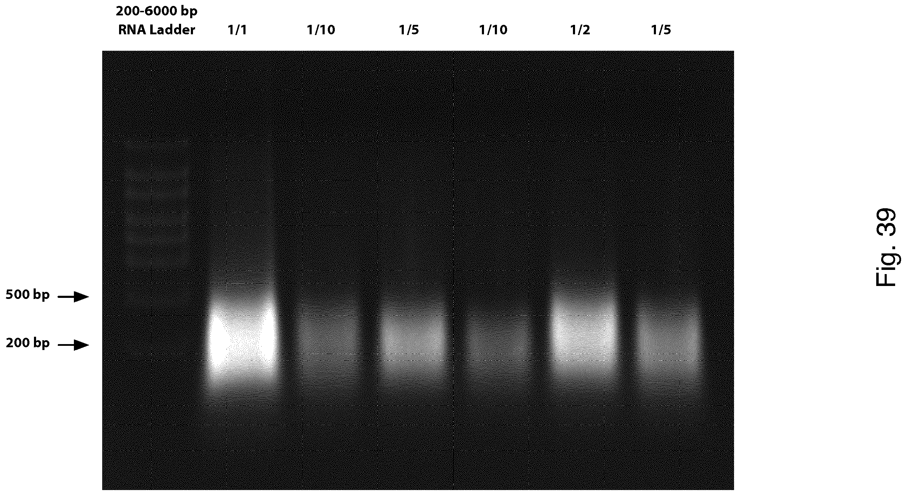

**Figure 39. Example 7 - gDNA to ssRNA with chPrimer (Transcription):**

1- RNA Ladder (200-6000bp)

2- Transcription product of 200-300bp sheared gDNA with chPrimer, Undiluted

3- Transcription product of 200-300bp sheared gDNA with chPrimer, 1/10 diluted

4- Transcription product of 200-300bp sheared gDNA with chPrimer, 1/5 diluted

5- Transcription product of 200-300bp sheared gDNA with chPrimer, 1/10 diluted

6- Transcription product of 200-300bp sheared gDNA with chPrimer, 1/2 diluted

7- Transcription product of 200-300bp sheared gDNA with chPrimer, 1/5 diluted

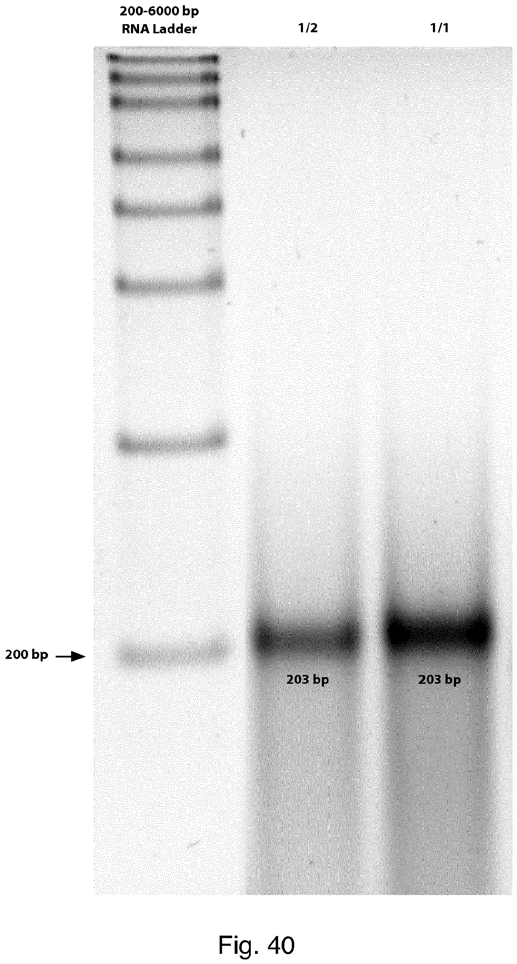

**Figure 40. Example 8 - sDNA to asRNA with dsPrimer (Transcription):**

1- RNA Ladder (200-6000bp)

2- Transcription of 200bp ssDNA with dsPrimer, 1/2 diluted

3- Transcription of 200bp ssDNA with dsPrimer, undiluted

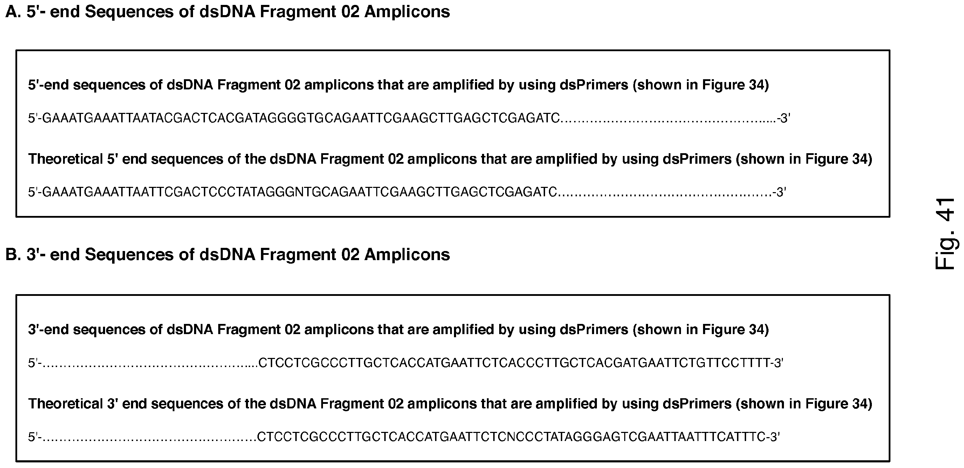

**Figure 41. Sequence Data of dsDNA Fragment 02 Amplicons Amplified by dsPrimer:**

Sequencing data of both 3'- and 5'- ends (last 60 nucleotides) of DNA Fragment 02 amplicons that are amplified by using dsPrimer method (shown in Figure 34).

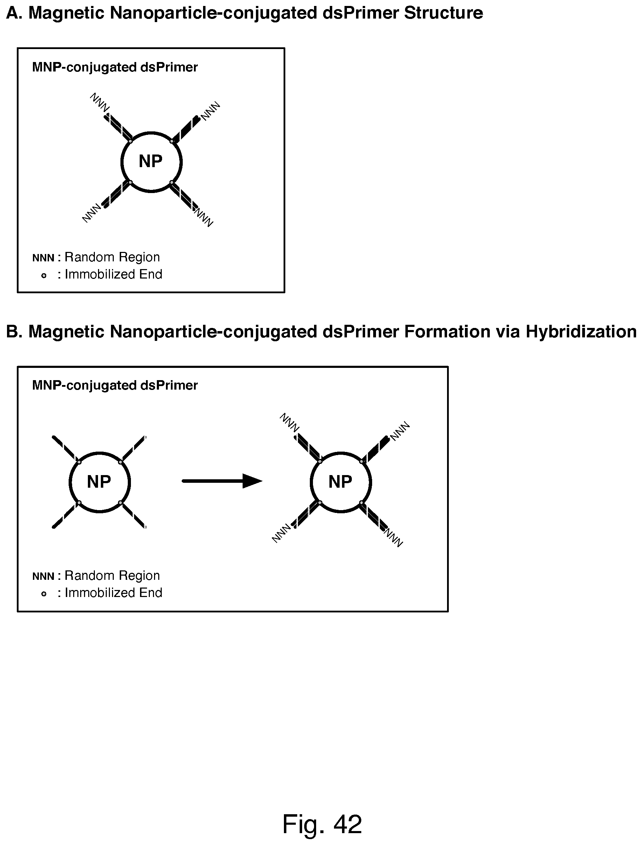

**Figure 42. Design NP1 Magnetic Nanoparticle-conjugated dsPrimer Structure**

The molecular structure of the magnetic nanoparticle-conjugated dsPrimers comprises a single stranded primer region (called "asPrimer") located in the upper strand of the dsPrimer (which also contains a degenerate 3' overhang region, e.g. hexamer) and complementary sequences in the lower strand (called "sPrimer"). dsPrimers are immobilized to the surface of magnetic nanoparticle through their 3' end lower (non-overhang) strand, while the upper (3' overhang containing) strand is not attached to the bead.

- **A. Magnetic Nanoparticle-conjugated dsPrimer Structure:**
  dsPrimers can be immobilized to the reactive surface of the magnetic nanoparticle in double-structured form via the modified 5' end of the upper strand or the modified 3' end of the lower strand.

- **B. Magnetic Nanoparticle-conjugated dsPrimer Formation via Hybridization:**
  The single-stranded 3' end of lower strand of dsPrimer structures can be immobilized in a standalone manner to the reactive surface of the magnetic nanoparticle and the complementary upper strand hybridized after this immobilization step.

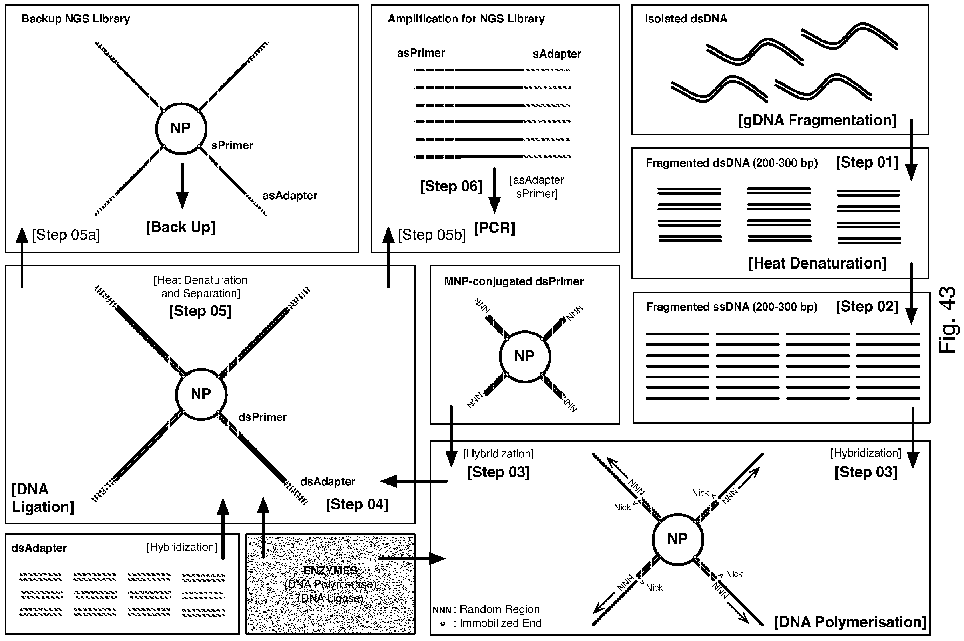

**Figure 43. Design NP2 Construction of Backup NGS Library**

The molecular structure of the magnetic nanoparticle-conjugated dsPrimers may contain a single stranded primer region (called "asPrimer") located in the upper strand of the dsPrimer (which also contains a degenerate 3' overhang region, e.g. hexamer) and complementary sequences in the lower strand (called "sPrimer") dsPrimers may be immobilized to the surface of magnetic nanoparticle through their 3' end lower (non-overhang) strand, while the upper (overhang containing) strand is not attached to the bead.

The molecular structure of the dsAdaptor, which takes the form of a conventional double stranded Adaptor molecule, contains a DNA primer site as an upper strand that contains a hydroxyl group on its 3' end, and complementary lower strand that contains a phosphate group on its 5' end. The 5' end of the upper strand and the 3' end of the lower strand lack phosphate and hydroxyl groups, respectively, which provides specificity.

The procedure begins after end-repaired fragmented DNA is obtained.

- Step 01: Genomic DNA is fragmented and end-repaired via any preferred method known in the art.
- Step 02: Fragmented dsDNA is denatured applying heat.
- Step 03: Fragmented ssDNA is hybridized with magnetic nanoparticle-conjugated dsPrimers via the random nucleotide overhang region and the resulting nick is ligated via DNA ligase while DNA polymerase fills the overhang region on the nanoparticle.
- Step 04: dsAdaptors are ligated via DNA ligase to the ds DNA fragment on the nanoparticle.
- Step 05: dsDNA fragments containing both dsAdaptor and dsPrimer regions are heat denatured.
- Step 05a: Magnetic nanoparticles containing the ssDNA templates are separated and stored as a backup NGS library. The stored backup NGS library can be used for later amplification or for the creation of the same library, or any other desired application.
- Step 05b: ssDNA fragments released via heat denaturation are separated from the nanoparticles and ready to enter sequencing, for example NGS, workflow as desired.
- Step 06: Separated ssDNA containing the added sAdaptor and asPrimer regions are amplified via PCR or other amplification methods known in the art such as Strand Displacement Amplification, Loop-mediated Isothermal Amplification, Helicase-dependant Amplification, Transcription etc.

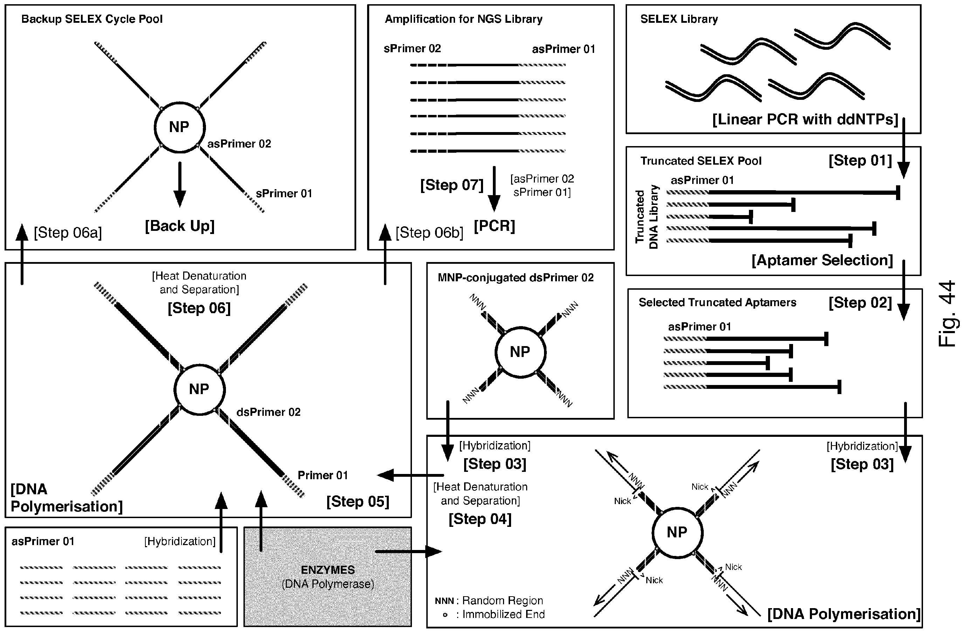

## Figure 44. Design NP3 Construction of Backup SELEX Cycle Pool

The molecular structure of the magnetic nanoparticle-conjugated dsPrimers contain a single stranded primer region (called "asPrimer") located in the upper strand of the dsPrimer (which also contains a degenerate 3' overhang region, e.g. hexamer) and complementary sequences in the lower strand (called "sPrimer").

The dsPrimers are immobilized to the surface of the magnetic nanoparticle through the 5' end of the upper (3' overhang containing) strand, while the lower strand is not attached to the bead.

- Step 01: Linear amplification of the DNA SELEX Library is performed via Linear PCR using the asPrimer 01, with ddNTPs in the reaction mixture. Resulting in truncated ssDNA molecules form the initial Truncated SELEX Pool.
- Step 02: Aptamer candidate molecules against the desired ligand(s) are selected from the single base truncated SELEX Pool using any preferred selection/separation method and secondary structures are denatured via heat and maintained at 0°C, for example, kept on ice.
- Step 03: Selected truncated ssDNA molecules are hybridized with magnetic nanoparticle-conjugated dsPrimer02s via the random nucleotide overhang region. The overhang region is filled with DNA polymerase.
- Step 04: The resulting dsDNA is heat denatured and the strand with the nick is separated from the strand on the nanoparticle. The separated nanoparticle with the immobilized ssDNA is transferred to a new tube for further processing.
- Step 05: asPrimer 01s are hybridized to the ssDNA containing dsPrimer02s on the nanoparticle and elongated by DNA polymerase to produce dsDNA containing nanoparticles.
- Step 06: dsDNA on the nanoparticles is denatured via heat and ssDNA immobilized nanoparticles are separated.
- Step 06a: The nanoparticles containing the immobilized DNA molecules are collected for storage. Stored nanoparticles can be used for repeating the cycle in which they are produced, sequencing, for example NGS, experiments, or for any other desired reaction.
- Step 06b: Free ssDNA molecules are separated and ready for, for example, linear amplification via linear PCR with DNA chain terminators and a new cycle is started by returning to first step, or linear PCR amplification is performed without chain terminators and the resulting ssDNA molecules are ready to entering sequencing, for example NGS, workflow.
- Step 07: PCR or other amplification methods known in the art such as Strand Displacement Amplification, Loop-mediated Isothermal Amplification, Helicase-dependant Amplification, Transcription etc. of ssDNA is performed.

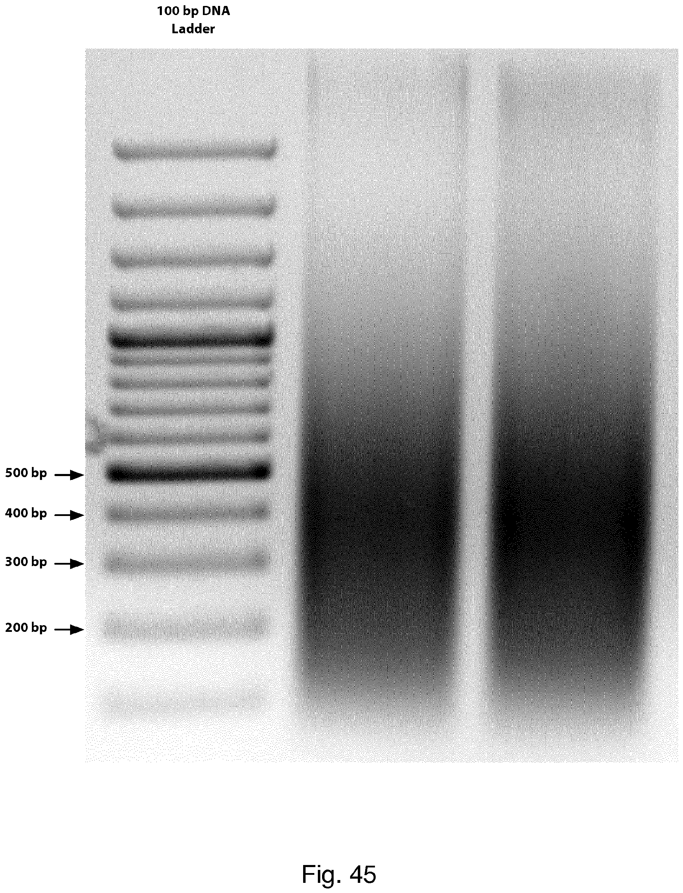

## Figure 45. Example 10A - gDNA Fragmentation

Human genomic DNA was fragmented using NEBNext dsDNA Fragmentase (New England Biolabs), run on 1% agarose after purification. 25$\mu$l of the same sample were loaded into each well. Approximately 250bp genomic DNA fragments were extracted from gel afterwards.

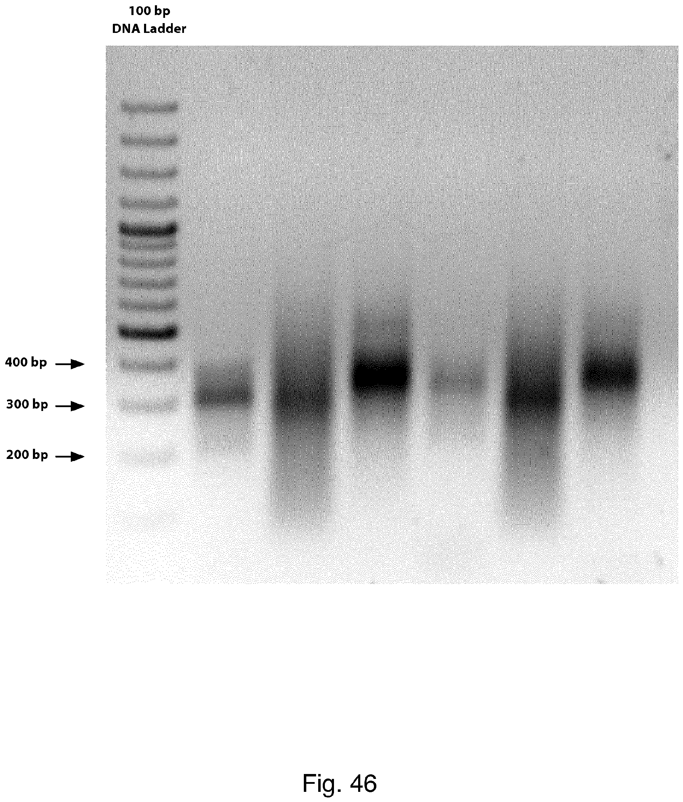

**Figure 46. Example 10C** - **gDNA to gDNA with dsPrimer (Ion Torrent Adaptation)**

Human genomic DNA fragments of approximately 250bp were amplified with dsPrimers containing Ion Torrent™ (Life Technologies, Inc) adaptor sequences. 2 different modified Ion Torrent primers with different $MgCl_2$ concentrations were used. Primer PAm adds 60 bases, Primer PBm adds 142 bases to the amplified product.

   1- 100bp plus ladder
   2- PrimerA with 2mM MgCl2. *(Approximately 310bp)*
   3- PrimerA and PrimerB with 2mM MgCl2.
   4- PrimerB with 2mM MgCl2. *(Approximately 392bp)*
   5- PrimerA with 1.5 MgCl2. *(Approximately 310bp)*
   6- PrimerA and PrimerB with 1.5mM MgCl2.
   7- PrimerB with 1.5mM MgCl2. *(Approximately 392bp)*

As discussed above several Design protocols are included in order to illustrate the methods of the invention and some preferred steps and embodiments. However, it is envisaged that a skilled person could readily derive alternative methods of the invention by combining various and appropriate steps shown in the schematic designs. In order that a skilled person can readily carry out all of the described protocols and any alternatives, the description below contains exemplary protocols for 20 of the fundamental steps (described herein as "bricks") of the technology.

**Brick 01** - **Hybridization of dsPrimer (dsPromoter, dsTerminator etc.) molecules to single-stranded nucleic acid fragments**

- Add 1μl of dsPrimer (100pmol/μl) into the reaction tube including denatured template + nuclease free $H_2O$.
- Add 2μl of 10X reaction buffer 01 into the reaction tube.
- Incubate at 25°C for 10 minutes.
- Immediately transfer the reaction tube into ice.

**Brick 02a** - **Elongation of hybridized dsPrimer (dsPromoter, dsTerminator etc.) molecules by a DNA polymerase with strand displacement activity to form double-stranded nucleic acid fragments.**

- Add 1μl of dNTP mix (10mM) into the reaction tube.
- Add 0.3μl of DNA Polymerase (10u/μl into the reaction tube.
- Incubate at 25°C for 10 minutes.
- Incubate at 37°C for 40 minutes.
- Incubate at 75°C for 10 minutes.

**Brick 02b** - **Elongation of hybridized dsPrimer (dsPromoter, dsTerminator etc.) molecules by a DNA polymerase without any strand displacement activity to form double-stranded nucleic acid fragments**

- Add 1μl of dNTP mix (10mM) into the reaction tube.

- Add 1μl of DNA polymerase (200u/μl) into the reaction tube.

- Incubate at 25°C for 10 minutes.

- Incubate at 45°C for 60 minutes.

- Incubate at 95°C for 5 minutes.

**Brick 03** - **RNA amplification by DNA-dependent RNA polymerase (RNAP) with standard NTPs**

- Add 22.25μl nuclease free H$_2$O into a nuclease free 0.2ml reaction tube.
- Add 10μl of 5X reaction buffer 02 into the reaction tube.
- Add 10μl of NTP mix (10mM) into the reaction tube.
- Add 5μl of amplification product into the reaction tube.
- Add 1.25μl of RNase inhibitor (40u/μl) into the reaction tube.
- Add 1.5μl of RNA Polymerase (20u/μl) into the reaction tube.
- Incubate at 37°C for 2 hours.

**Brick 04** - **Isothermal DNA amplification with RNase H and RNA priming by phi29 DNA polymerase with standard dNTPs**

- Add 28.85μl nuclease free H$_2$O into a 0.2ml nuclease free reaction tube.
- Add 5μl of 10X reaction buffer 03 into the reaction tube.
- Add 5μl of Elongation reaction into the reaction tube.
- Add 5μl of primer (100pmol/μl) into the reaction tube.
- Incubate the reaction tube at 95°C for 5 minutes.
- Immediately transfer the reaction tube into ice.
- Add 5μl of dNTP into the reaction tube.
- Add 0.75μl of phi29 DNA Polymerase (10u/μl) into the reaction tube.
- Add 0.4μl of RNase H (5u/μl) into the reaction tube.
- Incubate at 37°C for 4 hours.
- Incubate at 75°C for 10 minutes.

**Brick 05** - **Heat denaturation of double-stranded DNA (dsDNA) molecules followed up with ice treatment to preserve single-stranded structure of denatured fragments**

- Add 5μl of double-stranded DNA (50ng/μl) into a nuclease free 0.2ml reaction tube.

- Add 10.7μl nuclease free H$_2$O into the reaction tube.

- Incubate at 94°C for 15 minutes.

- Immediately transfer the reaction tube into ice and incubate for 3 minutes.

**Brick 06** - **Heat denaturation of double-stranded nucleic acid fragments after dsPrimer (dsPromoter, dsTerminator etc.) insertion without any ice treatment**

- Incubate the reaction tube at 95°C for 5 minutes.

**Brick 07a** - **Annealing of single-stranded nucleic acid fragments (to each other) and polymerase chain reaction (PCR) primers (to single-stranded nucleic acid fragments) after heat denaturation**

- Add 0.75 μl of each primer (100pmol/μl) into the reaction mixture from brick02.

- Incubate at 50°C for 5 minutes.

- Incubate at room temperature for 5 minutes.

**Brick 07b** - **Annealing of single-stranded nucleic acid fragments to each other after heat denaturation**

- Incubate the reaction tube at room temperature for 5 minutes.

**Brick 08a** - **Elongation of annealed single-stranded nucleic acid fragments and polymerase chain reaction (PCR) primers by a DNA polymerase to form double-stranded nucleic acid molecules**

- Add 10µl of annealing reaction from brick07a into a nuclease free 0.2ml reaction tube.
- Add 7.4µl nuclease free $H_2O$ into the reaction tube.
- Add 2µl of 10X reaction buffer 01 into the reaction tube.
- Add 0.3µl of dNTP mix (10mM) into the reaction tube.
- Add 0.3µl of DNA polymerase (10u/µl) into the reaction tube.
- Incubate at 37°C for 20 minutes.
- Incubate at 75°C for 20 minutes.

**Brick 08b** - **Elongation of annealed single-stranded nucleic acid fragments by a DNA polymerase to form double-stranded nucleic acid molecules**

- Add 7µl of annealing reaction from brick07b into a nuclease free 0.2ml reaction tube.
- Add 10.4µl nuclease free $H_2O$ into the reaction tube.
- Add 2µl of 10X reaction buffer 01 into the reaction tube.
- Add 0.3µl of dNTP mix (10mM) into the reaction tube.
- Add 0.3µl of DNA Polymerase (10u/µl) into the reaction tube.
- Incubate at 37°C for 15 minutes.
- Incubate at 75°C for 20 minutes.

**Brick 09a** - **Hot Start-Touch Down polymerase chain reaction (PCR) with one primer**

- Add 5µl of Elongation reaction mixture from brick08b into a nuclease free 0.2ml reaction tube.
- Add 72µl nuclease free $H_2O$ into the reaction tube.
- Add 10µl of 10X reaction buffer 04 into the reaction tube.
- Add 8µl of $MgCl_2$ (25mM) into the reaction tube.
- Add 2µl of dNTP mix (10mM) into the reaction tube.
- Add 2.4µl of primer (100pmol/µl) into the reaction tube.
- Add 0.6µl of Hot Start DNA Polymerase (5u/µl) into the reaction tube.
- Use the Thermal Cycling Protocol below:

  ○ Initial Denaturation: 95°C 4 minutes

  ○ 15 cycles of:

    ▪ 95°C 45 seconds

    ▪ 55-45°C 45 seconds

    ▪ 72°C 45 seconds

  ○ 20 cycles of:

- 95°C 45 seconds
- 45°C 45 seconds
- 72°C 45 seconds

○ Final Extension: 72°C 5 minutes

**Brick 09b** - **Standard polymerase chain reaction (PCR) with one primer**

- Set up the reaction on ice.
- Add 5μl of Elongation reaction mixture from brick08b into a nuclease free 0.2ml reaction tube.
- Add 72μl nuclease free $H_2O$ into the reaction tube.
- Add 10μl of 10X reaction buffer 05 into the reaction tube.
- Add 8μl of $MgCl_2$ (25mM) into the reaction tube.
- Add 2μl of dNTP mix (10mM) into the reaction tube.
- Add 2.4μl of primer (100pmol/μl) into the reaction tube.
- Add 0.6μl of DNA Polymerase (5u/μl) into the reaction tube.
- Use the Thermal Cycling Protocol below:

  ○ Initial Denaturation: 95°C 3 minutes

  ○ 30 cycles of:

  - 95°C 45 seconds
  - 45°C 45 seconds
  - 72°C 45 seconds

  ○ Final Extension: 72°C 5 minutes

**Brick 10** - **Degradation of DNA hybridized RNA molecules with RNase H**

- Add 0.2μl of RNase H (5u/μl) into the reaction tube.
- Incubate at 15°C for 1 hour.
- Incubate at 65°C for 10 minutes.

**Brick 11a** - **Hot Start-Touch Down polymerase chain reaction (PCR) with two primers**

- Add 5μl of Elongation reaction mixture from brick08b into a nuclease free 0.2ml reaction tube.
- Add 73.8μl nuclease free $H_2O$ into the reaction tube.
- Add 10μl of 10X reaction buffer 04 into the reaction tube.
- Add 7μl of $MgCl_2$ (25mM) into the reaction tube.
- Add 2μl of dNTP mix (10mM) into the reaction tube.
- Add 0.8μl of each primer (100pmol/μl) into the reaction tube.
- Add 0.6μl of Hot Start DNA Polymerase (5u/μl) into the reaction tube.
- Use the Thermal Cycling Protocol below:

  ○ Initial Denaturation: 95°C 4 minutes

  ○ 15 cycles of:

- 95°C 45 seconds
- 60-50°C 45 seconds
- 72°C 45 seconds

○ 20 cycles of:

- 95°C 45 seconds
- 50°C 45 seconds
- 72°C 45 seconds

○ Final Extension: 72°C 5 minutes

**Brick 11b** - **Standard polymerase chain reaction (PCR) with two primers**

- Set up the reaction on ice.
- Add 5μl of Elongation reaction mixture from brick08b into a nuclease free 0.2ml reaction tube.
- Add 73.8μl nuclease free $H_2O$ into the reaction tube.
- Add 10μl of 10X reaction buffer 05 into the reaction tube.
- Add 7μl of $MgCl_2$ (25mM) into the reaction tube.
- Add 2μl of dNTP mix (10mM) into the reaction tube.
- Add 0.8μl of each primer (100pmol/μl) into the reaction tube.
- Add 0.6μl of DNA Polymerase (5u/μl) into the reaction tube.
- Use the Thermal Cycling Protocol below:

○ Initial Denaturation: 95°C 3 minutes

○ 30 cycles of:

- 95°C 45 seconds
- 50°C 45 seconds
- 72°C 45 seconds

○ Final Extension: 72°C 5 minutes

**Brick 12** - **RNA amplification by DNA-dependent RNA polymerase (RNAP) with ddNTPs**

- Add 21.25μl nuclease free H2O into a nuclease free 0.2ml reaction tube.
- Add 10μl of 5X reaction buffer 02 into the reaction tube.
- Add 10μl of NTP mix (10mM) into the reaction tube.
- Add 1μl of ddNTP mix (1mM) into the reaction tube.
- Add 5μl of amplification product into the reaction tube.
- Add 1.25μl of RNase inhibitor (40u/μl) into the reaction tube.
- Add 1.5μl of RNA Polymerase (20u/μl) into the reaction tube.
- Incubate at 37°C for 2 hours.

**Brick 13a** - **Hybridization of Lock Primer after heat denaturation**

- Add 1μl of Lock Primer into the reaction tube.
- Incubate at 50°C for 10 minutes.

**Brick 13b** - **Hybridization of Lock Primer T after heat denaturation**

- Add 1μl of Lock Primer into the reaction tube.
- Incubate at 50°C for 10 minutes.

**Brick 14** - **Hybridization of oligo dT or primer region extended oligo dT molecules**

- Add 1μl of oligo dT (100pmol/μl) into the reaction tube.
- Add 2μl of 10X reaction buffer 06 into the reaction tube.
- Incubate at 42°C for 10 minutes.

**Brick 15** - **Elongation of hybridized oligo dT or primer region extended oligo dT molecules by a DNA polymerase to form double-stranded nucleic acid molecules**

- Add 1μl of dNTP mix (10mM) into the reaction tube from brick14.
- Add 1μl of DNA polymerase (200u/μl) into the reaction tube.
- Incubate at 42°C for 60 minutes.
- Incubate at 95°C for 5 minutes.

**Brick 16a** - **Polyadenylation of single-stranded RNA (ssRNA) molecules with Poly (A) polymerase**

- Add 5μl nuclease free $H_2O$ into a 0.2ml nuclease free reaction tube.
- Add 2 μl of 10X reaction buffer 07 into the reaction tube.
- Add 10 μl ssRNA (50ng/μl) into the reaction tube.
- Add 2μl of ATP (10mM) into the reaction tube.
- Add 1μl of Poly(A) Polymerase (5u/μl) into the reaction tube.
- Incubate at 37°C for 10 minutes.
- PolyA polymerase is active in RT buffer.
- For inactivation: add EDTA at a final concentration of 10mM)

**Brick 16b** - **Polyadenylation of single-stranded DNA (ssDNA) molecules with Terminal Transferase**

- Add 3.2μl nuclease free $H_2O$ into a nuclease free reaction tube.
- Add 4μl of 5X reaction buffer 08 into the reaction tube.
- Add 10μl of ssDNA (50ng/μl) into the reaction tube.
- Add 1.3μl of dATP (100 μM) into the reaction tube.
- Add 1.5μl of Terminal Transferase (20u/μl) into the reaction tube.
- Incubate at 37°C for 15 minutes.
- Incubate at 70°C for 10 minutes.

**Brick 17** - **Replication of phi6 promoter region single-stranded RNA (ssRNA) molecules by phi6 RNA-dependent RNA polymerase (RNAP) with standard NTPs**

- Add μl nuclease free $H_2O$ into a 0.2ml nuclease free reaction tube.
- Add 10μl of ssRNA (100ng/μl) into the reaction tube.
- Add 2μl of 10X reaction buffer 09 into the reaction tube.

- Add 0.4μl of ATP (10mM) into the reaction tube.
- Add 0.4μl of CTP (10mM) into the reaction tube.
- Add 0.4μl of UTP (10mM) into the reaction tube.
- Add 1.2μl of GTP (10mM) into the reaction tube.
- Incubate the reaction tube at 95°C for 2 minutes.
- Immediately transfer the reaction tube on ice, incubate for 3 minutes.
- Add 1μl of Phi6 RNA Replicase (1u/μl) into the reaction tube.
- Incubate at 32°C for 2 hours.

**Brick 18** - **Hybridization of amplified single-stranded RNA (ssRNA) fragments with each other to form double-stranded RNA (dsRNA) molecules**

- Incubate the amplification reaction at 80°C for 10 minutes.
- Incubate at room temperature for 5 minutes.

**Brick 19** - **Degradation of phosphate group (-PO$_3$) contained fragment of double-stranded DNA (dsDNA) molecules by Lambda exonuclease to obtain single-stranded DNA (ssDNA) fragments**

- Add 24μl H$_2$O into a nuclease free 0.2ml reaction tube.
- Add 5μl of 10X reaction buffer 10 into the reaction tube.
- Add 20μl of amplification product into the reaction tube.
- Add 1μl of Lambda exonuclease (10u/μl) into the reaction tube.
- Incubate at 37°C for 30 minutes.
- Incubate at 94°C for 10 minutes.

**Brick 20** - **Annealing of single-stranded nucleic acid fragments to each other after Lambda exonuclease denaturation**

- Incubate the reaction tube from brick19 at room temperature for 5 minutes.

**Brick 21** - **Sealing of the nick created by hybridizing the fragmented ssDNA with Magnetic Nanoparticle-conjugated dsPrimer and elongation of the overhang region.**

- Add 1μl of dNTP mix (10mM) into the reaction tube.
- Add 0.3μl of DNA Polymerase (10u/μl) into the reaction tube.
- Incubate at 25°C for 20 minutes.
- Add 2.33μl of ATP (10mM).
- Add 1μl DNA Ligase (3000u/μl).
- Incubate at 25°C for 30 minutes.
- Incubate at 75°C for 10 minutes

**Brick 22** - **Addition of the dsAdaptor to the dsPrimer added dsDNA fragment on the Magnetic Nanoparticle**

- Add 1.67μl nuclease free H$_2$O
- Add 1μl of 10X reaction buffer 01 into the reaction tube.
- Add 1μl dsAdaptor (100pmol/μl) into the raction tube.
- Add 1.5μl of ATP (10mM).
- Add 1μl DNA Ligase (3000u/μl).

- Incubate at 25°C for 30 minutes.
- Incubate at 75°C for 10 minutes..

**Brick 23** - **Heat denaturation of nanoparticule immobilized dsDNA**

- Incubate at 95°C for 15 minutes.
- Immediately transfer the reaction tube on to ice and incubate for 3 minutes.

**Brick 24** - **Linear amplification via linear PCR with ddNTPs for producing Truncated SELEX Pool**

- Set up the reaction on ice.
- Add 10µl of template into a nuclease free 0.2ml reaction tube.
- Add 66.4µl nuclease free $H_2O$ into the reaction tube.
- Add 10µl of 10X reaction buffer 05 into the reaction tube.
- Add 8µl of $MgCl_2$ (25mM) into the reaction tube.
- Add 2µl of dNTP mix (10mM) into the reaction tube.
- Add 1µl of ddNTP mix (0.5mM) into the reaction tube.
- Add 2µl of primer (100pmol/µl) into the reaction tube.
- Add 0.6µl of DNA Polymerase (5u/µl) into the reaction tube.
- Use the Thermal Cycling Protocol below:

    ◦ Initial Denaturation: 95°C 3 minutes
    ◦ 36 cycles of:

        ▪ 95°C 45 seconds
        ▪ (Annealing temperature for the desired primer) 45 seconds
        ▪ 72°C 20 seconds

    ◦ Final Extension: 72°C 3 minutes

**Brick 25** - **Heat denaturation of ssDNA to denature secondary structures**

- Add 10µl of selected ssDNA into a nuclease free 0.2ml reaction tube.
- Add 4.7µl nuclease free $H_2O$ into the reaction tube.
- Incubate at 94°C for 5 minutes.
- Immediately transfer the reaction tube into ice and incubate for 3 minutes.

**Brick 26** - **Hybridization of asPrimer 01 and elongation via DNA polymerase**

- Add 1µl of asPrimer (100pmol/µl) 01 into the reaction.
- Add 1µl of dNTP mix (10mM) into the reaction tube.
- Add 1µl of DNA polymerase (200u/µl) into the reaction tube.
- Incubate at 45°C for 45 minutes.
- Incubate at 95°C for 5 minutes.

**Brick 27** - **Amplification of ssDNA via PCR**

- Set up the reaction on ice.
- Add 10μl of template into a nuclease free 0.2ml reaction tube.
- Add 66.4μl nuclease free H$_2$O into the reaction tube.
- Add 10μl of 10X reaction buffer 05 into the reaction tube.
- Add 8μl of MgCl$_2$ (25mM) into the reaction tube.
- Add 2μl of dNTP mix (10mM) into the reaction tube.
- Add 1μl of ddNTP mix (0.5mM) into the reaction tube.
- Add 1μl of asPrimer 02 (100pmol/μl) into the reaction tube.
- Add 1μl of sPrimer 01 (100pmol/μl) into the reaction tube.
- Add 0.6μl of DNA Polymerase (5u/μl) into the reaction tube.
- Use the Thermal Cycling Protocol below:

  ◦ Initial Denaturation: 95°C 3 minutes
  ◦ 30 cycles of:

    ▪ 95°C 45 seconds
    ▪ (Annealing temperature for the desired primer) 45 seconds
    ▪ 72°C 20 seconds

  ◦ Final Extension: 72°C 3 minutes

**Reaction Buffers:**

- **10X reaction buffer 01:** 500 mM Tris-HCl (pH 8.0 at 25°C), 50 mM MgCl$_2$, 10 mM DTT.

- **5X reaction buffer 02:** 200 mM Tris-HCl (pH 7.9 at 25°C), 30 mM MgCl$_2$,

- 50 mM DTT, 50 mM NaCl and 10 mM spermidine.

- **10X reaction buffer 03:** 330 mM Tris-acetate (pH 7.9 at 37°C), 100 mM Mg-acetate, 660 mM K-acetate, 1% (v/v) Tween 20, 10 mM DTT.

- **10X reaction buffer 04:** 200 mM Tris-HCl (pH 8.3 at 25°C), 200 mM KCl, 50 mM (NH$_4$)$_2$SO$_4$.

- **10X reaction buffer 05:** 750 mM Tris-HCl (pH 8.8 at 25°C), 200 mM (NH$_4$)$_2$SO$_4$, 0.1% (v/v) Tween 20.

- **10X reaction buffer 06:** 500 mM Tris-HCl (pH 8.3), 500 mM KCl, 40 mM MgCl$_2$, 100 mM DTT.

- **10X reaction buffer 07:** 500 mM Tris-HCl(pH 7.9 at 25°C), 2,5M NaCl, 100 mM MgCl$_2$.

- **5X reaction buffer 08:** 1 M potassium cacodylate, 0.125 M Tris, 0.05% (v/v) Triton X-100, 5 mM CoCl$_2$ (pH 7.2 at 25°C).

- **10X reaction buffer 09:** 500 mM Tris-acetate (pH 8.75 at 21°C), 500 mM NH$_4$Ac, 15 mM MnCl$_2$.

- **10X reaction buffer 10:** 670 mM glycine-KOH (pH 9.4), 25 mM MgCl$_2$, 0.1% (v/v) Triton X-100.

**Clauses**

Further embodiments of the invention are set out in the numbered clauses below.

1. A method of nucleic acid manipulation comprising:

(i) hybridizing a double stranded primer to the 3' end of a single stranded nucleic acid template, wherein said primer comprises a double stranded region and a single stranded region, wherein the single stranded region is a 3' overhang region and wherein the 3' overhang region enables the double stranded primer to target and hybridize to the 3' end of the nucleic acid template, wherein said single stranded 3' overhang region comprises a degenerate sequence or a sequence comprising universal bases, and wherein the double stranded primer is made up of two separate strands;

(ii) at least one round of polymerization using a polypeptide with 5' to 3' DNA polymerization activity, wherein at least the first round of polymerization comprises using a polypeptide with 5' to 3' DNA polymerization activity to carry out a primer extension reaction to synthesise nucleotides in a template dependent manner from the 3' end of the single stranded region of said hybridized double stranded primer;

wherein the hybridizing step (i) and at least the first primer extension reaction from the 3' end of the single stranded region of said hybridized double stranded primer in step (ii) takes place without the formation of a phosphodiester bond between the 3' end of the nucleic acid template and the double stranded primer of step (i).

2. A method of nucleic acid amplification comprising:

(i) hybridizing a double stranded primer to the 3' end of a single stranded nucleic acid template, wherein said primer comprises a double stranded region and a single stranded region, wherein the single stranded region is a 3' overhang region and wherein the 3' overhang region enables the double stranded primer to target and hybridize to the 3' end of the nucleic acid template, wherein said single stranded 3' overhang region comprises a degenerate sequence or a sequence comprising universal bases, and wherein the double stranded primer is made up of two separate strands;

(ii) at least one round of polymerization using a polypeptide with 5' to 3' DNA polymerization activity in order to form an amplification template, wherein at least the first round of polymerization comprises using a polypeptide with 5' to 3' DNA polymerization activity to carry out a primer extension reaction to synthesise nucleotides in a template dependent manner from the 3' end of the single stranded region of said hybridized double stranded primer; and

(iii) at least one amplification step to form an amplified nucleic acid product;

wherein the hybridizing step (i) and at least the first primer extension reaction from the 3' end of the single stranded region of said hybridized double stranded primer in step (ii) takes place without the formation of a phosphodiester bond between the 3' end of the nucleic acid template and the double stranded primer of step (i).

3. The method of clause 1 or clause 2, wherein said double stranded region of said double stranded primer comprises a nucleic acid fragment to be joined to said nucleic acid template, a primer binding site, a control region, a promoter, a terminator, an operator, a repressor, an enhancer, a silencer, an insulator or other response element, a sequencing adaptor region or other element permitting or facilitating sequencing, a restriction site, a label, an identifier sequence, a barcode, a forensic signature, or combinations thereof.

4. The method of any one of clauses 1 to 3, wherein said double stranded primer comprises a further single stranded 3' overhang region, and preferably wherein said double stranded region of said double stranded primer comprises one or more of a nucleic acid fragment to be joined to said nucleic acid template, a primer binding site, a control region, a promoter, a terminator, an operator, a repressor, an enhancer, a silencer, an insulator or other response element, a sequencing adaptor region or other element permitting or facilitating sequencing, a restriction site, a label, an identifier sequence, a barcode, or a forensic signature, which may be the same or different, or combinations thereof.

5. The method of any one of clauses 1 to 4, wherein said single stranded 3' overhang region comprises universal bases selected from the group consisting of inosine, 2-amino purine, diaminopurine, 5-nitroindole, 5-methyl isodeoxycytosine, iso deoxyguanine, or a mixture thereof.

6. The method of any one of clauses 1 to 5, wherein said single stranded 3' overhang region is up to 6 or 12 base pairs in length.

7. The method of any one of clauses 1 to 6 wherein a single type of double stranded primer is used.

8. The method of any one of clauses 1 to 7 wherein two or more different types of double stranded primers are used.

9. The method of clause 8, wherein the two or more different types of double stranded primer contain different primer binding sites, or wherein one double stranded primer contains a promoter region and the other a terminator region, or wherein one double stranded primer contains a promoter region and the other a primer binding site.

10. The method of any one of clauses 1 to 9, wherein one or more of the double stranded primers is a hybrid double stranded primer wherein one strand comprises DNA and the other strand comprises RNA.

11. The method of any one of clauses 1 to 10, wherein said method further comprises the use of a lock primer which cannot be subjected to chain elongation.

12. The method of any one of clauses 1 to 11, wherein said method is carried out under isothermal conditions.

13. The method of any one of clauses 1 to 12, wherein said method is used in sequencing applications, nucleic acid labelling, joining nucleic acid fragments together, or for incorporation of primer sites, promoter sites, terminator sites and/or restriction sites.

14. The method of any one of clauses 1 to 13, wherein one or more strands of one or more of the double stranded primers comprises a chimeric RNA/DNA or DNA/RNA sequence.

15. The method of any one of clauses 1 to 14, wherein the double stranded primer is immobilized to a solid phase before step (i) is carried out.

16. The method of clause 15, wherein the solid phase is a nanoparticle, preferably a magnetic nanoparticle.

17. A method of nucleic acid manipulation comprising a hybridizing step wherein a double stranded primer is hybridized to the 3' end of a single stranded nucleic acid template, wherein said primer comprises a double stranded region and a single stranded region, wherein the single stranded region is a 3' overhang region and wherein the 3' overhang region enables the double stranded primer to target and hybridize to the 3' end of the nucleic acid template, wherein said single stranded 3' overhang region comprises a degenerate sequence or a sequence comprising universal bases, and wherein the double stranded primer is made up of two separate strands;
wherein said hybridizing step takes place without the formation of a phosphodiester bond between the 3' end of the nucleic acid template and the double stranded primer of the hybridizing step.

18. A method of nucleic acid manipulation comprising a hybridizing step wherein a double stranded primer is hybridised to the 5' end of a single stranded nucleic acid template, wherein said primer comprises a double stranded region and a single stranded region, wherein the single stranded region is a 5' overhang region and wherein the 5' overhang region enables the double stranded primer to target and hybridize to the 5' end of the nucleic acid template, wherein said single stranded 5' overhang region comprises a degenerate sequence or a sequence comprising universal bases, and wherein the double stranded primer is made up of two separate strands;
wherein said hybridizing step takes place without the formation of a phosphodiester bond between the 5' end of the nucleic acid template and the double stranded primer of the hybridising step.

19. A method of Systematic Evolution of Ligands by Exponential Enrichment (SELEX) comprising:

(a) contacting a library of single stranded nucleic acid molecules with a ligand of interest, wherein said library is a library of truncated nucleic acid molecules in which the 3' ends of the nucleic acid molecules do not have a 3'OH group;
(b) selecting for nucleic acid molecules which bind said ligand;
(c) use of a double stranded primer to amplify the nucleic acid molecules which bind to said ligand, wherein said double stranded primer hybridizes to the 3' end of the single stranded molecules selected in step (b), wherein said primer comprises a double stranded region and a single stranded region, wherein the single stranded region is a 3' overhang region and wherein the 3' overhang region enables the double stranded primer to target and hybridize to the 3' end of the molecules selected in step (b), wherein said single stranded 3' overhang region comprises a degenerate sequence or a sequence comprising universal bases, and wherein the double stranded primer is made up of two separate strands; and optionally
(d) use said amplified nucleic acid molecules as a new library of nucleic acid molecules to repeat steps (a) to (c) one or more times.

20. The method of clause 19, wherein the libraries of step (a) are produced using a polypeptide with RNA or DNA polymerase activity in the presence of chain terminators.

21. The method of clause 19 or clause 20, wherein said amplification of nucleic acid molecules in step (c) is carried out in accordance with any one of clauses 2 to 16.

22. The method of any one of clauses 19 to 21, wherein said library of step (a) contains nucleic acid molecules of a predetermined fixed length and nucleic acid molecules with decreasing lengths from said predetermined fixed length.

23. The method of any one of clauses 19 to 22, wherein the amplification products produced in step (c) are nucleic acid molecules which do not contain additional oligonucleotide regions, such as constant or fixed primer binding sites, promoter, terminator, adaptor regions or other element permitting or facilitating function as a primer binding site, at the 5' or 3' end of the molecule, or only contain such additional oligonucleotide regions at the 5' end of the molecule.

24. The method of any one of clauses 19 to 23, wherein said method step (c) is carried out without the formation of a phosphodiester bond between the 3' end of the nucleic acid molecules selected in step (b) and the double stranded primer.

25. The method of any one of clauses 19 to 24, wherein a single stranded nucleic acid molecule which can specifically bind to said ligand is identified.

26. A solid phase comprising immobilized double stranded primers, wherein said double stranded primers comprise a double stranded region and a single stranded region, wherein the single stranded region is a 3' overhang region and wherein the 3' overhang region enables the double stranded primer to target and hybridize to the 3' end of a single stranded nucleic acid template, wherein said single stranded 3' overhang region comprises a degenerate sequence or a sequence comprising universal bases, and wherein the double stranded primer is made up of two separate strands.

27. The solid phase of clause 26, wherein said double stranded primers are immobilized to the solid phase through either the 5' end of the strand containing the 3' overhang region or the 3' end of the non-overhang strand.

28. The solid phase of clause 26 or clause 27, wherein said solid phase is a nanoparticle, preferably a magnetic nanoparticle.

29. A method of preparing a library, comprising the following steps:

(A) hybridizing single stranded nucleic acid templates to the solid phase of clause 27 or clause 28;
(B) polymerization using a polypeptide with 5' to 3' DNA polymerization activity to carry out a primer extension reaction to synthesize nucleotides in a template dependent manner from the 3' end of the single stranded region of the dsPrimer;
(C) removing the non-immobilized strands from the solid phase, wherein said non-immobilized strands form a library of single stranded molecules; optionally
(D) retaining the immobilized single stranded molecules as a backup library.

30. The method of clause 29 comprising one or more of the following additional steps:

(E) after the hybridization step (A) and before step (C) carrying out a step in which the gap between the hybridized single stranded nucleic acid molecules and the 5' end of the strand of the dsPrimer which does not contain the 3' overhang region is repaired;
(F) after the polymerization step (B) and before step (C) ligating a double stranded adaptor molecule to the free ends of the immobilized double stranded polymerized product which are not in contact with the solid phase.

31. A method of preparing a library, comprising the following steps:

(A) hybridizing single stranded nucleic acid templates, wherein said templates comprise a known primer sequence at the 5' end, to the solid phase of clause 27 or clause 28, wherein the double stranded primers of said solid phase are immobilized to the solid phase through the 5' end of the strand containing the 3' overhang region;
(B) polymerization using a polypeptide with 5' to 3' DNA polymerization activity to carry out a primer extension reaction to synthesize nucleotides in a template dependent manner from the 3' end of the single stranded region of the dsPrimer;

(C) removing the non-immobilized strands containing the nucleic acid template molecules from the solid phase;

(D) resynthesizing the nucleic acid template molecules using a single stranded primer which hybridizes to the complement of the known primer site which has been incorporated at the 3' end of the polymerized strand generated in step (B), hybridizing said primer, after which an enzyme with 5' to 3' DNA polymerization activity is used to carry out a primer extension reaction to synthesize nucleotides in a template dependent manner from the 3' end of said single stranded primer;

(E) removing the non-immobilized strands from the solid phase, wherein said non-immobilized strands form a library of single stranded molecules; optionally

(F) retaining the immobilized single stranded molecules as a backup library.

32. The method of clause 31, wherein the single stranded nucleic acid templates in step (A) are candidate SELEX aptamers, preferably truncated SELEX aptamers.

33. A Library or backup library produced by the methods of any one of clauses 29 to 32.

34. A double stranded primer which comprises a double stranded region and two single stranded regions, wherein the single stranded regions are 3' overhang regions which are present on the 3' end of each strand of the double stranded primer, wherein said single stranded 3' overhang regions comprise a degenerate sequence or a sequence comprising universal bases, and wherein the double stranded primer is made up of two separate strands.

35. The double stranded primer of clause 34 wherein said double stranded region of said double stranded primer comprises a nucleic acid fragment to be joined to said nucleic acid template, a primer binding site, a control region, a promoter, a terminator, an operator, a repressor, an enhancer, a silencer, an insulator or other response element, a sequencing adaptor region or other element permitting or facilitating sequencing, a restriction site, a label, an identifier sequence a barcode or a forensic signature, or combinations thereof.

36. The double stranded primer of any one of clauses 34 or 35, wherein said single stranded 3' overhang region comprises universal bases selected from the group consisting of inosine, 2-amino purine, diaminopurine, 5-nitroindole, 5-methyl isodeoxycytosine, iso deoxyguanine, or a mixture thereof.

37. The double stranded primer of any one of clauses 34 to 36, wherein said single stranded 3' overhang region is up to 6 or 12 base pairs in length.

38. The double stranded primer of any one of clauses 34 to 37, wherein the double stranded primers is a hybrid double stranded primer wherein one strand comprises DNA and the other strand comprises RNA.

39. The double stranded primer of any one of clauses 34 to 38, wherein the double stranded primer is a lock primer which cannot be subjected to chain elongation.

40. The double stranded primer of any one of clauses 34 to 39, wherein one or more strands of the double stranded primer comprises a chimeric RNA/DNA or DNA/RNA sequence

41. A double stranded primer comprising a double stranded region and a single stranded region, wherein the single stranded region is a 3' overhang region comprising a degenerate sequence or a sequence comprising universal bases, wherein when only one of the strands has a 3' overhang region then the strand which does not contain the 3' overhang region does not have a 5'- $PO_3$ group, and wherein when both of the strands have a 3' overhang region then one or both of the strands does not have a 5'- $PO_3$ group.

42. The double stranded primer of clause 41 wherein said lack of a 5'- $PO_3$ group prevents incorporation of said strand which lacks the 5'- $PO_3$ group into a nucleic acid template primed by the primer.

43. A composition comprising the double stranded primer of clause 41 or clause 42 and a nucleic acid template wherein the double stranded primer is hybridized to the 3' end of the nucleic acid template but the strand which lacks the 5'- $PO_3$ group is not incorporated into the nucleic acid template with a covalent bond and may be removed by heat denaturation, strand displacement or nick translation.

44. The double stranded primer of any one of clauses 41 to 43 wherein the 3' overhang region enables the double stranded primer to target and hybridize to the 3' end of a nucleic acid template.

45. The double stranded primer of any one of clauses 41 to 44, wherein the double stranded region of said double stranded primer comprises: a nucleic acid fragment to be joined to said nucleic acid template, a primer binding site, a control region, a promoter, a terminator, an operator, a repressor, an enhancer, a silencer, an insulator or other response element, a sequencing adaptor region or other element permitting or facilitating sequencing, a restriction site, a label, an identifier sequence, a barcode, a forensic signature, or combinations thereof.

46. A kit or composition comprising the solid phase of any one of clauses 26 to 28 or the double stranded primer of any one of clauses 34 to 42 or 44 to 45

47. A kit or composition comprising:

(i) a double stranded primer which comprises a double stranded region and a single stranded region, wherein the single stranded region is a 3' overhang region and wherein the 3' overhang region enables the double stranded primer to target and hybridize to the 3' end of a single stranded nucleic acid template, wherein said single stranded 3' overhang region comprises a degenerate sequence or a sequence comprising universal bases, wherein the double stranded primer is made up of two separate strands, and wherein said double stranded region of said double stranded primer comprises a promoter region; and
(ii) a double stranded primer which comprises a double stranded region and a single stranded region, wherein the single stranded region is a 3' overhang region and wherein the 3' overhang region enables the double stranded primer to target and hybridize to the 3' end of a single stranded nucleic acid template, wherein said single stranded 3' overhang region comprises a degenerate sequence or a sequence comprising universal bases, wherein the double stranded primer is made up of two separate strands, and wherein said double stranded region of said double stranded primer comprises a terminator region or a primer binding site.

48. A kit or composition comprising:

(i) a single type of double stranded primer wherein said double stranded primer comprises a double stranded region and a single stranded region, wherein the single stranded region is a 3' overhang region and wherein the 3' overhang region enables the double stranded primer to target and hybridize to the 3' end of a single stranded nucleic acid template, wherein said single stranded 3' overhang region comprises a degenerate sequence or a sequence comprising universal bases, wherein the double stranded primer is made up of two separate strands and wherein said double stranded region of said double stranded primer comprises a primer binding site; and, optionally
(ii) a single type of single stranded primer which can hybridise to said primer binding site.

49. A SELEX kit or composition comprising:

(i) a double stranded primer which comprises a double stranded region and a single stranded region, wherein the single stranded region is a 3' overhang region and wherein the 3' overhang region enables the double stranded primer to target and hybridize to the 3' end of a single stranded nucleic acid template, wherein said single stranded 3' overhang region comprises a degenerate sequence or a sequence comprising universal bases, wherein the double stranded primer is made up of two separate strands; and
(ii) a library of single stranded nucleic acid aptamers, wherein said library is a library of truncated nucleic acid molecules in which the 3' ends of the nucleic acid molecules do not have a 3'OH group.

50. The solid phase of any one of clauses 26 to 28 or the double stranded primer of any one of clauses 34 to 42 or 44 to 45 for use in sequencing applications, nucleic acid labelling, joining nucleic acid fragments together, or for incorporation of primer sites, terminator sites and/or restriction sites.

The invention and some preferred embodiments will now be described in more detail in the following non-limiting Examples with reference to the Figures 33 to 46.

**Examples**

**Example 1. dsDNA to dsDNA with dsPrimer (Optimization):**

DNA Fragment 02 was primed with dsPrimer and amplification primers were inserted into the fragment with the following procedure:

- 5$\mu$l of fragment 02 (50ng/$\mu$l) added to a nuclease free 0.2ml reaction tube.
- 10.7$\mu$l nuclease free $H_2O$ was added to the reaction tube.
- The sample was Incubated at 94°C for 15 minutes and immediately transferred on to ice and incubated for 3 minutes.
- 1$\mu$l of dsPrimer (100pmol/$\mu$l) was added
- 2$\mu$l of 10X reaction buffer (500mM Tris-Hcl (pH 8.0 at 25°C), 50mM $MgCl_2$, 10mM DTT) was added to the reaction tube.
- The sample was incubated at 25°C for 10 minutes and immediately transferred on to ice.
- 1$\mu$l of dNTP mix (10mM) and 0.3$\mu$l of DNA Polymerase (10u/$\mu$l, DNA polymerase I, Large (Klenow) Fragment with strand displacement activity) was added to the sample and incubated with the following thermal cycling protocol:

  ○ 25°C for 10 minutes.
  ○ 37°C for 40 minutes.
  ○ 95°C for 5 minutes.
  ○ Hold at 95°C.

- The sample was incubated at room temperature for 5 minutes.
- 7$\mu$l of the sample was transferred into a new nuclease free 0.2ml reaction tube.
- 10.4$\mu$l nuclease-free $H_2O$ was added to the reaction tube.
- 2$\mu$l of 10X reaction buffer (500mM Tris-Hcl (pH 8.0 at 25°C), 50mM MgCl2, 10mM DTT) was added to the reaction tube.
- 0.3$\mu$l dNTP mix (10mM) and 0.3$\mu$l DNA Polymerase (10u/$\mu$l) was added to the reaction tube and the sample was incubated with the following thermal cycling protocol:

  ○ 37°C for 15 minutes
  ○ 75°C for 20 minutes

The obtained dsPrimer inserted dsDNA fragment was amplified with the following Hot-Start Touch-Down protocol (with 2mM $MgCl_2$ and 2.4$\mu$M primer reaction lane 5)

- Template: 5$\mu$l (from previous reaction)
- Nuclease-free $H_2O$: 72$\mu$l
- 10X reaction buffer: 10$\mu$l (200 mM Tris-HCl (pH 8.3 at 25°C), 200 mM KCl, 50 mM $(NH_4)_2SO_4$)
- $MgCl_2$(25mM) : 8$\mu$l
- dNTP mix (10mM): 2$\mu$l
- Primer (100pmol/$\mu$l) : 2.4$\mu$l
- Hot Start DNA Polymerase (5u/$\mu$l): 0.6$\mu$l
- Thermal Cycling protocol:

  ○ Initial Denaturation: 95°C 4 minutes
  ○ 15 cycles:

    ▪ 95°C 45 seconds
    ▪ 55-45°C 45 seconds (Touch-down)
    ▪ 72°C 45 seconds

  ○ 20 cycles:

    ▪ 95°C 45 seconds
    ▪ 45°C 45 seconds
    ▪ 72°C 45 seconds

◦ Final Extension 72°C 5minutes
◦ Final Hold: 10°C

5μl of each sample were run with 1kb DNA ladder on 1% agarose gel 5V/cm for 45 minutes (results shown in Figure 33). The MgCl$_2$ and primer concentrations are varied in each sample in order to optimize the PCR conditions. Sequences of oligonucleotides are shown below:

*DNA Fragment 02*

Source: EGFP region of Clontech's pEGFP-C1 vector.
Size: 764 bp dsDNA region.
Sequence:
>EGFP

TGCAGAATTCGAAGCTTGAGCTCGAGATCTGAGTCCGGACTTGTACAGCTCGT
CCATGCCGAGAGTGATCCCGGCGGCGGTCACGAACTCCAGCAGGACCATGT
GATCGCGCTTCTCGTTGGGGTCTTTGCTCAGGGCGGACTGGGTGCTCAGGTA
GTGGTTGTCGGGCAGCAGCACGGGGCCGTCGCCGATGGGGGTGTTCTGCTG
GTAGTGGTCGGCGAGCTGCACGCTGCCGTCCTCGATGTTGTGGCGGATCTTG
AAGTTCACCTTGATGCCGTTCTTCTGCTTGTCGGCCATGATATAGACGTTGTG
GCTGTTGTAGTTGTACTCCAGCTTGTGCCCCAGGATGTTGCCGTCCTCCTTGA
AGTCGATGCCCTTCAGCTCGATGCGGTTCACCAGGGTGTCGCCCTCGAACTT
CACCTCGGCGCGGGTCTTGTAGTTGCCGTCGTCCTTGAAGAAGATGGTGCGC
TCCTGGACGTAGCCTTCGGGCATGGCGGACTTGAAGAAGTCGTGCTGCTTCA
TGTGGTCGGGGTAGCGGCTGAAGCACTGCACGCCGTAGGTCAGGGTGGTCA
CGAGGGTGGGCCAGGGCACGGGCAGCTTGCCGGTGGTGCAGATGAACTTCA
GGGTCAGCTTGCCGTAGGTGGCATCGCCCTCGCCCTCGCCGGACACGCTGA
ACTTGTGGCCGTTTACGTCGCCGTCCAGCTCGACCAGGATGGGCACCACCCC
GGTGAACAGCTCCTCGCCCTTGCTCACCATGAATTCTC

*dsPrimer*

Source: Artificial
Size: Upper strand contains 36 DNA bp with 6 degenerate DNA bases. Lower strand contains 30 DNA bp. It also contain a T7 promoter (bold and underlined).
Sequence:

**T7 Promoter**

5'-GAAATGAAATTAA**TACGACTCACTATAGGG**NNNNNN-3'

3'-CTTTACTTTAATTATGCTGAGTGATATCCC-5'

*Single stranded Primer (for use with dsPrimer)*

Source: Artificial
Size: Single stranded primer contains artificially synthesized 20 DNA bp.
Sequence:
5'-GAAATGAAATTAATACGACT-3'

**Example 2. dsDNA to dsDNA with dsPrimer (Size comparison to standard PCR):**

dsDNA fragment 02 was amplified using the protocol given in Example 1: dsDNA to dsDNA with dsPrimer (Optimization)
dsDNA fragment 02 was also amplified using the DNA Fragment 02 Primers with the following Hot-Start PCR protocol:

- Template: 2μl (10ng/μl)
- Nuclease-free $H_2O$: 73,4μl
- 10X reaction buffer: 10μl (200 mM Tris-HCl (pH 8.3 at 25°C), 200 mM KCl, 50 mM $(NH_4)_2SO_4$)
- $MgCl_2$(25mM): 10μl
- dNTP mix (10mM): 2μl
- Forward primer(100pmol/μl): 1μl
- Reverse Primer (100pmol/μl) : 1μl
- Hot Start DNA Polymerase(5u/μl): 0.6μl
- Thermal Cycling protocol:

  ○ Initial Denaturation: 95°C 4 minutes
  ○ 32 cycles:

     ▪ 95°C 45 seconds
     ▪ 50°C 45 seconds
     ▪ 72°C 45 seconds

  ○ Final Extension 72°C 5minutes
  ○ Final Hold: 10°C

5μl of each sample were run with 50bp DNA ladder on 1.2% Agarose Gel 5V/cm for 60 minutes (results shown in Figure 34). In one experiment, a double-stranded DNA fragment was amplified with dsPrimer, and in another experiment with the fragment specific primers to compare the sizes of the bands; the size difference due to the primer regions formed at both ends by dsPrimer is evident.

Sequences and details of DNA Fragment 02, dsPrimer and Single stranded Primer (for use with dsPrimer) are as shown in Example 1.

*DNA Fragment 02 Primer Sequences*

Source: Artificial
Size: Forward primer contains artificially synthesized 19 DNA bp and reverse primer contains artificially synthesized 20 DNA bp.
Sequence:

Forward Primer: 5'-CATGGTGAGCAAGGGCGAG-3'
Reverse Primer: 5'-TGCAGAATTCGAAGCTTGAG-3'

**Example 3. dsDNA to ssRNA with dsPrimer (Transcription):**

dsDNA fragment 02 was amplified using the protocol given in Example 2: dsDNA to dsDNA with dsPrimer, the resulting PCR product was used as the template for the following transcription protocol:

- Nuclease-free H$_2$O : 22.25$\mu$l
- 5x Reaction Buffer: 10$\mu$l (200 mM Tris-HCl (pH 7.9 at 25°C), 30 mM MgCl$_2$, 50 mM DTT, 50 mM NaCl and 10 mM spermidine).
- NTP Mix (10mM): 10$\mu$l
- Template: 5$\mu$l
- Rnase inhibitor (40u/$\mu$l): 1.25$\mu$l
- RNA Polymerase (20u/$\mu$l): 1.5$\mu$l
- Incubate at 37°C for 2 hours

5$\mu$l of the transcription product was run with RNA ladder (100-1000bp) on 1.5% agarose gel at 5V/cm for 1 hour (results shown in Figure 35). This experiment shows successful in-vitro transcription of a double-stranded DNA amplicon that has been amplified by the T7 promoter containing dsPrimer.

Sequences and details of DNA Fragment 02, dsPrimer and Single stranded Primer (for use with dsPrimer) are as shown in Example 1.

**Example 4. gDNA to gDNA with chPrimer:**

Sheared gDNA of several different size ranges was amplified using the protocol given in gDNA to gDNA with chPrimer (Example 5).

5$\mu$l of each sample was run with 100bp DNA ladder on 1.2% agarose gel at 5V/cm for 45 minutes (results shown in Figure 36). This experiment shows that gDNA sequences of different sizes were successfully amplified with chPrimer, with the range of the bands having increased by the length of chPrimers.

Sequences and details of Genomic DNA (gDNA), chPrimer and Single stranded Primer Sequences (for use with chPrimer) are as shown in Example 5.

**Example 5. gDNA to gDNA with chPrimer (Size comparison of single and multiple primer sites):**

Sheared gDNA of 300-400bp and 400-500bp was primed with chPrimer and amplification primers were inserted into the sheared gDNA using the following procedure:

- 5$\mu$l of sheared gDNA (25ng/$\mu$l) was added to a nuclease free 0.2ml reaction tube.
- 10.7$\mu$l nuclease free H$_2$O was added to the reaction tube.
- The sample was incubated at 94°C for 15 minutes and immediately transferred onto ice and incubated for 3 minutes.
- 1$\mu$l of chPrimer (100pmol/$\mu$l) was added
- 2$\mu$l of 10X reaction buffer (500mM Tris-Hcl (pH 8.0 at 25°C), 50mM MgCl$_2$, 10mM DTT) was added to the reaction tube.
- The sample was incubated at 25°C for 10 minutes and immediately transferred onto ice.
- 1$\mu$l of dNTP mix (10mM) and 0.3$\mu$l of DNA Polymerase (10u/$\mu$l) was added to the sample and incubated with the following thermal cycling protocol:

  ○ 25°C for 10 minutes
  ○ 37°C for 40 minutes
  ○ 95°C for 5 minutes
  ○ Hold at 95°C

- The sample was incubated at room temperature for 5 minutes.
- 10$\mu$l of the sample was transferred into a new nuclease free 0.2ml reaction tube.
- 7.4$\mu$l nuclease-free H$_2$O was added to the reaction tube.
- 2$\mu$l of 10X reaction buffer (500mM Tris-Hcl (pH 8.0 at 25°C), 50mM MgCl$_2$, 10mM DTT) was added to the reaction tube.
- 0.3$\mu$l dNTP mix (10mM) and 0.3$\mu$l DNA Polymerase (10u/$\mu$l) was added to the reaction tube and the sample was incubated with the following thermal cycling protocol:

  ○ 37°C for 15 minutes
  ○ 75°C for 20 minutes

The obtained chPrimer inserted sheared gDNA was amplified with the following Hot-Start Touch-Down protocol, using sP1 and sP2 primers for single primer sites, asP1 and asP2 primers for multiple primer sites:

- Template: 5µl (from previous reaction)
- Nuclease-free H$_2$O: 72.4µl
- 10X reaction buffer: 10µl (200 mM Tris-HCl (pH 8.3 at 25°C), 200 mM KCl, 50 mM (NH$_4$)$_2$SO$_4$)
- MgCl$_2$(25mM) : 8µl
- dNTP mix (10mM): 2µl
- Primer 1 (100pmol/µl) 1µl
- Primer 2 (100pmol/µl) : 1µl
- Hot Start DNA Polymerase (5u/µl): 0.6µl
- Thermal Cycling protocol:

  ○ Initial Denaturation: 95°C 4 minutes
  ○ 16 cycles:

    ▪ 95°C 45 seconds
    ▪ 58-48°C 45 seconds (Touch-down)
    ▪ 72°C 45 seconds

  ○ 22 cycles:

    ▪ 95°C 45 seconds
    ▪ 48°C 45 seconds
    ▪ 72°C 45 seconds

  ○ Final Extension 72°C 5minutes
  ○ Final Hold: 10°C

5µl of each sample were run with 100bp DNA ladder on 1.5% agarose gel 5V/cm for 45 minutes (results shown in Figure 37). The difference in size by amplification with sense primers (single primer site included in the final sequence) and antisense primers (both primer sites included in the final sequence) can be observed in the gel image.

Sequences of oligonucleotides are shown below:

**Genomic DNA (gDNA)**

Source: Homo sapiens. Sheared by using NEB dsDNA Fragmentase enzyme.
Size: 100bp, 100-200bp 200-300 bp, 300-400 bp and 400-500 bp gDNA regions isolated and purified from the agarose gel of the sheared gDNA.

**chPrimer**

Source: Artificial
Size: Upper strand contains 58 DNA bp with 6 degenerate DNA bases at the 3'-end. Lower strand contains 58 DNA bp with 6 degenerate DNA bases at the 3'-end. A T7 promoter is also present (bold and underlined).
Sequence:

                                                                    **T7 Promoter**
5'-        AGTCGTTAGGCAATTCGATACAGAAATGAAAT**TAATACGACTCACTATAGGG**NNNNNN-3'
3'- NNNNNNTCAGCAATCCGTTAAGCTATGTCTTTACTTTAATTATGCTGAGTGATATCCC                -5'

**Single stranded Primer Sequences (for use with chPrimer)**

Source: Artificial
Size: sP1 primer contains artificially synthesized 21 DNA bp, sP2 primer contains artificially synthesized 21 DNA bp, asP1 primer contains artificially synthesized 26 DNA bp and asP2 contains artificially synthesized 26 DNA bp.
Sequence:

sP1 Primer: 5'-GTATCGAATTGCCTAACGACT-3'
sP2 Primer: 5'-AGTCGTTAGGCAATTCGATAC-3'
asP1 Primer: 5'-CCCTATAGTGAGTCGTATTAATTTCA-3'
asP2 Primer: 5'-TGAAATTAATACGACTCACTATAGGG-3'

**Example 6. gDNA to gDNA with dsPrimer:**

Sheared gDNA of 100-200bp was primed with dsPrimer and amplification primers were inserted into the sheared gDNA using the following procedure:

- 5μl of sheared gDNA (25ng/μl) was added to a nuclease free 0.2ml reaction tube.
- 10.7μl nuclease free $H_2O$ was added to the reaction tube.
- The sample was incubated at 94°C for 15 minutes and immediately transferred onto ice and incubated for 3 minutes.
- 1μl of dsPrimer (100pmol/μl) was added.
- 2μl of 10X reaction buffer (500mM Tris-Hcl (pH 8.0 at 25°C), 50mM $MgCl_2$, 10mM DTT) was added to the reaction tube.
- The sample was incubated at 25°C for 10 minutes and immediately transferred onto ice.
- 1μl of dNTP mix (10mM) and 0.3μl of DNA Polymerase (10u/μl) was added to the sample and incubated with the following thermal cycling protocol:

    ◦ 25°C for 10 minutes
    ◦ 37°C for 40 minutes
    ◦ 95°C for 5 minutes
    ◦ Hold at 95°C

- The sample was incubated at room temperature for 5 minutes.
- 10μl of the sample was transferred into a new nuclease free 0.2ml reaction tube.
- 7.4μl nuclease-free $H_2O$ was added to the reaction tube.
- 2μl of 10X reaction buffer (500mM Tris-Hcl (pH 8.0 at 25°C), 50mM $MgCl_2$, 10mM DTT) was added to the reaction tube.
- 0.3μl dNTP mix (10mM) and 0.3μl DNA Polymerase (10u/μl) was added to the reaction tube and the sample was incubated with the following thermal cycling protocol:

    ◦ 37°C for 15 minutes
    ◦ 75°C for 20 minutes

The obtained dsPrimer inserted sheared gDNA was amplified with the following Hot-Start Touch-Down protocol (with 2mM $MgCl_2$):

- Template: 5μl (from previous reaction)
- Nuclease-free $H_2O$: 72μl
- 10X reaction buffer: 10μl (200 mM Tris-HCl (pH 8.3 at 25°C), 200 mM KCl, 50 mM $(NH_4)_2SO_4$)
- $MgCl_2$(25mM) : 8μl
- dNTP mix (10mM): 2μl
- Primer (100pmol/μl) : 2.4μl
- Hot Start DNA Polymerase(5u/μl): 0.6μl
- Thermal Cycling protocol 1:

    ◦ Initial Denaturation: 95°C 4 minutes
    ◦ 16 cycles:

        ▪ 95°C 45 seconds
        ▪ 57-47°C 45 seconds (Touch-down)

- 72°C 30 seconds

  ○ 22 cycles:

  - 95°C 45 seconds
  - 47°C 45 seconds
  - 72°C 30 seconds

  ○ Final Extension 72°C 5minutes
  ○ Final Hold: 10°C

- Thermal Cycling protocol 2:

  ○ Initial Denaturation: 95°C 4 minutes
  ○ 16 cycles:

  - 95°C 45 seconds
  - 55-45°C 45 seconds (Touch-down)
  - 72°C 30 seconds

  ○ 22 cycles:

  - 95°C 45 seconds
  - 45°C 45 seconds
  - 72°C 30 seconds

  ○ Final Extension 72°C 5minutes
  ○ Final Hold: 10°C

5μl of each sample were run with 100bp DNA ladder on 1.2% agarose gel 5V/cm for 45 minutes (results shown in Figure 38). This experiment shows that unknown sequences of gDNA were amplified successfully through binding of the dsPrimers and amplifying the samples with primers specific to dsPrimers at differing thermal cycle conditions. Sequences and details of Genomic DNA (gDNA) are as shown in Example 5. Sequences and details of dsPrimer and Single Stranded Primer (for use with dsPrimer) are as shown in Example 1.

**Example 7. gDNA to ssRNA with chPrimer (Transcription):**

Sheared gDNA of 200-300bp size range was amplified using the protocol given in Example 4: gDNA to gDNA with chPrimer.

The obtained product was used as template for the following transcription reaction:

- Nuclease-free $H_2O$ : 22.25μl
- 5x Reaction Buffer: 10μl (200 mM Tris-HCl (pH 7.9 at 25°C), 30 mM $MgCl_2$, 50 mM DTT, 50 mM NaCl and 10 mM spermidine).
- NTP Mix (10mM): 10μl
- Template: 5μl
- RNase inhibitor (40u/μl): 1.25μl
- RNA Polymerase (20u/μl): 1.5μl
- Incubate at 37°C for 4 hours

The transcription product was diluted by 50%, 1/5, and 1/10 and 5μl of the transcription product was run with RNA ladder (200-6000 bp) on 1% agarose gel at 5V/cm for 45 minutes (results shown in Figure 39). This figure shows the successful binding of chPrimer to the gDNA since the products were transcribed into RNA through the promoter included in the chPrimer.

Sequences and details of Genomic DNA (gDNA), chPrimer and Single stranded Primer Sequences (for use with chPrimer) are as shown in Example 5.

**Example 8. sDNA to asRNA with dsPrimer (Transcription):**

dsPrimer was integrated to the 200bp ssDNA fragment 01 and a transcription reaction was performed using the following procedure:

- 1$\mu$l of fragment 01 (100pmol/$\mu$l) was added to a nuclease free 0.2ml reaction tube.
- 14.7$\mu$l nuclease free H$_2$O was added to the reaction tube.
- The sample was Incubated at 95°C for 5 minutes and immediately transferred on to ice and incubated for 3 minutes.
- 1$\mu$l of dsPrimer (100pmol/$\mu$l) was added
- 2$\mu$l of 10X reaction buffer (500mM Tris-Hcl (pH 8.0 at 25°C), 50mM MgCl$_2$, 10mM DTT) was added to the reaction tube.
- The sample was incubated at 25°C for 10 minutes and immediately transferred onto ice.
- 1$\mu$l of dNTP mix (10mM) and 0.3$\mu$l of DNA Polymerase (10u/$\mu$l) was added to the sample and incubated with the following thermal cycling protocol:

  - 25°C for 10 minutes
  - 37°C for 40 minutes
  - 75°C for 20 minutes

- 10$\mu$l of the sample was transferred into a new nuclease free 0.2ml reaction tube.
- 7.4$\mu$l nuclease-free H$_2$O was added to the reaction tube.
- 2$\mu$l of 10X reaction buffer (500mM Tris-Hcl (pH 8.0 at 25°C), 50mM MgCl2, 10mM DTT) was added to the reaction tube.
- 0.3$\mu$l dNTP mix (10mM) and 0.3$\mu$l DNA Polymerase (10u/$\mu$l) was added to the reaction tube and the sample was incubated with the following thermal cycling protocol:

  - 37°C for 15 minutes
  - 75°C for 20 minutes

5$\mu$l of the sample was used as template for the following transcription reaction

- Nuclease-free H$_2$O : 22.25$\mu$l
- 5x Reaction Buffer: 10$\mu$l (200 mM Tris-HCl (pH 7.9 at 25°C), 30 mM MgCl$_2$, 50 mM DTT, 50 mM NaCl and 10 mM spermidine).
- NTP Mix (10mM): 10$\mu$l
- Template: 5$\mu$l
- Rnase inhibitor(40u/$\mu$l): 1.25$\mu$l
- RNA Polymerase(20u/$\mu$l): 1.5$\mu$l
- Incubate at 37°C for 4 hours

The transcription product was diluted by 50%, and 5$\mu$l of the transcription product was run with RNA ladder (200-6000) on 1% agarose gel at 5V/cm for 45 minutes (results shown in Figure 40). This figure shows that dsPrimers with T7 promoter bound to the DNA fragment and were successfully transcribed into RNA. This is shown by the fact that the size has increased by 3 bps due to the 3 G's added during transcription by RNA Polymerase.

Sequences and details of dsPrimer and Single Stranded Primer (for use with dsPrimer) are as shown in Example 1.

***DNA Fragment 01***

Source: Artificial
Size: Template strand contains artificially synthesized 200 DNA bp.
Sequence:
>Template

GCATCGAGAGTAGAAGTATTCCATAGCGGTGGCTCCGACACTCTCGTTACTCG
GCTACGATACTGATACGGTAGCTTGATCGATCCTTCAGGCACTGCCTAGTACT
TCACCATCGAATGAGAATGGTTACATCGCCATTCGAAGTTCATGGCACGGCTC
TTATGTACGACTTGCATCCCACTACCAATTCGCCACGCCAT

**Example 9. Sequence Data of dsDNA Fragment 02 Amplicons Amplified by dsPrimer:**

dsDNA fragment 02 was amplified using the protocol given in Example 2: dsDNA to dsDNA with dsPrimer, the resulting PCR product was used as the template for the Sanger sequencing reactions to prove that dsPrimers targeted the ends, the amplified DNA Fragment 02 was sequenced with various primers. Sequencing of the dsDNA fragment 02 was done with three different primers that target the inside of the template (sequencing primer 1, sequencing primer 2, sequencing primer 3) in order to obtain and verify the full sequence. The fragment was also sequenced by the single stranded primer of dsPrimer - since both ends had the same sequence of the dsPrimer, single primer sequencing targeted both ends, giving matching data for both 3'- and 5'- ends (last 60 nucleotides) of DNA Fragment 02 amplicons that are amplified by using dsPrimer method (results shown in Figure 41). Here the sequence data of the amplicon amplified by dsPrimer shows a high accuracy rate.

Sequences and details of DNA Fragment 02, dsPrimer and Single Stranded Primer (for use with dsPrimer) are as shown in Example 1. Sequences and details of DNA Fragment 02 Primer Sequences are as shown in Example 2.

***Sanger Sequencing Primers***

Source: Artificial
Size: Sequencing primer 01 contains artificially synthesized 20 DNA bp, sequencing primer 02 contains artificially synthesized 20 DNA bp and sequencing primer 03 contains artificially synthesized 20 DNA bp.
Sequence:

Sequencing Primer 01: 5'-GATGAACTTCAGGGTCAGCT-3'

Sequencing Primer 02: 5'-CTTGAAGAAGTCGTGCTGCT-3'

Sequencing Primer 03: 5'-TCAAGATCCGCCACAACATC-3'

**Example 10. gDNA to gDNA with dsPrimer (Ion Torrent Adaptation)**

Fragments of approximately 250bp, isolated from NEBNext dsDNA Fragmentase treated Human Genomic DNA were amplified using Ion Torrent™ (Life Technologies, Inc) adaptor sequences comprising a dsPrimer structure.

**A. Fragmentization: NEBNext dsDNA Fragmentase (New England Biolabs, Inc) used following the manufacturer's instructions.**

- 20µl of Human Genomic DNA in nuclease free $H_2O$ (250ng/µl) was added into a nuclease free 0.2ml reaction tube.
- 59µl nuclease free $H_2O$ was added into the reaction tube.
- 10µl of 10X Fragmentase Reaction Buffer was added into the reaction tube.
- 1µl of 100X BSA was added into the reaction tube.
- Reaction tube was vortexed thoroughly.
- Reaction tube was incubated on ice for 5 minutes.
- 10µl of dsDNA Fragmentase was added.
- Reaction tube was vortexed thoroughly.
- Reaction tube was incubated for 17 minutes at 37°C for 17 minutes.
- 5µl of 0.5M EDTA was immediately added to stop the reaction.
- The fragmentated DNA was purified using GeneJET Gel Extraction Kit (Thermo Scientific), according to the manufacturer's instructions:

- The reaction was transferred into a nuclease free 1.5ml reaction tube.
- 105µl Binding Buffer was added into the reaction tube and mixed thoroughly.
- The reaction was transferred into a GeneJET purification column and centrifuged for 1 minute at 12000g and flow-through was discarded.
- 700µl of Wash Buffer was added to the column and centrifuged for 1 minute at 12000g, flow-through discarded.
- Empty column was centrifuged for 1 minute at 12000g and transferred into a nuclease free 1.5ml reaction tube.
- 25µl nuclease free water was added to the centre of the column and incubated at room temperature for 1min, then centrifuged for 1 minute at 12000g.
- Previous step was repeated.

**B. Size selection & purification: Size selection and purification was performed using GeneJET Gel Extraction Kit (Thermo Scientific), according to the manufacturer's instructions:**

- 2X 25µl eluted DNA was loaded on a 1% agarose gel along with a 100bp DNA ladder and run for 45 minutes at 80V.
- Approximately 250bp fragment containing gel slices obtained from both wells were weighted and placed into a nuclease free 1.5ml reaction tube.
- 255µl of Binding Buffer was added to the reaction tube, vortexed and incubated at 60°C for 10 minutes.
- 255µl 100% isopropanol was added into the reaction tube and mixed thoroughly.
- Reaction tube was vortexed then transferred into a GeneJET purification column and centrifuged for 1 minute at 12000g and flow-through was discarded.
- 700µl of Wash Buffer was added to the column and centrifuged for 1 minute at 12000g, flow-through discarded.
- Empty column was centrifuged for 1 minute at 12000g and transferred into a nuclease free 1.5ml reaction tube.
- 20µl nuclease free water was added to the center of the column and incubated at room temperature for 1min, then centrifuged for 1 minute at 12000g.
- Previous step was repeated.

**C. dsPrimer Amplification:**
Approximately 250bp Human gDNA fragments were primed with dsPrimer and amplification primers were inserted into the fragment with the following procedure:

- 10µl of approximately 250bp Human gDNA (15ng/µl) were added to a nuclease free 0.2ml reaction tube.
- 5.7µl nuclease free $H_2O$ was added to the reaction tube. The sample was incubated at 94°C for 15 minutes and immediately transferred on to ice and incubated for 3 minutes.
- 1µl of dsPrimer (100pmol/µl), which was incubated at 45°C for 5 minutes just before the addition, was added
- 2µl of 10X reaction buffer (500mM Tris-Hcl (pH 8.0 at 25°C), 50mM $MgCl_2$, 10mM DTT) was added to the reaction tube.
- The sample was incubated at 20°C for 10 minutes and immediately transferred on to ice.
- 1µl of dNTP mix (10mM) and 0.3µl of DNA Polymerase (10u/µl) was added to the sample and incubated with the following thermal cycling protocol:

    - 20°C for 15 minutes.
    - 37°C for 40 minutes.
    - 95°C for 5 minutes.
    - Hold at 95°C.

- The sample was incubated at room temperature for 5 minutes.
- 9µl of the sample was transferred into a new nuclease free 0.2ml reaction tube.
- 8.4µl nuclease-free $H_2O$ was added to the reaction tube.
- 2µl of 10X reaction buffer (500mM Tris-Hcl (pH 8.0 at 25°C), 50mM

MgCl2, 10mM DTT) was added to the reaction tube.

- 0.3µl dNTP mix (10mM) and 0.3µl DNA Polymerase (10u/µl) was added to the reaction tube and the sample was incubated with the following thermal cycling protocol:

○ 37°C for 15 minutes
○ 75°C for 20 minutes

The obtained dsPrimer inserted dsDNA fragment was amplified with the following Hot-Start protocol:

- Template: 5μl (from previous reaction)
- Nuclease-free H$_2$O: 72.4μl
- 10X reaction buffer: 10μl (200 mM Tris-HCl (pH 8.3 at 25°C), 200 mM KCl, 50 mM (NH$_4$)$_2$SO$_4$)
- MgCl$_2$(25mM) : 8μl
- dNTP mix (10mM): 2μl
- Primer (100pmol/μl) : 2μl
- Hot Start DNA Polymerase (5u/μl): 0.6μl
- Thermal Cycling protocol:

  ○ Initial Denaturation: 95°C 4 minutes
  ○ 30 cycles:

    ▪ 95°C 45 seconds
    ▪ 58°C 45 seconds
    ▪ 72°C 25 seconds

  ○ Final Extension 72°C 3minutes
  ○ Final Hold: 10°C

10μl of each sample were run with 100bp plus DNA ladder on 1.5% agarose gel 5V/cm for 1 hour.
Sequences of oligonucleotides are shown below:

*Genomic DNA (gDNA)*

Source: Homo sapiens. Sheared by using NEB dsDNA Fragmentase enzyme. Size: 250bp gDNA regions isolated and purified from the agarose gel of the sheared gDNA.

*dsPrimer*

Source: Artificial
Size: Upper strand contains 77 DNA bp with 6 degenerate DNA bases at the 3'-end. Lower strand contains 71 DNA bp. Sequence:

5'-
CCACTACGCCTCCGCTTTCCTCTCTATGGGCAGTCGGTGATCCATCTCATCCCTGCGTGTCTCCGACTCAGNN
NNNN-3'
3'-

GGTGATGCGGAGGCGAAAGGAGAGATACCCGTCAGCCACTAGGTAGAGTAGGGACGCACAGAGGCTGAGTC
-5'

*Single stranded Primer Sequences*

Source: Artificial
Size: Primer PAm contains artificially synthesized 23 DNA bp and primer PBm contains artificially synthesized 21 DNA bp.
Sequence:

Primer PAm: 5'- CCATCTCATCCCTGCGTGTCTCC-3'
Primer PBm: 5'- CCACTACGCCTCCGCTTTCCT-3'

**Claims**

1. A method of nucleic acid manipulation comprising:

   (i) hybridizing a double stranded primer to the 3' end of a single stranded nucleic acid template, wherein said primer comprises a double stranded region and a single stranded region, wherein the single stranded region is a 3' overhang region and wherein the 3' overhang region enables the double stranded primer to target and hybridize to the 3' end of the nucleic acid template, wherein said single stranded 3' overhang region comprises a degenerate sequence or a sequence comprising universal bases, and wherein the double stranded primer is made up of two separate strands;
   (ii) at least one round of polymerization using a polypeptide with 5' to 3' DNA polymerization activity, wherein at least the first round of polymerization comprises using a polypeptide with 5' to 3' DNA polymerization activity to carry out a primer extension reaction to synthesise nucleotides in a template dependent manner from the 3' end of the single stranded region of said hybridized double stranded primer;

   wherein the hybridizing step (i) and at least the first primer extension reaction from the 3' end of the single stranded region of said hybridized double stranded primer in step (ii) takes place without the formation of a phosphodiester bond between the 3' end of the nucleic acid template and the double stranded primer of step (i) and wherein said single stranded 3' overhang region is up to 12 bases in length.

2. The method of claim 1, wherein said double stranded region of said double stranded primer comprises a nucleic acid fragment to be joined to said nucleic acid template, a primer binding site, a control region, a promoter, a terminator, an operator, a repressor, an enhancer, a silencer, an insulator or other response element, a sequencing adaptor region or other element permitting or facilitating sequencing, a restriction site, a label, an identifier sequence, a barcode, a forensic signature, or combinations thereof.

3. The method of either claim 1 or 2, wherein said single stranded 3' overhang region comprises universal bases selected from the group consisting of inosine, 2-amino purine, diaminopurine, 5-nitroindole, 5-methyl isodeoxycytosine, iso deoxyguanine, or a mixture thereof.

4. The method of any one of claims 1 to 3, wherein said single stranded 3' overhang region is up to 6 bases in length.

5. The method of any one of claims 1 to 4 wherein two or more different types of double stranded primers are used.

6. The method of claim 5, wherein the two or more different types of double stranded primer contain different primer binding sites, or wherein one double stranded primer contains a promoter region and the other a terminator region, or wherein one double stranded primer contains a promoter region and the other a primer binding site.

7. The method of any one of claims 1 to 6, wherein one or more of the double stranded primers is a hybrid double stranded primer wherein one strand comprises DNA and the other strand comprises RNA.

8. The method of any one of claims 1 to 7, wherein said method further comprises the use of a lock primer which cannot be subjected to chain elongation.

9. The method of any one of claims 1 to 8, wherein said method is used in sequencing applications, nucleic acid labelling, joining nucleic acid fragments together, or for incorporation of primer sites, promoter sites, terminator sites and/or restriction sites.

10. The method of any one of claims 1 to 9, wherein the double stranded primer is immobilized to a solid phase before step (i) is carried out.

11. The method of claim 10, wherein the solid phase is a nanoparticle, preferably a magnetic nanoparticle.

12. A double stranded primer comprising a double stranded region and a single stranded region, wherein the single stranded region is a 3' overhang region comprising a degenerate sequence or a sequence comprising universal bases, wherein when only one of the strands has a 3' overhang region then the strand which does not contain the 3' overhang region does not have a 5'- $PO_3$ group, wherein when both of the strands have a 3' overhang region then one or both of the strands does not have a 5'- $PO_3$ group and wherein the single stranded 3' overhang region is up to 12 bases in length.

13. The double stranded primer of claim 12 wherein said lack of a 5'- $PO_3$ group prevents incorporation of said strand which lacks the 5'- $PO_3$ group into a nucleic acid template primed by the primer.

14. The double stranded primer of either claim 12 or 13 wherein the 3' overhang region enables the double stranded primer to target and hybridize to the 3' end of a nucleic acid template.

15. The double stranded primer of any one of claims 12 to 14, wherein the single stranded 3' overhang region is up to 6 bases in length.

## dsPrimer Structure

## dsPrimer Silencing

Fig. 1

**dsPromoter Structure**

**dsPromoter Silencing**

Fig. 2

Fig. 3

Fig. 4A

Fig. 4B

Fig. 5A

Fig. 5B

Fig. 6

Fig. 7A

Fig. 7B

Fig. 8A

EP 3 633 046 A1

Fig. 8B

Fig. 9

Fig. 10

Fig. 11

Fig. 12

Fig. 13

Fig. 14

Fig. 15A

Fig. 15B

5'  ————————————————————————————————————  3'
                        sDNA

                        Step 01  ⬇  **Terminal Transferase**
                                    (Polyadenylation)

5'  ————————————————————————————————————  AAAAAA...AAA  3'
                        sDNA

                        Step 02  ⬇  **Oligo dT**
                                    (Annealing)

                                              **Oligo dT Primer**
                                              VTTTTTT  5'
3'  ————————————————————————————————————  AAAAAA...AAA  3'
5'                      sDNA

                        Step 03  ⬇  **DNA Polymerase**
                                    (DNA Polymerization)

                                              **Oligo dT Primer**
3' ◄- - - - - - - - - - - - - - - - - - - - - VTTTTTT  5'
5'  ————————————————————————————————————  AAAAAA...AAA  3'
                        sDNA

                        Step 03  ⬇  **DNA Polymerase**
                                    (DNA Polymerization)

3'  ————————————————————————————————————  **Oligo dT Primer**
                        asDNA               TTTTTT  5'
5'  ————————————————————————————————————  AAAAAA...AAA  3'
                        sDNA

                        Step 04              **dsDNA**
                                             (Denaturation)
                                      AAA...AAAAAA  3'

5'  ————————————————————————————————————  AAA...AAAAAA  3'
                        sDNA                                    **[Continued]**

                        Step 05  ⬇  **Lock Primer T**
                                    (No -OH Group)
                                    (Annealing)
                                                    **Lock Primer T** DNA 5'
3'  ————————————————————————————————————         TTTTTT-
5'  ————————————————————————————————————  AAA...AAAAAA        DNA 3'
                        sDNA

                                 ⬇

                        **No Reaction**

Fig. 16A

Fig. 16B

Fig. 17

Fig. 18A

Fig. 18B

Fig. 19A

3' ──────────────── asDNA ──────────────── TTTTTT ▨▨▨ 5'
**Oligo dT Primer**

**Step 05** ⬇ **Lock Primer T**
(No -OH Group)
(Annealing)

3' ──────────────── asDNA ──────────────── TTTTTT ▨▨▨ 5'
**Oligo dT Primer**

**Step 06** ⬇ **dsPromoter**
(Annealing)

5' DNA **T7 Promoter** ▨▨▨─NNNNNN──── asDNA ──── TTTTTT ▨▨▨ 3'
3' RNA ▨▨▨ ∨
**Nick** **Oligo dT Primer** 5'

**Step 07** ⬇ **DNA Polymerase**
(DNA Polymerization)

5' **T7 Promoter** ▨▨▨─NNNNNN─ ─ ─ ─ ─ ─ ─ ─ ─ ─ ─➤ 3'
3' ◄─ ─ ─ ─ ─ ─── asDNA ──── TTTTTT ▨▨▨
**Oligo dT Primer** 5'

**Step 07** ⬇ **DNA Polymerase**
(Strand Displacement)

5' DNA **T7 Promoter** ▨▨▨──── sDNA ──── AAAAA ▨▨ DNA 3'
3' DNA ▨▨▨──── asDNA ──── TTTTTT ▨▨ DNA
**Oligo dT Primer** 5'

**Step 07** ⬇ **RNA Polymerase**
(Amplification)

5' GGG ▨▨▨▨▨▨ sRNA ▨▨▨▨▨▨ AAAAA ▨▨▨ 3'
**Oligo dT Primer**

Fig. 19B

114

Fig. 20A

Fig. 20B

Fig. 21A

Fig. 21B

Fig. 22A

Fig. 22B

Fig. 23A

Fig. 23B

Fig. 24A

Fig. 24B

Fig. 25A

Fig. 25B

Fig. 26A

Fig. 26B

Fig. 27A

[Figure 27A]

**Step 04**
DNA Polymerase
(ddNTP)
(Strand Displacement)
(Amplification)

Truncated
DNA Library

5′ PO₃⁻ ————————————— 3′
5′ PO₃⁻ ————————————— 3′
5′ PO₃⁻ ————— 3′
5′ PO₃⁻ ———————— 3′
5′ PO₃⁻ ——————————————— 3′

**Step 05**
Ligand
(Enrichment)

**Ligand
Enrichment**

**Step 06**
DNA Aptamer
(Evolution)

**SELEX**

**Step 07**
DNA Aptamer
(Selection)
(Separation)

[Continued]

Fig. 27B

[Figure 27B]

Fig. 27C

Fig. 27D

Fig. 28

EP 3 633 046 A1

**chPrimer Structure**

**chPrimer Silencing**

Fig. 29

Fig. 30

Fig. 31A

Fig. 31B

Fig. 32A

**Step 04**

chPrimer
(Denaturation)
(Annealing)

3' —————asDNA————— sP1 (DNA) asP2 (DNA) 5'
5' —————sDNA————— 3'

3' ————————asDNA———————— 5'
5' asP1 (DNA) sP2 (DNA) ————sDNA———— 3'

**Step 05** ↓ DNA Polymerase
(DNA Polymerization)

**Step 05** ↓ DNA Polymerase
(DNA Polymerization)

3' —————asDNA————— sP1 (DNA) asP2 (DNA) 5'
5' —————sDNA————— - - - - → 3'

3' ←- - - - —————asDNA————— 5'
5' asP1 (DNA) sP2 (DNA) ————sDNA———— 3'

**Step 05** ↓ DNA Polymerase
(Strand Displacement)

**Step 05** ↓ DNA Polymerase
(Strand Displacement)

3' —————asDNA————— sP1 (DNA) asP2 (DNA) 5'
5' —————sDNA————— asP1 (DNA) sP2 (DNA) 3'

3' sP1 (DNA) asP2 (DNA) ————asDNA———— 5'
5' asP1 (DNA) sP2 (DNA) ————sDNA———— 3'

**Step 06** asP2, asP1
(Denaturation)
(Annealing)

**PCR**

↓

5' asP1 (DNA) sP2 (DNA) ————sDNA———— asP1 (DNA) sP2 (DNA) 3'
3' sP1 (DNA) asP2 (DNA) ————asDNA———— sP1 (DNA) asP2 (DNA) 5'

Fig. 32B

139

Fig. 33

Fig. 34

**200-6000 bp
RNA Ladder**       **1/1**          **1/10**          **1/100**

1000 bp ➜

500 bp ➜

200 bp ➜

Fig. 35

Fig. 36

Fig. 37

Fig. 38

Fig. 39

Fig. 40

## A. 5'- end Sequences of dsDNA Fragment 02 Amplicons

EP 3 633 046 A1

5'-end sequences of dsDNA Fragment 02 amplicons that are amplified by using dsPrimers (shown in Figure 34)

5'-GAAATGAAATTAATACGACTCACGATAGGGGTGCAGAATTCGAAGCTTGAGCTCGAGATC..................................................-3'

Theoretical 5' end sequences of the dsDNA Fragment 02 amplicons that are amplified by using dsPrimers (shown in Figure 34)

5'-GAAATGAAATTAATTCGACTCCCTATAGGGNTGCAGAATTCGAAGCTTGAGCTCGAGATC...............................................-3'

## B. 3'- end Sequences of dsDNA Fragment 02 Amplicons

3'-end sequences of dsDNA Fragment 02 amplicons that are amplified by using dsPrimers (shown in Figure 34)

5'-............................................CTCCTCGCCCTTGCTCACCATGAATTCTCACCCTTGCTCACGATGAATTCTGTTCCTTTT-3'

Theoretical 3' end sequences of the dsDNA Fragment 02 amplicons that are amplified by using dsPrimers (shown in Figure 34)

5'-............................................CTCCTCGCCCTTGCTCACCATGAATTCTCNCCCTATAGGGAGTCGAATTAATTTCATTTC-3'

Fig. 41

148

## A. Magnetic Nanoparticle-conjugated dsPrimer Structure

**MNP-conjugated dsPrimer**

NNN : Random Region
o   : Immobilized End

## B. Magnetic Nanoparticle-conjugated dsPrimer Formation via Hybridization

**MNP-conjugated dsPrimer**

NNN : Random Region
o   : Immobilized End

## Fig. 42

Fig. 43

Fig. 44

Fig. 45

Fig. 46

Europäisches
Patentamt

European
Patent Office

Office européen
des brevets

# EUROPEAN SEARCH REPORT

**Application Number**

EP 19 17 0382

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/103018 A2 (EINSTEIN COLL MED [US]; LOUDIG OLIVIER [US]) 13 September 2007 (2007-09-13) | 12 | INV. C12Q1/68 C12N15/10 C12Q1/6844 |
| Y | * Fig. 1 * | 1-11, 13-15 | |
| A | WO 01/79549 A2 (INCYTE PHARMA INC [US]) 25 October 2001 (2001-10-25) * claims 1, 3, 7, 8; page 2, line 29 - page 3, line 13; page 2, lines 19-26; page 11, line 17 - page 12, line 7 Examples 1-3 * | 1-12 | |
| Y | WO 01/36680 A2 (EASTMAN ERIC M [US]; MILLSTEIN LARRY S [US]) 25 May 2001 (2001-05-25) * page 5, lines 18-25; page 7, lines 10-18; Fig. 2 and 3 * | 1-11 | |
| Y | WO 93/04199 A2 (SCIENT GENERICS LTD [GB]) 4 March 1993 (1993-03-04) * Fig. 1-4 * | 1-11 | **TECHNICAL FIELDS SEARCHED (IPC)** C12N C12Q |
| A | WO 99/39001 A2 (AMERSHAM PHARM BIOTECH AB [SE]; ULFENDAHL PER JOHAN [SE]) 5 August 1999 (1999-08-05) * Fig. 1 and 2 * | 1-12 | |
| Y | WO 2009/055732 A1 (ROSETTA INPHARMATICS LLC [US]; RAYMOND CHRISTOPHER K [US]; ARMOUR CHRI) 30 April 2009 (2009-04-30) * claims 42, 44 * | 1-11 | |
| X | US 5 866 336 A (NAZARENKO IRINA A [US] ET AL) 2 February 1999 (1999-02-02) | 12 | |
| Y | * Fig. 8A and 8B * | 13-15 | |

The present search report has been drawn up for all claims

| Place of search | Date of completion of the search | Examiner |
|---|---|---|
| Munich | 10 February 2020 | Leber, Thomas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 17 0382

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2020

| Patent document cited in search report | | Publication date | Patent family member(s) | | Publication date |
|---|---|---|---|---|---|
| WO 2007103018 | A2 | 13-09-2007 | US | 2007208997 A1 | 06-09-2007 |
| | | | US | 2012009570 A1 | 12-01-2012 |
| | | | WO | 2007103018 A2 | 13-09-2007 |
| WO 0179549 | A2 | 25-10-2001 | US | 6387624 B1 | 14-05-2002 |
| | | | US | 2002142331 A1 | 03-10-2002 |
| | | | WO | 0179549 A2 | 25-10-2001 |
| WO 0136680 | A2 | 25-05-2001 | AU | 1601801 A | 30-05-2001 |
| | | | WO | 0136680 A2 | 25-05-2001 |
| WO 9304199 | A2 | 04-03-1993 | NONE | | |
| WO 9939001 | A2 | 05-08-1999 | AU | 3027699 A | 16-08-1999 |
| | | | EP | 1051523 A2 | 15-11-2000 |
| | | | JP | 2002501760 A | 22-01-2002 |
| | | | US | 6280954 B1 | 28-08-2001 |
| | | | WO | 9939001 A2 | 05-08-1999 |
| WO 2009055732 | A1 | 30-04-2009 | CN | 102124126 A | 13-07-2011 |
| | | | EP | 2209912 A1 | 28-07-2010 |
| | | | JP | 2011500092 A | 06-01-2011 |
| | | | US | 2010029511 A1 | 04-02-2010 |
| | | | US | 2011039732 A1 | 17-02-2011 |
| | | | US | 2013252823 A1 | 26-09-2013 |
| | | | WO | 2009055732 A1 | 30-04-2009 |
| US 5866336 | A | 02-02-1999 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8071311 B
- US 8143001 B
- US 8034568 B
- US 7939258 B
- US 8192941 B
- US 6855496 B
- WO 2011055737 A
- EP 2377929 A

**Non-patent literature cited in the description**

- **VAN PELT-VERKUI et al.** Principles and Technical Aspects of PCR Amplification. Springer, 2008
- **MCGINNESS ; JOYCE.** *J. Biol. Chem.,* 2002, vol. 277, 2987-2991
- **ARNAUD-BARBE et al.** *Nucleic Acids Research,* 1998, vol. 26, 3550-3554
- **VEGA, MIGUEL et al.** Improvement of $\varphi$29 DNA polymerase amplification performance by fusion of DNA binding motifs. *PNAS,* 2010, 16506-16511

SEQUENCE LISTING

<110> Selvi, Ozan
      Tokso, Sila
      Orcan, Serkan

<120> Primer Technology

<130> 69.114123

<150> GB1301857.7
<151> 2013-02-01

<160> 26

<170> PatentIn version 3.5

<210> 1
<211> 18
<212> DNA
<213> Artificial Sequence

<220>
<223> T7 RNA polymerase promoter sequence

<400> 1
taatacgact cactatag                                                    18


<210> 2
<211> 20
<212> DNA
<213> Artificial Sequence

<220>
<223> T7 RNA promoter sequence

<400> 2
taatacgact cactataggg                                                  20


<210> 3
<211> 764
<212> DNA
<213> Artificial Sequence

<220>
<223> EGFP region of Clontech's pEGFP-C1 vector

<400> 3
tgcagaattc gaagcttgag ctcgagatct gagtccggac ttgtacagct cgtccatgcc     60

gagagtgatc ccggcggcgg tcacgaactc cagcaggacc atgtgatcgc gcttctcgtt     120

ggggtctttg ctcagggcgg actgggtgct caggtagtgg ttgtcgggca gcagcacggg     180

gccgtcgccg atggggggtgt ctgctggta gtggtcggcg agctgcacgc tgccgtcctc     240

gatgttgtgg cggatcttga agttcacctt gatgccgttc ttctgcttgt cggccatgat     300

atagacgttg tggctgttgt agttgtactc cagcttgtgc cccaggatgt tgccgtcctc     360

cttgaagtcg atgcccttca gctcgatgcg gttcaccagg gtgtcgccct cgaacttcac     420

```
ctcggcgcgg gtcttgtagt tgccgtcgtc cttgaagaag atggtgcgct cctggacgta          480

gccttcgggc atggcggact tgaagaagtc gtgctgcttc atgtggtcgg ggtagcggct          540

gaagcactgc acgccgtagg tcagggtggt cacgagggtg ggccagggca cgggcagctt          600

gccggtggtg cagatgaact tcagggtcag cttgccgtag gtggcatcgc cctcgccctc          660

gccggacacg ctgaacttgt ggccgtttac gtcgccgtcc agctcgacca ggatgggcac          720

caccccggtg aacagctcct cgcccttgct caccatgaat tctc                          764
```

```
<210>   4
<211>   36
<212>   DNA
<213>   Artificial Sequence

<220>
<223>   ds primer 5'-3'


<220>
<221>   misc_feature
<222>   (31)..(36)
<223>   n is a, c, g, or t

<400>   4
gaaatgaaat taatacgact cactataggg nnnnnn                                     36


<210>   5
<211>   30
<212>   DNA
<213>   Artificial Sequence

<220>
<223>   ds primer 3'-5'

<400>   5
ctttacttta attatgctga gtgatatccc                                           30


<210>   6
<211>   20
<212>   DNA
<213>   Artificial Sequence

<220>
<223>   ss primer 5'-3'

<400>   6
gaaatgaaat taatacgact                                                      20


<210>   7
<211>   19
<212>   DNA
<213>   Artificial Sequence

<220>
<223>   DNA fragment 02 forward primer sequence
```

```
<400>  7
catggtgagc aagggcgag                                                        19


<210>  8
<211>  20
<212>  DNA
<213>  Artificial Sequence

<220>
<223>  DNA fragment 02 reverse primer sequence

<400>  8
tgcagaattc gaagcttgag                                                       20


<210>  9
<211>  58
<212>  DNA
<213>  Artificial Sequence

<220>
<223>  chPrimer 5'-3'


<220>
<221>  misc_feature
<222>  (53)..(58)
<223>  n is a, c, g, or t

<400>  9
agtcgttagg caattcgata cagaaatgaa attaatacga ctcactatag ggnnnnnn            58


<210>  10
<211>  58
<212>  DNA
<213>  Artificial Sequence

<220>
<223>  chPrimer 3'-5'


<220>
<221>  misc_feature
<222>  (1)..(6)
<223>  n is a, c, g, or t

<400>  10
nnnnnntcag caatccgtta agctatgtct ttactttaat tatgctgagt gatatccc           58


<210>  11
<211>  21
<212>  DNA
<213>  Artificial Sequence

<220>
<223>  ss primer sP1

<400>  11
gtatcgaatt gcctaacgac t                                                     21
```

<210> 12
<211> 21
<212> DNA
<213> Artificial Sequence

<220>
<223> ss primer sP2

<400> 12
agtcgttagg caattcgata c                                                    21


<210> 13
<211> 26
<212> DNA
<213> Artificial Sequence

<220>
<223> ss primer asP1

<400> 13
ccctatagtg agtcgtatta atttca                                               26


<210> 14
<211> 26
<212> DNA
<213> Artificial Sequence

<220>
<223> ss primer asP2

<400> 14
tgaaattaat acgactcact ataggg                                               26


<210> 15
<211> 200
<212> DNA
<213> Artificial Sequence

<220>
<223> DNA fragment 01

<400> 15
gcatcgagag tagaagtatt ccatagcggt ggctccgaca ctctcgttac tcggctacga         60

tactgatacg gtagcttgat cgatccttca ggcactgcct agtacttcac catcgaatga        120

gaatggttac atcgccattc gaagttcatg gcacggctct tatgtacgac ttgcatccca        180

ctaccaattc gccacgccat                                                     200


<210> 16
<211> 20
<212> DNA
<213> Artificial Sequence

<220>
<223> sanger sequencing primer

```
<400> 16
gatgaacttc agggtcagct                                                        20


<210>  17
<211>  20
<212>  DNA
<213>  Artificial Sequence

<220>
<223>  sanger sequencing primer

<400> 17
cttgaagaag tcgtgctgct                                                        20


<210>  18
<211>  20
<212>  DNA
<213>  Artificial Sequence

<220>
<223>  sanger sequencing primer

<400> 18
tcaagatccg ccacaacatc                                                        20


<210>  19
<211>  60
<212>  DNA
<213>  Artificial Sequence

<220>
<223>  DNA fragment 02 amplicons amplified by dsPrimer 5' end

<400> 19
gaaatgaaat taatacgact cacgataggg gtgcagaatt cgaagcttga gctcgagatc           60


<210>  20
<211>  60
<212>  DNA
<213>  Artificial Sequence

<220>
<223>  ds DNA fragment 02 amplicons amplified by dsPrimer 3' end

<400> 20
ctcctcgccc ttgctcacca tgaattctca cccttgctca cgatgaattc tgttcctttt          60


<210>  21
<211>  60
<212>  DNA
<213>  Artificial Sequence

<220>
<223>  dsDNA fragment 02 Amplicons Amplified by dsPrimer- theoretical 5'
       end sequence
```

<220>
<221> misc_feature
<222> (31)..(31)
<223> n is a, c, g, or t

<400> 21
gaaatgaaat taattcgact ccctataggg ntgcagaatt cgaagcttga gctcgagatc        60


<210> 22
<211> 60
<212> DNA
<213> Artificial Sequence

<220>
<223> dsDNA fragment 02 amplicons amplified by dsPrimer theoretical 3'
      end


<220>
<221> misc_feature
<222> (30)..(30)
<223> n is a, c, g, or t

<400> 22
ctcctcgccc ttgctcacca tgaattctcn ccctataggg agtcgaatta atttcatttc        60


<210> 23
<211> 77
<212> DNA
<213> Artificial Sequence

<220>
<223> dsPrimer 5'-3'


<220>
<221> misc_feature
<222> (72)..(77)
<223> n is a, c, g, or t

<400> 23
ccactacgcc tccgctttcc tctctatggg cagtcggtga tccatctcat ccctgcgtgt        60

ctccgactca gnnnnnn                                                       77


<210> 24
<211> 71
<212> DNA
<213> Artificial Sequence

<220>
<223> dsPrimer 3'-5'

<400> 24
ggtgatgcgg aggcgaaagg agagataccc gtcagccact aggtagagta gggacgcaca        60

gaggctgagt c                                                             71


<210> 25

```
<211>  23
<212>  DNA
<213>  Artificial Sequence

<220>
<223>  ss primer PAm

<400>  25
ccatctcatc cctgcgtgtc tcc                                          23


<210>  26
<211>  21
<212>  DNA
<213>  Artificial Sequence

<220>
<223>  ss primer  PBm

<400>  26
ccactacgcc tccgctttcc t                                            21
```